# EUROPEAN PATENT SPECIFICATION

(11) **EP 3 126 348 B1**
(45) Date of publication and mention of the grant of the patent: **29.11.2017**
(21) Application number: 15715216.6
(22) Date of filing: 02.04.2015
(51) Int. Cl.: C07D 401/12

(54) **DIAMINOTRIAZINE COMPOUND USEFUL AS HERBICIDE**
ALS HERBIZID NUTZBARE DIAMINOTRIAZINVERBINDUNG
COMPOSE DE DIAMINOTRIAZINE UTILE COMME HERBICIDE

(30) Priority: 03.04.2014 EP 14163356
(43) Date of publication of application: 08.02.2017
(73) Proprietor: BASF SE, 67056 Ludwigshafen am Rhein (DE)
(72) Inventor: MAJOR, Julia, 67251 Freinsheim (DE); VOGT, Florian, 68167 Mannheim (DE); CALO, Frederick, 40223 Düsseldorf (DE); SEITZ, Thomas, 68519 Viernheim (DE); SCHACHTSCHABEL, Doreen, 68165 Mannheim (DE); NEWTON, Trevor William, 67435 Neustadt (DE); HANZLIK, Kristin, 67273 Bobenheim am Berg (DE); KREUZ, Klaus, 79211 Denzlingen (DE); TRESCH, Stefan, 67281 Kirchheim (DE); HUTZLER, Johannes, 67165 Waldsee (DE)
(74) Representative: BASF IP Association
(86) International application number: PCT/EP2015/057360
(87) International publication number: WO 2015/150541

(56) References cited:
- EP-B1- 0 171 708
- WO-A2-02/10160
- US-A- 4 816 064

## Description

The present invention relates to diaminotriazine compounds and to their use as herbicides. The present invention also relates to agrochemical compositions for crop protection and to a method for controlling unwanted vegetation.

US 3,816,419 describes 4-haloalkyl or 4-haloalkenyl-2,4-diaminotriazines and their use as herbicides. Simlar compounds are known from US 3,932,167

DE 197 44 711 describes herbicidally active 2,4-diamino-1,3,5-triazine compounds, which carry a group A-Z in the 6-position, where A is alkylene and Z is a carbocylic or heterocyclic radical.

DE 198 30 902 describes amino-chloro-triazine compounds and their use as herbicides.

EP 0545 149 describes 6-triflouromethyl-1,3,5-triazine compounds and their use as intermediates for crop protecting agents.

EP 2 147 600 describes 2,4-diaminotriazines compounds, which are N-substituted with a bicyclic radical and their use as an agrochemical ingredients.

US 4,816,064 describes diamino-1,3,5-triazine derivatives, wherein the triazine ring in its 6 position carries an oxygen and wherein the amino-groups, attached to the triazine ring may substituted by alkyl, alkenyl, alkynyl, cycloalkyl, phenyl or aralkyl. The amino groups of these compounds do not carry any heteroaryl radical.

WO 02/10160 describes diamino-1,3,5-triazine derivatives, wherein the triazine ring in its 6 position carries a substituted alkyl radical.

EP 0171708 describes diamino-1,3,5-triazine derivatives, wherein the triazine ring in its 6 position carries SCHF₂ and wherein the amino-groups, attached to the triazine ring may substituted by alkyl substituted aryl. In particular the amino-groups may substituted by benzyl, phenethyl, 3-tolylpropyl or 1-methyl-4-tolylbutyl.

However, the herbicidal properties of the known triazine type compounds are not always entirely satisfactory.

Earlier filed EP 12189762.3 describes 2-(2-fluorophenyl)amino-6-aminotriazine compounds having herbicide activity.

Earlier filed EP 13176634.7 describes 2-(hetaryl)amino-6-aminotriazine compounds having herbicide activity.

It is therefore an object of the present invention to provide compounds having improved herbicidal action, in particular good herbicide activity at low application rates. Moreover, the herbicids should be sufficiently compatible with crop plants for commercial utilization.

These and further objects are achieved by diaminotriazine compounds of formula (I), defined below, and by their agriculturally suitable salts.

Accordingly, the present invention relates to diaminotriazine compounds of formula (I) 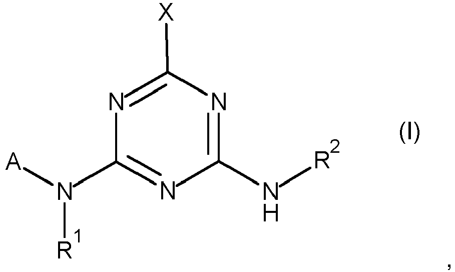 wherein
- A: is 5- or 6-membered monocyclic heteroaryl, which comprises 1, 2 or 3 heteroatoms as ring members, which are selected from O, S and N, where heteroaryl is substituted by 1, 2, 3 or 4 substituents R^{A}, which are identical or different and which are selected from the group consisting of halogen, OH, CN, amino, NO₂, C₁-C₆-alkyl, C₂-C₆-alkenyl, C₂-C₆-alkynyl, C₁-C₆-alkoxy, C₂-C₆-alkenyloxy, C₂-C₆-alkynyloxy, (C₁-C₆-alkoxy)-C₁-C₆-alkyl, (C₁-C₆-alkoxy)-C₁-C₆-alkoxy, (C₁-C₆-alkoxy)-C₂-C₆-alkenyl, (C₁-C₆-alkoxy)-C₂-C₆-alkynyl, C₁-C₆-alkylthio, (C₁-C₆-alkyl)sulfinyl, (C₁-C₆-alkyl)sulfonyl, (C₁-C₆-alkyl)amino, di(C₁-C₆-alkyl)amino, (C₁-C₆-alkyl)-carbonyl, (C₁-C₆-alkoxy)-carbonyl, (C₁-C₆-alkyl)-carbonyloxy, C₃-C₆-cycloalkyl, C₃-C₆-cycloalkoxy, (C₃-C₆-cycloalkyl)-C₁-C₄-alkyl, (C₃-C₆-cycloalkyl)-C₁-C₄-alkoxy, where the aliphatic and cycloaliphatic parts of the 22 aforementioned radicals are unsubstituted, partly or completely halogenated and where the cycloaliphatic parts of the last 4 mentioned radicals may carry 1, 2, 3, 4, 5 or 6 methyl groups;
- R¹: is selected from the group consisting of H, OH, S(O)₂NH₂, CN, C₁-C₆-alkyl, C₂-C₆-alkenyl, C₂-C₆-alkynyl, (C₃-C₆-cycloalkyl)-C₁-C₄-alkyl, C₁-C₆-alkoxy, (C₁-C₆-alkoxy)-C₁-C₆-alkyl, (C₁-C₆-alkyl)-carbonyl, (C₁-C₆-alkoxy)carbonyl, (C₁-C₆-alkyl)sulfonyl, (C₁-C₆-alkylamino)carbonyl, di(C₁-C₆-alkyl)aminocarbonyl, (C₁-C₆-alkylamino)sulfonyl, di(C₁-C₆-alkyl)aminosulfonyl and (C₁-C₆-alkoxy)sulfonyl, where the aliphatic and cycloaliphatic parts of the 14 aforementioned radicals are unsubstituted, partly or completely halogenated,
phenyl, phenyl-C₁-C₆-alkyl, phenylsulfonyl, phenylaminosulfonyl, phenylcarbonyl and phenoxycarbonyl,
wherein phenyl in the last 6 mentioned radicals are unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy;
- R²: is selected from the group consisting of H, OH, S(O)₂NH₂, CN, C₁-C₆-alkyl, C₂-C₆-alkenyl, C₂-C₆-alkynyl, (C₃-C₆-cycloalkyl)-C₁-C₄-alkyl, C₁-C₆-alkoxy, (C₁-C₆-alkoxy)-C₁-C₆-alkyl, (C₁-C₆-alkyl)-carbonyl, (C₁-C₆-alkoxy)carbonyl, (C₁-C₆-alkyl)sulfonyl, (C₁-C₆-alkylamino)carbonyl, di(C₁-C₆-alkyl)aminocarbonyl, (C₁-C₆-alkylamino)sulfonyl, di(C₁-C₆-alkyl)aminosulfonyl and (C₁-C₆-alkoxy)sulfonyl, where the aliphatic and cycloaliphatic parts of the 14 aforementioned radicals are unsubstituted, partly or completely halogenated,
phenyl, phenylsulfonyl, phenylaminosulfonyl, phenyl-C₁-C₆ alkyl, phenoxy, phenylcarbonyl and phenoxycarbonyl,
wherein phenyl in the last 6 mentioned radicals is unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy;
- X: is NR^{3a}R^{3b}, OR^{3c} or S(O)ₖR^{3d},
wherein R^{3a}, R^{3b}, R^{3c} or R^{3d} are independently of one another are selected from the group consisting of H, CN, S(O)₂NH₂, C₁-C₆-alkyl, C₂-C₆-alkenyl, C₂-C₆-alkynyl, C₃-C₆-cycloalkyl, (C₃-C₆-cycloalkyl)-C₁-C₆-alkyl, (C₁-C₆-alkoxy)-C₁-C₆-alkyl, (C₁-C₆-alkyl)-carbonyl, (C₁-C₆-alkoxy)carbonyl, (C₁-C₆-alkyl)sulfonyl, C₁-C₆-alkylamino)carbonyl, di(C₁-C₆-alkyl)aminocarbonyl, (C₁-C₆-alkylamino)sulfonyl, di(C₁-C₆-alkyl)aminosulfonyl and (C₁-C₆-alkoxy)sulfonyl, where the aliphatic and cycloaliphatic parts of the 14 aforementioned radicals are unsubstituted, partly or completely halogenated,
phenyl, phenylsulfonyl, phenyl-C₁-C₆ alkyl, phenylaminosulfonyl, phenylcarbonyl and phenoxycarbonyl, wherein phenyl in the last 6 mentioned radicals is unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy, or
R^{3a}, R^{3b} together with the nitrogen atom, to which they are bound, form an N-bound, mono - or bicyclic heterocyclic radical, which may have1, 2, 3 or 4 further heteroatoms which are selected from N, O and S, which is unsubstituted or substituted by one or more identical or different substituents selected from the group consisting of halogen, CN, NO₂, C₁-C₆-alkyl, C₁-C₆-haloalkyl, C₁-C₆-alkoxy and C₁-C₆-haloalkoxy,
one of R^{3a}, R^{3b} may also be OH, C₁-C₆-alkoxy, C₃-C₆-cycloalkoxy, (C₃-C₆-cycloalkyl)-C₁-C₄-alkoxy C₂-C₆-alkenyloxy, C₂-C₆-alkynyloxy, (C₁-C₆-alkoxy)-C₁-C₆-alkoxy, where the aliphatic and cycloaliphatic parts of the 7 aforementioned radicals are unsubstituted, partly or completely halogenated,
or phenoxy, which is unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy;
k is 0, 1 or 2,
including their agriculturally acceptable salts.

The present invention also relates to agrochemical compositions comprising at least one diaminotriazine compound of formula (I) and at least one auxiliary customary for formulating crop protection agents.

The present invention also relates to the use of diaminotriazine compound of formula (I) as herbicides, i.e. for controlling unwanted and/or harmful vegetation or plants.

The present invention furthermore provides a method for controlling unwanted plants. The method includes allowing a herbicidally effective amount of at least one diaminotriazine compound of the formula (I) to act on the unwanted plants or vegetation, their seeds and/or their habitat. Application can be done before, during and/or after, preferably during and/or after, the emergence of the unwanted plants.

Moreover, the invention relates to processes for preparing diaminotriazine compound of formula (I) and to intermediates.

Further embodiments of the present invention are evident from the claims, the description and the examples. It is to be understood that the features mentioned above and still to be illustrated below of the subject matter of the invention can be applied not only in the combination given in each particular case but also in other combinations, without leaving the scope of the invention.

As used herein, the terms "controlling" and "combating" are synonyms.

As used herein, the terms "undesirable vegetation", "unwanted vegetation", unwanted plants" and "harmful plants" are synonyms.

In the context of substituents, the term "one or more substitutents" means that the number of substituents is e.g. from 1 to 10, in particular 1, 2, 3, 4, 5, 6, 7 or 8.

If the diaminotriazine compounds of formula (I) as described herein is capable of forming geometrical isomers, for example E/Z isomers, the invention relates to both the pure isomers and mixtures thereof. Likeweise, the invention relates to the use of the pure pure isomers and to the use of their mixtures and to compositions containing the pure isomers or mixtures thereof.

If the diaminotriazine compounds of formula (I) as described herein have one or more centres of chirality and, as a consequence, are present as enantiomers or diastereomers, the invention relates to both the pure enantiomers or diastereomers, and mixtures thereof. Likeweise, the invention relates to the use of the pure enantiomers or diasteremers and to the use of the mixtures thereof and to compositions containing the pure enantiomers or diastereomers or mixtures thereof.

If the diaminotriazine compounds of formula (I) as described herein have ionizable functional groups, they can also be employed in the form of their agriculturally acceptable salts. Suitable are, in general, the salts of those cations and the acid addition salts of those acids whose cations and anions, respectively, have no adverse effect on the activity of the active compounds.

Preferred cations are the ions of the alkali metals, preferably of lithium, sodium and potassium, of the alkaline earth metals, preferably of calcium and magnesium, and of the transition metals, preferably of manganese, copper, zinc and iron, further ammonium and substituted ammonium in which one to four hydrogen atoms are replaced by C₁-C₄-alkyl, hydroxy-C₁-C₄-alkyl, (C₁-C₄-alkoxy)-C₁-C₄-alkyl, hydroxy-(C₁-C₄-alkoxy)-C₁-C₄-alkyl, phenyl or benzyl, preferably ammonium, methylammonium, isopropylammonium, dimethylammonium, diisopropylammonium, trimethylammonium, heptylammonium, dodecylammonium, tetradecylammonium, tetramethylammonium, tetraethylammonium, tetrabutylammonium, 2-hydroxyethyl-ammonium (olamine salt), 2-(2-hydroxyeth-1-oxy)eth-1-ylammonium (diglycolamine salt), di(2-hydroxyeth-1-yl)-ammonium (diolamine salt), tris(2-hydroxyethyl)ammonium (trolamine salt), tris(2-hydroxypropyl)ammonium, benzyltrimethylammonium, benzyltriethylammonium, N,N,N-trimethylethanolammonium (choline salt), furthermore phosphonium ions, sulfonium ions, preferably tri(C₁-C₄-alkyl)sulfonium, such as trimethylsulfonium, and sulfoxonium ions, preferably tri(C₁-C₄-alkyl)sulfoxonium, and finally the salts of polybasic amines such as N,N-bis-(3-aminopropyl)methylamine and diethylenetriamine.

Anions of useful acid addition salts are primarily chloride, bromide, fluoride, iodide, hydrogensulfate, methylsulfate, sulfate, dihydrogenphosphate, hydrogenphosphate, nitrate, bicarbonate, carbonate, hexafluorosilicate, hexafluorophosphate, benzoate and also the anions of C₁-C₄-alkanoic acids, preferably formate, acetate, propionate and butyrate.

Further embodiments of the present invention are evident from the claims, the description and the examples. It is to be understood that the features mentioned above and still to be illustrated below of the subject matter of the invention can be applied not only in the combination given in each particular case but also in other combinations, without leaving the scope of the invention.

The organic moieties mentioned in the definition of the variables, e.g. A, R^{A}, R¹, R², R^{3a}, R^{3b}, R^{3c}, R^{3d}, X are - like the term halogen - collective terms for individual enumerations of the individual group members. The term halogen denotes in each case fluorine, chlorine, bromine or iodine. All hydrocarbon chains, i.e. all alkyl, haloalkyl, alkenyl, alkynyl, alkenyloxy, alkynyloxy, alkoxy, alkylthio, alkylsulfinyl, alkylsulfonyl, (alkyl)amino, di(alkyl)amino, (alkyl)aminocarbonyl, di(alkyl)aminocarbonyl, (alkyl)aminosulfony, di(alkyl)aminosulfonyl, alkoxyalkyl, alkoxy, (alky)carbonyl, (alkoxy)carbonyl chains can be straight-chain or branched, the prefix Cₙ-Cₘ denoting in each case the possible number of carbon atoms in the group. The same applies to composed radicals, such as cycloalkylalkyl and phenylalkyl.

Examples of such meanings are:
- C₁-C₄-alkyl and also the C₁-C₄-alkyl moieties of C₁-C₄-alkoxy, C₁-C₄-alkylthio, C₁-C₄-alkylsulfonyl, (C₁-C₄-alkyl)carbonyl, (C₁-C₄-alkyl)carbonyl, (C₁-C₄-alkoxy)carbonyl, (C₁-C₄-alkyl)carbonyloxy, C₁-C₄-alkyoxy-C₁-C₄-alkyl, C₃-C₆-cycloalkyl-C₁-C₄-alkyl, (C₁-C₄-alkylamino)carbonyl, di(C₁-C₄-alkyl)aminocarbonyl, (C₁-C₄-alkylamino)sulfonyl, di(C₁-C₄-alkyl)aminosulfonyl or phenyl-C₁-C₄-alkyl: for example CH₃, C₂H₅, n-propyl, CH(CH₃)₂, n-butyl, CH(CH₃)-C₂H₅, CH₂-CH(CH₃)₂ and C(CH₃)₃;
- C₁-C₆-alkyl and also the C₁-C₆-alkyl moieties of C₁-C₆-alkoxy, C₁-C₆-alkylthio, C₁-C₆-alkylsulfonyl, (C₁-C₆-alkyl)carbonyl, (C₁-C₆-alkyl)carbonyl, (C₁-C₆-alkoxy)carbonyl, (C₁-C₆-alkyl)carbonyloxy, C₁-C₆-alkyoxy-C₁-C₆-alkyl, C₃-C₆-cycloalkyl-C₁-C₆-alkyl, (C₁-C₆-alkylamino)carbonyl, di(C₁-C₆-alkyl)aminocarbonyl, (C₁-C₆-alkylamino)sulfonyl, di(C₁-C₆-alkyl)aminosulfonyl or phenyl-C₁-C₆-alkyl: C₁-C₄-alkyl as mentioned above, and also, for example, n-pentyl, 1-methylbutyl, 2-methylbutyl, 3-methylbutyl, 2,2-dimethylpropyl, 1-ethylpropyl, n-hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl or 1-ethyl-2-methylpropyl, preferably methyl, ethyl, n-propyl, 1-methylethyl, n-butyl, 1,1-dimethylethyl, n-pentyl or n-hexyl;
- C₂-C₆-alkenyl and also the C₂-C₆-alkenyl moieties of (C₁-C₆-alkoxy)-C₂-C₆-alkenyl: a linear or branched ethylenically unsaturated hydrocarbon group having 2 to 6 carbon atoms and a C=C-double bond in any position, such as ethenyl, 1-propenyl, 2-propenyl, 1-methyl-ethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl;
- C₂-C₆-alkynyl and also the C₂-C₆-alkynyl moieties of (C₁-C₆-alkoxy)-C₂-C₆-alkynyl: linear or branched unsaturated hydrocarbon group having 2 to 6 carbon atoms and containing at least one C-C-triple bond, such as ethynyl, 1-propynyl, 2-propynyl (propargyl), 1-butynyl, 2-butynyl, 3-butynyl, 1-methyl-2-propynyl and the like;
- C₁-C₄-haloalkyl: a C₁-C₄-alkyl radical as mentioned above which is partially or fully substituted by fluorine, chlorine, bromine and/or iodine, for example, chloro-methyl, dichloromethyl, trichloromethyl, fluoromethyl, difluoromethyl, trifluoromethyl, chlorofluoromethyl, dichlorofluoromethyl, chlorodifluoromethyl, bromomethyl, iodomethyl, 2-fluoroethyl, 2-chloroethyl, 2-bromoethyl, 2-iodoethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl, 2-fluoropropyl, 3-fluoropropyl, 2,2-difluoropropyl, 2,3-difluoropropyl, 2-chloropropyl, 3-chloropropyl, 2,3-dichloropropyl, 2-bromopropyl, 3-bromopropyl, 3,3,3-trifluoropropyl, 3,3,3-trichloropropyl, 2,2,3,3,3-pentafluoropropyl, heptafluoropropyl, 1-(fluoromethyl)-2-fluoroethyl, 1-(chloromethyl)-2-chloroethyl, 1-(bromomethyl)-2-bromoethyl, 4-fluorobutyl, 4-chlorobutyl, 4-bromobutyl, nonafluorobutyl, 1,1,2,2,-tetrafluoroethyl and 1-trifluoromethyl-1,2,2,2-tetrafluoroethyl;
- C₁-C₆-haloalkyl: C₁-C₄-haloalkyl as mentioned above, and also, for example, 5-fluoropentyl, 5-chloropentyl, 5-bromopentyl, 5-iodopentyl, undecafluoropentyl, 6-fluorohexyl, 6-chlorohexyl, 6-bromohexyl, 6-iodohexyl and dodecafluorohexyl;

- C₁-C₄-alkoxy: for example methoxy, ethoxy, propoxy, 1-methylethoxy butoxy, 1-methylpropoxy, 2-methylpropoxy and 1,1-dimethylethoxy;
- C₁-C₆-alkoxy and also the C₁-C₆-alkoxy moieties of (C₁-C₆-alkoxy)carbonyl, (C₁-C₆-alkoxy)sulfonyl, (C₁-C₆-alkoxy)-C₁-C₆-alkyl, (C₁-C₆-alkoxy)-C₁-C₆-alkoxy, (C₁-C₆-alkoxy)-C₂-C₆-alkenyl, (C₁-C₆-alkoxy)-C₂-C₆-alkynyl: C₁-C₄-alkoxy as mentioned above, and also, for example, pentoxy, 1-methylbutoxy, 2-methylbutoxy, 3-methoxylbutoxy, 1,1-dimethylpropoxy, 1,2-dimethylpropoxy, 2,2-dimethylpropoxy, 1-ethylpropoxy, hexoxy, 1-methylpentoxy, 2-methylpentoxy, 3-methylpentoxy, 4-methylpentoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-ethylbutoxy, 2-ethylbutoxy, 1,1,2-trimethylpropoxy, 1,2,2-trimethylpropoxy, 1-ethyl-1-methylpropoxy and 1-ethyl-2-methylpropoxy;
- C₁-C₄-haloalkoxy: a C₁-C₄-alkoxy radical as mentioned above which is partially or fully substituted by fluorine, chlorine, bromine and/or iodine, for example, chloro-methoxy, dichloromethoxy, trichloromethoxy, fluoromethoxy, difluoromethoxy, trifluoromethoxy, chlorofluoromethoxy, dichlorofluoromethoxy, chlorodifluoromethoxy2-fluoroethoxy, 2-chloroethoxy, 2-bromoethxoy, 2,2-difluoroethoxy, 2,2,2-trifluoroethoxy, 2-chloro-2-fluoroethoxy, 2-chloro-2,2-difluoroethoxy, 2,2-dichloro-2-fluoroethoxy, 2,2,2-trichloroethoxy, pentafluoroethoxy, 2-fluoropropoxy, 3-fluoropropoxy, 2,2-difluoropropoxy, 2,3-difluoropropoxy, 2-chloropropoxy, 3-chloropropoxy, 2,3-dichloropropoxy, 3,3,3-trifluoropropoxy, 3,3,3-trichloropropoxy, 2,2,3,3,3-pentafluoropropoxy, heptafluoropropoxy, 1-(fluoromethyl)-2-fluoroethoxy, 4-fluorobutoxy, nonafluorobutoxy, 1,1,2,2,-tetrafluoroethoxy and 1-trifluoromethyl-1,2,2,2-tetrafluoroethoxy;
- C₁-C₆-haloalkoxy: C₁-C₄-alkoxy as mentioned above: C₁-C₄-haloalkoxy as mentioned above, and also, for example, 5-fluoropentyl, 5-chloropentyl, 5-bromopentyl, 5-iodopentyl, undecafluoropentyl, 6-fluorohexyl, 6-chlorohexyl, 6-bromohexyl, 6-iodohexyl and dodecafluorohexyl;
- C₂-C₆-alkenyloxy: C₂-C₆-alkenyl as defined above, which is bound via an oxygen atom, such as ethenyloxy (vinyloxy), 1-propenyloxy, 2-propenyloxy (allyloxy), 1-butenyloxy, 2-butenyloxy, 3-butenyloxy 1-methyl-2-propenyloxy and the like;
- C₂-C₆-alkynyloxy: C₂-C₆-alkynyl as defined above, which is bound via an oxygen atom, such as ethynyloxy, 1-propynyl, 2-propynyloxy (propargyloxy), 1-butynyloxy, 2-butynyloxy, 3-butynyloxy 1-methyl-2-propynyloxy and the like;
- C₁-C₄-alkylthio: for example methylthio, ethylthio, propylthio, 1-methylethylthio, butylthio, 1-methylpropylthio, 2-methylpropylthio and 1,1-dimethylethylthio;
- C₁-C₆-alkylthio: C₁-C₄-alkylthio as mentioned above, and also, for example, pentylthio, 1-methylbutylthio, 2-methylbutylthio, 3-methylbutylthio, 2,2-dimethylpropylthio, 1-ethylpropylthio, hexylthio, 1,1-dimethylpropylthio, 1,2-dimethylpropylthio, 1-methylpentylthio, 2-methylpentylthio, 3-methylpentylthio, 4-methylpentylthio, 1,1-dimethylbutylthio, 1,2-dimethylbutylthio, 1,3-dimethylbutylthio, 2,2-dimethylbutylthio, 2,3-dimethylbutylthio, 3,3-dimethylbutylthio, 1-ethylbutylthio, 2-ethylbutylthio, 1,1,2-trimethylpropylthio, 1,2,2-trimethylpropylthio, 1-ethyl-1-methylpropylthio and 1- ethyl-2-methylpropylthio;
- C₁-C₆-alkylsulfinyl (C₁-C₆-alkyl-S(=O)-): z.B. methylsulfinyl, ethylsulfinyl, propylsulfinyl, 1-methylethylsulfinyl, butylsulfinyl, 1-methylpropylsulfinyl, 2-methylpropylsulfinyl, 1,1-dimethylethylsulfinyl, pentylsulfinyl, 1-methylbutylsulfinyl, 2-methylbutylsulfinyl, 3-methylbutylsulfinyl, 2,2-dimethylpropylsulfinyl, 1-ethylpropylsulfinyl, 1,1-dimethylpropylsulfinyl, 1,2-dimethylpropylsulfinyl, hexylsulfinyl, 1-methylpentylsulfinyl, 2-methylpentylsulfinyl, 3-methylpentylsulfinyl, 4-methylpentyl-sulfinyl, 1,1-dimethylbutylsulfinyl, 1,2-dimethylbutylsulfinyl, 1,3-dimethylbutyl-sulfinyl, 2,2-dimethylbutylsulfinyl, 2,3-dimethylbutylsulfinyl, 3,3-dimethylbutyl-sulfinyl, 1-ethylbutylsulfinyl, 2-ethylbutylsulfinyl, 1,1,2-trimethylpropylsulfinyl, 1,2,2-trimethylpropylsulfinyl, 1-ethyl-1-methylpropylsulfinyl and 1-ethyl-2-methylpropylsulfinyl;
- C₁-C₆-alkylsulfonyl (C₁-C₆-alkyl-S(O)₂-): for example methylsulfonyl, ethylsulfonyl, propylsulfonyl, 1-methylethylsulfonyl, butylsulfonyl, 1-methylpropylsulfonyl, 2-methylpropylsulfonyl, 1,1-dimethylethylsulfonyl, pentylsulfonyl, 1-methylbutylsulfonyl, 2-methylbutylsulfonyl, 3-methylbutylsulfonyl, 1,1-dimethylpropylsulfonyl, 1,2-dimethylpropylsulfonyl, 2,2-dimethylpropylsulfonyl, 1-ethylpropylsulfonyl, hexylsulfonyl, 1-methylpentylsulfonyl, 2-methylpentylsulfonyl, 3-methylpentylsulfonyl, 4-methylpentylsulfonyl, 1,1-dimethylbutylsulfonyl, 1,2-dimethylbutylsulfonyl, 1,3-dimethylbutylsulfonyl, 2,2-dimethylbutylsulfonyl, 2,3-dimethylbutylsulfonyl, 3,3-dimethylbutylsulfonyl, 1-ethylbutylsulfonyl, 2-ethylbutylsulfonyl, 1,1,2-trimethyl-propylsulfonyl, 1,2,2-trimethylpropylsulfonyl, 1-ethyl-1-methylpropylsulfonyl and 1-ethyl-2-methylpropylsulfonyl;
- (C₁-C₄-alkyl)amino and also the (C₁-C₄-alkylamino) moieties of (C₁-C₄-alkylamino)carbonyl or (C₁-C₄-alkylamino)sulfonyl: for example methylamino, ethylamino, propylamino, 1-methylethylamino, butylamino, 1-methylpropylamino, 2-methylpropylamino or 1,1-dimethylethylamino;
- (C₁-C₆-alkyl)amino and also the (C₁-C₆-alkylamino) moieties of (C₁-C₆-alkylamino)carbonyl or (C₁-C₆-alkylamino)sulfonyl: (C₁-C₄-alkyl)amino as mentioned above, and also, for example, pentylamino, 1-methylbutylamino, 2-methylbutylamino, 3-methylbutylamino, 2,2-dimethylpropylamino, 1-ethylpropylamino, hexylamino, 1,1-dimethylpropylamino, 1,2-dimethylpropylamino, 1-methylpentylamino, 2-methylpentylamino, 3-methylpentylamino, 4-methylpentylamino, 1,1-dimethylbutylamino, 1,2-dimethylbutylamino, 1,3-dimethylbutylamino, 2,2-dimethylbutylamino, 2,3-dimethylbutyl-amino 3,3-dimethylbutylamino, 1-ethylbutylamino, 2-ethylbutylamino, 1,1,2-trimethylpropylamino, 1,2,2-trimethyl-propylamino, 1-ethyl-1-methylpropylamino or 1-ethyl-2-methylpropylamino;
- di(C₁-C₄-alkyl)amino and also the di(C₁-C₄-alkylamino) moieties of di(C₁-C₄-alkylamino)carbonyl or di(C₁-C₄-alkylamino)sulfonyl: for example N,N-dimethylamino, N,N-diethylamino, N,N-di(1-methylethyl)amino, N,N-dipropylamino, N,N-dibutylamino, N,N-di(1-methylpropyl)amino, N,N-di(2-methylpropyl)amino, N,N-di(1,1-dimethylethyl)amino, N-ethyl-N-methylamino, N-methyl-N-propylamino, N-methyl-N-(1-methylethyl)amino, N-butyl-N-methylamino, N-methyl-N-(1-methylpropyl)amino, N-methyl-N-(2-methylpropyl)amino, N-(1,1-dimethylethyl)-N-methylamino, N-ethyl-N-propylamino, N-ethyl-N-(1-methylethyl)amino, N-butyl-N-ethylamino, N-ethyl-N-(1-methylpropyl)amino, N-ethyl-N-(2-methylpropyl)amino, N-ethyl-N-(1,1-dimethylethyl)amino, N-(1-methylethyl)-N-propylamino, N-butyl-N-propylamino, N-(1-methylpropyl)-N-propylamino, N-(2-methylpropyl)-N-propylamino, N-(1,1-dimethylethyl)-N-propylamino, N-butyl-N-(1-methylethyl)amino, N-(1-methylethyl)-N-(1-methylpropyl)amino, N-(1-methylethyl)-N-(2-methylpropyl)amino, N-(1,1-dimethylethyl)-N-(1-methylethyl)amino, N-butyl-N-(1-methylpropyl)amino, N-butyl-N-(2-methylpropyl)amino, N-butyl-N-(1,1-dimethylethyl)amino, N-(1-methylpropyl)-N-(2-methylpropyl)amino, N-(1,1-dimethylethyl)-N-(1-methylpropyl)amino or N-(1,1-dimethylethyl)-N-(2-methylpropyl)amino;
- di(C₁-C₆-alkyl)amino and also the di(C₁-C₆-alkylamino) moieties of di(C₁-C₆-alkylamino)carbonyl or di(C₁-C₆-alkylamino)sulfonyl: di(C₁-C₄-alkyl)amino as mentioned above, and also, for example, N-methyl-N-pentylamino, N-methyl-N-(1-methylbutyl)amino, N-methyl-N-(2-methylbutyl)amino, N-methyl-N-(3-methylbutyl)amino, N-methyl-N-(2,2-dimethylpropyl)amino, N-methyl-N-(1-ethylpropyl)amino, N-methyl-N-hexylamino, N-methyl-N-(1,1-dimethylpropyl)amino, N-methyl-N-(1,2-dimethylpropyl)amino, N-methyl-N-(1-methylpentyl)amino, N-methyl-N-(2-methylpentyl)amino, N-methyl-N-(3-methylpentyl)amino, N-methyl-N-(4-methylpentyl)amino, N-methyl-N-(1,1-dimethylbutyl)amino, N-methyl-N-(1,2-dimethylbutyl)amino, N-methyl-N-(1,3-dimethylbutyl)amino, N-methyl-N-(2,2-dimethylbutyl)amino, N-methyl-N-(2,3-dimethylbutyl)amino, N-methyl-N-(3,3-dimethylbutyl)amino, N-methyl-N-(1-ethylbutyl)amino, N-methyl-N-(2-ethylbutyl)amino, N-methyl-N-(1,1,2-trimethylpropyl)amino, N-methyl-N- (1,2,2-trimethylpropyl)amino, N-methyl-N-(1-ethyl-1-methylpropyl)amino, N-methyl-N- (1-ethyl-2-methylpropyl)amino, N-ethyl-N-pentylamino, N-ethyl-N-(1-methylbutyl)amino, N-ethyl-N-(2-methylbutyl)amino, N-ethyl-N-(3-methylbutyl)amino, N-ethyl-N-(2,2-dimethylpropyl)amino, N-ethyl-N-(1-ethylpropyl)amino, N-ethyl-N-hexylamino, N-ethyl-N-(1,1-dimethylpropyl)amino, N-ethyl-N-(1,2-dimethylpropyl)amino, N-ethyl-N-(1-methylpentyl)amino, N-ethyl-N-(2-methylpentyl)amino, N-ethyl-N-(3-methylpentyl)amino, N-ethyl-N-(4-methylpentyl)amino, N-ethyl-N-(1,1-dimethylbutyl)amino, N-ethyl-N-(1,2-dimethylbutyl)amino, N-ethyl-N-(1,3-dimethylbutyl)amino, N-ethyl-N-(2,2-dimethylbutyl)amino, N-ethyl-N-(2,3-dimethylbutyl)amino, N-ethyl-N-(3,3-dimethylbutyl)amino, N-ethyl-N-(1-ethylbutyl)amino, N-ethyl-N-(2-ethylbutyl)amino, N-ethyl-N-(1,1,2-trimethylpropyl)amino, N-ethyl-N-(1,2,2-trimethylpropyl)amino, N-ethyl-N-(1-ethyl-1-methylpropyl)amino, N-ethyl-N-(1-ethyl-2-methylpropyl)amino, N-propyl-N-pentylamino, N-butyl-N-pentylamino, N,N-dipentylamino, N-propyl-N-hexylamino, N-butyl-N-hexylamino, N-pentyl-N-hexylamino or N,N-dihexylamino;
- C₃-C₆-cyclolalkyl and also the C₃-C₆-cyclolalkyl moieties of (C₃-C₆-cyclolalkyl)-carbonyl, (C₃-C₆-cyclolalkyl)-C₁-C₆-alkyl and (C₃-C₆-cyclolalkyl)-C₁-C₆-alkoxy: a cycloaliphatic radical having 3 to 6 carbon atoms, such as cyclopropyl, cyclobutyl, cyclopentyl and cyclohexyl;
- C₃-C₆-cyclolalkoxy: a cycloaliphatic radical having 3 to 6 carbon atoms and bound via an oxygen atom, such as cyclopropyloxy, cyclobutyloxy, cyclopentyloxy and cyclohexyloxy;
- (C₃-C₆-cyclolalkyl)-C₁-C₆-alkyl: C₁-C₆-alkyl, in particular C₁-C₄-alkyl as defined above, such as methyl or ethyl, wherein 1 hydrogen atom is replaced by C₃-C₆-cyclolalkyl as defined above, examples including cyclopropylmethyl (CH₂-cyclopropyl), cyclobutylmethyl, cyclopentylmethyl, cycloexylmethyl, 1-cyclopropylethyl (CH(CH₃)-cyclopropyl), 1-cyclobutylethyl, 1-cyclopentylethyl, 1-cycloexylethyl, 2-cyclopropylethyl (CH₂CH₂-cyclopropyl), 2-cyclobutylethyl, 2-cyclopentylethyl or 2-cycloexylethyl;
- (C₃-C₆-cyclolalkyl)-C₁-C₆-alkoxy: C₁-C₆-alkoxy, in particular C₁-C₄-alkoxy as defined above, such as methoxy or ethoxy, wherein 1 hydrogen atom is replaced by C₃-C₆-cyclolalkyl as defined above, examples including cyclopropylmethoxy (OCH₂-cyclopropyl), cyclobutylmethoxy, cyclopentylmethoxy, cycloexylmethoxy, 1-cyclopropylethoxy (O-CH(CH₃)-cyclopropyl), 1-cyclobutylethoxy, 1-cyclopentylethoxy, 1-cycloexylethoxy, 2-cyclopropylethoxy (OCH₂CH₂)-cyclopropyl), 2-cyclobutylethoxy, 2-cyclopentylethoxy and 2-cycloexylethoxy;
- (C₁-C₆-alkoxy)-C₁-C₆-alkyl: C₁-C₆-alkyl, in particular C₁-C₄-alkyl as defined above, such as methyl, ethyl or isopropyl, wherein 1 hydrogen atom is replaced by C₁-C₆-alkoxy as defined above, examples including methoxymethyl, ethoxymethyl, n-propoxymethyl, butoxymethyl, 1-methoxyethyl, 1-ethoxyethyl, 1-(n-propoxy)ethyl, 1-butoxyethyl, 2-methoxyethyl, 2-ethoxyethyl, 2-(n-propoxy)ethyl, 2-butoxyethyl, 2-methoxypropyl, 2-ethoxypropyl, 2-(n-propoxy)propyl, 2-butoxypropyl;
- (C₁-C₆-alkoxy)-C₁-C₆-alkoxy: C₁-C₆-alkoxy, in particular C₁-C₄-alkoxy as defined above, such as methoxy or ethoxy, wherein 1 hydrogen atom is replaced by C₁-C₆-alkoxy as defined above, examples including methoxymethoxy, ethoxymethoxy, n-propoxymethoxy, butoxymethoxy, 2-methoxyethoxy, 2-ethoxyethoxy, 2-(n-propoxy)ethoxy and 2-butoxyethoxy;
- (C₁-C₆-alkoxy)-C₂-C₆-alkenyl: C₂-C₆-alkenyl, in particular C₂-C₄-alkenyl as defined above, such as ethenyl, propenyl, 1-butenyl or 2-butenyl, wherein 1 hydrogen atom is replaced by C₁-C₆-alkoxy as defined above;
- (C₁-C₆-alkoxy)-C₂-C₆-alkynyl: C₂-C₆-alkynyl, in particular C₂-C₄-alkynyl as defined above, such as ethynyl, propynyl or 2-butynyl, wherein 1 hydrogen atom is replaced by C₁-C₆-alkoxy as defined above;
- (C₁-C₆-alkyl)carbonyl: C₁-C₆-alkyl as mentioned above, which is bound to the remainder of the molecule by a carbonyl group;
- (C₁-C₆-alkoxy)carbonyl: C₁-C₆-alkyloxy as mentioned above, which is bound to the remainder of the molecule by a carbonyl group;
- (C₁-C₆-alkylamino)carbonyl: (C₁-C₆-alkyl)amino as mentioned above, which is bound to the remainder of the molecule by a carbonyl group;
- (C₁-C₆-alkylamino)sulfonyl: (C₁-C₆-alkyl)amino as mentioned above, which is bound to the remainder of the molecule by a sulfonyl group;
- di(C₁-C₆-alkylamino)carbonyl: di(C₁-C₆-alkyl)amino as mentioned above, which is bound to the remainder of the molecule by a carbonyl group;
- di(C₁-C₆-alkylamino)sulfonyl: di(C₁-C₆-alkyl)amino as mentioned above, which is bound to the remainder of the molecule by a sulfonyl group;
- phenyl-C₁-C₆-alkyl: C₁-C₆-alkyl, in particular C₁-C₄-alkyl as defined above, such as methyl or ethyl, wherein 1 hydrogen atom is replaced by phenyl, examples including benzyl, 1-phenylethyl, 2-phenylethyl, 1-phenylpropyl, 2-phenylpropyl, 1-phenyl-1-methylethyl etc.;
- three- to six-membered heterocyclyl: monocyclic saturated or partially unsaturated hydrocarbon having three to six ring members as mentioned above which, in addition to carbon atoms, contains one or two heteroatoms selected from O, S and N;
for example
saturated heterocycles such as 2-oxiranyl, 2-oxetanyl, 3-oxetanyl, 2-aziridinyl, 3-thietanyl, 1-azetidinyl, 2-azetidinyl, 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 2-piperidinyl, 3-piperidinyl, 4-piperidinyl, 1,3-dioxan-2-yl, 1,3-dioxan-4-yl, 1,3-dioxan-5-yl, 1,4-dioxan-2-yl, 1,3-dithian-2-yl, 1,3-dithian-4-yl, 1,4-dithian-2-yl, 1,3-dithian-5-yl, 2-tetrahydropyranyl, 3-tetrahydropyranyl, 4-tetrahydropyranyl, 2-tetrahydrothiopyranyl, 3-tetrahydrothiopyranyl, 4-tetrahydro-thiopyranyl, 3-hexahydropyridazinyl, 4-hexahydropyridazinyl, 2-hexahydropyrimidinyl, 4-hexahydropyrimidinyl, 5-hexahydropyrimidinyl, 2-piperazinyl, tetrahydro-1,3-oxazin-2-yl, tetrahydro-1,3-oxazin-6-yl, 2-morpholinyl, 3-morpholinyl or 4-morpholinyl, for example 2H-pyran-2-yl, 2H-pyran-3-yl, 2H-pyran-4-yl, 2H-pyran-5-yl, 2H-pyran-6-yl, 2H-thiopyran-2-yl, 2H-thiopyran-3-yl, 2H-thiopyran-4-yl, 2H-thiopyran-5-yl, 2H-thiopyran-6-yl;
partially unsaturated heterocycles such as 2,3-dihydrofur-2-yl, 2,3-dihydrofur-3-yl, 2,4-dihydrofur-2-yl, 2,4-dihydrofur-3-yl, 2,3-dihydrothien-2-yl, 2,3-dihydrothien-3-yl, 2,4-dihydrothien-2-yl, 2,4-dihydrothien-3-yl, 4,5-dihydropyrrol-2-yl, 4,5-dihydropyrrol-3-yl, 2,5-dihydropyrrol-2-yl, 2,5-dihydropyrrol-3-yl, 4,5-dihydroisoxazol-3-yl, 2,5-dihydroisoxazol-3-yl, 2,3-dihydroisoxazol-3-yl, 4,5-dihydroisoxazol-4-yl, 2,5-dihydroisoxazol-4-yl, 2,3-dihydroisoxazol-4-yl, 4,5-dihydroisoxazol-5-yl, 2,5-dihydroisoxazol-5-yl, 2,3-dihydroisoxazol-5-yl, 4,5-dihydroisothiazol-3-yl, 2,5-dihydroisothiazol-3-yl, 2,3-dihydroisothiazol-3-yl, 4,5-dihydroisothiazol-4-yl, 2,5-dihydroisothiazol-4-yl, 2,3-dihydroisothiazol-4-yl, 4,5-dihydroisothiazol-5-yl, 2,5-dihydroisothiazol-5-yl, 2,3-dihydroisothiazol-5-yl, 2,3-dihydropyrazol-2-yl, 2,3-dihydropyrazol-3-yl, 2,3-dihydropyrazol-4-yl, 2,3-dihydropyrazol-5-yl, 3,4-dihydropyrazol-3-yl, 3,4-dihydropyrazol-4-yl, 3,4-dihydropyrazol-5-yl, 4,5-dihydropyrazol-3-yl, 4,5-dihydropyrazol-4-yl, 4,5-dihydropyrazol-5-yl, 2,3-dihydroimidazol-2-yl, 2,3-dihydroimidazol-3-yl, 2,3-dihydroimidazol-4-yl, 2,3-dihydroimidazol-5-yl, 4,5-dihydroimidazol-2-yl, 4,5-dihydroimidazol-4-yl, 4,5-dihydroimidazol-5-yl, 2,5-dihydroimidazol-2-yl, 2,5-dihydroimidazol-4-yl, 2,5-dihydroimidazol-5-yl, 2,3-dihydrooxazol-3-yl, 2,3-dihydrooxazol-4-yl, 2,3-dihydrooxazol-5-yl, 3,4-dihydrooxazol-3-yl, 3,4-dihydrooxazol-4-yl, 3,4-dihydrooxazol-5-yl, 2,3-dihydrothiazol-3-yl, 2,3-dihydrothiazol-4-yl, 2,3-dihydrothiazol-5-yl, 3,4-dihydrothiazol-3-yl, 3,4-dihydrothiazol-4-yl, 3,4-dihydrothiazol-5-yl, 3,4-dihydrothiazol-2-yl, 3,4-dihydrothiazol-3-yl, 3,4-dihydrothiazol-4-yl, 3,6-dihydro-2H-pyran-2-yl, 3,6-dihydro-2H-pyran-3-yl, 3,6-dihydro-2H-pyran-4-yl, 3,6-dihydro-2H-pyran-5-yl, 3,6-dihydro-2H-pyran-6-yl, 3,4-dihydro-2H-pyran-3-yl, 3,4-dihydro-2H-pyran-4-yl, 3,4-dihydro-2H-pyran-6-yl, 5,6-dihydro-4H-1,3-oxazin-2-yl;

The preferred embodiments of the invention mentioned herein below have to be understood as being preferred either independently from each other or in combination with one another. Particular groups embodiments of the invention relate to those diaminotriazines of formula (I), wherein the variables R¹, R², A and X, either independently of one another or in combination with one another, have the following meanings:

Particular groups (a) of embodiments relate to the diaminotriazine compounds of formula (I), wherein
- A: is monocyclic heteroaryl having 5 or 6 ring members which, in addition to carbon atoms, contains 1, 2 or 3 nitrogen atoms, or alternatively 1 or 2 nitrogen atoms and an oxygen or sulfur atom, or an oxygen or a sulfur atom, which is unsubstituted or substituted by 1, 2, 3 or 4, in particular 2, 3 or 4 radicals R^{A}, and wherein
- R^{A}: is frequently selected from the group consisting of halogen, CN, amino, NO₂, C₁-C₆-alkyl, C₂-C₆-alkynyl, C₃-C₆-cycloalkyl, C₃-C₆-cycloalkoxy, (C₃-C₆-cycloalkyl)methoxy, C₁-C₆-alkoxy, (C₁-C₆-alkoxy)-C₁-C₆-alkyl, (C₁-C₆-alkoxy)-C₁-C₆-alkoxy, (C₁-C₆-alkoxy)-C₂-C₆-alkenyl, (C₁-C₆-alkoxy)-C₂-C₆-alkynyl, C₁-C₆-alkylthio, (C₁-C₆-alkyl)sulfinyl, (C₁-C₆-alkyl)sulfonyl, (C₁-C₆-alkyl)amino, di(C₁-C₆-alkyl)amino, (C₁-C₆-alkyl)carbonyl and (C₁-C₆-alkoxy)carbonyl, where the aliphatic and cycloaliphatic parts of the 17 aforementioned radicals are unsubstituted, partly or completely halogenated;
preferably selected from the group consisting of halogen, CN, C₁-C₆-alkyl, C₁-C₆-haloalkyl, C₁-C₆-alkoxy, C₁-C₆-haloalkoxy, C₃-C₆-cycloalkyl, C₃-C₆-cycloalkoxy, (C₃-C₆-cycloalkyl)methoxy, C₂-C₆-alkynyl, C₂-C₆-alkenyl, C₂-C₆-alkynyloxy and C₂-C₆-alkenyloxy; more particularly selected from halogen, such as F or Cl, C₁-C₄-alkyl, such as methyl, C₂-C₄-alkenyl such as ethenyl (= vinyl), C₂-C₄-alkynyl such as ethynyl, C₂-C₄-alkenyloxy, such as allyloxy, C₂-C₄-alkynyloxy, such as propargyloxy, C₁-C₄-haloalkyl, such as trifluoromethyl, C₁-C₄-alkoxy, such as methoxy, ethoxy, isopropyloxy or 2-butyloxy, C₁-C₄-haloalkoxy, such as trifluoromethoxy, C₃-C₆-cycloalkyl, such as cyclopropyl, cyclobutyl or cyclopentyl, C₃-C₆-cycloalkoxy, such as cyclopropyloxy, cyclobutyloxy or cyclopentyloxy, (C₃-C₆-cycloalkyl)methoxy, such as cyclopropylmethoxy and CN; even more particularly selected from halogen, C₁-C₄-alkyl, C₂-C₄-alkynyl, C₁-C₄-haloalkyl, C₁-C₄-alkoxy, C₁-C₄-haloalkoxy and CN;
even more particularly selected from halogen, C₁-C₄-haloalkyl, C₁-C₄-alkoxy, C₁-C₄-haloalkoxy and CN;
especially selected from the group consisting of F, Cl and CN.

Further particular groups (b) of embodiments relate to the diaminotriazine compounds of formula (I), wherein
- A: is 6-membered heteroaryl having 1, 2 or 3 nitrogen atoms, in particular 1 nitrogen atom as ring members, i.e. 2-, 3- or 4-pyridyl, where the 6-membered heteroaryl is unsubstituted or in particular substituted by 1, 2, 3 or 4 radicals R^{A}, in particular by 2, 3 or 4 radicals R^{A}, especially by 3 or 4 radicals R^{A},
especially 2-pyridyl or 4-pyridyl, which is substituted by 1, 2, 3 or 4 radicals R^{A}, in particular by 2, 3 or 4 radicals R^{A}, especially by 3 or 4 radicals R^{A}, where R^{A} is as defined above; and where
- R^{A}: is frequently selected from the group consisting of halogen, CN, amino, NO₂, C₁-C₆-alkyl, C₂-C₆-alkynyl, C₃-C₆-cycloalkyl, C₃-C₆-cycloalkoxy, (C₃-C₆-cycloalkyl)methoxy, C₁-C₆-alkoxy, (C₁-C₆-alkoxy)-C₁-C₆-alkyl, (C₁-C₆-alkoxy)-C₁-C₆-alkoxy, (C₁-C₆-alkoxy)-C₂-C₆-alkenyl, (C₁-C₆-alkoxy)-C₂-C₆-alkynyl, C₁-C₆-alkylthio, (C₁-C₆-alkyl)sulfinyl, (C₁-C₆-alkyl)sulfonyl, (C₁-C₆-alkyl)amino, di(C₁-C₆-alkyl)amino, (C₁-C₆-alkyl)carbonyl and (C₁-C₆-alkoxy)carbonyl, where the aliphatic and cycloaliphatic parts of the 17 aforementioned radicals are unsubstituted, partly or completely halogenated;
preferably selected from the group consisting of halogen, CN, C₁-C₆-alkyl, C₁-C₆-haloalkyl, C₁-C₆-alkoxy, C₁-C₆-haloalkoxy, C₃-C₆-cycloalkyl, C₃-C₆-cycloalkoxy, (C₃-C₆-cycloalkyl)methoxy, C₂-C₆-alkynyl, C₂-C₆-alkenyl, C₂-C₆-alkynyloxy and C₂-C₆-alkenyloxy; more particularly selected from halogen, such as F or Cl, C₁-C₄-alkyl, such as methyl, C₂-C₄-alkenyl such as ethenyl (= vinyl), C₂-C₄-alkynyl such as ethynyl, C₂-C₄-alkenyloxy, such as allyloxy, C₂-C₄-alkynyloxy, such as propargyloxy, C₁-C₄-haloalkyl, such as trifluoromethyl, C₁-C₄-alkoxy, such as methoxy, ethoxy, isopropyloxy or 2-butyloxy, C₁-C₄-haloalkoxy, such as trifluoromethoxy, C₃-C₆-cycloalkyl, such as cyclopropyl, cyclobutyl or cyclopentyl, C₃-C₆-cycloalkoxy, such as cyclopropyloxy, cyclobutyloxy or cyclopentyloxy, (C₃-C₆-cycloalkyl)methoxy, such as cyclopropylmethoxy and CN;
even more particularly selected from halogen, C₁-C₄-alkyl, C₂-C₄-alkynyl, C₁-C₄-haloalkyl, C₁-C₄-alkoxy, C₁-C₄-haloalkoxy and CN;
even more particularly selected from halogen, C₁-C₄-haloalkyl, C₁-C₄-alkoxy, C₁-C₄-haloalkoxy and CN;
especially selected from the group consisting of F, Cl and CN.

Further particular groups (c) of embodiments relate to the diaminotriazine compounds of formula (I), wherein
- A: is 5-membered heteroaryl having 1, 2 or 3 nitrogen atoms, or alternatively 0, 1 or 2 nitrogen atoms and on atom selected from O and S as ring members, where the 5-membered heteroaryl is unsubstituted or in particular substituted by 1, 2 or 3 radicals R^{A}, in particular by 2 or 3 radicals R^{A},
where R^{A} is as defined above; and where
- R^{A}: is frequently selected from the group consisting of halogen, CN, amino, NO₂, C₁-C₆-alkyl, C₂-C₆-alkynyl, C₃-C₆-cycloalkyl, C₃-C₆-cycloalkoxy, (C₃-C₆-cycloalkyl)methoxy, C₁-C₆-alkoxy, (C₁-C₆-alkoxy)-C₁-C₆-alkyl, (C₁-C₆-alkoxy)-C₁-C₆-alkoxy, (C₁-C₆-alkoxy)-C₂-C₆-alkenyl, (C₁-C₆-alkoxy)-C₂-C₆-alkynyl, C₁-C₆-alkylthio, (C₁-C₆-alkyl)sulfinyl, (C₁-C₆-alkyl)sulfonyl, (C₁-C₆-alkyl)amino, di(C₁-C₆-alkyl)amino, (C₁-C₆-alkyl)carbonyl and (C₁-C₆-alkoxy)carbonyl, where the aliphatic and cycloaliphatic parts of the 17 aforementioned radicals are unsubstituted, partly or completely halogenated;
preferably selected from the group consisting of halogen, CN, C₁-C₆-alkyl, C₁-C₆-haloalkyl, C₁-C₆-alkoxy, C₁-C₆-haloalkoxy, C₃-C₆-cycloalkyl, C₃-C₆-cycloalkoxy, (C₃-C₆-cycloalkyl)methoxy, C₂-C₆-alkynyl, C₂-C₆-alkenyl, C₂-C₆-alkynyloxy and C₂-C₆-alkenyloxy; more particularly selected from halogen, such as F or Cl, C₁-C₄-alkyl, such as methyl, C₂-C₄-alkenyl such as ethenyl (= vinyl), C₂-C₄-alkynyl such as ethynyl, C₂-C₄-alkenyloxy, such as allyloxy, C₂-C₄-alkynyloxy, such as propargyloxy, C₁-C₄-haloalkyl, such as trifluoromethyl, C₁-C₄-alkoxy, such as methoxy, ethoxy, isopropyloxy or 2-butyloxy, C₁-C₄-haloalkoxy, such as trifluoromethoxy, C₃-C₆-cycloalkyl, such as cyclopropyl, cyclobutyl or cyclopentyl, C₃-C₆-cycloalkoxy, such as cyclopropyloxy, cyclobutyloxy or cyclopentyloxy, (C₃-C₆-cycloalkyl)methoxy, such as cyclopropylmethoxy and CN; even more particularly selected from halogen, C₁-C₄-alkyl, C₂-C₄-alkynyl, C₁-C₄-haloalkyl, C₁-C₄-alkoxy, C₁-C₄-haloalkoxy and CN;
even more particularly selected from halogen, C₁-C₄-haloalkyl, C₁-C₄-alkoxy, C₁-C₄-haloalkoxy and CN;
especially selected from the group consisting of F, Cl and CN.

Further particular groups (b.1) of embodiments relate to the diaminotriazine compounds of formula (I), wherein
- A: is 2-pyridiyl, which is unsubstituted or in particular substituted by 1, 2, 3 or 4 radicals R^{A}, in particular by 2, 3 or 4 radicals R^{A}, especially by 3 or 4 radicals R^{A},
where R^{A} is as defined above; and where
- R^{A}: is frequently selected from the group consisting of halogen, CN, amino, NO₂, C₁-C₆-alkyl, C₂-C₆-alkynyl, C₃-C₆-cycloalkyl, C₃-C₆-cycloalkoxy, (C₃-C₆-cycloalkyl)methoxy, C₁-C₆-alkoxy, (C₁-C₆-alkoxy)-C₁-C₆-alkyl, (C₁-C₆-alkoxy)-C₁-C₆-alkoxy, (C₁-C₆-alkoxy)-C₂-C₆-alkenyl, (C₁-C₆-alkoxy)-C₂-C₆-alkynyl, C₁-C₆-alkylthio, (C₁-C₆-alkyl)sulfinyl, (C₁-C₆-alkyl)sulfonyl, (C₁-C₆-alkyl)amino, di(C₁-C₆-alkyl)amino, (C₁-C₆-alkyl)carbonyl and (C₁-C₆-alkoxy)carbonyl, where the aliphatic and cycloaliphatic parts of the 17 aforementioned radicals are unsubstituted, partly or completely halogenated;
preferably selected from the group consisting of halogen, CN, C₁-C₆-alkyl, C₁-C₆-haloalkyl, C₁-C₆-alkoxy, C₁-C₆-haloalkoxy, C₃-C₆-cycloalkyl, C₃-C₆-cycloalkoxy, (C₃-C₆-cycloalkyl)methoxy, C₂-C₆-alkynyl, C₂-C₆-alkenyl, C₂-C₆-alkynyloxy and C₂-C₆-alkenyloxy; more particularly selected from halogen, such as F or Cl, C₁-C₄-alkyl, such as methyl, C₂-C₄-alkenyl such as ethenyl (= vinyl), C₂-C₄-alkynyl such as ethynyl, C₂-C₄-alkenyloxy, such as allyloxy, C₂-C₄-alkynyloxy, such as propargyloxy, C₁-C₄-haloalkyl, such as trifluoromethyl, C₁-C₄-alkoxy, such as methoxy, ethoxy, isopropyloxy or 2-butyloxy, C₁-C₄-haloalkoxy, such as trifluoromethoxy, C₃-C₆-cycloalkyl, such as cyclopropyl, cyclobutyl or cyclopentyl, C₃-C₆-cycloalkoxy, such as cyclopropyloxy, cyclobutyloxy or cyclopentyloxy, (C₃-C₆-cycloalkyl)methoxy, such as cyclopropylmethoxy and CN;
even more particularly selected from halogen, C₁-C₄-alkyl, C₂-C₄-alkynyl, C₁-C₄-haloalkyl, C₁-C₄-alkoxy, C₁-C₄-haloalkoxy and CN;
even more particularly selected from halogen, C₁-C₄-haloalkyl, C₁-C₄-alkoxy, C₁-C₄-haloalkoxy and CN;
especially selected from the group consisting of F, Cl and CN.

Further particular groups (b.2) of embodiments relate to the diaminotriazine compounds of formula (I), wherein
- A: is 3-pyridiyl, which is unsubstituted or in particular substituted by 1, 2, 3 or 4 radicals R^{A}, in particular by 2, 3 or 4 radicals R^{A}, especially by 3 or 4 radicals R^{A}, where R^{A} is as defined above; and where
- R^{A}: is frequently selected from the group consisting of halogen, CN, amino, NO₂, C₁-C₆-alkyl, C₂-C₆-alkynyl, C₃-C₆-cycloalkyl, C₃-C₆-cycloalkoxy, (C₃-C₆-cycloalkyl)methoxy, C₁-C₆-alkoxy, (C₁-C₆-alkoxy)-C₁-C₆-alkyl, (C₁-C₆-alkoxy)-C₁-C₆-alkoxy, (C₁-C₆-alkoxy)-C₂-C₆-alkenyl, (C₁-C₆-alkoxy)-C₂-C₆-alkynyl, C₁-C₆-alkylthio, (C₁-C₆-alkyl)sulfinyl, (C₁-C₆-alkyl)sulfonyl, (C₁-C₆-alkyl)amino, di(C₁-C₆-alkyl)amino, (C₁-C₆-alkyl)carbonyl and (C₁-C₆-alkoxy)carbonyl, where the aliphatic and cycloaliphatic parts of the 17 aforementioned radicals are unsubstituted, partly or completely halogenated;
preferably selected from the group consisting of halogen, CN, C₁-C₆-alkyl, C₁-C₆-haloalkyl, C₁-C₆-alkoxy, C₁-C₆-haloalkoxy, C₃-C₆-cycloalkyl, C₃-C₆-cycloalkoxy, (C₃-C₆-cycloalkyl)methoxy, C₂-C₆-alkynyl, C₂-C₆-alkenyl, C₂-C₆-alkynyloxy and C₂-C₆-alkenyloxy; more particularly selected from halogen, such as F or Cl, C₁-C₄-alkyl, such as methyl, C₂-C₄-alkenyl such as ethenyl (= vinyl), C₂-C₄-alkynyl such as ethynyl, C₂-C₄-alkenyloxy, such as allyloxy, C₂-C₄-alkynyloxy, such as propargyloxy, C₁-C₄-haloalkyl, such as trifluoromethyl, C₁-C₄-alkoxy, such as methoxy, ethoxy, isopropyloxy or 2-butyloxy, C₁-C₄-haloalkoxy, such as trifluoromethoxy, C₃-C₆-cycloalkyl, such as cyclopropyl, cyclobutyl or cyclopentyl, C₃-C₆-cycloalkoxy, such as cyclopropyloxy, cyclobutyloxy or cyclopentyloxy, (C₃-C₆-cycloalkyl)methoxy, such as cyclopropylmethoxy and CN;
even more particularly selected from halogen, C₁-C₄-alkyl, C₂-C₄-alkynyl, C₁-C₄-haloalkyl, C₁-C₄-alkoxy, C₁-C₄-haloalkoxy and CN;
even more particularly selected from halogen, C₁-C₄-haloalkyl, C₁-C₄-alkoxy, C₁-C₄-haloalkoxy and CN; especially selected from the group consisting of F, Cl and CN.

Further particular groups (b.3) of embodiments relate to the diaminotriazine compounds of formula (I), wherein
- A: is 4-pyridiyl, which is unsubstituted or in particular substituted by 1, 2, 3 or 4 radicals R^{A}, in particular by 2, 3 or 4 radicals R^{A}, especially by 3 or 4 radicals R^{A}, where R^{A} is as defined above; and where
- R^{A}: is frequently selected from the group consisting of halogen, CN, amino, NO₂, C₁-C₆-alkyl, C₂-C₆-alkynyl, C₃-C₆-cycloalkyl, C₃-C₆-cycloalkoxy, (C₃-C₆-cycloalkyl)methoxy, C₁-C₆-alkoxy, (C₁-C₆-alkoxy)-C₁-C₆-alkyl, (C₁-C₆-alkoxy)-C₁-C₆-alkoxy, (C₁-C₆-alkoxy)-C₂-C₆-alkenyl, (C₁-C₆-alkoxy)-C₂-C₆-alkynyl, C₁-C₆-alkylthio, (C₁-C₆-alkyl)sulfinyl, (C₁-C₆-alkyl)sulfonyl, (C₁-C₆-alkyl)amino, di(C₁-C₆-alkyl)amino, (C₁-C₆-alkyl)carbonyl and (C₁-C₆-alkoxy)carbonyl, where the aliphatic and cycloaliphatic parts of the 17 aforementioned radicals are unsubstituted, partly or completely halogenated;
preferably selected from the group consisting of halogen, CN, C₁-C₆-alkyl, C₁-C₆-haloalkyl, C₁-C₆-alkoxy, C₁-C₆-haloalkoxy, C₃-C₆-cycloalkyl, C₃-C₆-cycloalkoxy, (C₃-C₆-cycloalkyl)methoxy, C₂-C₆-alkynyl, C₂-C₆-alkenyl, C₂-C₆-alkynyloxy and C₂-C₆-alkenyloxy; more particularly selected from halogen, such as F or Cl, C₁-C₄-alkyl, such as methyl, C₂-C₄-alkenyl such as ethenyl (= vinyl), C₂-C₄-alkynyl such as ethynyl, C₂-C₄-alkenyloxy, such as allyloxy, C₂-C₄-alkynyloxy, such as propargyloxy, C₁-C₄-haloalkyl, such as trifluoromethyl, C₁-C₄-alkoxy, such as methoxy, ethoxy, isopropyloxy or 2-butyloxy, C₁-C₄-haloalkoxy, such as trifluoromethoxy, C₃-C₆-cycloalkyl, such as cyclopropyl, cyclobutyl or cyclopentyl, C₃-C₆-cycloalkoxy, such as cyclopropyloxy, cyclobutyloxy or cyclopentyloxy, (C₃-C₆-cycloalkyl)methoxy, such as cyclopropylmethoxy and CN;
even more particularly selected from halogen, C₁-C₄-alkyl, C₂-C₄-alkynyl, C₁-C₄-haloalkyl, C₁-C₄-alkoxy, C₁-C₄-haloalkoxy and CN;
even more particularly selected from halogen, C₁-C₄-haloalkyl, C₁-C₄-alkoxy, C₁-C₄-haloalkoxy and CN;
especially selected from the group consisting of F, Cl and CN.

In groups (a), (b) and (c), and likewise in groups (b.1), (b.2) and (b.3) of embodiments, preferably at least one of the radicals R^{A} in particular at least 2 of the radicals R^{A} are selected from halogen, in particular from fluorine or chlorine, while any further radical R^{A} is as defined above and frequently selected from the group consisting of halogen, CN, amino, NO₂, C₁-C₆-alkyl, C₂-C₆-alkynyl, C₃-C₆-cycloalkyl, C₃-C₆-cycloalkoxy, (C₃-C₆-cycloalkyl)methoxy, C₁-C₆-alkoxy, (C₁-C₆-alkoxy)-C₁-C₆-alkyl, (C₁-C₆-alkoxy)-C₁-C₆-alkoxy, (C₁-C₆-alkoxy)-C₂-C₆-alkenyl, (C₁-C₆-alkoxy)-C₂-C₆-alkynyl, C₁-C₆-alkylthio, (C₁-C₆-alkyl)sulfinyl, (C₁-C₆-alkyl)sulfonyl, (C₁-C₆-alkyl)amino, di(C₁-C₆-alkyl)amino, (C₁-C₆-alkyl)carbonyl and (C₁-C₆-alkoxy)carbonyl, where the aliphatic and cycloaliphatic parts of the 17 aforementioned radicals are unsubstituted, partly or completely halogenated;
in particular selected from the group consisting of halogen, CN, C₁-C₆-alkyl, C₁-C₆-haloalkyl, C₁-C₆-alkoxy, C₁-C₆-haloalkoxy, C₃-C₆-cycloalkyl, C₃-C₆-cycloalkoxy, (C₃-C₆-cycloalkyl)methoxy, C₂-C₆-alkynyl, C₂-C₆-alkenyl, C₂-C₆-alkynyloxy and C₂-C₆-alkenyloxy;
more particularly selected from halogen, such as F or Cl, C₁-C₄-alkyl, such as methyl, C₂-C₄-alkenyl such as ethenyl (= vinyl), C₂-C₄-alkynyl such as ethynyl, C₂-C₄-alkenyloxy, such as allyloxy, C₂-C₄-alkynyloxy, such as propargyloxy, C₁-C₄-haloalkyl, such as trifluoromethyl, C₁-C₄-alkoxy, such as methoxy, ethoxy, isopropyloxy or 2-butyloxy, C₁-C₄-haloalkoxy, such as trifluoromethoxy, C₃-C₆-cycloalkyl, such as cyclopropyl, cyclobutyl or cyclopentyl, C₃-C₆-cycloalkoxy, such as cyclopropyloxy, cyclobutyloxy or cyclopentyloxy, (C₃-C₆-cycloalkyl)methoxy, such as cyclopropylmethoxy and CN;
even more particularly selected from halogen, C₁-C₄-alkyl, C₂-C₄-alkynyl, C₁-C₄-haloalkyl, C₁-C₄-alkoxy, C₁-C₄-haloalkoxy and CN;
even more particularly selected from halogen, C₁-C₄-haloalkyl, C₁-C₄-alkoxy, C₁-C₄-haloalkoxy and CN;
even more particularly selected from the group consisting of halogen and CN, and especially selected from the group consisting of F, Cl and CN.

Further particular groups (b.1), (b.2) and (b.3) of embodiments relate to the diaminotriazine compounds of formula (I), wherein A is selected from the radicals of formulae (A.1), (A.2) and (A.3), in particular from (A.1) and (A.3), even more particular (A.3): 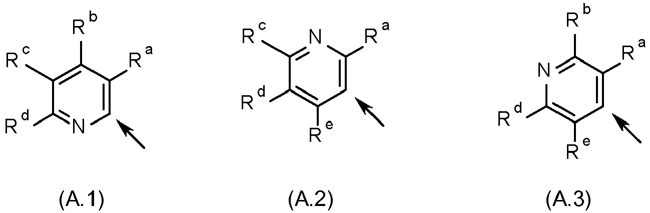 wherein the arrow indicates the atom, which is attached to the nitrogen atom in formula (I), and where
R^{a} and R^{e} independently of one another are as defined for R^{A} and in particular halogen, CN, NO₂, C₁-C₆-alkyl, C₁-C₆-haloalkyl, C₁-C₆-alkoxy, C₁-C₆-haloalkoxy, C₁-C₆-alkylthio, (C₁-C₆-alkyl)sulfinyl, (C₁-C₆-alkyl)sulfonyl, amino, (C₁-C₆-alkyl)amino, di(C₁-C₆-alkyl)amino, (C₁-C₆-alkyl)carbonyl, (C₁-C₆-alkoxy)carbonyl, C₃-C₆-cycloalkoxy, (C₃-C₆-cycloalkyl)methoxy, C₂-C₆-alkynyl, C₂-C₆-alkenyl, C₂-C₆-alkynyloxy and C₂-C₆-alkenyloxy; and
R^{b}, R^{c} and R^{d} independently of one another are hydrogen or as defined for R^{A}, in particular hydrogen, halogen, CN, NO₂, C₁-C₆-alkyl, C₁-C₆-haloalkyl, C₁-C₆-alkoxy, C₁-C₆-alkylthio, (C₁-C₆-alkyl)sulfinyl, (C₁-C₆-alkyl)sulfonyl, amino, (C₁-C₆-alkyl)amino, di(C₁-C₆-alkyl)amino, (C₁-C₆-alkyl)carbonyl, (C₁-C₆-alkoxy)carbonyl, C₃-C₆-cycloalkoxy, (C₃-C₆-cycloalkyl)methoxy, C₂-C₆-alkynyl, C₂-C₆-alkenyl, C₂-C₆-alkynyloxy and C₂-C₆-alkenyloxy;
and preferably where R^{a} and R^{e} independently of one another are halogen, CN, C₁-C₆-alkyl or C₁-C₆-alkoxy; and
R^{b}, R^{c} and R^{d} independently of one another are hydrogen, halogen, CN, NO₂, C₁-C₆-alkyl, C₁-C₆-haloalkyl, C₁-C₆-alkoxy or C₁-C₆-haloalkoxy;
and more preferably where R^{a} and R^{e} independently of one another are halogen or CN; and
R^{b}, R^{c} and R^{d} independently of one another are hydrogen, halogen, CN, C₁-C₆-alkyl, C₁-C₆-haloalkyl, C₁-C₆-alkoxy or C₁-C₆-haloalkoxy;
and especially where R^{a} and R^{e} independently of one another are especially halogen, especially fluorine or chlorine; and
R^{b}, R^{c} and R^{d} independently of one another are hydrogen, halogen, CN, NO₂, C₁-C₆-alkyl, C₁-C₆-haloalkyl, C₁-C₆-alkoxy or C₁-C₆-haloalkoxy, in particular hydrogen, halogen or CN and espcially hydrogen or halogen, such as fluorine or chlorine.

Even more preferred R^{a} and R^{e} are halogen, especially fluorine or chlorine, and R^{b}, R^{c} and R^{d} are hydrogen; or
R^{a}, R^{b}, R^{d} and R^{e} are halogen, especially fluorine or chlorine, and R^{c} is hydrogen, or
R^{a}, R^{b}, R^{c}, R^{d} and R^{e} are halogen, especially fluorine or chlorine.

In very special groups of embodiments (b.1), the radical A is of formula (A.1) wherein R^{a} is halogen, especially fluorine or chlorine, or CN and R^{b}, R^{c} and R^{d} are hydrogen, halogen, especially fluorine or chlorine, or CN, in particular hydrogen or halogen, especially hydrogen, fluorine or chlorine;
and where in particular R^{a}, R^{b} and R^{d} are halogen, especially F or Cl, and R^{c} is H or halogen, especially hydrogen or fluorine.

In yet further very special groups of embodiments (b.1a), the radical A is 4-chloro-3,5,6-trifluoro-2-pyridyl.

In yet further very special groups of embodiments (b.1b), the radical A is 4-chloro-3,6-difluoro-2-pyridyl.

In yet further very special groups of embodiments (b.1c), the radical A is 5-chloro-3,6-difluoro-2-pyridyl.

In yet further very special groups of embodiments (b.1d), the radical A is 3,4,6-trifluoro-2-pyridyl.

In yet further very special groups of embodiments (b.1e), the radical A is 3,5,6-trifluoro-2-pyridyl.

In yet further very special groups of embodiments (b.1f), the radical A is 3,4,5,6-tetrafluoro-2-pyridyl.

In further very special groups of embodiments (b.2), the radical A is of formula (A.2) wherein R^{a} and R^{e} are halogen, such as fluorine or chlorine, or CN and R^{c} and R^{d} are hydrogen, halogen, such as fluorine or chlorine, or CN, in particular hydrogen or halogen, especially hydrogen, fluorine or chlorine;
or where in particular R^{a}, R^{d} and R^{e} are halogen, especially F or Cl, and R^{c} is H or halogen, especially hydrogen or fluorine.

In yet further very special groups of embodiments (b.2a), the radical A is 2,4,5,6-tetrafluoro-3-pyridyl.

In yet further very special groups of embodiments (b.2b), the radical A is 2,4,5-trifluoro-3-pyridyl.

In yet further very special groups of embodiments (b.2c), the radical A is 6-chloro-2,4,5-trifluoro-3-pyridyl.

In yet further very special groups of embodiments (b.2d), the radical A is 2,4,6-trifluoro-3-pyridyl.

In yet further very special groups of embodiments (b.2e), the radical A is 5-chloro-2,4,6-trifluoro-3-pyridyl.

In further very special groups of embodiments (b.3), the radical A is of formula (A.3) wherein R^{a} and R^{e} are halogen or CN and R^{b} and R^{d} are hydrogen, halogen, such as fluorine or chlorine, or CN, in particular hydrogen or halogen, such as fluorine or chlorine; and where R^{a} is in particular halogen, especially F or Cl, R^{b} and R^{d} are hydrogen halogen, especially H, F or Cl, and Re is CN or halogen, especially CN, chlorine or fluorine; and where especially R^{a}, R^{b}, R^{d} and R^{e} are halogen, in particular chlorine or fluorine.

In yet a further very special groups of embodiments (b.3a), the radical A is 2,3,5,6-tetraflouro-4-pyridyl.

Particular groups of embodiments relate to the diaminotriazine compounds of formula (I), wherein;
- R¹: is H, CN, C₁-C₆-alkyl, (C₁-C₆-alkoxy)-C₁-C₆-alkyl, (C₁-C₆-alkyl)carbonyl, (C₁-C₆-alkyl)sulfonyl, C₁-C₆-alkoxy, (C₁-C₆-alkoxy)carbonyl, (C₁-C₆-alkylamino)carbonyl, di(C₁-C₆-alkyl)aminocarbonyl, (C₁-C₆-alkylamino)sulfonyl, di(C₁-C₆-alkyl)aminosulfonyl, where the aliphatic parts of the 10 aforementioned radicals are unsubstituted, partly or completely halogenated,
phenyl, phenylcarbonyl and phenyl-C₁-C₆ alkyl, wherein phenyl in the last 3 mentioned radical is unsubstituted or substituted by 1, 2, 3, 4, or 5 identical or different substituents selected from the group consisting of halogen, CN, NO₂, C₁-C₆-alkyl, C₁-C₆-haloalkyl, C₁-C₆-alkoxy and C₁-C₆-haloalkoxy; preferably, H, CN, C₁-C₆-alkyl, (C₁-C₆-alkoxy)-C₁-C₆-alkyl, (C₁-C₆-alkyl)carbonyl or (C₁-C₆-alkyl)sulfonyl, where the aliphatic parts of the 4 aforementioned radicals unsubstituted
partly or completely halogenated,
phenyl and phenyl-C₁-C₆ alkyl,
wherein phenyl in the last 2 mentioned radical is unsubstituted or substituted by 1, 2, 3, 4, or 5 identical or different substituents selected from the group consisting of halogen, CN, NO₂, C₁-C₆-alkyl, C₁-C₆-haloalkyl, C₁-C₆-alkoxy and C₁-C₆-haloalkoxy;
in particular H, CN, C₁-C₆-alkyl, C₁-C₆-haloalkyl, (C₁-C₆-alkoxy)-C₁-C₆-alkyl, C₁-C₆-alkoxy, (C₁-C₆-alkyl)carbonyl, (C₁-C₆-haloalkyl)carbonyl, (C₁-C₆-alkyl)sulfonyl or (C₁-C₆-haloalkyl)sulfonyl;
more particularly H, CN, C₁-C₄-alkyl, (C₁-C₄-alkoxy)-C₁-C₄-alkyl, C₁-C₄-alkoxy, (C₁-C₄-alkyl)carbonyl or (C₁-C₄-alkyl)sulfonyl;even more particularly H, CN, CH₃, CH₂OCH₃, OCH₃, C(O)CH₃ or SO₂CH₃; especially hydrogen.

Further particular groups of embodiments relate to the diaminotriazine compounds of formula (I), wherein;
- R²: is H, CN, C₁-C₆-alkyl, (C₁-C₆-alkoxy)-C₁-C₆-alkyl, (C₁-C₆-alkyl)carbonyl, (C₁-C₆-alkyl)sulfonyl, (C₁-C₆-alkoxy)carbonyl, (C₁-C₆-alkylamino)carbonyl, di(C₁-C₆-alkyl)aminocarbonyl, (C₁-C₆-alkylamino)sulfonyl, di(C₁-C₆-alkyl)aminosulfonyl, where the aliphatic parts of the 9 aforementioned radicals are unsubstituted, partly or completely halogenated, phenyl, phenylcarbonyl and C₁-C₆ alkylphenyl,
wherein phenyl in the last 3 mentioned radical is unsubstituted or substituted by 1, 2, 3, 4, or 5 identical or different substituents selected from the group consisting of halogen, CN, NO₂, C₁-C₆-alkyl, C₁-C₆-haloalkyl, C₁-C₆-alkoxy and C₁-C₆-haloalkoxy; preferably, H, CN, C₁-C₆-alkyl, (C₁-C₆-alkox_{Y})-C₁-C₆-alkyl, (C₁-C₆-alkyl)carbonyl or (C₁-C₆-alkyl)sulfonyl, where the aliphatic parts of the 4 aforementioned radicals unsubstituted
partly or completely halogenated;
phenyl and phenyl-C₁-C₆ alkyl,
wherein phenyl in the last 2 mentioned radical is unsubstituted or substituted by 1, 2, 3, 4, or 5 identical or different substituents selected from the group consisting of halogen, CN, NO₂, C₁-C₆-alkyl, C₁-C₆-haloalkyl, C₁-C₆-alkoxy and C₁-C₆-haloalkoxy in particular H, CN, C₁-C₆-alkyl, (C₁-C₆-alkoxy)-C₁-C₆-alkyl, (C₁-C₆-alkyl)carbonyl or (C₁-C₆-alkyl)sulfonyl;
more particularly H, CN, C₁-C₄-alkyl, (C₁-C₄-alkoxy)-C₁-C₄-alkyl, (C₁-C₄-alkyl)carbonyl or (C₁-C₄-alkyl)sulfonyl;
even more particularly H, CN, CH₃, CH₂OCH₃, C(O)CH₃ or SO₂CH₃; especially hydrogen.

Further particular groups (1) of embodiments relate to the diaminotriazine compounds of formula (I), wherein
- X: is OR^{3c}, where
- R^{3c}: is in particular selected from the group consisting of C₁-C₆-alkyl, (C₁-C₆-alkoxy)-C₁-C₆-alkyl, C₃-C₆-cycloalkyl, (C₁-C₆-alkyl)-carbonyl, (C₁-C₆-alkoxy)carbonyl, (C₁-C₆-alkyl)sulfonyl, where the aliphatic parts of the 6 aforementioned radicals unsubstituted, partly or completely halogenated;
phenyl, phenylsulfonyl or phenyl-C₁-C₆ alkyl,
wherein phenyl in the last 3 mentioned radicals is unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy
more particularly from the group consisting of C₁-C₄-alkyl, (C₁-C₄-alkoxy)-C₁-C₄-alkyl, C₃-C₄-cycloalkyl, (C₁-C₄-alkyl)-carbonyl, (C₁-C₄-alkoxy)carbonyl, (C₁-C₄-alkyl)sulfonyl, where the aliphatic parts of the 6 aforementioned radicals unsubstituted, partly or completely halogenated;
especially C₁-C₄-alkyl, such as CH₃, CH₃CH₂, CH(CH₃)₂, CH₂CH(CH₃)₂, C(CH₃)₃,, or C₁-C₄-haloalkyl, such as CH₂CF₃, CF(CH₃)₂, CH(CF₃)₂ or CH(CH₃)CF₃.

Further particular groups (2) of embodiments relate to the diaminotriazine compounds of formula (I), wherein
- X: is S(O)ₖR^{3d}, wherein k and R^{3d} are as defined above where
- R^{3d}: is in particular selected from the group consisting of H, C₁-C₆-alkyl, (C₁-C₆-alkoxy)-C₁-C₆-alkyl, C₃-C₆-cycloalkyl, (C₁-C₆-alkyl)-carbonyl, (C₁-C₆-alkoxy)carbonyl, (C₁-C₆-alkyl)sulfonyl, where the aliphatic parts of the 7 aforementioned radicals unsubstituted, partly or completely halogenated;
phenyl, phenylsulfonyl or phenyl-C₁-C₆ alkyl,
wherein phenyl in the last 3 mentioned radicals is unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy
more particularly from the group consisting of C₁-C₄-alkyl, (C₁-C₄-alkoxy)-C₁-C₄-alkyl, C₃-C₄-cycloalkyl, (C₁-C₄-alkyl)-carbonyl, (C₁-C₄-alkoxy)carbonyl, (C₁-C₄-alkyl)sulfonyl, where the aliphatic parts of the 6 aforementioned radicals unsubstituted, partly or completely halogenated
especially C₁-C₄-alkyl, such as CH₃, CH₃CH₂, CH(CH₃)₂, CH₂CH(CH₃)₂, C(CH₃)₃,, or C₁-C₄-haloalkyl, such as CH₂CF₃, CF(CH₃)₂, CH(CF₃)₂ or CH(CH₃)CF₃.

In this particular group (2) of embodiments, where X is S(O)ₖR^{3d} the variable k is preferably 0 or 2 and especially 0.

Further particular groups (3) of embodiments relate to the diaminotriazine compounds of formula (I), wherein
- X is: NR^{3a}R^{3b}, wherein R^{3a} and R^{3b} are as defined above and wherein at least one of R^{3a} and R^{3b} is preferably different from H, and where
- R^{3a} R^{3b}: are independently of one another H, CN, S(O)₂NH₂, C₁-C₆-alkyl, C₂-C₆-alkenyl, C₂-C₆-alkynyl, C₃-C₆-cycloalkyl, C₃-C₆-cycloalkyl-C₁-C₆-alkyl, (C₁-C₆-alkoxy)-C₁-C₆-alkyl, (C₁-C₆-alkyl)-carbonyl, (C₁-C₆-alkoxy)carbonyl, (C₁-C₆-alkyl)sulfonyl, C₁-C₆-alkylamino)carbonyl, di(C₁-C₆-alkyl)aminocarbonyl, (C₁-C₆-alkylamino)sulfonyl, di(C₁-C₆-alkyl)aminosulfonyl and (C₁-C₆-alkoxy)sulfonylwhere the aliphatic parts of the 15 aforementioned radicals are unsubstituted, partly or completely halogenated,
phenyl, phenylsulfonyl or phenyl-C₁-C₆ alkyl,
wherein phenyl in the last 3 mentioned radicals is unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy,
one of R^{3a}, R^{3b} may also be OH, C₁-C₆-alkoxy, C₃-C₆-cycloalkoxy, (C₁-C₆-alkoxy)-C₁-C₆-alkoxy, where the aliphatic and cycloaliphatic parts of the 3 aforementioned radicals are unsubstituted, partly or completely halogenated;
- R^{3a} R^{3b}: are independently of one another more particularly selected from the group consisting of H, C₁-C₄-alkyl, such as CH₃, CH₃CH₂, CH(CH₃)₂, CH₂CH(CH₃)₂, or C(CH₃)₃, C₁-C₄-haloalkyl, such as CH₂CF₃, CF(CH₃)₂, CH(CF₃)₂ or CH(CH₃)CF₃, phenyl and phenyl-C₁-C₄ alkyl, such as benzyl, 1-phenylethyl or 2-phenylethyl, wherein phenyl in phenyl and phenyl-C₁-C₄ alkyl is unsubstituted or substituted by 1, 2, 3, 4 or 5 identical or different substituents selected from the group consisting of halogen, CN, C₁-C₄-alkyl, C₃-C₄-cycloalkyl, C₁-C₄-haloalkyl, C₁-C₄-alkoxy and C₁-C₄-haloalkoxy;
especially from the group consisting of hydrogen, C₁-C₄-alkyl, such as CH₃, CH₃CH₂, CH(CH₃)₂, CH₂CH(CH₃)₂, C(CH₃)₃, phenyl and benzyl.

Further particular groups of (3a) embodiments relate to the diaminotriazine compounds of formula (I), wherein
- X is: NR^{3a}R^{3b}, wherein
- R^{3a}, R^{3b}: together with the nitrogen atom, to which they are bound, form an N bound saturated or unsaturated mono - or bicyclic heterocyclic radical, which may have 1, 2, 3 or 4, in particular 1 or 2, further heteroatoms which are selected from N, O and S, which heterocyclic radical is substituted or unsubstituted by one or more, e. g. 1, 2, 3, 4, 5 ,6, 7 or 8 identical or different substituents selected from the group consisting of halogen, CN, NO₂, C₁-C₆-alkyl, C₁-C₆-haloalkyl, C₁-C₆-alkoxy, (C₁-C₆-alkoxy)-C₁-C₆-alkyl and C₁-C₆-haloalkoxy, in particular selected from the group consisting halogen, CN, NO₂, C₁-C₂-alkyl, C₁-C₂-haloalkyl, (C₁-C₂-alkoxy)-C₁-C₂-alkyl, C₁-C₂-alkoxy and C₁-C₂-haloalkoxy;
R^{3a}, R^{3b} together with the nitrogen atom, to which they are bound, in particular form an N bound saturated monoheterocyclic radical, which may have 1 or 2 further heteroatoms which are selected from N and O, which heterocyclic radical is substituted or
unsubstituted by one or more, e. g. 1, 2, 3, 4, 5 ,6, 7 or 8 identical or different substituents selected from the group consisting of halogen, CN, NO₂, C₁-C₆-alkyl, C₁-C₆-haloalkyl, C₁-C₆-alkoxy, (C₁-C₆-alkoxy)-C₁-C₆-alkyl and C₁-C₆-haloalkoxy, in particular selected from the group consisting halogen, CN, NO₂, C₁-C₂-alkyl, C₁-C₂-haloalkyl, (C₁-C₂-alkoxy)-C₁-C₂-alkyl, C₁-C₂-alkoxy and C₁-C₂-haloalkoxy;
R^{3a}, R^{3b} together with the nitrogen atom, to which they are bound, more particularly form an N bound saturated mono- or bicyclic heterocyclic radical, which is selected from the group consisting of 1-aziridinyl, 1-azetidinyl, 1-piperidinyl, 1-pyrrolidinyl, azepan-1-yl, azocan-1-yl, morpholin-4-yl, isoxazolidine-2-yl, oxazolidine-3-yl, piperazine-1-yl, octahydroisoindol-2-yl, octahydroindol-1-yl, octahydro-2H-quinolin-1-yl, azabicyclo[2.2.1]heptan-3-yl and azabicyclo[2.2.1]heptan-7-yl, where the aforementioned radicals are unsubstituted or substituted by one or more identical or different substituents selected from the group consisting of halogen, CN, NO₂, C₁-C₂-alkyl, C₁-C₂-haloalkyl, (C₁-C₂-alkoxy)-C₁-C₂-alkyl, C₁-C₂-alkoxy and C₁-C₂-haloalkoxy;
R^{3a}, R^{3b} together with the nitrogen atom, to which they are bound, especially form an N-bound saturated monocyclic heterocyclic radical, which is selected from the group consisting of 1-piperidinyl, 1-pyrrolidinyl, azepan-1-yl, azocan-1-yl, morpholin-4-yl, where the aforementioned radicals are unsubstituted or substituted by one or more identical or different substituents selected from the group consisting of halogen, CN, NO₂, C₁-C₂-alkyl, C₁-C₂-haloalkyl, (C₁-C₂-alkoxy)-C₁-C₂-alkyl, C₁-C₂-alkoxy and C₁-C₂-haloalkoxy.

Particularly preferred are the diaminotriazine compounds of formula (I), and likewise the diaminotriazine compounds of formula (I) according to embodiment groups (1), (2), (3) and (3a), wherein
- R¹: is hydrogen; and
- R²: is hydrogen.

Particularly preferred are the diaminotriazine compounds of formula (I), and likewise the diaminotriazine compounds of formula (I) according to embodiment groups (1), (2), (3) and (3a), wherein
- R¹: is hydrogen;
- R²: is hydrogen; and.
- A: is as defined for group (b) of embodiments.

Particularly preferred are the diaminotriazine compounds of formula (I), and likewise the diaminotriazine compounds of formula (I) according to embodiment groups (1), (2), (3) and (3a), wherein
- R¹: is hydrogen;
- R²: is hydrogen; and.
- A: is as defined for group (b.1) of embodiments.

Particularly preferred are the diaminotriazine compounds of formula (I), and likewise the diaminotriazine compounds of formula (I) according to embodiment groups (1), (2), (3) and (3a), wherein
- R¹: is hydrogen;
- R²: is hydrogen; and
- A: is as defined for group (b.2) of embodiments.

Particularly preferred are the diaminotriazine compounds of formula (I), and likewise the diaminotriazine compounds of formula (I) according to embodiment groups (1), (2), (3) and (3a), wherein
- R¹: is hydrogen;
- R²: is hydrogen; and.
- A: is as defined for group (b.3) of embodiments.

More preferred are the diaminotriazine compounds of formula (I), and likewise the diaminotriazine compounds of formula (I) according to embodiment groups (1), (2), (3) and (3a), wherein
- R¹: is hydrogen;
- R²: is hydrogen; and.
- A: is of the formula (A.1), where R^{a}, R^{b}, R^{c} and R^{d} are as defined above and where preferably R^{a} is halogen or CN and R^{b}, R^{c} and R^{d} are hydrogen, halogen or CN, in particular hydrogen or halogen;
and where in particular R^{a}, R^{b} and R^{d} are halogen, especially F or Cl, and R^{c} is H or halogen, especially hydrogen or fluorine.

Also preferred are the diaminotriazine compounds of formula (I), and likewise the diaminotriazine compounds of formula (I) according to embodiment groups (1), (2), (3) and (3a), wherein
- R¹: is hydrogen;
- R²: is hydrogen; and.
- A: is of the formula (A.2), where R^{a}, R^{b}, Re and R^{d} are as defined above and where preferably R^{a} and R^{e} are halogen or CN and R^{c} and R^{d} are hydrogen, halogen or CN, in particular hydrogen or halogen;
and where in particular R^{a}, R^{e} and R^{d} are halogen, especially F or Cl, and R^{c} is H or halogen, especially hydrogen or fluorine.

Even more preferred are the diaminotriazine compounds of formula (I), and likewise the diaminotriazine compounds of formula (I) according to embodiment groups (1), (2), (3) and (3a), wherein
- R¹: is hydrogen;
- R²: is hydrogen; and.
- A: is of the formula (A.3), where R^{a}, R^{b}, R^{d} and Re are as defined above and where preferably R^{a} and Re are halogen or CN and R^{b} and R^{d} are hydrogen, halogen or CN, in particular hydrogen or halogen;
and where in particular R^{a} is halogen, especially F or Cl, R^{b} and R^{d} are hydrogen halogen, especially H, F or Cl, and Re is CN or halogen, especially CN, chlorine or fluorine; and where especially R^{a}, R^{b}, R^{d} and Re are halogen, in particular chlorine or fluorine.

Especially preferred are the diaminotriazine compounds of formula (I), and likewise the diaminotriazine compounds of formula (I) according to embodiment groups (1), (2), (3) and (3a), wherein
- R¹: is hydrogen;
- R²: is hydrogen; and.
- A: is 4-chloro-3,5,6-trifluoro-2-pyridyl.

Especially preferred are the diaminotriazine compounds of formula (I), and likewise the diaminotriazine compounds of formula (I) according to embodiment groups (1), (2), (3) and (3a), wherein
- R¹: is hydrogen;
- R²: is hydrogen; and.
- A: is 2,3,5,6-tetrafluoro-4-pyridyl.

Particular preference is given to azines of formula (I.a), which correspond to diaminotriazines of formula (I) wherein A is (A.1) with R^{b} is F, R^{c} is H, and R¹ and R² are H: 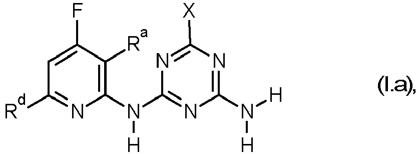 wherein the variables R^{a}, R^{d} and X have the above meanings, in particular the preferred meanings, as defined above. Particularly preferred examples of compounds of formula (I.a) include the compounds I.a.1 to I.a.600, where X, R^{a} and R^{d} are as defined in the rows of the following table A.

**Table A**

| | R^{a} | R^{d} | X |
|---|---|---|---|
| 1 | F | H | NH-CH₃ |
| 2 | F | H | N(CH₃)₂ |
| 3 | F | H | NH-C₂H₅ |
| 4 | F | H | N(CH₃)-C₂H₅ |
| 5 | F | H | N(C₂H₅)₂ |
| 6 | F | H | NH-CH(CH₃)₂ |
| 7 | F | H | NH-C(CH₃)₃ |
| 8 | F | H | NH-OCH₃ |
| 9 | F | H | N(CH₃)-OCH₃ |
| 10 | F | H | NH-OC₂H₅ |
| 11 | F | H | NH-C₆H₅ |
| 12 | F | H | NH-CH₂C₆H₅ |
| 13 | F | H | N(CH₃)C₆H₅ |
| 14 | F | H | N1 |
| 15 | F | H | N2 |
| 16 | F | H | N3 |
| 17 | F | H | N4 |
| 18 | F | H | N5 |
| 19 | F | H | N6 |
| 20 | F | H | N7 |
| 21 | F | H | (*R*/*S*)-N8 |
| 22 | F | H | (*R*)-N8 |
| 23 | F | H | (*S*)-N8 |
| 24 | F | H | *E*-N9 |
| 25 | F | H | *Z*-N9 |
| 26 | F | H | *E-*N10 |
| 27 | F | H | *Z*-N10 |
| 28 | F | H | N11 |
| 29 | F | H | N12 |
| 30 | F | H | N13 |
| 31 | F | H | (*R*/*S*)-N14 |
| 32 | F | H | (*R*)-N14 |
| 33 | F | H | (*S*)-N14 |
| 34 | F | H | O-CH₃ |
| 35 | F | H | O-C₂H₅ |
| 36 | F | H | O-CH₂CF₃ |
| 37 | F | H | O-CH(CH₃)₂ |
| 38 | F | H | O-C(CH₃)₃ |
| 39 | F | H | O-CH(C₂H₅)₂ |
| 40 | F | H | O-CH₂CH(CH₃)₂ |
| 41 | F | H | O-CH(CH₃)(CF₃) |
| 42 | F | H | O-CF(CH₃)₂ |
| 43 | F | H | O-C(CF₃)(CH₃)₂ |
| 44 | F | H | O-CH₂OCH₃ |
| 45 | F | H | O-CH₂CH₂OCH₃ |
| 46 | F | H | O-C₆H₅ |
| 47 | F | H | O-c-C₃H₅ |
| 48 | F | H | O-c-C₅H₉ |
| 49 | F | H | O-c-C₆H₁₁ |
| 50 | F | H | O-O1 |
| 51 | F | H | O-O2 |
| 52 | F | H | O-O3 |
| 53 | F | H | O-CH₂-c-C₃H₅ |
| 54 | F | H | O-CH₂-c-C₅H₉ |
| 55 | F | H | S-CH₃ |
| 56 | F | H | S-C₂H₅ |
| 57 | F | H | S-CH₂CF₃ |
| 58 | F | H | S-CH(CH₃)₂ |
| 59 | F | H | S-C(CH₃)₃ |
| 60 | F | H | S-CH(C₂H₅)₂ |
| 61 | F | H | S-CH₂CH(CH₃)₂ |
| 62 | F | H | S-CH(CH₃)(CF₃) |
| 63 | F | H | S-CF(CH₃)₂ |
| 64 | F | H | S-C(CF₃)(CH₃)₂ |
| 65 | F | H | S-CH₂OCH₃ |
| 66 | F | H | S-CH₂CH₂OCH₃ |
| 67 | F | H | S-C₆H₅ |
| 68 | F | H | S-c-C₃H₅ |
| 69 | F | H | S-c-C₅H₉ |
| 70 | F | H | S-c-C₆H₁₁ |
| 71 | F | H | S-O1 |
| 72 | F | H | S-O2 |
| 73 | F | H | S-O3 |
| 74 | F | H | S-CH₂-c-C₃H₅ |
| 75 | F | H | S-CH₂-c-C₅H₉ |
| 76 | F | F | NH-CH₃ |
| 77 | F | F | N(CH₃)₂ |
| 78 | F | F | NH-C₂H₅ |
| 79 | F | F | N(CH₃)-C₂H₅ |
| 80 | F | F | N(C₂H₅)₂ |
| 81 | F | F | NH-CH(CH₃)₂ |
| 82 | F | F | NH-C(CH₃)₃ |
| 83 | F | F | NH-OCH₃ |
| 84 | F | F | N(CH₃)-OCH₃ |
| 85 | F | F | NH-OC₂H₅ |
| 86 | F | F | NH-C₆H₅ |
| 87 | F | F | NH-CH₂C₆H₅ |
| 88 | F | F | N(CH₃)C₆H₅ |
| 89 | F | F | N1 |
| 90 | F | F | N2 |
| 91 | F | F | N3 |
| 92 | F | F | N4 |
| 93 | F | F | N5 |
| 94 | F | F | N6 |
| 95 | F | F | N7 |
| 96 | F | F | (*R*/*S*)-N8 |
| 97 | F | F | (*R*)-N8 |
| 98 | F | F | (*S*)-N8 |
| 99 | F | F | *E*-N9 |
| 100 | F | F | *Z*-N9 |
| 101 | F | F | *E-*N10 |
| 102 | F | F | *Z*-N10 |
| 103 | F | F | N11 |
| 104 | F | F | N12 |
| 105 | F | F | N13 |
| 106 | F | F | (*R*/*S*)-N14 |
| 107 | F | F | (*R*)-N14 |
| 108 | F | F | (*S*)-N14 |
| 109 | F | F | O-CH₃ |
| 110 | F | F | O-C₂H₅ |
| 111 | F | F | O-CH₂CF₃ |
| 112 | F | F | O-CH(CH₃)₂ |
| 113 | F | F | O-C(CH₃)₃ |
| 114 | F | F | O-CH(C₂H₅)₂ |
| 115 | F | F | O-CH₂CH(CH₃)₂ |
| 116 | F | F | O-CH(CH₃)(CF₃) |
| 117 | F | F | O-CF(CH₃)₂ |
| 118 | F | F | O-C(CF₃)(CH₃)₂ |
| 119 | F | F | O-CH₂OCH₃ |
| 120 | F | F | O-CH₂CH₂OCH₃ |
| 121 | F | F | O-C₆H₅ |
| 122 | F | F | O-c-C₃H₅ |
| 123 | F | F | O-c-C₅H₉ |
| 124 | F | F | O-c-C₆H₁₁ |
| 125 | F | F | O-O1 |
| 126 | F | F | O-O2 |
| 127 | F | F | O-O3 |
| 128 | F | F | O-CH₂-c-C₃H₅ |
| 129 | F | F | O-CH₂-c-C₅H₉ |
| 130 | F | F | S-CH₃ |
| 131 | F | F | S-C₂H₅ |
| 132 | F | F | S-CH₂CF₃ |
| 133 | F | F | S-CH(CH₃)₂ |
| 134 | F | F | S-C(CH₃)₃ |
| 135 | F | F | S-CH(C₂H₅)₂ |
| 136 | F | F | S-CH₂CH(CH₃)₂ |
| 137 | F | F | S-CH(CH₃)(CF₃) |
| 138 | F | F | S-CF(CH₃)₂ |
| 139 | F | F | S-C(CF₃)(CH₃)₂ |
| 140 | F | F | S-CH₂OCH₃ |
| 141 | F | F | S-CH₂CH₂OCH₃ |
| 142 | F | F | S-C₆H₅ |
| 143 | F | F | S-c-C₃H₅ |
| 144 | F | F | S-c-C₅H₉ |
| 145 | F | F | S-c-C₆H₁₁ |
| 146 | F | F | S-O1 |
| 147 | F | F | S-O2 |
| 148 | F | F | S-O3 |
| 149 | F | F | S-CH₂-c-C₃H₅ |
| 150 | F | F | S-CH₂-c-C₅H₉ |
| 151 | F | Cl | NH-CH₃ |
| 152 | F | Cl | N(CH₃)₂ |
| 153 | F | Cl | NH-C₂H₅ |
| 154 | F | Cl | N(CH₃)-C₂H₅ |
| 155 | F | Cl | N(C₂H₅)₂ |
| 156 | F | Cl | NH-CH(CH₃)₂ |
| 157 | F | Cl | NH-C(CH₃)₃ |
| 158 | F | Cl | NH-OCH₃ |
| 159 | F | Cl | N(CH₃)-OCH₃ |
| 160 | F | Cl | NH-OC₂H₅ |
| 161 | F | Cl | NH-C₆H₅ |
| 162 | F | Cl | NH-CH₂C₆H₅ |
| 163 | F | Cl | N(CH₃)C₆H₅ |
| 164 | F | Cl | N1 |
| 165 | F | Cl | N2 |
| 166 | F | Cl | N3 |
| 167 | F | Cl | N4 |
| 168 | F | Cl | N5 |
| 169 | F | Cl | N6 |
| 170 | F | Cl | N7 |
| 171 | F | Cl | (*R*/*S*)-N8 |
| 172 | F | Cl | (*R*)-N8 |
| 173 | F | Cl | (*S*)-N8 |
| 174 | F | Cl | *E*-N9 |
| 175 | F | Cl | *Z*-N9 |
| 176 | F | Cl | *E-*N10 |
| 177 | F | Cl | *Z*-N10 |
| 178 | F | Cl | N11 |
| 179 | F | Cl | N12 |
| 180 | F | Cl | N13 |
| 181 | F | Cl | (*R*/*S*)-N14 |
| 182 | F | Cl | (*R*)-N14 |
| 183 | F | Cl | (*S*)-N14 |
| 184 | F | Cl | O-CH₃ |
| 185 | F | Cl | O-C₂H₅ |
| 186 | F | Cl | O-CH₂CF₃ |
| 187 | F | Cl | O-CH(CH₃)₂ |
| 188 | F | Cl | O-C(CH₃)₃ |
| 189 | F | Cl | O-CH(C₂H₅)₂ |
| 190 | F | Cl | O-CH₂CH(CH₃)₂ |
| 191 | F | Cl | O-CH(CH₃)(CF₃) |
| 192 | F | Cl | O-CF(CH₃)₂ |
| 193 | F | Cl | O-C(CF₃)(CH₃)₂ |
| 194 | F | Cl | O-CH₂OCH₃ |
| 195 | F | Cl | O-CH₂CH₂OCH₃ |
| 196 | F | Cl | O-C₆H₅ |
| 197 | F | Cl | O-c-C₃H₅ |
| 198 | F | Cl | O-c-C₅H₉ |
| 199 | F | Cl | O-c-C₆H₁₁ |
| 200 | F | Cl | O-O1 |
| 201 | F | Cl | O-O2 |
| 202 | F | Cl | O-O3 |
| 203 | F | Cl | O-CH₂-c-C₃H₅ |
| 204 | F | Cl | O-CH₂-c-C₅H₉ |
| 205 | F | Cl | S-CH₃ |
| 206 | F | Cl | S-C₂H₅ |
| 207 | F | Cl | S-CH₂CF₃ |
| 208 | F | Cl | S-CH(CH₃)₂ |
| 209 | F | Cl | S-C(CH₃)₃ |
| 210 | F | Cl | S-CH(C₂H₅)₂ |
| 211 | F | Cl | S-CH₂CH(CH₃)₂ |
| 212 | F | Cl | S-CH(CH₃)(CF₃) |
| 213 | F | Cl | S-CF(CH₃)₂ |
| 214 | F | Cl | S-C(CF₃)(CH₃)₂ |
| 215 | F | Cl | S-CH₂OCH₃ |
| 216 | F | Cl | S-CH₂CH₂OCH₃ |
| 217 | F | Cl | S-C₆H₅ |
| 218 | F | Cl | S-c-C₃H₅ |
| 219 | F | Cl | S-c-C₅H₉ |
| 220 | F | Cl | S-c-C₆H₁₁ |
| 221 | F | Cl | S-O1 |
| 222 | F | Cl | S-O2 |
| 223 | F | Cl | S-O3 |
| 224 | F | Cl | S-CH₂-c-C₃H₅ |
| 225 | F | Cl | S-CH₂-c-C₅H₉ |
| 226 | F | CN | NH-CH₃ |
| 227 | F | CN | N(CH₃)₂ |
| 228 | F | CN | NH-C₂H₅ |
| 229 | F | CN | N(CH₃)-C₂H₅ |
| 230 | F | CN | N(C₂H₅)₂ |
| 231 | F | CN | NH-CH(CH₃)₂ |
| 232 | F | CN | NH-C(CH₃)₃ |
| 233 | F | CN | NH-OCH₃ |
| 234 | F | CN | N(CH₃)-OCH₃ |
| 235 | F | CN | NH-OC₂H₅ |
| 236 | F | CN | NH-C₆H₅ |
| 237 | F | CN | NH-CH₂C₆H₅ |
| 238 | F | CN | N(CH₃)C₆H₅ |
| 239 | F | CN | N1 |
| 240 | F | CN | N2 |
| 241 | F | CN | N3 |
| 242 | F | CN | N4 |
| 243 | F | CN | N5 |
| 244 | F | CN | N6 |
| 245 | F | CN | N7 |
| 246 | F | CN | (*R*/*S*)-N8 |
| 247 | F | CN | (*R*)-N8 |
| 248 | F | CN | (*S*)-N8 |
| 249 | F | CN | *E*-N9 |
| 250 | F | CN | *Z*-N9 |
| 251 | F | CN | *E*-N10 |
| 252 | F | CN | *Z*-N10 |
| 253 | F | CN | N11 |
| 254 | F | CN | N12 |
| 255 | F | CN | N13 |
| 256 | F | CN | (*R*/*S*)-N14 |
| 257 | F | CN | (*R*)-N14 |
| 258 | F | CN | (*S*)-N14 |
| 259 | F | CN | O-CH₃ |
| 260 | F | CN | O-C₂H₅ |
| 261 | F | CN | O-CH₂CF₃ |
| 262 | F | CN | O-CH(CH₃)₂ |
| 263 | F | CN | O-C(CH₃)₃ |
| 264 | F | CN | O-CH(C₂H₅)₂ |
| 265 | F | CN | O-CH₂CH(CH₃)₂ |
| 266 | F | CN | O-CH(CH₃)(CF₃) |
| 267 | F | CN | O-CF(CH₃)₂ |
| 268 | F | CN | O-C(CF₃)(CH₃)₂ |
| 269 | F | CN | O-CH₂OCH₃ |
| 270 | F | CN | O-CH₂CH₂OCH₃ |
| 271 | F | CN | O-C₆H₅ |
| 272 | F | CN | O-c-C₃H₅ |
| 273 | F | CN | O-C-C₅H₉ |
| 274 | F | CN | O-c-C₆H₁₁ |
| 275 | F | CN | O-O1 |
| 276 | F | CN | O-O2 |
| 277 | F | CN | O-O3 |
| 278 | F | CN | O-CH₂-c-C₃H₅ |
| 279 | F | CN | O-CH₂-c-C₅H₉ |
| 280 | F | CN | S-CH₃ |
| 281 | F | CN | S-C₂H₅ |
| 282 | F | CN | S-CH₂CF₃ |
| 283 | F | CN | S-CH(CH₃)₂ |
| 284 | F | CN | S-C(CH₃)₃ |
| 285 | F | CN | S-CH(C₂H₅)₂ |
| 286 | F | CN | S-CH₂CH(CH₃)₂ |
| 287 | F | CN | S-CH(CH₃)(CF₃) |
| 288 | F | CN | S-CF(CH₃)₂ |
| 289 | F | CN | S-C(CF₃)(CH₃)₂ |
| 290 | F | CN | S-CH₂OCH₃ |
| 291 | F | CN | S-CH₂CH₂OCH₃ |
| 292 | F | CN | S-C₆H₅ |
| 293 | F | CN | S-c-C₃H₅ |
| 294 | F | CN | S-c-C₅H₉ |
| 295 | F | CN | S-c-C₆H₁₁ |
| 296 | F | CN | S-O1 |
| 297 | F | CN | S-O2 |
| 298 | F | CN | S-O3 |
| 299 | F | CN | S-CH₂-c-C₃H₅ |
| 300 | F | CN | S-CH₂-c-C₅H₉ |
| 301 | Cl | H | NH-CH₃ |
| 302 | Cl | H | N(CH₃)₂ |
| 303 | Cl | H | NH-C₂H₅ |
| 304 | Cl | H | N(CH₃)-C₂H₅ |
| 305 | Cl | H | N(C₂H₅)₂ |
| 306 | Cl | H | NH-CH(CH₃)₂ |
| 307 | Cl | H | NH-C(CH₃)₃ |
| 308 | Cl | H | NH-OCH₃ |
| 309 | Cl | H | N(CH₃)-OCH₃ |
| 310 | Cl | H | NH-OC₂H₅ |
| 311 | Cl | H | NH-C₆H₅ |
| 312 | Cl | H | NH-CH₂C₆H₅ |
| 313 | Cl | H | N(CH₃)C₆H₅ |
| 314 | Cl | H | N1 |
| 315 | Cl | H | N2 |
| 316 | Cl | H | N3 |
| 317 | Cl | H | N4 |
| 318 | Cl | H | N5 |
| 319 | Cl | H | N6 |
| 320 | Cl | H | N7 |
| 321 | Cl | H | (*R*/*S*)-N8 |
| 322 | Cl | H | (*R*)-N8 |
| 323 | Cl | H | (*S*)-N8 |
| 324 | Cl | H | *E*-N9 |
| 325 | Cl | H | *Z*-N9 |
| 326 | Cl | H | *E*-N10 |
| 327 | Cl | H | *Z*-N10 |
| 328 | Cl | H | N11 |
| 329 | Cl | H | N12 |
| 330 | Cl | H | N13 |
| 331 | Cl | H | (*R*/*S*)-N14 |
| 332 | Cl | H | (*R*)-N14 |
| 333 | Cl | H | (*S*)-N14 |
| 334 | Cl | H | O-CH₃ |
| 335 | Cl | H | O-C₂H₅ |
| 336 | Cl | H | O-CH₂CF₃ |
| 337 | Cl | H | O-CH(CH₃)₂ |
| 338 | Cl | H | O-C(CH₃)₃ |
| 339 | Cl | H | O-CH(C₂H₅)₂ |
| 340 | Cl | H | O-CH₂CH(CH₃)₂ |
| 341 | Cl | H | O-CH(CH₃)(CF₃) |
| 342 | Cl | H | O-CF(CH₃)₂ |
| 343 | Cl | H | O-C(CF₃)(CH₃)₂ |
| 344 | Cl | H | O-CH₂OCH₃ |
| 345 | Cl | H | O-CH₂CH₂OCH₃ |
| 346 | Cl | H | O-C₆H₅ |
| 347 | Cl | H | O-C-C₃H₅ |
| 348 | Cl | H | O-c-C₅H₉ |
| 349 | Cl | H | O-c-C₆H₁₁ |
| 350 | Cl | H | O-O1 |
| 351 | Cl | H | O-O2 |
| 352 | Cl | H | O-O3 |
| 353 | Cl | H | O-CH₂-c-C₃H₅ |
| 354 | Cl | H | O-CH₂-c-C₅H₉ |
| 355 | Cl | H | S-CH₃ |
| 356 | Cl | H | S-C₂H₅ |
| 357 | Cl | H | S-CH₂CF₃ |
| 358 | Cl | H | S-CH(CH₃)₂ |
| 359 | Cl | H | S-C(CH₃)₃ |
| 360 | Cl | H | S-CH(C₂H₅)₂ |
| 361 | Cl | H | S-CH₂CH(CH₃)₂ |
| 362 | Cl | H | S-CH(CH₃)(CF₃) |
| 363 | Cl | H | S-CF(CH₃)₂ |
| 364 | Cl | H | S-C(CF₃)(CH₃)₂ |
| 365 | Cl | H | S-CH₂OCH₃ |
| 366 | Cl | H | S-CH₂CH₂OCH₃ |
| 367 | Cl | H | S-C₆H₅ |
| 368 | Cl | H | S-c-C₃H₅ |
| 369 | Cl | H | S-c-C₅H₉ |
| 370 | Cl | H | S-c-C₆H₁₁ |
| 371 | Cl | H | S-O1 |
| 372 | Cl | H | S-O2 |
| 373 | Cl | H | S-O3 |
| 374 | Cl | H | S-CH₂-c-C₃H₅ |
| 375 | Cl | H | S-CH₂-c-C₅H₉ |
| 376 | Cl | F | NH-CH₃ |
| 377 | Cl | F | N(CH₃)₂ |
| 378 | Cl | F | NH-C₂H₅ |
| 379 | Cl | F | N(CH₃)-C₂H₅ |
| 380 | Cl | F | N(C₂H₅)₂ |
| 381 | Cl | F | NH-CH(CH₃)₂ |
| 382 | Cl | F | NH-C(CH₃)₃ |
| 383 | Cl | F | NH-OCH₃ |
| 384 | Cl | F | N(CH₃)-OCH₃ |
| 385 | Cl | F | NH-OC₂H₅ |
| 386 | Cl | F | NH-C₆H₅ |
| 387 | Cl | F | NH-CH₂C₆H₅ |
| 388 | Cl | F | N(CH₃)C₆H₅ |
| 389 | Cl | F | N1 |
| 390 | Cl | F | N2 |
| 391 | Cl | F | N3 |
| 392 | Cl | F | N4 |
| 393 | Cl | F | N5 |
| 394 | Cl | F | N6 |
| 395 | Cl | F | N7 |
| 396 | Cl | F | (*R*/*S*)-N8 |
| 397 | Cl | F | (*R*)-N8 |
| 398 | Cl | F | (*S*)-N8 |
| 399 | Cl | F | *E*-N9 |
| 400 | Cl | F | *Z*-N9 |
| 401 | Cl | F | *E*-N10 |
| 402 | Cl | F | *Z*-N10 |
| 403 | Cl | F | N11 |
| 404 | Cl | F | N12 |
| 405 | Cl | F | N13 |
| 406 | Cl | F | (*R*/*S*)-N14 |
| 407 | Cl | F | (*R*)-N14 |
| 408 | Cl | F | (*S*)-N14 |
| 409 | Cl | F | O-CH₃ |
| 410 | Cl | F | O-C₂H₅ |
| 411 | Cl | F | O-CH₂CF₃ |
| 412 | Cl | F | O-CH(CH₃)₂ |
| 413 | Cl | F | O-C(CH₃)₃ |
| 414 | Cl | F | O-CH(C₂H₅)₂ |
| 415 | Cl | F | O-CH₂CH(CH₃)₂ |
| 416 | Cl | F | O-CH(CH₃)(CF₃) |
| 417 | Cl | F | O-CF(CH₃)₂ |
| 418 | Cl | F | O-C(CF₃)(CH₃)₂ |
| 419 | Cl | F | O-CH₂OCH₃ |
| 420 | Cl | F | O-CH₂CH₂OCH₃ |
| 421 | Cl | F | O-C₆H₅ |
| 422 | Cl | F | O-c-C₃H₅ |
| 423 | Cl | F | O-c-C₅H₉ |
| 424 | Cl | F | O-c-C₆H₁₁ |
| 425 | Cl | F | O-O1 |
| 426 | Cl | F | O-O2 |
| 427 | Cl | F | O-O3 |
| 428 | Cl | F | O-CH₂-c-C₃H₅ |
| 429 | Cl | F | O-CH₂-c-C₅H₉ |
| 430 | Cl | F | S-CH₃ |
| 431 | Cl | F | S-C₂H₅ |
| 432 | Cl | F | S-CH₂CF₃ |
| 433 | Cl | F | S-CH(CH₃)₂ |
| 434 | Cl | F | S-C(CH₃)₃ |
| 435 | Cl | F | S-CH(C₂H₅)₂ |
| 436 | Cl | F | S-CH₂CH(CH₃)₂ |
| 437 | Cl | F | S-CH(CH₃)(CF₃) |
| 438 | Cl | F | S-CF(CH₃)₂ |
| 439 | Cl | F | S-C(CF₃)(CH₃)₂ |
| 440 | Cl | F | S-CH₂OCH₃ |
| 441 | Cl | F | S-CH₂CH₂OCH₃ |
| 442 | Cl | F | S-C₆H₅ |
| 443 | Cl | F | S-c-C₃H₅ |
| 444 | Cl | F | S-c-C₅H₉ |
| 445 | Cl | F | S-c-C₆H₁₁ |
| 446 | Cl | F | S-O1 |
| 447 | Cl | F | S-O2 |
| 448 | Cl | F | S-O3 |
| 449 | Cl | F | S-CH₂-c-C₃H₅ |
| 450 | Cl | F | S-CH₂-c-C₅H₉ |
| 451 | CN | H | NH-CH₃ |
| 452 | CN | H | N(CH₃)₂ |
| 453 | CN | H | NH-C₂H₅ |
| 454 | CN | H | N(CH₃)-C₂H₅ |
| 455 | CN | H | N(C₂H₅)₂ |
| 456 | CN | H | NH-CH(CH₃)₂ |
| 457 | CN | H | NH-C(CH₃)₃ |
| 458 | CN | H | NH-OCH₃ |
| 459 | CN | H | N(CH₃)-OCH₃ |
| 460 | CN | H | NH-OC₂H₅ |
| 461 | CN | H | NH-C₆H₅ |
| 462 | CN | H | NH-CH₂C₆H₅ |
| 463 | CN | H | N(CH₃)C₆H₅ |
| 464 | CN | H | N1 |
| 465 | CN | H | N2 |
| 466 | CN | H | N3 |
| 467 | CN | H | N4 |
| 468 | CN | H | N5 |
| 469 | CN | H | N6 |
| 470 | CN | H | N7 |
| 471 | CN | H | (*R*/*S*)-N8 |
| 472 | CN | H | (R)-N8 |
| 473 | CN | H | (S)-N8 |
| 474 | CN | H | *E*-N9 |
| 475 | CN | H | *Z*-N9 |
| 476 | CN | H | *E*-N10 |
| 477 | CN | H | *Z*-N10 |
| 478 | CN | H | N11 |
| 479 | CN | H | N12 |
| 480 | CN | H | N13 |
| 481 | CN | H | (*R*/*S*)-N14 |
| 482 | CN | H | (*R*)-N14 |
| 483 | CN | H | (*S*)-N14 |
| 484 | CN | H | O-CH₃ |
| 485 | CN | H | O-C₂H₅ |
| 486 | CN | H | O-CH₂CF₃ |
| 487 | CN | H | O-CH(CH₃)₂ |
| 488 | CN | H | O-C(CH₃)₃ |
| 489 | CN | H | O-CH(C₂H₅)₂ |
| 490 | CN | H | O-CH₂CH(CH₃)₂ |
| 491 | CN | H | O-CH(CH₃)(CF₃) |
| 492 | CN | H | O-CF(CH₃)₂ |
| 493 | CN | H | O-C(CF₃)(CH₃)₂ |
| 494 | CN | H | O-CH₂OCH₃ |
| 495 | CN | H | O-CH₂CH₂OCH₃ |
| 496 | CN | H | O-C₆H₅ |
| 497 | CN | H | O-c-C₃H₅ |
| 498 | CN | H | O-c-C₅H₉ |
| 499 | CN | H | O-c-C₆H₁₁ |
| 500 | CN | H | O-O1 |
| 501 | CN | H | O-O2 |
| 502 | CN | H | O-O3 |
| 503 | CN | H | O-CH₂-c-C₃H₅ |
| 504 | CN | H | O-CH₂-c-C₅H₉ |
| 505 | CN | H | S-CH₃ |
| 506 | CN | H | S-C₂H₅ |
| 507 | CN | H | S-CH₂CF₃ |
| 508 | CN | H | S-CH(CH₃)₂ |
| 509 | CN | H | S-C(CH₃)₃ |
| 510 | CN | H | S-CH(C₂H₅)₂ |
| 511 | CN | H | S-CH₂CH(CH₃)₂ |
| 512 | CN | H | S-CH(CH₃)(CF₃) |
| 513 | CN | H | S-CF(CH₃)₂ |
| 514 | CN | H | S-C(CF₃)(CH₃)₂ |
| 515 | CN | H | S-CH₂OCH₃ |
| 516 | CN | H | S-CH₂CH₂OCH₃ |
| 517 | CN | H | S-C₆H₅ |
| 518 | CN | H | S-c-C₃H₅ |
| 519 | CN | H | S-c-C₅H₉ |
| 520 | CN | H | S-c-C₆H₁₁ |
| 521 | CN | H | S-O1 |
| 522 | CN | H | S-O2 |
| 523 | CN | H | S-O3 |
| 524 | CN | H | S-CH₂-c-C₃H₅ |
| 525 | CN | H | S-CH₂-c-C₅H₉ |
| 526 | CN | F | NH-CH₃ |
| 527 | CN | F | N(CH₃)₂ |
| 528 | CN | F | NH-C₂H₅ |
| 529 | CN | F | N(CH₃)-C₂H₅ |
| 530 | CN | F | N(C₂H₅)₂ |
| 531 | CN | F | NH-CH(CH₃)₂ |
| 532 | CN | F | NH-C(CH₃)₃ |
| 533 | CN | F | NH-OCH₃ |
| 534 | CN | F | N(CH₃)-OCH₃ |
| 535 | CN | F | NH-OC₂H₅ |
| 536 | CN | F | NH-C₆H₅ |
| 537 | CN | F | NH-CH₂C₆H₅ |
| 538 | CN | F | N(CH₃)C₆H₅ |
| 539 | CN | F | N1 |
| 540 | CN | F | N2 |
| 541 | CN | F | N3 |
| 542 | CN | F | N4 |
| 543 | CN | F | N5 |
| 544 | CN | F | N6 |
| 545 | CN | F | N7 |
| 546 | CN | F | (*R*/*S*)-N8 |
| 547 | CN | F | (*R*)-N8 |
| 548 | CN | F | (*S*)-N8 |
| 549 | CN | F | *E*-N9 |
| 550 | CN | F | *Z*-N9 |
| 551 | CN | F | *E*-N10 |
| 552 | CN | F | *Z*-N10 |
| 553 | CN | F | N11 |
| 554 | CN | F | N12 |
| 555 | CN | F | N13 |
| 556 | CN | F | (*R*/*S*)-N14 |
| 557 | CN | F | (*R*)-N14 |
| 558 | CN | F | (*S*)-N14 |
| 559 | CN | F | O-CH₃ |
| 560 | CN | F | O-C₂H₅ |
| 561 | CN | F | O-CH₂CF₃ |
| 562 | CN | F | O-CH(CH₃)₂ |
| 563 | CN | F | O-C(CH₃)₃ |
| 564 | CN | F | O-CH(C₂H₅)₂ |
| 565 | CN | F | O-CH₂CH(CH₃)₂ |
| 566 | CN | F | O-CH(CH₃)(CF₃) |
| 567 | CN | F | O-CF(CH₃)₂ |
| 568 | CN | F | O-C(CF₃)(CH₃)₂ |
| 569 | CN | F | O-CH₂OCH₃ |
| 570 | CN | F | O-CH₂CH₂OCH₃ |
| 571 | CN | F | O-C₆H₅ |
| 572 | CN | F | O-c-C₃H₅ |
| 573 | CN | F | O-c-C₅H₉ |
| 574 | CN | F | O-c-C₆H₁₁ |
| 575 | CN | F | O-O1 |
| 576 | CN | F | O-O2 |
| 577 | CN | F | O-O3 |
| 578 | CN | F | O-CH₂-c-C₃H₅ |
| 579 | CN | F | O-CH₂-c-C₅H₉ |
| 580 | CN | F | S-CH₃ |
| 581 | CN | F | S-C₂H₅ |
| 582 | CN | F | S-CH₂CF₃ |
| 583 | CN | F | S-CH(CH₃)₂ |
| 584 | CN | F | S-C(CH₃)₃ |
| 585 | CN | F | S-CH(C₂H₅)₂ |
| 586 | CN | F | S-CH₂CH(CH₃)₂ |
| 587 | CN | F | S-CH(CH₃)(CF₃) |
| 588 | CN | F | S-CF(CH₃)₂ |
| 589 | CN | F | S-C(CF₃)(CH₃)₂ |
| 590 | CN | F | S-CH₂OCH₃ |
| 591 | CN | F | S-CH₂CH₂OCH₃ |
| 592 | CN | F | S-C₆H₅ |
| 593 | CN | F | S-c-C₃H₅ |
| 594 | CN | F | S-c-C₅H₉ |
| 595 | CN | F | S-c-C₆H₁₁ |
| 596 | CN | F | S-O1 |
| 597 | CN | F | S-O2 |
| 598 | CN | F | S-O3 |
| 599 | CN | F | S-CH₂-c-C₃H₅ |
| 600 | CN | F | S-CH₂-c-C₅H₉ |

In tables A and B the abbreviations given in the following table A1 are used:

**Table A1**

| | |
|---|---|
| c-C₃H₅ | cyclopropyl |
| c-C₅H₉ | cyclopentyl |
| c-C₆H₁₁ | cyclohexyl |
| C₆H₅ | Phenyl |
| N1 | azetidin-1-yl |
| N2 | aziridin-1-yl |
| N3 | pyrrolidin-1-yl |
| N4 | piperidin-1-yl |
| N5 | azepan-1-yl |
| N6 | azocan-1-yl |
| N7 | azonan-1-yl |
| (*R*/*S*)-N8 | (*R*/*S*)-2-methylpyrrolidin-1-yl |
| (*R*)-N8 | (*R*)-2-methylpyrrolidin-1-yl |
| (*S*)-N8 | (*S*)-2-methylpyrrolidin-1-yl |
| *E*-N9 | *E*-2,5-dimethylpyrrolidin-1-yl |
| *Z*-N9 | *Z*-2,5-dimethylpyrrolidin-1-yl |
| *E*-N10 | *E*-3,5-dimethylpiperidin-1-yl |
| *Z*-N10 | *S*-3,5-dimethylpiperidin-1-yl |
| N11 | morpholin-4-yl |
| N12 | 4-methylpiperazin-1-yl |
| N13 | Isoxazolidin-2-yl |
| (*R*/*S*)-N14 | (2*R*)-2-(methoxymethyl)pyrrolidin-1-yl |
| (*R*)-N14 | (2*S*)-2-(methoxymethyl)pyrrolidin-1-yl |
| (*S*)-N14 | (2*R*/*S*)-2-(methoxymethyl)pyrrolidin-1-yl |
| O1 | oxetan-2-yl |
| O2 | oxetan-3-yl |
| O3 | oxan-4-yl |

Likewise preferred are the diaminotriazines of formula (I.b), which correspond to compounds of formula (I), where R¹ and R² are H and A is A.1, wherein R^{b} is Cl and R^{c} is H: 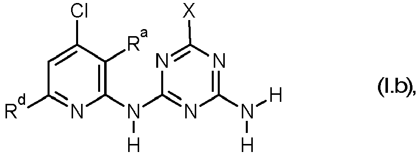 wherein the variables R^{a}, R^{d} and X have the above meanings, in particular the preferred meanings, as defined above. Particularly preferred examples of compounds of formula (I.b) include the compounds I.b.1 to I.b.600, where X, R^{a} and R^{d} are as defined in the rows of table A above and in particular as defined in rows 76 to 150.

Likewise preferred are the diaminotriazines of formula (I.c), which correspond to compounds of formula (I), where R¹ and R² are H and A is A.1, wherein R^{b} is Br and R^{c} is H: 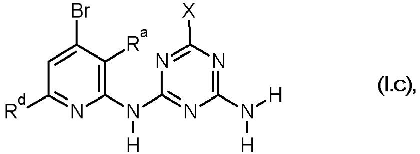 wherein the variables R^{a}, R^{d} and X have the above meanings, in particular the preferred meanings, as defined above. Particularly preferred examples of compounds of formula (I.c) include the compounds I.c.1 to I.c.600, where X, R^{a} and R^{d} are as defined in the rows of table A above and in particular as defined in rows 76 to 150.

Likewise preferred are the diaminotriazines of formula (I.d), which correspond to compounds of formula (I), where R¹ and R² are H and A is A.1, wherein R^{b} is F and R^{c} is F: 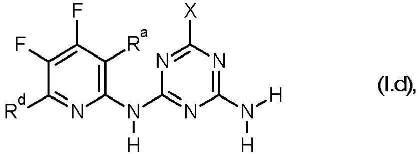 wherein the variables R^{a}, R^{d} and X have the above meanings, in particular the preferred meanings, as defined above. Particularly preferred examples of compounds of formula (I.d) include the compounds I.d.1 to I.d.600, where X, R^{a} and R^{d} are as defined in the rows of table A above. in particular as defined in rows 76 to 150.

Likewise preferred are the diaminotriazines of formula (I.e), which correspond to compounds of formula (I), where R¹ and R² are H and A is A.1, wherein R^{b} is Cl and R^{c} is F: 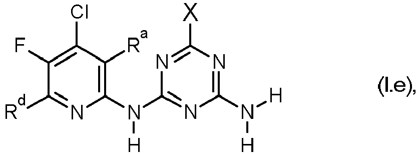 wherein the variables R^{a}, R^{d} and X have the above meanings, in particular the preferred meanings, as defined above. Particularly preferred examples of compounds of formula (I.e) include the compounds I.e.1 to I.e.600, where X, R^{a} and R^{d} are as defined in the rows of table A above and in particular as defined in rows 76 to 150.

Likewise preferred are the diaminotriazines of formula (I.f), which correspond to compounds of formula (I), where R¹ and R² are H and A is A.1, wherein R^{b} is Br and R^{c} is Cl: 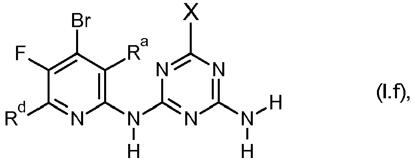 wherein the variables R^{a}, R^{d} and X have the above meanings, in particular the preferred meanings, as defined above. Particularly preferred examples of compounds of formula (I.f) include the compounds I.f.1 to I.f.600, where X, R^{a} and R^{d} are as defined in the rows of table A above and in particular as defined in rows 76 to 150.

Likewise preferred are the diaminotriazines of formula (I.g), which correspond to compounds of formula (I), where R¹ and R² are H and A is A.2, wherein R^{c} is H and Re is F: 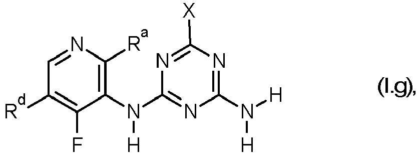 wherein the variables R^{a}, R^{d} and X have the above meanings, in particular the preferred meanings, as defined above. Particularly preferred examples of compounds of formula (I.g) include the compounds I.g.1 to I.g.600, where X, R^{a} and R^{d} are as defined in the rows of table A above and in particular as defined in rows 76 to 150.

Likewise preferred are the diaminotriazines of formula (I.h), which correspond to compounds of formula (I), where R¹ and R² are H and A is A.2, wherein R^{c} is F and Re is F: 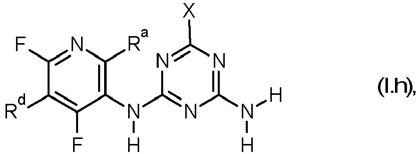 wherein the variables R^{a}, R^{d} and X have the above meanings, in particular the preferred meanings, as defined above. Particularly preferred examples of compounds of formula (I.h) include the compounds I.h.1 to I.h.600, where X, R^{a} and R^{d} are as defined in the rows of table A above and in particular as defined in rows 76 to 150.

Likewise preferred are the diaminotriazines of formula (I.i), which correspond to compounds of formula (I), where R¹ and R² are H and A is A.3, wherein R^{b} is H and Re is F: 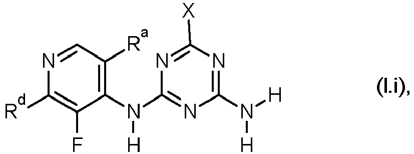 wherein the variables R^{a}, R^{d} and X have the above meanings, in particular the preferred meanings, as defined above. Particularly preferred examples of compounds of formula (I.i) include the compounds I.i.1 to I.i.600, where X, R^{a} and R^{d} are as defined in the rows of table A above and in particular as defined in rows 76 to 150.

Likewise preferred are the diaminotriazines of formula (I.k), which correspond to compounds of formula (I), where R¹ and R² are H and A is A.3, wherein R^{b} is F and Re is F: 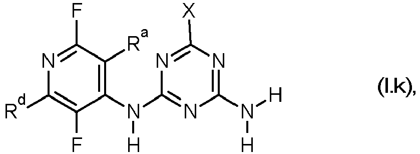 wherein the variables R^{a}, R^{d} and X have the above meanings, in particular the preferred meanings, as defined above. Particularly preferred examples of compounds of formula (I.k) include the compounds I.k.1 to I.k.600, where X, R^{a} and R^{d} are as defined in the rows of table A above and in particular as defined in rows 76 to 150.

Likewise preferred are the diaminotriazines of formula (I.l), which correspond to compounds of formula (I), where R¹ and R² are H and A is A.3, wherein R^{b} is F and Re is Cl: 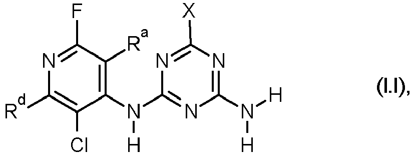 wherein the variables R^{a}, R^{d} and X have the above meanings, in particular the preferred meanings, as defined above. Particularly preferred examples of compounds of formula (I.l) include the compounds I.l.1 to I.l.600, where X, R^{a} and R^{d} are as defined in the rows of table A above and in particular as defined in rows 76 to 150.

Especially preferred compound is 6-pyrrolidin-1-yl-N4-(2,3,5,6-tetrafluoro-4-pyridyl)-1,3,5-triazine-2,4-diamine.

The diaminotriazine compounds of formula (I) according to the invention can be prepared by standard processes of organic chemistry, for example by the following processes:

### Process A)

The diaminotriazine compounds of formula (I), wherein R² is as defined above and in particular H, C₁-C₆-alkyl or (C₁-C₆-alkoxy)-C₁-C₆-alkyl, can be prepared by reacting halotriazines of formula (II) with amines of formula (III) in the presence of a base and a catalyst as depicted in the following scheme 1:

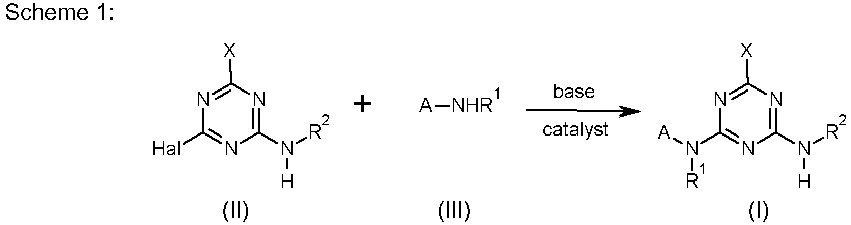

In scheme 1, the variables R¹, R², A and X have the above meanings while Hal is halogen, in particular bromine or chlorine and especially chlorine.

Compounds of formula (II) are novel and valuable intermediates in the production of the diaminotriazine compounds of formula (I) and therefore form part of the present invention.

Particular embodiments of the halotriazines of formual (II) relate to compounds, where the variables Hal, R¹, R² and X have in particular the following meanings:
Hal preferably Cl or Br, especially Cl;
R² is as defined above and in particular H, C₁-C₆-alkyl, (C₁-C₆-alkoxy)-C₁-C₆-alkyl; more particularly H, C₁-C₄-alkoxy-C₁-C₄-alkyl, such as CH₂OCH₃; especially hydrogen;
X is as defined above and in particular as defined in embodiments (1), (2), (3) or (3a).

Particular embodiments relate to the halotriazines of formula (II.a), which correspond to the halotriazines of formula (II) wherein R² is hydrogen and Hal is Cl. Further particular embodiments relate to the halotriazines of formula (II.b), which correspond to the halotriazines of formula (II) wherein R² is hydrogen and Hal is Br:

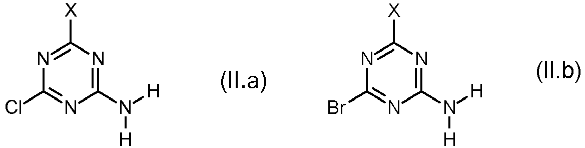

Particular examples of the compounds of formulae (II.a) and (II.b) are those, wherein X is as defined in the following table B.

**Table B:**

| # | X |
|---|---|
| 1 | NH-CH₃ |
| 2 | N(CH₃)₂ |
| 3 | NH-C₂H₅ |
| 4 | N(CH₃)-C₂H₅ |
| 5 | N(C₂H₅)₂ |
| 6 | NH-CH(CH₃)₂ |
| 7 | NH-C(CH₃)₃ |
| 8 | NH-OCH₃ |
| 9 | N(CH₃)-OCH₃ |
| 10 | NH-OC₂H₅ |
| 11 | NH-C₆H₅ |
| 12 | NH-CH₂C₆H₅ |
| 13 | N(CH₃)C₆H₅ |
| 14 | azetidin-1-yl |
| 15 | aziridin-1-yl |
| 16 | pyrrolidin-1-yl |
| 17 | piperidin-1-yl |
| 18 | azepan-1-yl |
| 19 | azocan-1-yl |
| 20 | azonan-1-yl |
| 21 | (*R*/*S*)-2-methylpyrrolidin-1-yl |
| 22 | (*R*)-2-methylpyrrolidin-1-yl |
| 23 | (*S*)-2-methylpyrrolidin-1-yl |
| 24 | *E-*2,5-dimethylpyrrolidin-1-yl |
| 25 | *Z*-2,5-dimethylpyrrolidin-1-yl |
| 26 | *E*-3,5-dimethylpiperidin-1-yl |
| 27 | *S*-3,5-dimethylpiperidin-1-yl |
| 28 | morpholin-4-yl |
| 29 | 4-methylpiperazin-1-yl |
| 30 | Isoxazolidin-2-yl |
| 31 | (2*R*)-2-(methoxymethyl)pyrrolidin-1-yl |
| 32 | (2*S*)-2-(methoxymethyl)pyrrolidin-1-yl |
| 33 | (2*R*/*S*)-2-(methoxymethyl)pyrrolidin-1-yl |
| 34 | NH-C₆H₅ |
| 35 | NH-CH₂C₆H₅ |
| 36 | N(CH₃)C₆H₅ |
| 37 | O-CH₃ |
| 38 | O-C₂H₅ |
| 39 | O-CH₂CF₃ |
| 40 | O-CH(CH₃)₂ |
| 41 | O-C(CH₃)₃ |
| 42 | O-CH(C₂H₅)₂ |
| 43 | O-CH₂CH(CH₃)₂ |
| 44 | O-CH(CH₃)(CF₃) |
| 45 | O-CF(CH₃)₂ |
| 46 | O-C(CF₃)(CH₃)₂ |
| 47 | O-CH₂OCH₃ |
| 48 | O-CH₂CH₂OCH₃ |
| 49 | O-C₆H₅ |
| 50 | O-c-C₃H₅ |
| 51 | O-c-C₅H₉ |
| 52 | O-c-C₆H₁₁ |
| 53 | O-(oxetan-2-yl) |
| 54 | O-(oxetan-3-yl) |
| 55 | O-(oxan-4-yl) |
| 56 | O-CH₂-c-C₃ |
| 57 | O-CH₂-c-C₅H₉ |
| 58 | S-CH₃ |
| 59 | S-C₂H₅ |
| 60 | S-CH₂CF₃ |
| 61 | S-CH(CH₃)₂ |
| 62 | S-C(CH₃)₃ |
| 63 | S-CH(C₂H₅)₂ |
| 64 | S-CH₂CH(CH₃)₂ |
| 65 | S-CH(CH₃)(CF₃) |
| 66 | S-CF(CH₃)₂ |
| 67 | S-C(CF₃)(CH₃)₂ |
| 68 | S-CH₂OCH₃ |
| 69 | S-CH₂CH₂OCH₃ |
| 70 | S-C₆H₅ |
| 71 | S-C-C₃H₅ |
| 72 | S-c-C₅H₉ |
| 73 | S-c-C₆H₁₁ |
| 74 | S-(oxetan-2-yl) |
| 75 | S-(oxetan-3-yl) |
| 76 | S-(oxan-4-yl) |
| 77 | S-CH₂-c-C₃H₅ |
| 78 | S-CH₂-c-C₅H₉ |

In formula (III)
- R¹: is in particular H, C₁-C₆-alkyl, (C₁-C₆-alkoxy)-C₁-C₆-alkyl;
more particularly H, (C₁-C₄-alkoxy)-C₁-C₄-alkyl, such as CH₂OCH₃;
especially hydrogen; and
- A: is as defined above.

The reaction of the halotriazines of formula (II) with the amines of formula (III) is usually carried out at temperatures in the range from from 50°C to the boiling point of the reaction mixture, preferably from 50°C to 150°C, particularly preferably from 60°C to 100°C, in an inert organic solvent (e.g. P. Dao et al., Tetrahedron 2012, 68, 3856 - 3860).

The reaction can be carried out at atmospheric pressure or under elevated pressure, if appropriate, under an inert gas, continuously or batchwise.

In one embodiment of the process according to the invention, the halotriazines of formula (II) and the amines of formula (III) are used in equimolar amounts.

In another embodiment of the process according to the invention, the amines of formula (III) are used in excess with regard to the halotriazines of formula (II).

Preferably the molar ratio of the amines of formula (III) to the halotriazines of formula (II) is in the range from 2 : 1 to 1 : 1, preferably 1.5 : 1 to 1 : 1, especially preferred 1.2 : 1.

The reaction of the halotriazines of formula (II) with the amines of formula (III) is usually carried out in an organic solvent. Suitable in principle are all solvents which are capable of dissolving the halotriazines of formula (II) and the amines of formula (II) at least partly and preferably fully under reaction conditions.Examples of suitable solvents are aliphatic hydrocarbons such as pentane, hexane, cyclohexane, nitromethane and mixtures of C₅-C₈-alkanes, aromatic hydrocarbons such as benzene, chlorobenzene, toluene, cresols, o-, m- and p-xylene, halogenated hydrocarbons such as dichloromethane, 1,2-dichloroethane, chloroform, carbon tetrachloride and chlorobenzene, ethers such as diethyl ether, diisopropyl ether, tert.-butyl methylether (TBME), dioxane, anisole and tetrahydrofuran (THF), esters such as ethyl acetate and butyl acetate; nitriles such as acetonitrile and propionitrile, as well as dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethylpropylene urea (DMPU), dimethyl sulfoxide (DMSO) and 1-methyl-2 pyrrolidinone (NMP). Preferred solvents are ethers as defined above. The term solvent as used herein also includes mixtures of two or more of the above compounds.

The reaction of the halotriazines of formula (II) with the amines of formula (III) is usually carried out in the presence of a base. Examples of suitable bases include metal-containing bases and nitrogen-containing bases. Examples of suitable metal-containing bases are inorganic compounds such as alkali metal and alkaline earth metal hydroxides, and other metal hydroxides, such as lithium hydroxide, sodium hydroxide, potassium hydroxide, magnesium hydroxide, calcium hydroxide and aluminum hydroxide; alkali metal and alkaline earth metal oxide, and other metal oxides, such as lithium oxide, sodium oxide, potassium oxide, magnesium oxide, calcium oxide and magnesium oxide, iron oxide, silver oxide; alkali metal and alkaline earth metal hydrides such as lithium hydride, sodium hydride, potassium hydride and calcium hydride, alkali metal and alkaline earth metal formates, acetates and other metal salts of carboxylic acids, such as sodium formate, sodium benzoate, lithium acetate, sodium acetate, potassium acetate, magnesium acetate, and calcium acetate; alkali metal and alkaline earth metal carbonates such as lithium carbonate, sodium carbonate, potassium carbonate, magnesium carbonate, and calcium carbonate, as well as alkali metal hydrogen carbonates (bicarbonates) such as lithium hydrogen carbonate, sodium hydrogen carbonate, potassium hydrogen carbonate; alkali metal and alkaline earth metal phosphates such as sodium phosphate, potassium phosphate and calcium phosphate; alkali metal and alkaline earth metal alkoxides such as sodium methoxide, sodium ethoxide, potassium ethoxide, potassium tert-butoxide, potassium tert-pentoxide and dimethoxymagnesium; and furthermore organic bases, such as tertiary amines such as tri-C₁-C₆-alkylamines, for example triethylamine, trimethylamine, N-ethyldiisopropylamine, and N-methylpiperidine, pyridine, substituted pyridines such as collidine, lutidine, N-methylmorpholine and also bicyclic amines such as 1,8-diazabicyclo[5.4.0]undec-7-ene (DBU) or 1,5-diazabicyclo[4.3.0]non-5-ene (DBN). Preferred bases are alkali metal and alkaline earth metal alkoxides as defined above. The term base as used herein also includes mixtures of two or more, preferably two of the above compounds. Particular preference is given to the use of one base. The bases can be used in excess, preferably from 1 to 10, especially preferred from 2 to 4 base equivalents based on the halotriazines of formula (II), and they may also be used as the solvent.

The reaction of the halotriazines of formula (II) with the amines of formula (III) may be carried out in the presence of a catalyst. Examples of suitable catalysts include for example, palladium based catalysts like, for example, Palladium(II)acetate, tetrakis(triphenylphosphine)palladium(0), bis(triphenylphosphine)palladium(II)chloride or (1,1,-bis(diphenylphosphino)ferrocene)-dichloropalladium(II),
and optionally suitable additives such as, for example, phosphines like, for example, P(o-tolyl)₃, triphenylphosphine or BINAP (2,2'-Bis(diphenylphospino)-1,1'-binaphthyl). The amount of catalyst is usually 10 to 20 mol % (0.1 to 0.2 equivalents) based on the halotriazines of formula (II).

The end of the reaction can easily be determined by the skilled worker by means of routine methods.

The reaction mixtures are worked up in a customary manner, for example by mixing with water, separation of the phases and, if appropriate, chromatographic purification of the crude product.

The amines of formula (III) used for the preparation of diaminotriazine compounds of formula (I), wherein R¹ is H, C₁-C₆-alkyl, (C₁-C₆-alkoxy)-C₁-C₆-alkyl or C₁-C₆-alkoxy, are commercially available and/or can be prepared by analogy to known literature.

The halotriazines of formula (II) required for the preparation of diaminotriazine compounds of formula (I), wherein R² is H, C₁-C₆-alkyl, (C₁-C₆-alkoxy)-C₁-C₆-alkyl or C₁-C₆-alkoxy, can be prepared by analogy (e.g. J. K.Chakrabarti et al., Tetrahedron 1975, 31, 1879 - 1882) by reacting thiotriazines of formula (IV) with a halogen, as depicted in scheme 2:

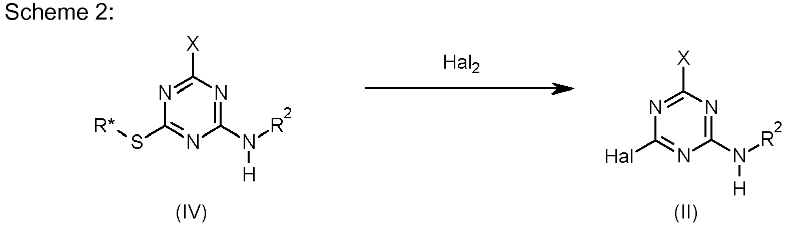

The variable X in formulae (II) and (VI) has the meanings, in particular the preferred meanings, as defined above in context of formula (I).
- R* is: C₁-C₆-alkyl, C₂-C₆-haloalkyl or phenyl;
in particular C₁-C₄-alkyl or C₂-C₄-haloalkyl;
more particularly C₁-C₄-alkyl;
especially CH₃; and

Compounds of formula (IV) are novel and valuable intermediates in the production of the diaminotriazine compounds of formula (I) and therefore also form part of the present invention.

Particular embodiments of the compounds of formual (IV) relate to compounds, where the variables R*, R² and X have in particular the following meanings:
- R* is: C₁-C₆-alkyl, C₂-C₆-haloalkyl or phenyl;
in particular C₁-C₄-alkyl or C₂-C₄-haloalkyl;
more particularly C₁-C₄-alkyl;
especially CH₃; and
- R² is: in particular H, C₁-C₆-alkyl, (C₁-C₆-alkoxy)-C₁-C₆-alkyl;
more particularly H, (C₁-C₄-alkoxy)-C₁-C₄-alkyl, such as CH₂OCH₃;
especially hydrogen;
- X: is as defined above and in particular as defined in embodiments (1), (2), (3) or (3a).

Particular embodiments relate to the compounds of formula (IV.a), which correspond to the compounds of formula (IV) wherein R² is hydrogen and R* is CH₃.

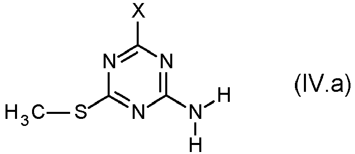

Particular examples of the compounds of formulae (IV.a) are those, wherein X is as defined in the table B above.

The reaction of the thiotriazines of formula (IV) with the halogen is usually carried out from 0°C to the boiling point of the reaction mixture, preferably from 15°C to the boiling point of the reaction mixture, particularly preferably from 15°C to 40°C, in an inert organic solvent (e.g. J. K. Chakrabarti et al., Tetrahedron 1975, 31, 1879 - 1882).

The reaction can be carried out at atmospheric pressure or under elevated pressure, if appropriate under an inert gas, continuously or batchwise.

In the reaction of the thiotriazines of formula (IV) with, halogen is generally used in excess with regard to the thiotriazines of formula (IV).

The reaction of the thiotriazines of formula (IV) with the halogen is usually carried out in an organic solvent.

Suitable in principle are all solvents which are capable of dissolving the thiotriazines of formula (IV) and the halogen at least partly and preferably fully under reaction conditions. Examples of suitable solvents are aliphatic hydrocarbons such as pentane, hexane, cyclohexane and mixtures of C₅-C₈-alkanes, halogenated hydrocarbons such as dichloromethane, 1,2-dichloroethane, chloroform and carbon tetrachloride; ethers such as diethyl ether, diisopropyl ether, tert.-butyl methylether (TBME), dioxane, anisole and tetrahydrofuran (THF), alcohols such as methanol, ethanol, n-propanol, isopropanol, n-butanol and tert.-butanol, as well as organic acids like formic acid, acetic acid, propionic acid, oxalic acid, citric acid, trifluoroacetic acid. Preferred solvents are halogenated hydrocarbons and organic acids as defined above. The term solvent as used herein also includes mixtures of two or more of the above compounds. The end of the reaction can easily be determined by the skilled worker by means of routine methods. The reaction mixtures are worked up in a customary manner, for example by mixing with water, separation of the phases and, if appropriate, chromatographic purification of the crude product.

The halotriazines of formula (II) required for the preparation of diaminotriazine compounds of formula (I), wherein R² is H, C₁-C₆-alkyl, (C₁-C₆-alkoxy)-C₁-C₆-alkyl or C₁-C₆-alkoxy, can also be prepared by reacting 2,4-dichlorotriazines of formula (VII) with a an amine H₂N-R², in particular with ammonia, as depicted in scheme 3:

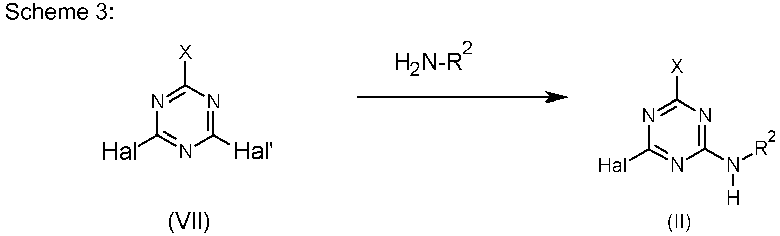

The variable X in formulae (II) and (VII) has the meanings, in particular the preferred meanings, as defined above in context of formula (I).

Hal and Hal' are each, indepentently, halogen, in particular bromine or chlorine, especially chlorine.

The reaction depicted in scheme 3 can be performed by simply mixing the required amounts of the compound of formula (VII) with the amine H₂N-R² or by analogy to the reaction depicted in step 1. Replacing the the amine H₂N-R² by a mercaptan
R*-SH will result in the compound of formula (IV).

Preferably the molar ratio of the amine to the halotriazines of formula (II) is in the range from 10 : 1 to 1 : 1, preferably 5: 1 to 1 : 1.

The reaction depicted in scheme 3 is preferably carried out in an inert solvent. Examples of suitable solvents are nitromethane, aromatic hydrocarbons such as benzene, chlorobenzene, toluene, cresols, o-, m- and p-xylene, halogenated hydrocarbons such as dichloromethane, 1,2-dichloroethane, chloroform, carbon tetrachloride and chlorobenzene, ethers such as diethyl ether, diisopropyl ether, tert.-butyl methylether (TBME), dioxane, anisole and tetrahydrofuran (THF), esters such as ethyl acetate and butyl acetate; nitriles such as acetonitrile and propionitrile, as well as dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethylpropylene urea (DMPU), dimethyl sulfoxide (DMSO) and 1-methyl-2 pyrrolidinone (NMP) and mixtures thereof with with water or with aliphatic hydrocarbons such as pentane, hexane, cyclohexane or with mixtures of C₅-C₈-alkane. Preferred solvents are ethers as defined above and mixtures thereof with water. The term solvent as used herein also includes mixtures of two or more of the above compounds.

The reaction depicted in scheme 1 may be performed in the presence of an auxiliary base. Suitable bases are those mentioned in context with the reaction depicted in scheme 1. However, the amine H₂N-R² may itself serve as an auxiliary base. In this case, usually an excess of the amine H₂N-R² is used.

### Process B)

The diaminotriazine compounds of formula (I), wherein
- R²: is different from hydrogen, e.g. C₁-C₆-alkyl, (C₁-C₆-alkoxy)-C₁-C₆-alkyl, CN, (C₁-C₆-alkyl)carbonyl, (C₁-C₆-alkoxy)carbonyl, (C₁-C₆-alkyl)sulfonyl, phenylsulfonyl, phenyl, phenyl-C₁-C₆ alkyl, phenylcarbonyl or phenoxycarbonyl,
wherein the phenyl is unsubstituted or substituted as defined above for the respective radicals in formula (I);
can be prepared by reacting azines of formula (I), wherein R² is hydrogen with a compound of formula (V) as depicted in scheme 4:

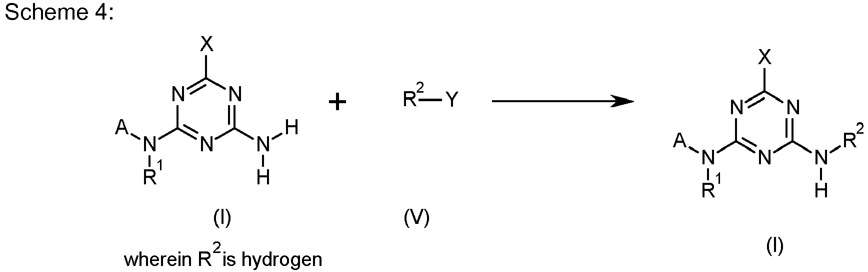

The variables A, R¹ and X have the meanings, in particular the preferred meanings, as in formula (I) mentioned above,
- R²: is different from hydrogen, e.g. C₁-C₆-alkyl, (C₁-C₆-alkoxy)-C₁-C₆-alkyl, CN, (C₁-C₆-alkyl)carbonyl, (C₁-C₆-alkoxy)carbonyl, (C₁-C₆-alkyl)sulfonyl, phenylsulfonyl, phenyl, phenyl-C₁-C₆ alkyl, phenylcarbonyl or phenoxycarbonyl,
wherein the phenyl is unsubstituted or substituted as defined above for the respective radicals in formula (I);
in particular C₁-C₄-alkyl, CN, (C₁-C₆-alkyl)carbonyl, (C₁-C₆-alkoxy)carbonyl or (C₁-C₆-alkyl)sulfonyl;
especially CN, COCH₃, COOCH₃ or SO₂CH₃; and
- Y is: halogen or oxycarbonyl-C₁-C₆-alkyl;
in particular halogen;
especially Cl, Br or I.

### Process C)

The diaminotriazine compoundsof formula (I), wherein
- R¹: is is different from hydrogen, e.g. C₁-C₆-alkyl, (C₁-C₆-alkoxy)-C₁-C₆-alkyl, CN, (C₁-C₆-alkyl)carbonyl, (C₁-C₆-alkoxy)carbonyl, (C₁-C₆-alkyl)sulfonyl, phenylsulfonyl, phenyl, phenyl-C₁-C₆ alkyl, phenylcarbonyl or phenoxycarbonyl
wherein the phenyl is unsubstituted or substituted by one to five substituents selected from the group consisting of halogen, CN, NO₂, C₁-C₆-alkyl, C₁-C₆-haloalkyl and C₁-C₆-alkoxy;
can be prepared by reacting azines of formula (I), wherein R¹ is hydrogen with a compound of formula (VI), as depecited in scheme 5:

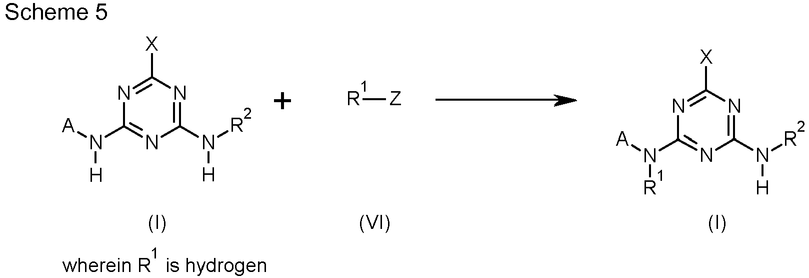

The variables A, R² and X have the meanings, in particular the preferred meanings, as in formula (I) mentioned above,
- R¹: is different from hydrogen, e.g. C₁-C₆-alkyl, (C₁-C₆-alkoxy)-C₁-C₆-alkyl, CN, (C₁-C₆-alkyl)carbonyl, (C₁-C₆-alkoxy)carbonyl, (C₁-C₆-alkyl)sulfonyl, phenylsulfonyl, phenyl, phenyl-C₁-C₆ alkyl, phenylcarbonyl or phenoxycarbonyl,
wherein the phenyl is unsubstituted or substituted as defined above for the respective radicals in formula (I);
in particular C₁-C₄-alkyl, CN, (C₁-C₆-alkyl)carbonyl, (C₁-C₆-alkoxy)carbonyl or (C₁-C₆-alkyl)sulfonyl;
especially CN, COCH₃, COOCH₃ or SO₂CH₃; and
- Z is: halogen or oxycarbonyl-C₁-C₆-alkyl;
in particular halogen;
especially Cl, I or Br.

Both processes B and C independently of one another usually carried out at from 0°C to the boiling point of the reaction mixture, preferably from 23°C to 130°C, particularly preferably from 23°C to 100°C, (e.g. Y. Yuki et al., Polym. J. 1992, 24, 791-799).

Both processes B and C independently of one another can be carried out at atmospheric pressure or under elevated pressure, if appropriate under an inert gas, continuously or batchwise.

In one embodiment of processes B and C according to the invention independently of one another, the diaminotriazine compounds of formula (I), wherein R², or R¹ respectively, is hydrogen are used in excess with regard to the compound of formula (V), or (VI) respectively.

In another embodiment of processes B and C according to the invention independently of one another, the diaminotriazine compounds of formula (I), wherein R², or R¹ respectively, is hydrogen and the compound of formula (V), or (VI) respectively, are used in equimolar amounts.

Preferably the molar ratio of the diaminotriazine compounds of formula (I), wherein R², or R¹ respectively, is hydrogen to the compound of formula (V), or (VI) respectively is in the range from 1 : 1.5 to 1 : 1, preferably 1 : 1.2 to 1 : 1, especially preferred 1 : 1.

Both processes B and C independently of one another are usually carried out in an organic solvent. Suitable in principle are all solvents which are capable of dissolving the diaminotriazine compounds of formula (I), wherein R², or R¹ respectively, is hydrogen and the compound of formula (V), or (VI) respectively, at least partly and preferably fully under reaction conditions. Examples of suitable solvents are halogenated hydrocarbons such as dichloromethane, 1,2-dichloroethane, chloroform, carbon tetrachloride and chlorobenzene; ethers such as diethyl ether, diisopropyl ether, tert.-butyl methylether (TBME), dioxane, anisole and tetrahydrofuran (THF); nitriles such as acetonitrile and propionitrile; alcohols such as methanol, ethanol, n-propanol, isopropanol, n-butanol and tert.-butanol; organic acids like formic acid, acetic acid, propionic acid, oxalic acid, methylbenzenesulfonic acid, benzenesulfonic acid, camphorsulfonic acid, citric acid, trifluoroacetic acid as well as dipolar aprotic solvents such as sulfolane, dimethylsulfoxide, N,N-dimethylformamide (DMF), N,N-dimethylacetamide (DMAC), 1,3-dimethyl-2-imidazolidinone (DMI), N,N'-dimethylpropylene urea (DMPU), dimethyl sulfoxide (DMSO) and 1-methyl-2 pyrrolidinone (NMP). Preferred solvents are halogenated hydrocarbons, ethers and dipolar aprotic solvents as mentioned above. More preferred solvents are dichloromethane or dioxane. It is also possible to use mixtures of the solvents mentioned. The term solvent as used herein also includes mixtures of two or more of the above compounds.

Both processes B and C independently of one another are optionally carried out in the presence of a base. Examples of suitable bases include metal-containing bases and nitrogen-containing bases. Examples of suitable metal-containing bases are inorganic compounds such as alkali metal and alkaline earth metal hydrides such as lithium hydride, sodium hydride, potassium hydride and calcium hydride, alkali metal and alkaline earth metal carbonates such as lithium carbonate, sodium carbonate, potassium carbonate, magnesium carbonate, and calcium carbonate, as well as alkali metal hydrogen carbonates (bicarbonates) such as lithium hydrogen carbonate, sodium hydrogen carbonate, potassium hydrogen carbonate; alkali metal and alkaline earth metal phosphates such as sodium phosphate, potassium phosphate and calcium phosphate; and furthermore organic bases, such as tertiary amines such as tri-C₁-C₆-alkylamines, for example triethylamine, trimethylamine, N-ethyldiisopropylamine, and N-methylpiperidine, pyridine, substituted pyridines such as collidine, lutidine, N-methylmorpholine and 4-dimethylaminopyridine (DMAP), and also bicyclic amines such as 1,8-diazabicyclo[5.4.0]undec-7-ene (DBU) or 1,5-diazabicyclo[4.3.0]non-5-ene (DBN). Preferred bases are organic bases and alkali metal carbonates as mentioned above. Especially preferred bases are organic bases as mentioned above. The term base as used herein also includes mixtures of two or more, preferably two of the above compounds. The bases are generally employed in excess; however they can also be employed in equimolar amounts, or, if appropriate, can be used as solvent. Preferably from 1 to 5 base equivalents, particularly preferred 3 base equivalents of base are used, based on the diaminotriazine compounds of formula (I).

Work-up of the reaction mixture is performed by standard procedures.

The compounds of formula (V), or (VI) respectively, are known compounds. They are commercially available or can be prepared in analogy to known methods.

The compounds of formula (I) have herbicidal activity. Therefore, they can be used for controlling unwanted or undesired plants or vegetation. They can also be used in a method for controlling unwanted or undesired plants or vegetation, which method comprises allowing at least one compound of formula (I) or a salt thereof to act on plants, their environment or on seed. In order to allow the compound of formula (I) or a salt thereof to act on plants, their environment or on seed the compounds of the invention are applied to the plants, their environment or to the seed of said plants.

To widen the spectrum of action and to achieve synergistic effects, the diaminotriazine compounds of formula (I) may be mixed with a large number of representatives of other herbicidal or growth-regulating active ingredient groups and then applied concomitantly.

Suitable components for mixtures are, for example, herbicides from the classes of the acetamides, amides, aryloxyphenoxypropionates, benzamides, benzofuran, benzoic acids, benzothiadiazinones, bipyridylium, carbamates, chloroacetamides, chlorocarboxylic acids, cyclohexanediones, dinitroanilines, dinitrophenol, diphenyl ether, glycines, imidazolinones, isoxazoles, isoxazolidinones, nitriles, N-phenylphthalimides, oxadiazoles, oxazolidinediones, oxyacetamides, phenoxycarboxylic acids, phenylcarbamates, phenylpyrazoles, phenylpyrazolines, phenylpyridazines, phosphinic acids, phosphoroamidates, phosphorodithioates, phthalamates, pyrazoles, pyridazinones, pyridines, pyridinecarboxylic acids, pyridinecarboxamides, pyrimidinediones, pyrimidinyl(thio)benzoates, quinolinecarboxylic acids, semicarbazones, sulfonylaminocarbonyltriazolinones, sulfonylureas, tetrazolinones, thiadiazoles, thiocarbamates, triazines, triazinones, triazoles, triazolinones, triazolocarboxamides, triazolopyrimidines, triketones, uracils, ureas.

The invention also relates to combinations of diaminotriazine compounds of formula (I) with at least one further herbicide B and/or at least one safener C).

The further herbicidal compound B (component B) is in particular selected from the herbicides of class b1) to b15):
b1) lipid biosynthesis inhibitors;
b2) acetolactate synthase inhibitors (ALS inhibitors);
b3) photosynthesis inhibitors;
b4) protoporphyrinogen-IX oxidase inhibitors,
b5) bleacher herbicides;
b6) enolpyruvyl shikimate 3-phosphate synthase inhibitors (EPSP inhibitors);
b7) glutamine synthetase inhibitors;
b8) 7,8-dihydropteroate synthase inhibitors (DHP inhibitors);
b9) mitosis inhibitors;
b10) inhibitors of the synthesis of very long chain fatty acids (VLCFA inhibitors);
b11) cellulose biosynthesis inhibitors;
b12) decoupler herbicides;
b13) auxinic herbicides;
b14) auxin transport inhibitors; and
b15) other herbicides selected from the group consisting of bromobutide, chlorflurenol, chlorflurenol-methyl, cinmethylin, cumyluron, dalapon, dazomet, difenzoquat, difenzoquat-metilsulfate, dimethipin, DSMA, dymron, endothal and its salts, etobenzanid, flamprop, flamprop-isopropyl, flamprop-methyl, flamprop-M-isopropyl, flamprop-M-methyl, flurenol, flurenol-butyl, flurprimidol, fosamine, fosamine-ammonium, indanofan, indaziflam, maleic hydrazide, mefluidide, metam, methiozolin (CAS 403640-27-7), methyl azide, methyl bromide, methyl-dymron, methyl iodide, MSMA, oleic acid, oxaziclomefone, pelargonic acid, pyributicarb, quinoclamine, triaziflam, tridiphane and 6-chloro-3-(2-cyclopropyl-6-methylphenoxy)-4-pyridazinol (CAS 499223-49-3) and its salts and esters;
including their agriculturally acceptable salts or derivatives such as ethers, esters or amides.

In one embodiment of the present invention the compositions according to the present invention comprise at least one diaminotriazine compound of formula (I) and at least one further active compound B (herbicide B).

According to a further embodiment of the invention the compositions contain at least one inhibitor of the lipid biosynthesis (herbicide b1). These are compounds that inhibit lipid biosynthesis. Inhibition of the lipid biosynthesis can be affected either through inhibition of acetylCoA carboxylase (hereinafter termed ACC herbicides) or through a different mode of action (hereinafter termed non-ACC herbicides). The ACC herbicides belong to the group A of the HRAC classification system whereas the non-ACC herbicides belong to the group N of the HRAC classification.

According to a further embodiment of the invention the compositions contain at least one ALS inhibitor (herbicide b2). The herbicidal activity of these compounds is based on the inhibition of acetolactate synthase and thus on the inhibition of the branched chain amino acid biosynthesis. These inhibitors belong to the group B of the HRAC classification system.

According to a further embodiment of the invention the compositions contain at least one inhibitor of photosynthesis (herbicide b3). The herbicidal activity of these compounds is based either on the inhibition of the photosystem II in plants (so-called PSII inhibitors, groups C1, C2 and C3 of HRAC classification) or on diverting the electron transfer in photosystem I in plants (so-called PSI inhibitors, group D of HRAC classification) and thus on an inhibition of photosynthesis. Amongst these, PSII inhibitors are preferred.

According to a further embodiment of the invention the compositions contain at least one inhibitor of protoporphyrinogen-IX-oxidase (herbicide b4). The herbicidal activity of these compounds is based on the inhibition of the protoporphyrinogen-IX-oxidase. These inhibitors belong to the group E of the HRAC classification system.

According to a further embodiment of the invention the compositions contain at least one bleacher-herbicide (herbicide b5). The herbicidal activity of these compounds is based on the inhibition of the carotenoid biosynthesis. These include compounds which inhibit carotenoid biosynthesis by inhibition of phytoene desaturase (so-called PDS inhibitors, group F1 of HRAC classification), compounds that inhibit the 4-hydroxyphenyl-pyruvate-dioxygenase (HPPD inhibitors, group F2 of HRAC classification), compounds that inhibit DOXsynthase (group F4 of HRAC class) and compounds which inhibit carotenoid biosynthesis by an unknown mode of action (bleacher - unknown target, group F3 of HRAC classification).

According to a further embodiment of the invention the compositions contain at least one EPSP synthase inhibitor (herbicide b6). The herbicidal activity of these compounds is based on the inhibition of enolpyruvyl shikimate 3-phosphate synthase, and thus on the inhibition of the amino acid biosynthesis in plants. These inhibitors belong to the group G of the HRAC classification system.

According to a further embodiment of the invention the compositions contain at least one glutamine synthetase inhibitor (herbicide b7). The herbicidal activity of these compounds is based on the inhibition of glutamine synthetase, and thus on the inhibition of the aminoacid biosynthesis in plants. These inhibitors belong to the group H of the HRAC classification system.

According to an further embodiment of the invention the compositions contain at least one DHP synthase inhibitor (herbicide b8). The herbicidal activity of these compounds is based on the inhibition of 7,8-dihydropteroate synthase. These inhibitors belong to the group I of the HRAC classification system.

According to a further embodiment of the invention the compositions contain at least one mitosis inhibitor (herbicide b9). The herbicidal activity of these compounds is based on the disturbance or inhibition of microtubule formation or organization, and thus on the inhibition of mitosis. These inhibitors belong to the groups K1 and K2 of the HRAC classification system. Among these, compounds of the group K1, in particular dinitroanilines, are preferred.

According to a further embodiment of the invention the compositions contain at least one VLCFA inhibitor (herbicide b10). The herbicidal activity of these compounds is based on the inhibition of the synthesis of very long chain fatty acids and thus on the disturbance or inhibition of cell division in plants. These inhibitors belong to the group K3 of the HRAC classification system.

According to an further embodiment of the invention the compositions contain at least one cellulose biosynthesis inhibitor (herbicide b11). The herbicidal activity of these compounds is based on the inhibition of the biosynthesis of cellulose and thus on the inhibition of the synthesis of cell walls in plants. These inhibitors belong to the group L of the HRAC classification system.

According to a further embodiment of the invention the compositions contain at least one decoupler herbicide (herbicide b12). The herbicidal activity of these compounds is based on the disruption of the cell membrane. These inhibitors belong to the group M of the HRAC classification system.

According to a further embodiment of the invention the compositions contain at least one auxinic herbicide (herbicide b13). These include compounds that mimic auxins, i.e. plant hormones, and affect the growth of the plants. These compounds belong to the group O of the HRAC classification system.

According to a further embodiment of the invention the compositions contain at least one auxin transport inhibitor (herbicide b14). The herbicidal activity of these compounds is based on the inhibition of the auxin transport in plants. These compounds belong to the group P of the HRAC classification system.

As to the given mechanisms of action and classification of the active substances, see e.g. "HRAC, Classification of Herbicides According to Mode of Action", http://www.plantprotection.org/hrac/MOA.html).

Preference is given to those compositions according to the present invention comprising at least one herbicide B selected from herbicides of class b1, b6, b9, b10 and b11.

Preference is also given to those compositions according to the present invention comprising at least one herbicide B selected from herbicides of class b2, b3, b4, b5, b6, b9 and b10.

Specific preference is given to those compositions according to the present invention which comprise at least one herbicide B selected from the herbicides of class b4, b6 b9 and b10.

Particular preference is given to those compositions according to the present invention which comprise at least one herbicide B selected from the herbicides of class b4, b6 and b10.

Examples of herbicides B which can be used in combination with the diaminitriazine compounds of formula (I) according to the present invention are:
b1) from the group of the lipid biosynthesis inhibitors:
   ACC-herbicides such as alloxydim, alloxydim-sodium, butroxydim, clethodim, clodinafop, clodinafop-propargyl, cycloxydim, cyhalofop, cyhalofop-butyl, diclofop, diclofop-methyl, fenoxaprop, fenoxaprop-ethyl, fenoxaprop-P, fenoxaprop-P-ethyl, fluazifop, fluazifop-butyl, fluazifop-P, fluazifop-P-butyl, haloxyfop, haloxyfop-methyl, haloxyfop-P, haloxyfop-P-methyl, metamifop, pinoxaden, profoxydim, propaquizafop, quizalofop, quizalofop-ethyl, quizalofop-tefuryl, quizalofop-P, quizalofop-P-ethyl, quizalofop-P-tefuryl, sethoxydim, tepraloxydim, tralkoxydim,
   4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl-[1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); and non ACC herbicides such as benfuresate, butylate, cycloate, dalapon, dimepiperate, EPTC, esprocarb, ethofumesate, flupropanate, molinate, orbencarb, pebulate, prosulfocarb, TCA, thiobencarb, tiocarbazil, triallate and vernolate;
b2) from the group of the ALS inhibitors:
   sulfonylureas such as amidosulfuron, azimsulfuron, bensulfuron, bensulfuron-methyl, chlorimuron, chlorimuron-ethyl, chlorsulfuron, cinosulfuron, cyclosulfamuron, ethametsulfuron, ethametsulfuron-methyl, ethoxysulfuron, flazasulfuron, flucetosulfuron, flupyrsulfuron, flupyrsulfuron-methyl-sodium, foramsulfuron, halosulfuron, halosulfuron-methyl, imazosulfuron, iodosulfuron, iodosulfuron-methyl-sodium, iofensulfuron, iofensulfuron-sodium, mesosulfuron, metazosulfuron, metsulfuron, metsulfuron-methyl, nicosulfuron, orthosulfamuron, oxasulfuron, primisulfuron, primisulfuron-methyl, propyrisulfuron, prosulfuron, pyrazosulfuron, pyrazosulfuron-ethyl, rimsulfuron, sulfometuron, sulfometuron-methyl, sulfosulfuron, thifensulfuron, thifensulfuron-methyl, triasulfuron, tribenuron, tribenuron-methyl, trifloxysulfuron, triflusulfuron, triflusulfuron-methyl and tritosulfuron,
   imidazolinones such as imazamethabenz, imazamethabenz-methyl, imazamox, imazapic, imazapyr, imazaquin and imazethapyr, triazolopyrimidine herbicides and sulfonanilides such as cloransulam, cloransulam-methyl, diclosulam, flumetsulam, florasulam, metosulam, penoxsulam, pyrimisulfan and pyroxsulam,
   pyrimidinylbenzoates such as bispyribac, bispyribac-sodium, pyribenzoxim, pyriftalid, pyriminobac, pyriminobac-methyl, pyrithiobac, pyrithiobac-sodium, 4-[[[2-[(4,6-dimethoxy-2-pyrimidinyl)oxy]phenyl]methyl]amino]-benzoic acid-1-methylethyl ester (CAS 420138-41-6), 4-[[[2-[(4,6-dimethoxy-2-pyrimidinyl)oxy]phenyl]methyl]amino]-benzoic acid propyl ester (CAS 420138-40-5), N-(4-bromophenyl)-2-[(4,6-dimethoxy-2-pyrimidinyl)oxy]benzenemethanamine (CAS 420138-01-8),
   sulfonylaminocarbonyl-triazolinone herbicides such as flucarbazone, flucarbazone-sodium, propoxycarbazone, propoxycarbazone-sodium, thiencarbazone and thiencarbazone-methyl; and triafamone;
   among these, a preferred embodiment of the invention relates to those compositions comprising at least one imidazolinone herbicide;
b3) from the group of the photosynthesis inhibitors:
   amicarbazone, inhibitors of the photosystem II, e.g. triazine herbicides, including of chlorotriazine, triazinones, triazindiones, methylthiotriazines and pyridazinones such as ametryn, atrazine, chloridazone, cyanazine, desmetryn, dimethametryn,hexazinone, metribuzin, prometon, prometryn, propazine, simazine, simetryn, terbumeton, terbuthylazin, terbutryn and trietazin, aryl urea such as chlorobromuron, chlorotoluron, chloroxuron, dimefuron, diuron, fluometuron, isoproturon, isouron, linuron, metamitron, methabenzthiazuron, metobenzuron, metoxuron, monolinuron, neburon, siduron, tebuthiuron and thiadiazuron, phenyl carbamates such as desmedipham, karbutilat, phenmedipham, phenmedipham-ethyl, nitrile herbicides such as bromofenoxim, bromoxynil and its salts and esters, ioxynil and its salts and esters, uraciles such as bromacil, lenacil and terbacil, and bentazon and bentazon-sodium, pyridate, pyridafol, pentanochlor and propanil and inhibitors of the photosystem I such as diquat, diquat-dibromide, paraquat, paraquat-dichloride and paraquat-dimetilsulfate. Among these, a preferred embodiment of the invention relates to those compositions comprising at least one aryl urea herbicide. Among these, likewise a preferred embodiment of the invention relates to those compositions comprising at least one triazine herbicide. Among these, likewise a preferred embodiment of the invention relates to those compositions comprising at least one nitrile herbicide;
b4) from the group of the protoporphyrinogen-IX oxidase inhibitors:
   acifluorfen, acifluorfen-sodium, azafenidin, bencarbazone, benzfendizone, bifenox, butafenacil, carfentrazone, carfentrazone-ethyl, chlomethoxyfen, cinidon-ethyl, fluazolate, flufenpyr, flufenpyr-ethyl, flumiclorac, flumiclorac-pentyl, flumioxazin, fluoroglycofen, fluoroglycofen-ethyl, fluthiacet, fluthiacet-methyl, fomesafen, halosafen, lactofen, oxadiargyl, oxadiazon, oxyfluorfen, pentoxazone, profluazol, pyraclonil, pyraflufen, pyraflufen-ethyl, saflufenacil, sulfentrazone, thidiazimin, tiafenacil, trifludimoxazin (BAS 850 H), ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100; Sumitomo; LS 5296489), N-ethyl-3-(2,6-dichloro-4-trifluoromethylphenoxy)-5-methyl-1H-pyrazole-1-carboxamide (CAS 452098-92-9), N-tetrahydrofurfuryl-3-(2,6-dichloro-4-trifluoromethylphenoxy)-5-methyl-1H-pyrazole-1-carboxamide (CAS 915396-43-9), N-ethyl-3-(2-chloro-6-fluoro-4-trifluoromethylphenoxy)-5-methyl-1H-pyrazole-1-carboxamide (CAS 452099-05-7), N-tetrahydrofurfuryl-3-(2-chloro-6-fluoro-4-trifluoromethylphenoxy)-5-methyl-1H-pyrazole-1-carboxamide (CAS 452100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7) (LS 4061013), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0) (LS 567 0033 = F2-Flumioxazin), 1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0) (LS 568 1323 = Uracil-F2-PPO), methyl (E)-4-[2-chloro-5-[4-chloro-5-(difluoromethoxy)-1H-methyl-pyrazol-3-yl]-4-fluoro-phenoxy]-3-methoxy-but-2-enoate (CAS 948893-00-3) (Isagro, IR6396), and 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4) (FMC Trifluoromethyluracil);
b5) from the group of the bleacher herbicides:
   PDS inhibitors: beflubutamid, diflufenican, fluridone, flurochloridone, flurtamone, norflurazon, picolinafen, and 4-(3-trifluoromethylphenoxy)-2-(4-trifluoromethylphenyl)pyrimidine (CAS 180608-33-7), HPPD inhibitors: benzobicyclon, benzofenap, bicyclopyrone, clomazone, fenquintrione, isoxaflutole, mesotrione, pyrasulfotole, pyrazolynate, pyrazoxyfen, sulcotrione, tefuryltrione, tembotrione, tolpyralate, topramezone, bleacher, unknown target: aclonifen, amitrole and flumeturon;
b6) from the group of the EPSP synthase inhibitors:
   glyphosate, glyphosate-isopropylammonium, glyposate-potassium and glyphosate-trimesium (sulfosate);
b7) from the group of the glutamine synthase inhibitors:
   bilanaphos (bialaphos), bilanaphos-sodium, glufosinate, glufosinate-P and glufosinate-ammonium;
b8) from the group of the DHP synthase inhibitors:
   asulam;
b9) from the group of the mitosis inhibitors:
   compounds of group K1: dinitroanilines such as benfluralin, butralin, dinitramine, ethalfluralin, fluchloralin, oryzalin, pendimethalin, prodiamine and trifluralin, phosphoramidates such as amiprophos, amiprophos-methyl, and butamiphos, benzoic acid herbicides such as chlorthal, chlorthal-dimethyl, pyridines such as dithiopyr and thiazopyr, benzamides such as propyzamide and tebutam; compounds of group K2: carbetamide, chlorpropham, flamprop, flamprop-isopropyl, flamprop-methyl, flamprop-M-isopropyl, flamprop-M-methyl and propham ; among these, compounds of group K1, in particular dinitroanilines are preferred;
b10) from the group of the VLCFA inhibitors:
   chloroacetamides such as acetochlor, alachlor, butachlor, dimethachlor, dimethenamid, dimethenamid-P, metazachlor, metolachlor, metolachlor-S, pethoxamid, pretilachlor, propachlor, propisochlor and thenylchlor, oxyacetanilides such as flufenacet and mefenacet, acetanilides such as diphenamid, naproanilide, napropamide and napropamide-M, tetrazolinones such fentrazamide, and other herbicides such as anilofos, cafenstrole, fenoxasulfone, ipfencarbazone, piperophos, pyroxasulfone and isoxazoline compounds of the formulae II.1, II.2, II.3, II.4, II.5, II.6, II.7, II.8 and II.9 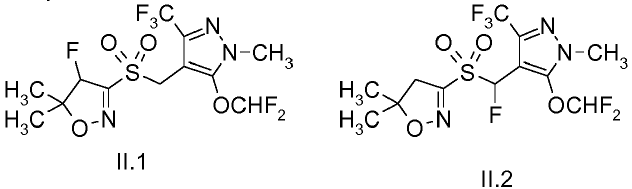 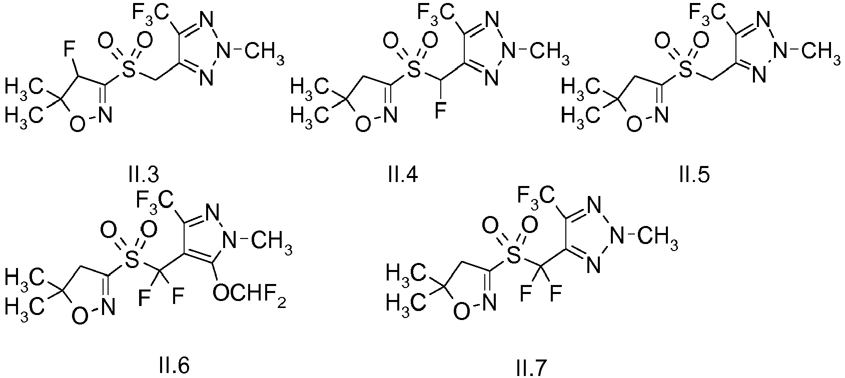 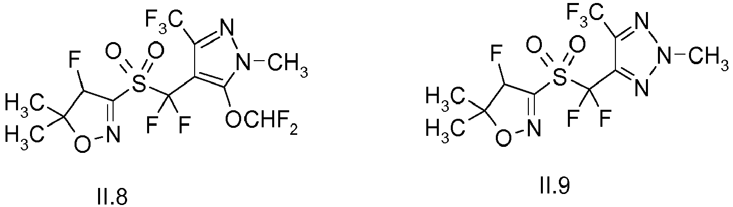 the isoxazoline compounds of the formula (I)I are known in the art, e.g. from WO 2006/024820, WO 2006/037945, WO 2007/071900 and WO 2007/096576;
   among the VLCFA inhibitors, preference is given to chloroacetamides and oxyacetamides;
b11) from the group of the cellulose biosynthesis inhibitors:
   chlorthiamid, dichlobenil, flupoxam, indaziflam, isoxaben, triaziflam and 1-cyclohexyl-5-pentafluorphenyloxy-1⁴-[1,2,4,6]thiatriazin-3-ylamine (CAS 175899-01-1);
b12) from the group of the decoupler herbicides:
   dinoseb, dinoterb and DNOC and its salts;
b13) from the group of the auxinic herbicides:
   2,4-D and its salts and esters such as clacyfos, 2,4-DB and its salts and esters, aminocyclopyrachlor and its salts and esters, aminopyralid and its salts such as aminopyralid-dimethylammonium, aminopyralid-tris(2-hydroxypropyl)ammonium and its esters, benazolin, benazolin-ethyl, chloramben and its salts and esters, clomeprop, clopyralid and its salts and esters, dicamba and its salts and esters, dichlorprop and its salts and esters, dichlorprop-P and its salts and esters, fluroxypyr, fluroxypyr-butometyl, fluroxypyr-meptyl, halauxifen and its salts and esters (CAS 943832-60-8 DOW, LS 566509); MCPA and its salts and esters, MCPA-thioethyl, MCPB and its salts and esters, mecoprop and its salts and esters, mecoprop-P and its salts and esters, picloram and its salts and esters, quinclorac, quinmerac, TBA (2,3,6) and its salts and esters, triclopyr and its salts and esters, 4-amino-3-chloro-6-(4-chloro-2-fluoro-3-methoxyphenyl)-5-fluoropyridine-2-carboxylic acid (DOW, "Rinskor-acid") and benzyl 4-amino-3-chloro-6-(4-chloro-2-fluoro-3-methoxyphenyl)-5-fluoropyridine-2-carboxylate (CAS 1390661-72-9) (DOW, "Rinskor");
b14) from the group of the auxin transport inhibitors: diflufenzopyr, diflufenzopyr-sodium, naptalam and naptalam-sodium;
b15) from the group of the other herbicides: bromobutide, chlorflurenol, chlorflurenol-methyl, cinmethylin, cumyluron, cyclopyrimorate (CAS 499223-49-3 Mitsui; SW-065; H-965) and its salts and esters, dalapon, dazomet, difenzoquat, difenzoquat-metilsulfate, dimethipin, DSMA, dymron, endothal and its salts, etobenzanid, flurenol, flurenol-butyl, flurprimidol, fosamine, fosamine-ammonium, indanofan, maleic hydrazide, mefluidide, metam, methiozolin (CAS 403640-27-7), methyl azide, methyl bromide, methyl-dymron, methyl iodide, MSMA, oleic acid, oxaziclomefone, pelargonic acid, pyributicarb, quinoclamine and tridiphane.

Preferred herbicides B that can be used in combination with the diaminotriazine compounds of the formula (I) according to the present invention are:
b1) from the group of the lipid biosynthesis inhibitors:
   clethodim, clodinafop-propargyl, cycloxydim, cyhalofop-butyl, diclofop-methyl, fenoxaprop-P-ethyl, fluazifop-P-butyl, haloxyfop-P-methyl, metamifop, pinoxaden, profoxydim, propaquizafop, quizalofop-P-ethyl, quizalofop-P-tefuryl, sethoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl-[1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl-[1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); benfuresate, dimepiperate, EPTC, esprocarb, ethofumesate, molinate, orbencarb, prosulfocarb, thiobencarb and triallate;
b2) from the group of the ALS inhibitors:
   amidosulfuron, azimsulfuron, bensulfuron-methyl, bispyribac-sodium, chlorimuron-ethyl, chlorsulfuron, cloransulam-methyl, cyclosulfamuron, diclosulam, ethametsulfuron-methyl, ethoxysulfuron, flazasulfuron, florasulam, flucarbazone-sodium, flucetosulfuron, flumetsulam, flupyrsulfuron-methyl-sodium, foramsulfuron, halosulfuron-methyl, imazamethabenz-methyl, imazamox, imazapic, imazapyr, imazaquin, imazethapyr, imazosulfuron, iodosulfuron, iodosulfuron-methyl-sodium, iofensulfuron, iofensulfuron-sodium, mesosulfuron, metazosulfuron, metosulam, metsulfuron-methyl, nicosulfuron, orthosulfamuron, oxasulfuron, penoxsulam, primisulfuron-methyl, propoxycarbazon-sodium, propyrisulfuron, prosulfuron, pyrazosulfuron-ethyl, pyribenzoxim, pyrimisulfan, pyriftalid, pyriminobac-methyl, pyrithiobac-sodium, pyroxsulam, rimsulfuron, sulfometuron-methyl, sulfosulfuron, thiencarbazone-methyl, thifensulfuron-methyl, triasulfuron, tribenuron-methyl, trifloxysulfuron, triflusulfuron-methyl, tritosulfuron and triafamone;
b3) from the group of the photosynthesis inhibitors:
   ametryn, amicarbazone, atrazine, bentazone, bentazone-sodium, bromoxynil and its salts and esters, chloridazone, chlorotoluron, cyanazine, desmedipham, diquat-dibromide, diuron, fluometuron, hexazinone, ioxynil and its salts and esters, isoproturon, lenacil, linuron, metamitron, methabenzthiazuron, metribuzin, paraquat, paraquat-dichloride, phenmedipham, propanil, pyridate, simazine, terbutryn, terbuthylazine and thidiazuron;
b4) from the group of the protoporphyrinogen-IX oxidase inhibitors:
   acifluorfen-sodium, bencarbazone, benzfendizone, butafenacil, carfentrazone-ethyl, cinidon-ethyl, flufenpyr-ethyl, flumiclorac-pentyl, flumioxazin, fluoroglycofen-ethyl, fomesafen, lactofen, oxadiargyl, oxadiazon, oxyfluorfen, pentoxazone, pyraflufen, pyraflufen-ethyl, saflufenacil, sulfentrazone, tiafenacil, trifludimoxazin (BAS 850 H), ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100; Sumitomo; LS 5296489), N-ethyl-3-(2,6-dichloro-4-trifluoromethylphenoxy)-5-methyl-1*H-*pyrazole-1-carboxamide (CAS 452098-92-9), N-tetrahydrofurfuryl-3-(2,6-dichloro-4-trifluoromethylphenoxy)-5-methyl-1*H*-pyrazole-1-carboxamide (CAS 915396-43-9), N-ethyl-3-(2-chloro-6-fluoro-4-trifluoromethylphenoxy)-5-methyl-1*H*-pyrazole-1-carboxamide (CAS 452099-05-7), N-tetrahydrofurfuryl-3-(2-chloro-6-fluoro-4-trifluoromethylphenoxy)-5-methyl-1*H-*pyrazole-1-carboxamide (CAS 452100-03-7), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7) (LS 4061013), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0) (LS 567 0033 = F2-Flumioxazin) ;1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0) ***(LS 568 1323 = Uracil-F2-PPO),*** and 3-[7-chloro-5-fluoro-2-(trifluoromethyl)-1H-benzimidazol-4-yl]-1-methyl-6-(trifluoromethyl)-1H-pyrimidine-2,4-dione (CAS 212754-02-4) (FMC Trifluoromethyluracil);
b5) from the group of the bleacher herbicides:
   aclonifen, amitrole, beflubutamid, benzobicyclon, bicyclopyrone, clomazone, diflufenican, fenquintrione, flumeturon, flurochloridone, flurtamone, isoxaflutole, mesotrione, norflurazon, picolinafen, pyrasulfotole, pyrazolynate, sulcotrione, tefuryltrione, tembotrione, tolpyralate, topramezone and 4-(3-trifluoromethylphenoxy)-2-(4-trifluoromethylphenyl)pyrimidine (CAS 180608-33-7);
b6) from the group of the EPSP synthase inhibitors:
   glyphosate, glyphosate-isopropylammonium, glyphosate-potassium and glyphosate-trimesium (sulfosate);
b7) from the group of the glutamine synthase inhibitors:
   glufosinate, glufosinate-P, glufosinate-ammonium;
b8) from the group of the DHP synthase inhibitors: asulam;
b9) from the group of the mitosis inhibitors:
   benfluralin, dithiopyr, ethalfluralin, flamprop, flamprop-isopropyl, flamprop-methyl, flamprop-M-isopropyl, flamprop-M-methyl, oryzalin, pendimethalin, thiazopyr and trifluralin;
b10) from the group of the VLCFA inhibitors:
   acetochlor, alachlor, anilofos, butachlor, cafenstrole, dimethenamid, dimethenamid-P, fentrazamide, flufenacet, mefenacet, metazachlor, metolachlor, S-metolachlor, naproanilide, napropamide, napropamide-M, pretilachlor, fenoxasulfone, ipfencarbazone, pyroxasulfone thenylchlor and isoxazoline-compounds of the formulae 11.1, II.2, II.3, II.4, II.5, II.6, II.7, II.8 and II.9 as mentioned above;
b11) from the group of the cellulose biosynthesis inhibitors: dichlobenil, flupoxam, indaziflam, isoxaben, triaziflam and 1-cyclohexyl-5-pentafluorphenyloxy-1⁴-[1,2,4,6]thiatriazin-3-ylamine (CAS 175899-01-1);
b13) from the group of the auxinic herbicides:
   2,4-D and its salts and esters, aminocyclopyrachlor and its salts and esters, aminopyralid and its salts such as aminopyralid-dimethylammonium, aminopyralid-tris(2-hydroxypropyl)ammonium and its esters, clopyralid and its salts and esters, dicamba and its salts and esters, dichlorprop-P and its salts and esters, fluroxypyr-meptyl, halauxifen and its salts and esters (CAS 943832-60-8 DOW, LS 566509), MCPA and its salts and esters, MCPB and its salts and esters, mecoprop-P and its salts and esters, picloram and its salts and esters, quinclorac, quinmerac, triclopyr and its salts and esters, 4-amino-3-chloro-6-(4-chloro-2-fluoro-3-methoxyphenyl)-5-fluoropyridine-2-carboxylic acid (DOW, "Rinskor-acid") and benzyl 4-amino-3-chloro-6-(4-chloro-2-fluoro-3-methoxyphenyl)-5-fluoropyridine-2-carboxylate (CAS 1390661-72-9) (DOW, "Rinskor");
b14) from the group of the auxin transport inhibitors: diflufenzopyr and diflufenzopyr-sodium;
b15) from the group of the other herbicides: bromobutide, cinmethylin, cumyluron, cyclopyrimorate (CAS 499223-49-3 Mitsui; SW-065; H-965) and its salts and esters, dalapon, difenzoquat, difenzoquat-metilsulfate, DSMA, dymron (= daimuron), indanofan, metam, methylbromide, MSMA, oxaziclomefone, pyributicarb and tridiphane.

Particularly preferred herbicides B that can be used in combination with the diaminotriazine compounds of the formula (I) according to the present invention are:
b1) from the group of the lipid biosynthesis inhibitors: clodinafop-propargyl, cycloxydim, cyhalofop-butyl, fenoxaprop-P-ethyl, pinoxaden, profoxydim, tepraloxydim, tralkoxydim, 4-(4'-Chloro-4-cyclopropyl-2'-fluoro[1,1'-biphenyl]-3-yl)-5-hydroxy-2,2,6,6-tetramethyl-2H-pyran-3(6H)-one (CAS 1312337-72-6); 4-(2',4'-Dichloro-4-cyclopropyl[1,1'-biphenyl]-3-yl)-5-hydroxy-2,2,6,6-tetramethyl-2H-pyran-3(6H)-one (CAS 1312337-45-3); 4-(4'-Chloro-4-ethyl-2'-fluoro[1,1'-biphenyl]-3-yl)-5-hydroxy-2,2,6,6-tetramethyl-2H-pyran-3(6H)-one (CAS 1033757-93-5); 4-(2',4'-Dichloro-4-ethyl[1,1'-biphenyl]-3-yl)-2,2,6,6-tetramethyl-2H-pyran-3,5(4H,6H)-dione (CAS 1312340-84-3); 5-(Acetyloxy)-4-(4'-chloro-4-cyclopropyl-2'-fluoro[1,1'-biphenyl]-3-yl)-3,6-dihydro-2,2,6,6-tetramethyl-2H-pyran-3-one (CAS 1312337-48-6); 5-(Acetyloxy)-4-(2',4'-dichloro-4-cyclopropyl-[1,1'-biphenyl]-3-yl)-3,6-dihydro-2,2,6,6-tetramethyl-2H-pyran-3-one; 5-(Acetyloxy)-4-(4'-chloro-4-ethyl-2'-fluoro[1,1'-biphenyl]-3-yl)-3,6-dihydro-2,2,6,6-tetramethyl-2H-pyran-3-one (CAS 1312340-82-1); 5-(Acetyloxy)-4-(2',4'-dichloro-4-ethyl[1,1'-biphenyl]-3-yl)-3,6-dihydro-2,2,6,6-tetramethyl-2H-pyran-3-one (CAS 1033760-55-2); 4-(4'-Chloro-4-cyclopropyl-2'-fluoro[1,1'-biphenyl]-3-yl)-5,6-dihydro-2,2,6,6-tetramethyl-5-oxo-2H-pyran-3-yl carbonic acid methyl ester (CAS 1312337-51-1); 4-(2',4'-Dichloro -4-cyclopropyl- [1,1'-biphenyl]-3-yl)-5,6-dihydro-2,2,6,6-tetramethyl-5-oxo-2H-pyran-3-yl carbonic acid methyl ester; 4-(4'-Chloro-4-ethyl-2'-fluoro[1,1'-biphenyl]-3-yl)-5,6-dihydro-2,2,6,6-tetramethyl-5-oxo-2H-pyran-3-yl carbonic acid methyl ester (CAS 1312340-83-2); 4-(2',4'-Dichloro-4-ethyl[1,1'-biphenyl]-3-yl)-5,6-dihydro-2,2,6,6-tetramethyl-5-oxo-2H-pyran-3-yl carbonic acid methyl ester (CAS 1033760-58-5); esprocarb, prosulfocarb, thiobencarb and triallate;
b2) from the group of the ALS inhibitors: bensulfuron-methyl, bispyribac-sodium, cyclosulfamuron, diclosulam, flumetsulam, flupyrsulfuron-methyl-sodium, foramsulfuron, imazamox, imazapic, imazapyr, imazaquin, imazethapyr, imazosulfuron, iodosulfuron, iodosulfuron-methyl-sodium, iofensulfuron, iofensulfuron-sodium, mesosulfuron, metazosulfuron, nicosulfuron, penoxsulam, propoxycarbazon-sodium, propyrisulfuron, pyrazosulfuron-ethyl, pyroxsulam, rimsulfuron, sulfosulfuron, thiencarbazon-methyl, tritosulfuron and triafamone;
b3) from the group of the photosynthesis inhibitors: ametryn, atrazine, diuron, fluometuron, hexazinone, isoproturon, linuron, metribuzin, paraquat, paraquat-dichloride, propanil, terbutryn and terbuthylazine;
b4) from the group of the protoporphyrinogen-IX oxidase inhibitors: flumioxazin, oxyfluorfen, pyraflufen, pyraflufen-ethyl, saflufenacil, sulfentrazone, trifludimoxazin (BAS 850 H), ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100; Sumitomo; LS 5296489), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7) **(LS 4061013),** 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0) (LS 567 0033 = F2-Flumioxazin), and 1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0) (LS 568 1323 = Uracil-F2-PPO);
b5) from the group of the bleacher herbicides: amitrole, bicyclopyrone, clomazone, diflufenican, fenquintrione, flumeturon, flurochloridone, isoxaflutole, mesotrione, picolinafen, sulcotrione, tefuryltrione, tembotrione, tolpyralate and topramezone;
b6) from the group of the EPSP synthase inhibitors: glyphosate, glyphosate-isopropylammonium and glyphosate-trimesium (sulfosate);
b7) from the group of the glutamine synthase inhibitors: glufosinate, glufosinate-P and glufosinate-ammonium;
b9) from the group of the mitosis inhibitors: pendimethalin and trifluralin;
b10) from the group of the VLCFA inhibitors: acetochlor, cafenstrole, dimethenamid-P, fentrazamide, flufenacet, mefenacet, metazachlor, metolachlor, S-metolachlor, fenoxasulfone, ipfencarbazone and pyroxasulfone; likewise, preference is given to isoxazoline compounds of the formulae II.1, II.2, II.3, II.4, II.5, II.6, II.7, II.8 and II.9 as mentioned above;
b11) from the group of the cellulose biosynthesis inhibitors: indaziflam, isoxaben and triaziflam;
b13) from the group of the auxinic herbicides: 2,4-D and its salts and esters such as clacyfos, and aminocyclopyrachlor and its salts and esters, aminopyralid and its salts and its esters, clopyralid and its salts and esters, dicamba and its salts and esters, fluroxypyr-meptyl, halauxifen, halauxifen-methyl, quinclorac, quinmerac, 4-amino-3-chloro-6-(4-chloro-2-fluoro-3-methoxyphenyl)-5-fluoropyridine-2-carboxylic acid (DOW, "Rinskor-acid") and benzyl 4-amino-3-chloro-6-(4-chloro-2-fluoro-3-methoxyphenyl)-5-fluoropyridine-2-carboxylate (CAS 1390661-72-9) (DOW, "Rinskor");
b14) from the group of the auxin transport inhibitors: diflufenzopyr and diflufenzopyr-sodium,
b15) from the group of the other herbicides: dymron (= daimuron), indanofan, oxaziclomefone.

Particularly preferred herbicides B are the herbicides B as defined above; in particular the herbicides B.1 - B.196 listed below in table B:

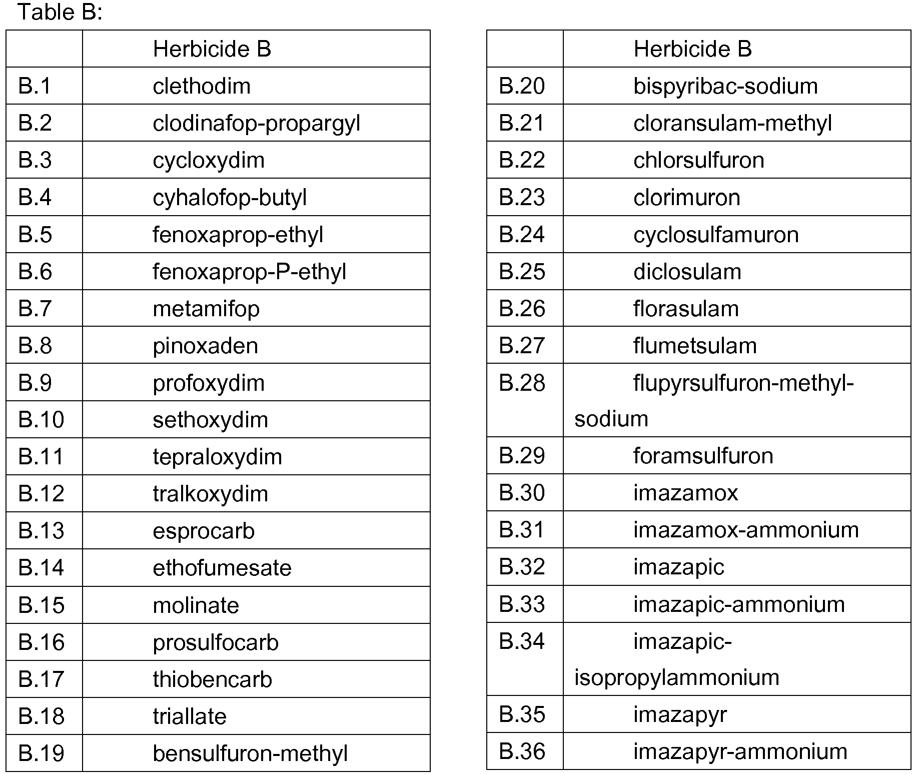

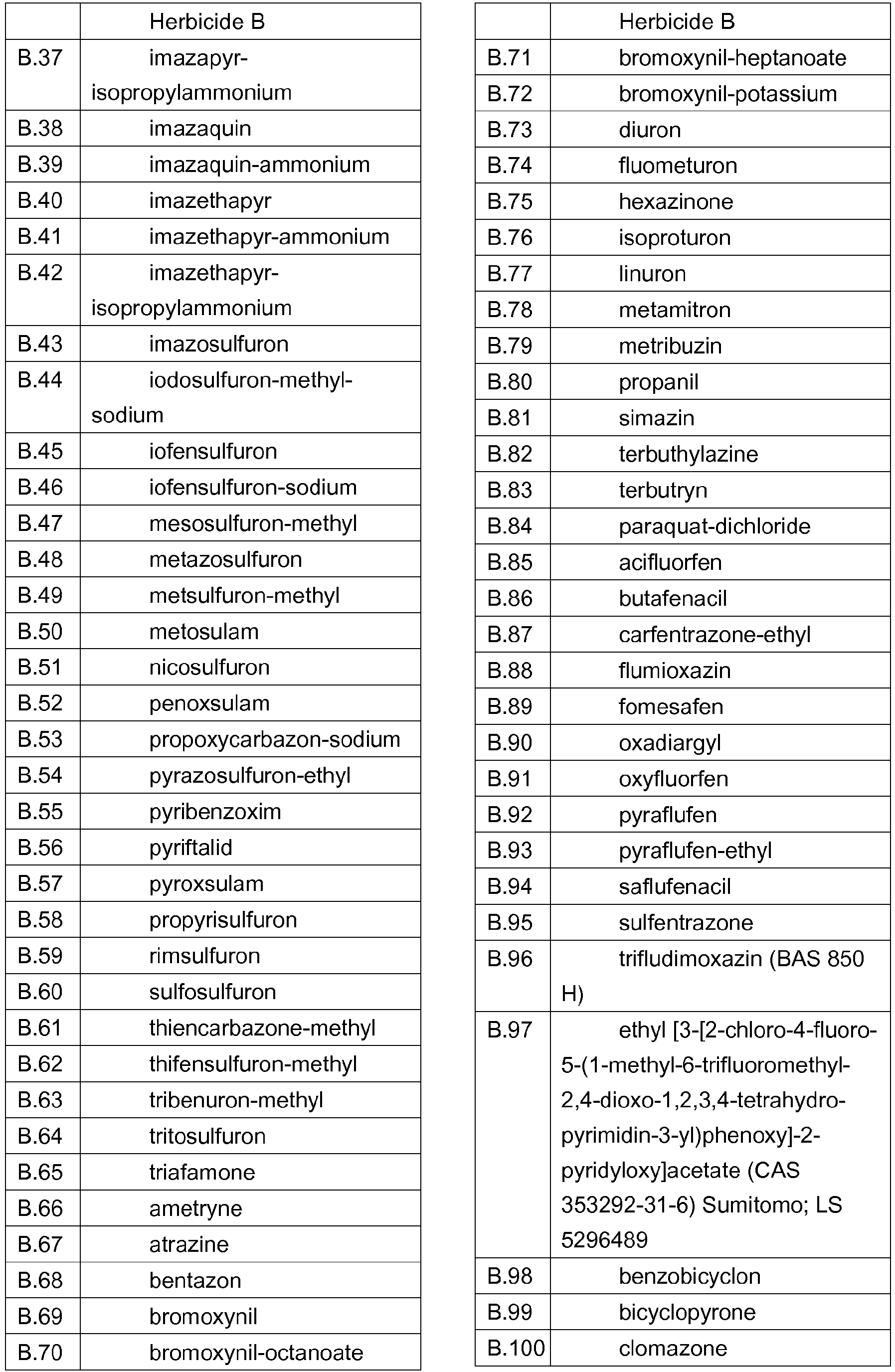

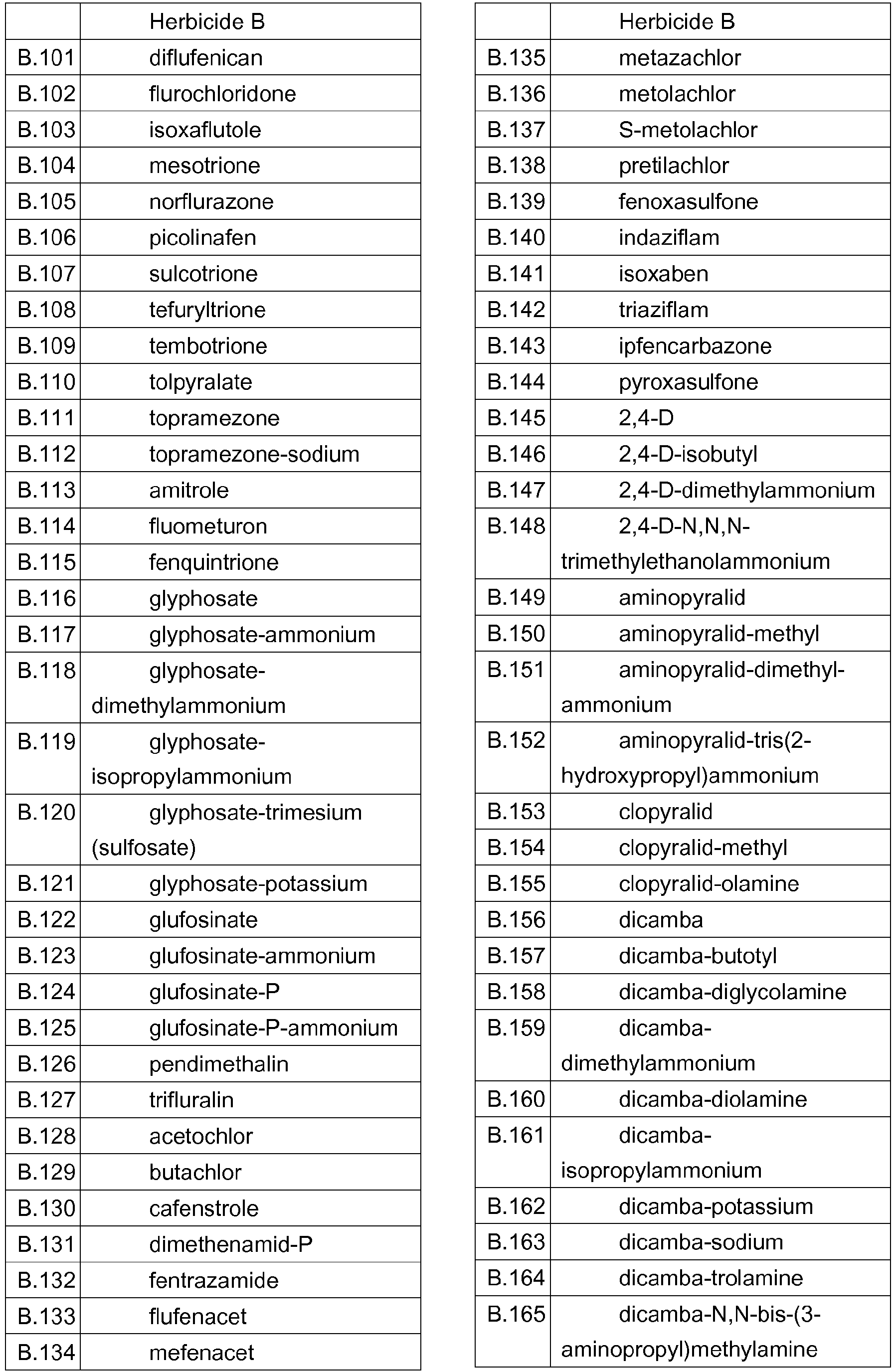

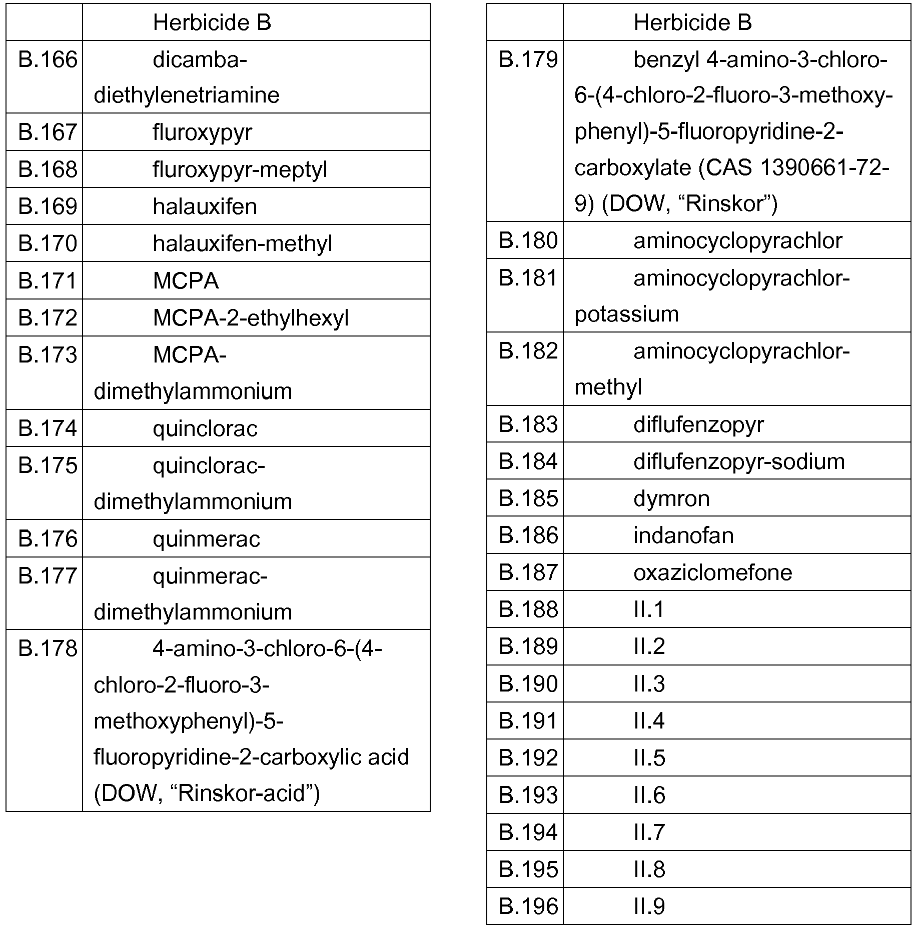

In another embodiment of the present invention the compositions according to the present invention comprise at least one diaminotriazine compound of formula (I) and at least one safener C.

Safeners are chemical compounds which prevent or reduce damage on useful plants without having a major impact on the herbicidal action of the herbicidal active components of the present compositions towards unwanted plants. They can be applied either before sowings (e.g. on seed treatments, shoots or seedlings) or in the pre-emergence application or post-emergence application of the useful plant. The safeners and the diaminotriazine compound of formula (I) and/or the herbicides B can be applied simultaneously or in succession.

Suitable safeners are e.g. (quinolin-8-oxy)acetic acids, 1-phenyl-5-haloalkyl-1H-1,2,4-triazol-3-carboxylic acids, 1-phenyl-4,5-dihydro-5-alkyl-1H-pyrazol-3,5-dicarboxylic acids, 4,5-dihydro-5,5-diaryl-3-isoxazol carboxylic acids, dichloroacetamides, alpha-oximinophenylacetonitriles, acetophenonoximes, 4,6-dihalo-2-phenylpyrimidines, N-[[4-(aminocarbonyl)phenyl]sulfonyl]-2-benzoic amides, 1,8-naphthalic anhydride, 2-halo-4-(haloalkyl)-5-thiazol carboxylic acids, phosphorthiolates and N-alkyl-O-phenylcarbamates and their agriculturally acceptable salts and their agriculturally acceptable derivatives such amides, esters, and thioesters, provided they have an acid group.

Examples of preferred safeners C are benoxacor, cloquintocet, cyometrinil, cyprosulfamide, dichlormid, dicyclonon, dietholate, fenchlorazole, fenclorim, flurazole, fluxofenim, furilazole, isoxadifen, mefenpyr, mephenate, naphthalic anhydride, oxabetrinil, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4) and N-(2-Methoxybenzoyl)-4-[(methylaminocarbonyl)amino]benzenesulfonamide (CAS 129531-12-0).

Especially preferred safeners C are benoxacor, cloquintocet, cyprosulfamide, dichlormid, fenchlorazole, fenclorim, flurazole, fluxofenim, furilazole, isoxadifen, mefenpyr, naphthalic anhydride, oxabetrinil, 4-(dichloroacetyl)-1-oxa-4-azaspiro-[4.5]decane (MON4660, CAS 71526-07-3), 2,2,5-trimethyl-3-(dichloroacetyl)-1,3-oxazolidine (R-29148, CAS 52836-31-4) and N-(2-Methoxybenzoyl)-4-[(methylaminocarbonyl)amino]benzenesulfonamide (CAS 129531-12-0).

Particularly preferred safeners C are benoxacor, cloquintocet, cyprosulfamide, dichlormid, fenchlorazole, fenclorim, furilazole, isoxadifen, mefenpyr, naphtalic anhydride, 4-(dichloroacetyl)-1-oxa-4-azaspiro[4.5]decane (MON4660, CAS 71526-07-3), 2,2,5-trimethyl-3-(dichloroacetyl)-1,3-oxazolidine (R-29148, CAS 52836-31-4) and N-(2-Methoxybenzoyl)-4-[(methylaminocarbonyl)amino]benzenesulfonamide (CAS 129531-12-0).

Particularly preferred safeners C, which, as component C, are constituent of the composition according to the invention are the safeners C as defined above; in particular the safeners C.1 - C.17 listed below in table C:

**Table C**

| | Safener C |
|---|---|
| C.1 | benoxacor |
| C.2 | cloquintocet |
| C.3 | cloquintocet-mexyl |
| C.4 | cyprosulfamide |
| C.5 | dichlormid |
| C.6 | fenchlorazole |
| C.7 | fenchlorazole-ethyl |
| C.8 | fenclorim |
| C.9 | furilazole |
| C.10 | isoxadifen |
| C.11 | isoxadifen-ethyl |
| C.12 | mefenpyr |
| C.13 | mefenpyr-diethyl |
| C.14 | naphtalic acid anhydride |
| C.15 | 4-(dichloroacetyl)-1-oxa-4-azaspiro[4.5]decane (MON4660, CAS 71526-07-3) |
| C.16 | 2,2,5-trimethyl-3-(dichloroacetyl)-1,3-oxazolidine (R-29148, CAS 52836-31-4) |
| C.17 | N-(2-Methoxybenzoyl)-4-[(methylaminocarbonyl)amino]benzenesulfonamide (CAS 129531-12-0) |

The active compounds B of groups b1) to b15) and the active compounds C are known herbicides and safeners, see, for example, The Compendium of Pesticide Common Names (http://www.alanwood.net/pesticides/); Farm Chemicals Handbook 2000 volume 86, Meister Publishing Company, 2000; B. Hock, C. Fedtke, R. R. Schmidt, Herbizide [Herbicides], Georg Thieme Verlag, Stuttgart 1995; W. H. Ahrens, Herbicide Handbook, 7th edition, Weed Science Society of America, 1994; and K. K. Hatzios, Herbicide Handbook, Supplement for the 7th edition, Weed Science Society of America, 1998. 2,2,5-Trimethyl-3-(dichloroacetyl)-1,3-oxazolidine [CAS No. 52836-31-4] is also referred to as R-29148. 4-(Dichloroacetyl)-1-oxa-4-azaspiro[4.5]decane [CAS No. 71526-07-3] is also referred to as AD-67 and MON 4660.

The assignment of the active compounds to the respective mechanisms of action is based on current knowledge. If several mechanisms of action apply to one active compound, this substance was only assigned to one mechanism of action.

Active compounds B and C having a carboxyl group can be employed in the form of the acid, in the form of an agriculturally suitable salt as mentioned above or else in the form of an agriculturally acceptable derivative in the compositions according to the invention.

In the case of dicamba, suitable salts include those, where the counterion is an agriculturally acceptable cation. For example, suitable salts of dicamba are dicamba-sodium, dicamba-potassium, dicamba-methylammonium, dicamba-dimethylammonium, dicamba-isopropylammonium, dicamba-diglycolamine, dicamba-olamine, dicamba-diolamine, dicamba-trolamine, dicamba-N,N-bis-(3-aminopropyl)methylamine and dicamba-diethylenetriamine. Examples of a suitable ester are dicamba-methyl and dicamba-butotyl.

Suitable salts of 2,4-D are 2,4-D-ammonium, 2,4-D-dimethylammonium, 2,4-D-diethylammonium, 2,4-D-diethanolammonium (2,4-D-diolamine), 2,4-D-triethanolammonium, 2,4-D-isopropylammonium, 2,4-D-triisopropanolammonium, 2,4-D-heptylammonium, 2,4-D-dodecylammonium, 2,4-D-tetradecylammonium, 2,4-D-triethylammonium, 2,4-D-tris(2-hydroxypropyl)ammonium, 2,4-D-tris(isopropyl)-ammonium, 2,4-D-trolamine, 2,4-D-lithium, 2,4-D-sodium. Examples of suitable esters of 2,4-D are 2,4-D-butotyl, 2,4-D-2-butoxypropyl, 2,4-D-3-butoxypropyl, 2,4-D-butyl, 2,4-D-ethyl, 2,4-D-ethylhexyl, 2,4-D-isobutyl, 2,4-D-isooctyl, 2,4-D-isopropyl, 2,4-D-meptyl, 2,4-D-methyl, 2,4-D-octyl, 2,4-D-pentyl, 2,4-D-propyl, 2,4-D-tefuryl and clacyfos.

Suitable salts of 2,4-DB are for example 2,4-DB-sodium, 2,4-DB-potassium and 2,4-DB-dimethylammonium. Suitable esters of 2,4-DB are for example 2,4-DB-butyl and 2,4-DB-isoctyl.

Suitable salts of dichlorprop are for example dichlorprop-sodium, dichlorprop-potassium and dichlorprop-dimethylammonium. Examples of suitable esters of dichlorprop are dichlorprop-butotyl and dichlorprop-isoctyl.

Suitable salts and esters of MCPA include MCPA-butotyl, MCPA-butyl, MCPA-dimethylammonium, MCPA-diolamine, MCPA-ethyl, MCPA-thioethyl, MCPA-2-ethylhexyl, MCPA-isobutyl, MCPA-isoctyl, MCPA-isopropyl, MCPA-isopropylammonium, MCPA-methyl, MCPA-olamine, MCPA-potassium, MCPA-sodium and MCPA-trolamine.

A suitable salt of MCPB is MCPB sodium. A suitable ester of MCPB is MCPB-ethyl.

Suitable salts of clopyralid are clopyralid-potassium, clopyralid-olamine and clopyralid-tris-(2-hydroxypropyl)ammonium. Example of suitable esters of clopyralid is clopyral id-methyl.

Examples of a suitable ester of fluroxypyr are fluroxypyr-meptyl and fluroxypyr-2-butoxy-1-methylethyl, wherein fluroxypyr-meptyl is preferred.

Suitable salts of picloram are picloram-dimethylammonium, picloram-potassium, picloram-triisopropanolammonium, picloram-triisopropylammonium and picloram-trolamine. A suitable ester of picloram is picloram-isoctyl.

A suitable salt of triclopyr is triclopyr-triethylammonium. Suitable esters of triclopyr are for example triclopyr-ethyl and triclopyr-butotyl.

Suitable salts and esters of chloramben include chloramben-ammonium, chloramben-diolamine, chloramben-methyl, chloramben-methylammonium and chloramben-sodium. Suitable salts and esters of 2,3,6-TBA include 2,3,6-TBA-dimethylammonium, 2,3,6-TBA-lithium, 2,3,6-TBA-potassium and 2,3,6-TBA-sodium.

Suitable salts and esters of aminopyralid include aminopyralid-potassium, aminopyralid-dimethylammonium, and aminopyralid-tris(2-hydroxypropyl)ammonium.

Suitable salts of glyphosate are for example glyphosate-ammonium, glyphosate-diammonium, glyphoste-dimethylammonium, glyphosate-isopropylammonium, glyphosate-potassium, glyphosate-sodium, glyphosate-trimesium as well as the ethanolamine and diethanolamine salts, preferably glyphosate-diammonium, glyphosate-isopropylammonium and glyphosate-trimesium (sulfosate).

A suitable salt of glufosinate is for example glufosinate-ammonium.

A suitable salt of glufosinate-P is for example glufosinate-P-ammonium.

Suitable salts and esters of bromoxynil are for example bromoxynil-butyrate, bromoxynil-heptanoate, bromoxynil-octanoate, bromoxynil-potassium and bromoxynil-sodium.

Suitable salts and esters of ioxonil are for example ioxonil-octanoate, ioxonil-potassium and ioxonil-sodium.

Suitable salts and esters of mecoprop include mecoprop-butotyl, mecoprop-dimethylammonium, mecoprop-diolamine, mecoprop-ethadyl, mecoprop-2-ethylhexyl, mecoprop-isoctyl, mecoprop-methyl, mecoprop-potassium, mecoprop-sodium and mecoprop-trolamine.

Suitable salts of mecoprop-P are for example mecoprop-P-butotyl, mecoprop-P-dimethylammonium, mecoprop-P-2-ethylhexyl, mecoprop-P-isobutyl, mecoprop-P-potassium and mecoprop-P-sodium.

A suitable salt of diflufenzopyr is for example diflufenzopyr-sodium.

A suitable salt of naptalam is for example naptalam-sodium.

Suitable salts and esters of aminocyclopyrachlor are for example aminocyclopyrachlor-dimethylammonium, aminocyclopyrachlor-methyl, aminocyclopyrachlor-triisopropanolammonium, aminocyclopyrachlor-sodium and aminocyclopyrachlor-potassium.

A suitable salt of quinclorac is for example quinclorac-dimethylammonium.

A suitable salt of quinmerac is for example quinmerac-dimethylammonium.

A suitable salt of imazamox is for example imazamox-ammonium.

Suitable salts of imazapic are for example imazapic-ammonium and imazapic-isopropylammonium.

Suitable salts of imazapyr are for example imazapyr-ammonium and imazapyr-isopropylammonium.

A suitable salt of imazaquin is for example imazaquin-ammonium.

Suitable salts of imazethapyr are for example imazethapyr-ammonium and imazethapyr-isopropylammonium.

A suitable salt of topramezone is for example topramezone-sodium.

According to a preferred embodiment of the invention, the composition comprises as herbicidal active compound B or component B at least one, preferably exactly one herbicide B.

According to another preferred embodiment of the invention, the composition comprises as herbicidal active compounds B or component B at least two, preferably exactly two herbicides B different from each other.

According to another preferred embodiment of the invention, the composition comprises as herbicidal active compounds B or component B at least three, preferably exactly three herbicides B different from each other.

According to another preferred embodiment of the invention, the composition comprises as safening component C or component C at least one, preferably exactly one safener C.

According to another preferred embodiment of the invention, the composition comprises as component B at least one, preferably exactly one herbicide B, and as component C at least one, preferably exactly one, safener C.

According to another preferred embodiment of the invention, the composition comprises at least two, preferably exactly two, herbicides B different from each other, and as component C at least one, preferably exactly one, safener C.

According to another preferred embodiment of the invention, the composition comprises at least three, preferably exactly three, herbicides B different from each other, and as component C at least one, preferably exactly one, safener C.

According to another preferred embodiment of the invention, the composition comprises at least one, preferably exactly one compound of formula (I) and as component B at least one, preferably exactly one, herbicide B.

According to another preferred embodiment of the invention, the composition comprises at least one, preferably exactly one compound of formula (I) and at least two, preferably exactly two, herbicides B different from each other.

According to another preferred embodiment of the invention, the composition comprises at least one, preferably exactly one compound of formula (I) and at least three, preferably exactly three, herbicides B different from each other.

According to another preferred embodiment of the invention, the composition comprises at least one, preferably exactly one compound of formula (I) and as component C at least one, preferably exactly one, safener C.

According to another preferred embodiment of the invention, the composition comprises at least one, preferably exactly one compound of formula (I) as component B at least one, preferably exactly one, herbicide B, and as component C at least one, preferably exactly one safener C.

According to another preferred embodiment of the invention, the composition comprises at least one, preferably exactly one compound of formula (I), at least two, preferably exactly two herbicides B different from each other, and as component C at least one, preferably exactly one, safener C.

According to another preferred embodiment of the invention, the composition comprises at least one, preferably exactly one compound of formula (I) at least three, preferably exactly three herbicides B different from each other, and as component C at least one, preferably exactly one, safener C.

According to another preferred embodiment of the invention, the composition comprises, in addition to a compounds of formula (I), at least one and especially exactly one herbicidally active compound from group b1), in particular selected from the group consisting of clodinafop-propargyl, cycloxydim, cyhalofop-butyl, fenoxaprop-P-ethyl, pinoxaden, profoxydim, tepraloxydim, tralkoxydim, esprocarb, prosulfocarb, thiobencarb and triallate.

According to another preferred embodiment of the invention, the composition comprises, in addition to a compounds of formula (I), at least one and especially exactly one herbicidally active compound from group b2), in particular selected from the group consisting of bensulfuron-methyl, bispyribac-sodium, cyclosulfamuron, diclosulam, flumetsulam, flupyrsulfuron-methyl-sodium, foramsulfuron, imazamox, imazapic, imazapyr, imazaquin, imazethapyr, imazosulfuron, iodosulfuron, iodosulfuron-methyl-sodium, mesosulfuron, metazosulfuron, nicosulfuron, penoxsulam, propoxycarbazon-sodium, pyrazosulfuron-ethyl, pyroxsulam, rimsulfuron, sulfosulfuron, thiencarbazon-methyl and tritosulfuron.

According to another preferred embodiment of the invention, the composition comprises, in addition to a compounds of formula (I), at least one and especially exactly one herbicidally active compound from group b3), in particular selected from the group consisting of ametryn, atrazine, diuron, fluometuron, hexazinone, isoproturon, linuron, metribuzin, paraquat, paraquat-dichloride, propanil, terbutryn and terbuthylazine.

According to another preferred embodiment of the invention, the composition comprises, in addition to a compounds of formula (I), at least one and especially exactly one herbicidally active compound from group b4), in particular selected from the group consisting of flumioxazin, oxyfluorfen, pyraflufen, pyraflufen-ethyl, saflufenacil, sulfentrazone, trifludimoxazin (BAS 850 H), ethyl [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetate (CAS 353292-31-6; S-3100; Sumitomo; LS 5296489), 3-[7-fluoro-3-oxo-4-(prop-2-ynyl)-3,4-dihydro-2H-benzo[1,4]oxazin-6-yl]-1,5-dimethyl-6-thioxo-[1,3,5]triazinan-2,4-dione (CAS 451484-50-7) LS 4061013), 2-(2,2,7-trifluoro-3-oxo-4-prop-2-ynyl-3,4-dihydro-2H-benzo[1,4]oxazin-6-yl)-4,5,6,7-tetrahydro-isoindole-1,3-dione (CAS 1300118-96-0) (LS 567 0033 = F2-Flumioxazin) and 1-methyl-6-trifluoromethyl-3-(2,2,7-trifluoro-3-oxo-4-prop-2-ynyl-3,4-dihydro-2H-benzo[1,4]oxazin-6-yl)-1H-pyrimidine-2,4-dione (CAS 1304113-05-0) (LS 568 1323 = Uracil-F2-PPO).

According to another preferred embodiment of the invention, the composition comprises, in addition to a compounds of formula (I), at least one and especially exactly one herbicidally active compound from group b5), in particular selected from the group consisting of amitrole, bicyclopyrone, clomazone, diflufenican, flumeturon, flurochloridone, isoxaflutole, mesotrione, picolinafen, sulcotrione, tefuryltrione, tembotrione, tolpyralate and topramezone.

According to another preferred embodiment of the invention, the composition comprises, in addition to a compounds of formula (I), at least one and especially exactly one herbicidally active compound from group b6), in particular selected from the group consisting of glyphosate, glyphosate-isopropylammonium and glyphosate-trimesium (sulfosate).

According to another preferred embodiment of the invention, the composition comprises, in addition to a compounds of formula (I), at least one and especially exactly one herbicidally active compound from group b7), in particular selected from the group consisting of glufosinate, glufosinate-P and glufosinate-ammonium.

According to another preferred embodiment of the invention, the composition comprises, in addition to a compounds of formula (I), at least one and especially exactly one herbicidally active compound from group b9), in particular selected from the group consisting of pendimethalin and trifluralin.

According to another preferred embodiment of the invention, the composition comprises, in addition to a compounds of formula (I), at least one and especially exactly one herbicidally active compound from group b10), in particular selected from the group consisting of acetochlor, cafenstrole, dimethenamid-P, fentrazamide, flufenacet, mefenacet, metazachlor, metolachlor, S-metolachlor, fenoxasulfone and pyroxasulfone. Likewise, preference is given to compositions comprising in addition to a compounds of formula (I), at least one and especially exactly one herbicidally active compound from group b10), in particular selected from the group consisting of isoxazoline compounds of the formulae II.1, II.2, II.3, II.4, II.5, II.6, II.7, II.8 and II.9, as defined above.

According to another preferred embodiment of the invention, the composition comprises, in addition to a compounds of formula (I), at least one and especially exactly one herbicidally active compound from group b11), in particular indaziflam, isoxaben and triaziflam.

According to another preferred embodiment of the invention, the composition comprises, in addition to a compounds of formula (I), at least one and especially exactly one herbicidally active compound from group b13), in particular selected from the group consisting of 2,4-D and its salts and esters, aminocyclopyrachlor and its salts and esters, aminopyralid and its salts such as aminopyralid-dimethylammonium, aminopyralid-tris(2-hydroxypropyl)ammonium and its esters, clopyralid and its salts and esters, dicamba and its salts and esters, fluroxypyr-meptyl, halauxifen, halauxifen-methyl, quinclorac, quinmerac, 4-amino-3-chloro-6-(4-chloro-2-fluoro-3-methoxyphenyl)-5-fluoropyridine-2-carboxylic acid (DOW, "Rinskor-acid") and benzyl 4-amino-3-chloro-6-(4-chloro-2-fluoro-3-methoxyphenyl)-5-fluoropyridine-2-carboxylate (CAS 1390661-72-9) (DOW, "Rinskor").

According to another preferred embodiment of the invention, the composition comprises, in addition to a compounds of formula (I), at least one and especially exactly one herbicidally active compound from group b14), in particular selected from the group consisting of diflufenzopyr and diflufenzopyr-sodium.

According to another preferred embodiment of the invention, the composition comprises, in addition to a compounds of formula (I), at least one and especially exactly one herbicidally active compound from group b15), in particular selected from the group consisting of dymron (= daimuron), indanofan and oxaziclomefone.

According to another preferred embodiment of the invention, the composition comprises, in addition to a compounds of formula (I), at least one and especially exactly one safener C, in particular selected from the group consisting of benoxacor, cloquintocet, cyprosulfamide, dichlormid, fenchlorazole, fenclorim, furilazole, isoxadifen, mefenpyr, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

Further preferred embodiments relate to ternary compositions which correspond to the binary compositions mentioned above and additionally comprise a safener C, in particular selected from the group consisting of benoxacor, cloquintocet, cyprosulfamide, dichlormid, fenchlorazole, fenclorim, furilazole, isoxadifen, mefenpyr, 4-(dichloroacetyl)-1-oxa-4-azaspiro[4.5]decane (MON4660, CAS 71526-07-3) and 2,2,5-trimethyl-3-(dichloroacetyl)-1,3-oxazolidine (R-29148, CAS 52836-31-4).

Here and below, the term "binary compositions" includes compositions comprising one or more, for example 1, 2 or 3, active compounds of the formula (I) and either one or more, for example 1, 2 or 3, herbicides B or one or more safeners C.

Correspondingly, the term "ternary compositions" includes compositions comprising one or more, for example 1, 2 or 3, active compounds of the formula (I), one or more, for example 1, 2 or 3, herbicides B and one or more, for example 1, 2 or 3, safeners C.

In binary compositions comprising at least one compound of the formula (I) as component A and at least one herbicide B, the weight ratio of the active compounds A:B is generally in the range of from 1:1000 to 1000:1, preferably in the range of from 1:500 to 500:1, in particular in the range of from 1:250 to 250:1 and particularly preferably in the range of from 1:75 to 75:1.

In binary compositions comprising at least one compound of the formula (I) as component A and at least one safener C, the weight ratio of the active compounds A:C is generally in the range of from 1:1000 to 1000:1, preferably in the range of from 1:500 to 500:1, in particular in the range of from 1:250 to 250:1 and particularly preferably in the range of from 1:75 to 75:1.

In ternary compositions comprising at least one compound of formula (I) as component A, at least one herbicide B and at least one safener C, the relative proportions by weight of the components A:B are generally in the range of from 1:1000 to 1000:1, preferably in the range of from 1:500 to 500:1, in particular in the range of from 1:250 to 250:1 and particularly preferably in the range of from 1:75 to 75:1, the weight ratio of the components A:C is generally in the range of from 1:1000 to 1000:1, preferably in the range of from 1:500 to 500:1, in particular in the range of from 1:250 to 250:1 and particularly preferably in the range of from 1:75 to 75:1, and the weight ratio of the components B:C is generally in the range of from 1:1000 to 1000:1, preferably in the range of from 1:500 to 500:1, in particular in the range of from 1:250 to 250:1 and particularly preferably in the range of from 1:75 to 75:1. The weight ratio of components A + B to component C is preferably in the range of from 1:500 to 500:1, in particular in the range of from 1:250 to 250:1 and particularly preferably in the range of from 1:75 to 75:1.

The weight ratios of the individual components in the preferred mixtures mentioned below are within the limits given above, in particular within the preferred limits.

Particularly preferred are the compositions mentioned below comprising the compounds of formula (I) as defined and the substance(s) as defined in the respective row of table 1;
especially preferred comprising as only herbicidal active compounds the compounds of formula (I) as defined and the substance(s) as defined in the respective row of table 1;
most preferably comprising as only active compounds the compounds of formula I as defined and the substance(s) as defined in the respective row of table 1.

Particularly preferred are compositions 1.1 to 1.3545, comprising the compounds of formula (I) and the substance(s) as defined in the respective row of table 1:

The following combinations indicated by the code I.X.Y.Z represent particular embodiments of the invention:
I.a.1.1 to I.a.600.3545
I.b.1.1 to I.b.600.3545
I.c.1.1 to I.c.600.3545
I.d.1.1 to I.d.600.3545
I.e.1.1 to I.e.600.3545
I.f.1.1 to I.f.600.3545
I.g.1.1 to I.g.600.3545
I.h.1.1 to I.h.600.3545
I.i.1.1 to I.i.600.3545
I.k.1.1 to I.k.600.3545
I.l.1.1 to I.l.600.3545

In the above codes I.x refers to the formulae I.a, I.b, I.c, I.d, I.e, I.f, I.g, I.h, I.i, I.k and I.l, respectively. The integer Y refers to the row of table A, while the integer Z refers to the row of table 1 below (compositions 1.1 to 1.3545).

Hence, the code I.a.1.1 refers to the combination of the compound of formula I.a, wherein X, R^{a} and R^{d} are as defined in row 1 of table A, with the combination of the herbicide B and and the safener C are as defined in combination no. 1.1 of table 1.

The code I.e.91.35 refers to the combination of the compound of formula I.e wherein X, R^{a} and R^{d} are as defined in row 91 of table A, with the combination of the herbicide B and and the safener C are as defined in combination no. 1.35 of table 1.

The code I.k.92.3401 refers to the combination of the compound of formula I.k wherein X, R^{a} and R^{d} are as defined in row 92 of table A, with the combination of the herbicide B and and the safener C are as defined in combination no. 1.3401 of table 1.

Further particluar examples are the following mixtures:
- mixtures I.e.79.1 to I.e.79.3545, i.e. the mixtures of the compound of formula I.e, where X, R^{a} and R^{d} are as defined in row 79 of table A and where the herbicide or herbicide safener combination is as defined in one of the rows 1.1 to 1.3545 of table 1;
- mixtures I.e.89.1 to I.e.89.3545, i.e. the mixtures of the compound of formula I.e, where X, R^{a} and R^{d} are as defined in row 89 of table A and where the herbicide or herbicide safener combination is as defined in one of the rows 1.1 to 1.3545 of table 1;
- mixtures I.e.90.1 to I.e.90.3545, i.e. the mixtures of the compound of formula I.e, where X, R^{a} and R^{d} are as defined in row 90 of table A and where the herbicide or herbicide safener combination is as defined in one of the rows 1.1 to 1.3545 of table 1;
- mixtures I.e.91.1 to I.e.91.3545, i.e. the mixtures of the compound of formula I.e, where X, R^{a} and R^{d} are as defined in row 91 of table A and where the herbicide or herbicide safener combination is as defined in one of the rows 1.1 to 1.3545 of table 1;
- mixtures I.e.92.1 to I.e.92.3545, i.e. the mixtures of the compound of formula I.e, where X, R^{a} and R^{d} are as defined in row 92 of table A and where the herbicide or herbicide safener combination is as defined in one of the rows 1.1 to 1.3545 of table 1;
- mixtures I.e.96.1 to I.e.96.3545, i.e. the mixtures of the compound of formula I.e, where X, R^{a} and R^{d} are as defined in row 96 of table A and where the herbicide or herbicide safener combination is as defined in one of the rows 1.1 to 1.3545 of table 1;
- mixtures I.e.97.1 to I.e.97.3545, i.e. the mixtures of the compound of formula I.e, where X, R^{a} and R^{d} are as defined in row 97 of table A and where the herbicide or herbicide safener combination is as defined in one of the rows 1.1 to 1.3545 of table 1;
- mixtures I.e.98.1 to I.e.98.3545, i.e. the mixtures of the compound of formula I.e, where X, R^{a} and R^{d} are as defined in row 98 of table A and where the herbicide or herbicide safener combination is as defined in one of the rows 1.1 to 1.3545 of table 1;
- mixtures I.e.109.1 to I.e.109.3545, i.e. the mixtures of the compound of formula I.e, where X, R^{a} and R^{d} are as defined in row 109 of table A and where the herbicide or herbicide safener combination is as defined in one of the rows 1.1 to 1.3545 of table 1;
- mixtures I.e.110.1 to I.e.110.3545, i.e. the mixtures of the compound of formula I.e, where X, R^{a} and R^{d} are as defined in row 110 of table A and where the herbicide or herbicide safener combination is as defined in one of the rows 1.1 to 1.3545 of table 1;
- mixtures I.e.111.1 to I.e.111.3545, i.e. the mixtures of the compound of formula I.e, where X, R^{a} and R^{d} are as defined in row 111 of table A and where the herbicide or herbicide safener combination is as defined in one of the rows 1.1 to 1.3545 of table 1;
- mixtures I.e.119.1 to I.e.119.3545, i.e. the mixtures of the compound of formula I.e, where X, R^{a} and R^{d} are as defined in row 119 of table A and where the herbicide or herbicide safener combination is as defined in one of the rows 1.1 to 1.3545 of table 1;
- mixtures I.e.130.1 to I.e.130.3545, i.e. the mixtures of the compound of formula I.e, where X, R^{a} and R^{d} are as defined in row 130 of table A and where the herbicide or herbicide safener combination is as defined in one of the rows 1.1 to 1.3545 of table 1;
- mixtures I.e.132.1 to I.e.132.3545, i.e. the mixtures of the compound of formula I.e, where X, R^{a} and R^{d} are as defined in row 132 of table A and where the herbicide or herbicide safener combination is as defined in one of the rows 1.1 to 1.3545 of table 1;
- mixtures I.k.79.1 to I.k.79.3545, i.e. the mixtures of the compound of formula I.k, where X, R^{a} and R^{d} are as defined in row 79 of table A and where the herbicide or herbicide safener combination is as defined in one of the rows 1.1 to 1.3545 of table 1;
- mixtures I.k.89.1 to I.k.89.3545, i.e. the mixtures of the compound of formula I.k, where X, R^{a} and R^{d} are as defined in row 89 of table A and where the herbicide or herbicide safener combination is as defined in one of the rows 1.1 to 1.3545 of table 1;
- mixtures I.k.90.1 to I.k.90.3545, i.e. the mixtures of the compound of formula I.k, where X, R^{a} and R^{d} are as defined in row 90 of table A and where the herbicide or herbicide safener combination is as defined in one of the rows 1.1 to 1.3545 of table 1;
- mixtures I.k.91.1 to I.k.91.3545, i.e. the mixtures of the compound of formula I.k, where X, R^{a} and R^{d} are as defined in row 91 of table A and where the herbicide or herbicide safener combination is as defined in one of the rows 1.1 to 1.3545 of table 1;
- mixtures I.k.92.1 to I.k.92.3545, i.e. the mixtures of the compound of formula I.k, where X, R^{a} and R^{d} are as defined in row 92 of table A and where the herbicide or herbicide safener combination is as defined in one of the rows 1.1 to 1.3545 of table 1;
- mixtures I.k.96.1 to I.k.96.3545, i.e. the mixtures of the compound of formula I.k, where X, R^{a} and R^{d} are as defined in row 96 of table A and where the herbicide or herbicide safener combination is as defined in one of the rows 1.1 to 1.3545 of table 1;
- mixtures I.k.97.1 to I.k.97.3545, i.e. the mixtures of the compound of formula I.k, where X, R^{a} and R^{d} are as defined in row 97 of table A and where the herbicide or herbicide safener combination is as defined in one of the rows 1.1 to 1.3545 of table 1;
- mixtures I.k.98.1 to I.k.98.3545, i.e. the mixtures of the compound of formula I.k, where X, R^{a} and R^{d} are as defined in row 98 of table A and where the herbicide or herbicide safener combination is as defined in one of the rows 1.1 to 1.3545 of table 1;
- mixtures I.k.109.1 to I.k.109.3545, i.e. the mixtures of the compound of formula I.k, where X, R^{a} and R^{d} are as defined in row 109 of table A and where the herbicide or herbicide safener combination is as defined in one of the rows 1.1 to 1.3545 of table 1;
- mixtures I.k.110.1 to I.k.110.3545, i.e. the mixtures of the compound of formula I.k, where X, R^{a} and R^{d} are as defined in row 110 of table A and where the herbicide or herbicide safener combination is as defined in one of the rows 1.1 to 1.3545 of table 1;
- mixtures I.k.111.1 to I.k.111.3545, i.e. the mixtures of the compound of formula I.k, where X, R^{a} and R^{d} are as defined in row 111 of table A and where the herbicide or herbicide safener combination is as defined in one of the rows 1.1 to 1.3545 of table 1;
- mixtures I.k.119.1 to I.k.119.3545, i.e. the mixtures of the compound of formula I.k, where X, R^{a} and R^{d} are as defined in row 119 of table A and where the herbicide or herbicide safener combination is as defined in one of the rows 1.1 to 1.3545 of table 1;
- mixtures I.k.130.1 to I.k.130.3545, i.e. the mixtures of the compound of formula I.k, where X, R^{a} and R^{d} are as defined in row 130 of table A and where the herbicide or herbicide safener combination is as defined in one of the rows 1.1 to 1.3545 of table 1;
- mixtures I.k.132.1 to 1.k.132.3545, i.e. the mixtures of the compound of formula I.k, where X, R^{a} and R^{d} are as defined in row 132 of table A and where the herbicide or herbicide safener combination is as defined in one of the rows 1.1 to 1.3545 of table 1.

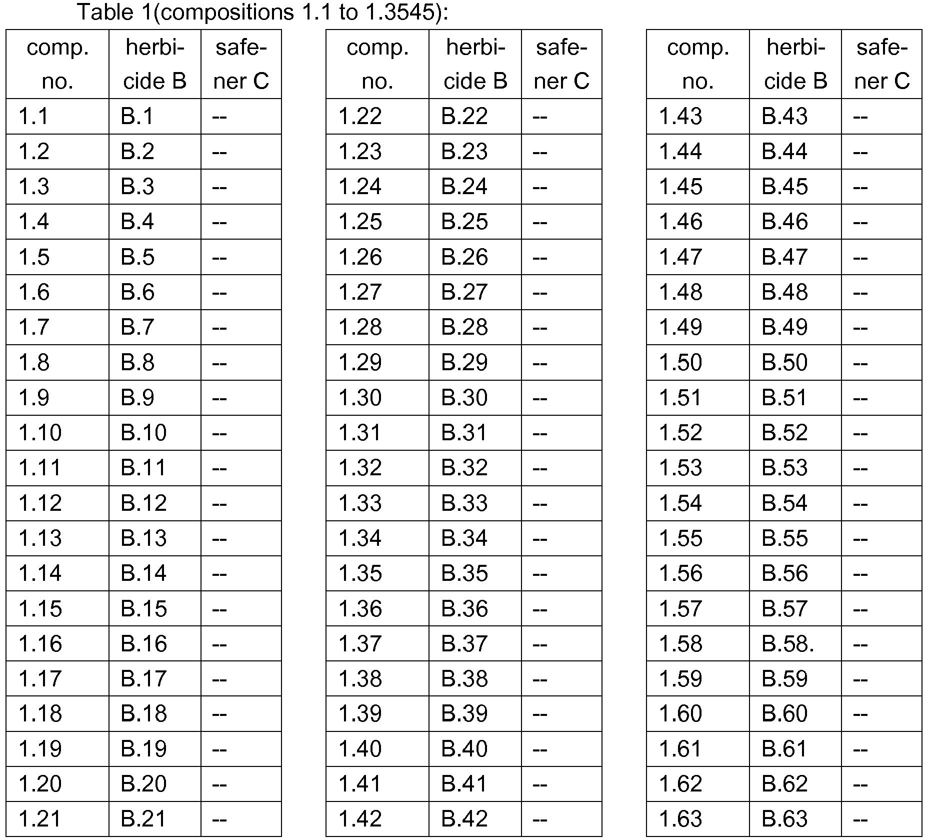

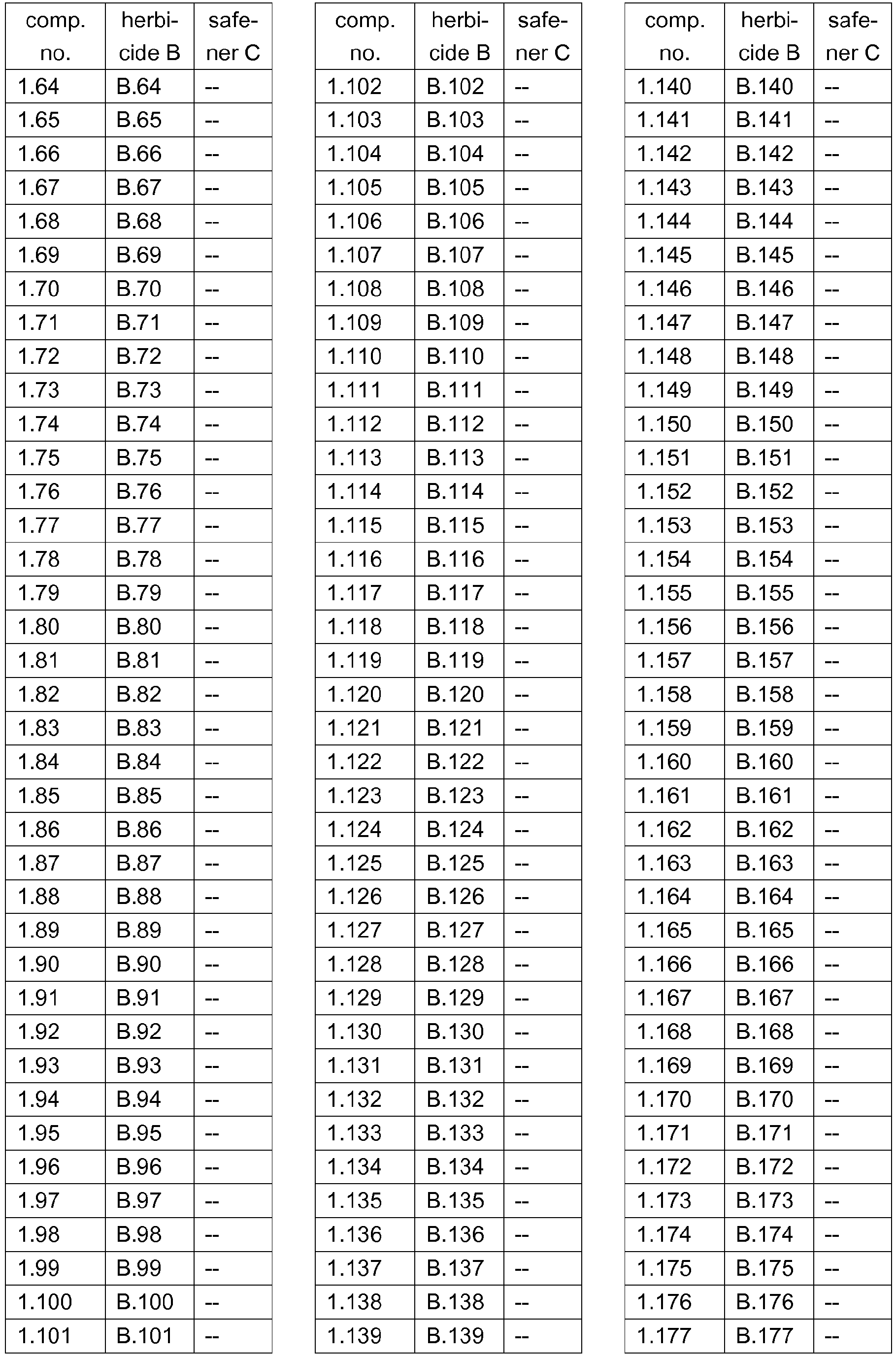

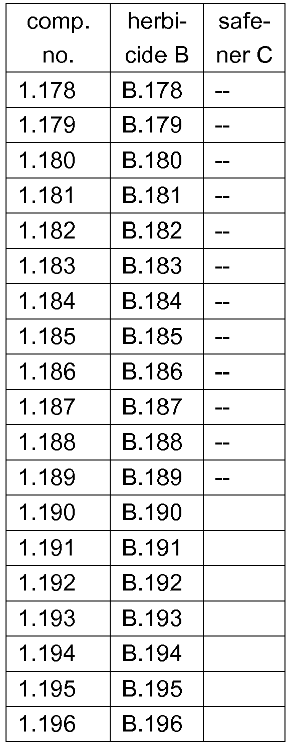

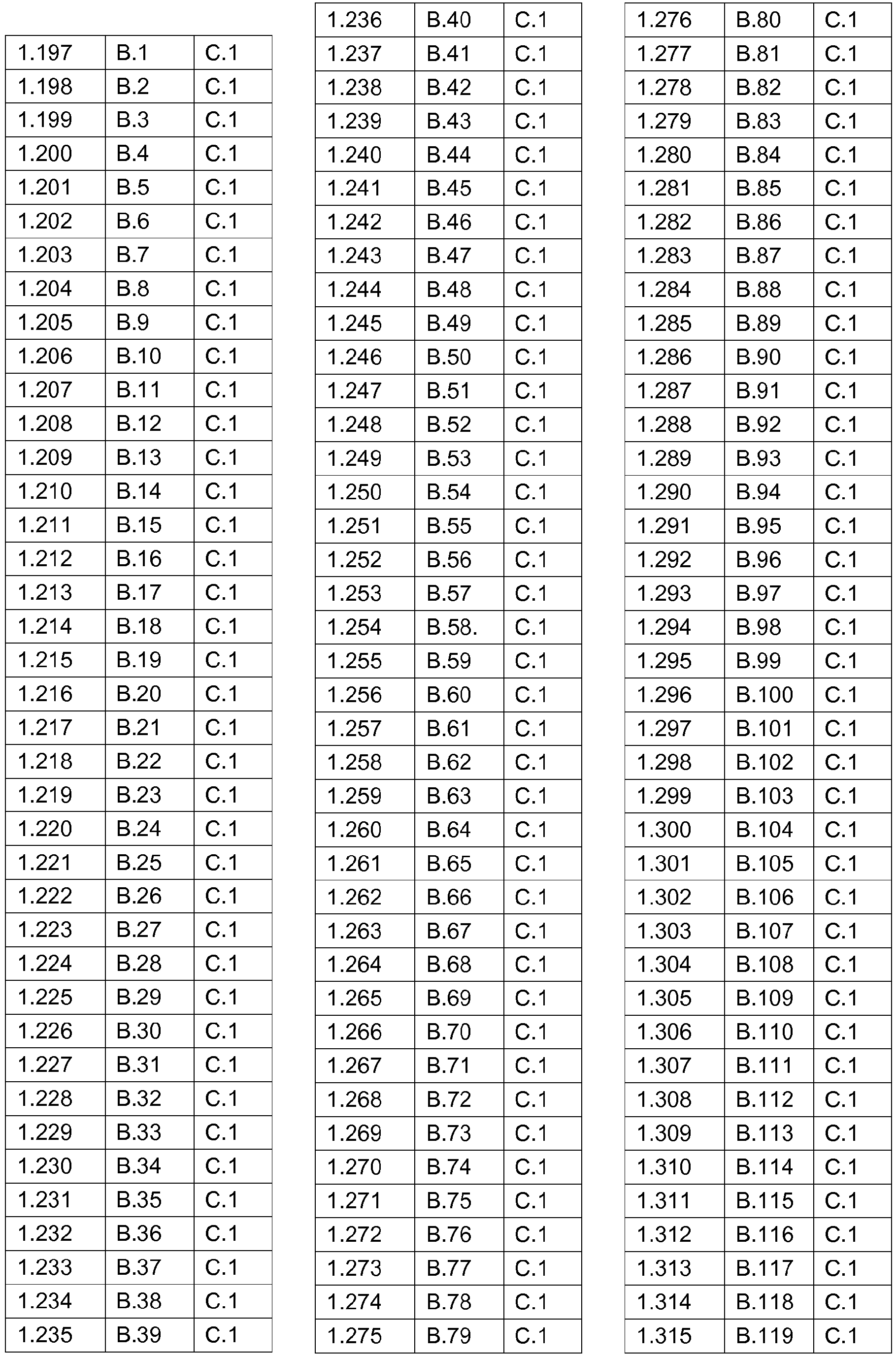

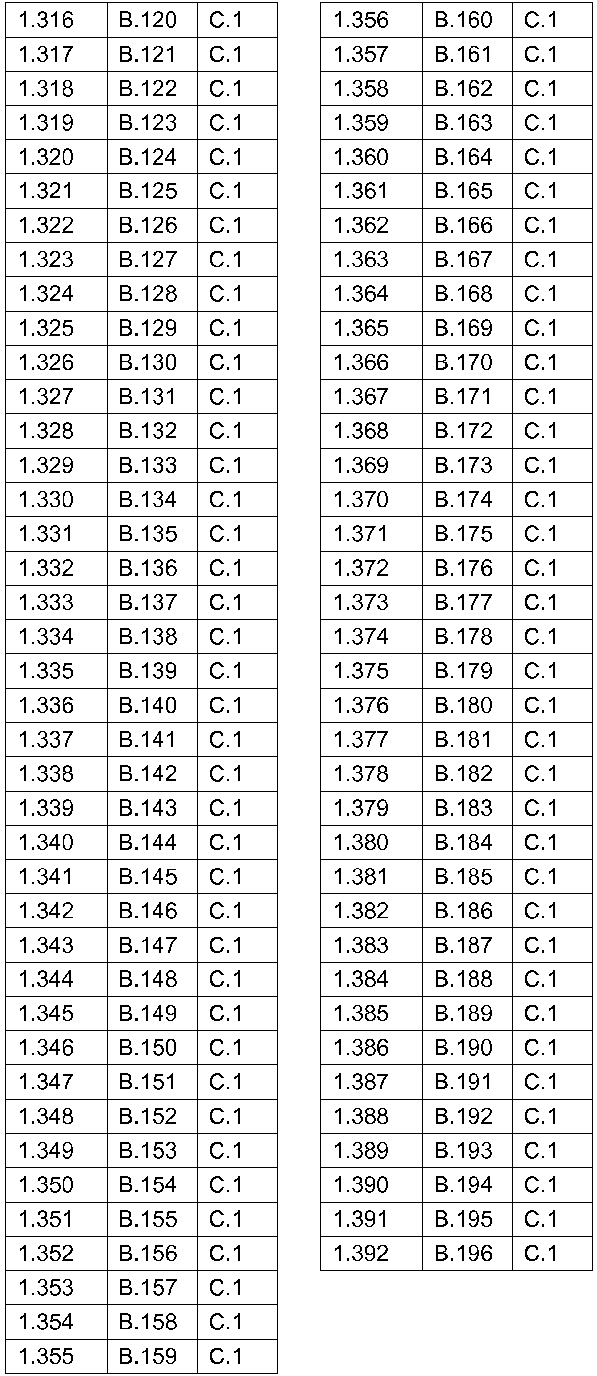

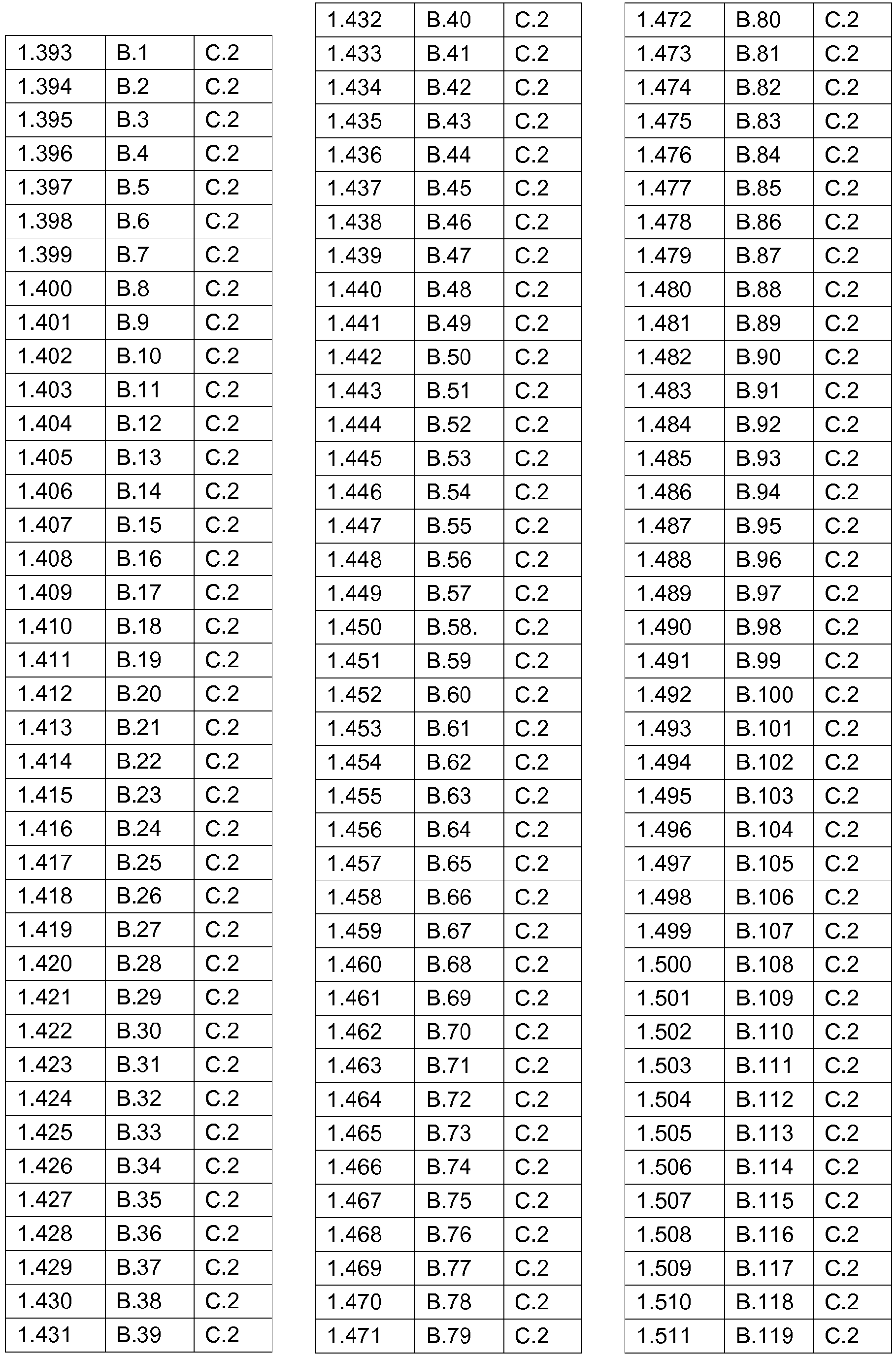

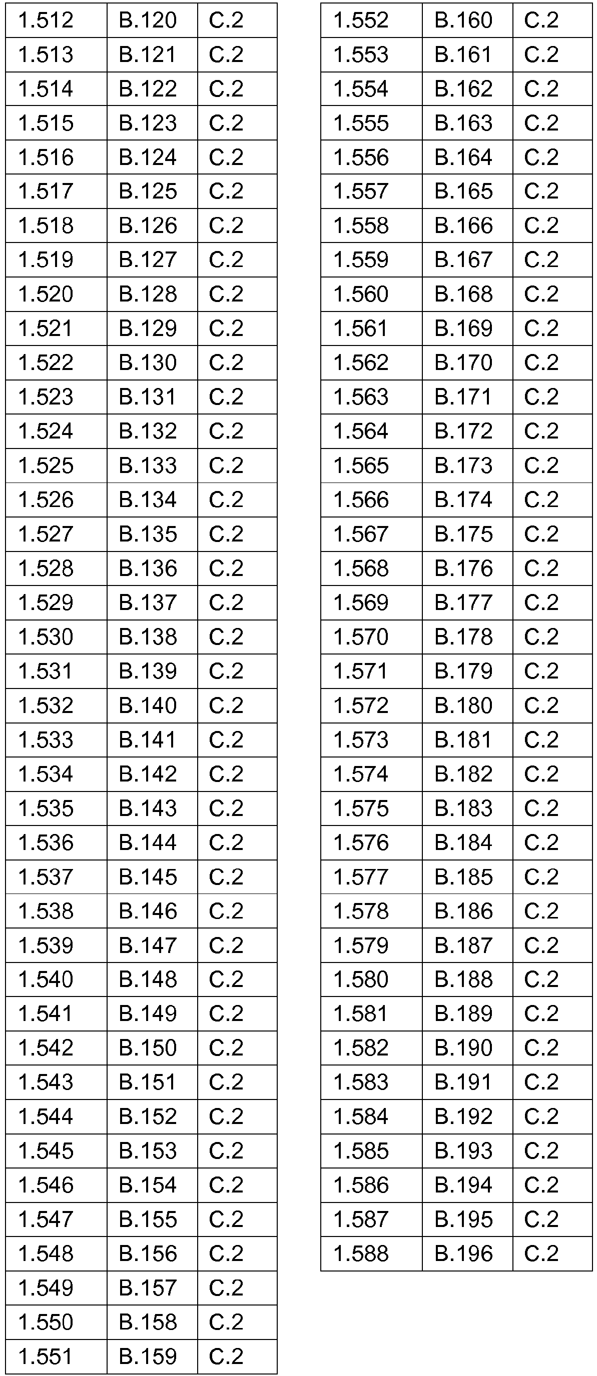

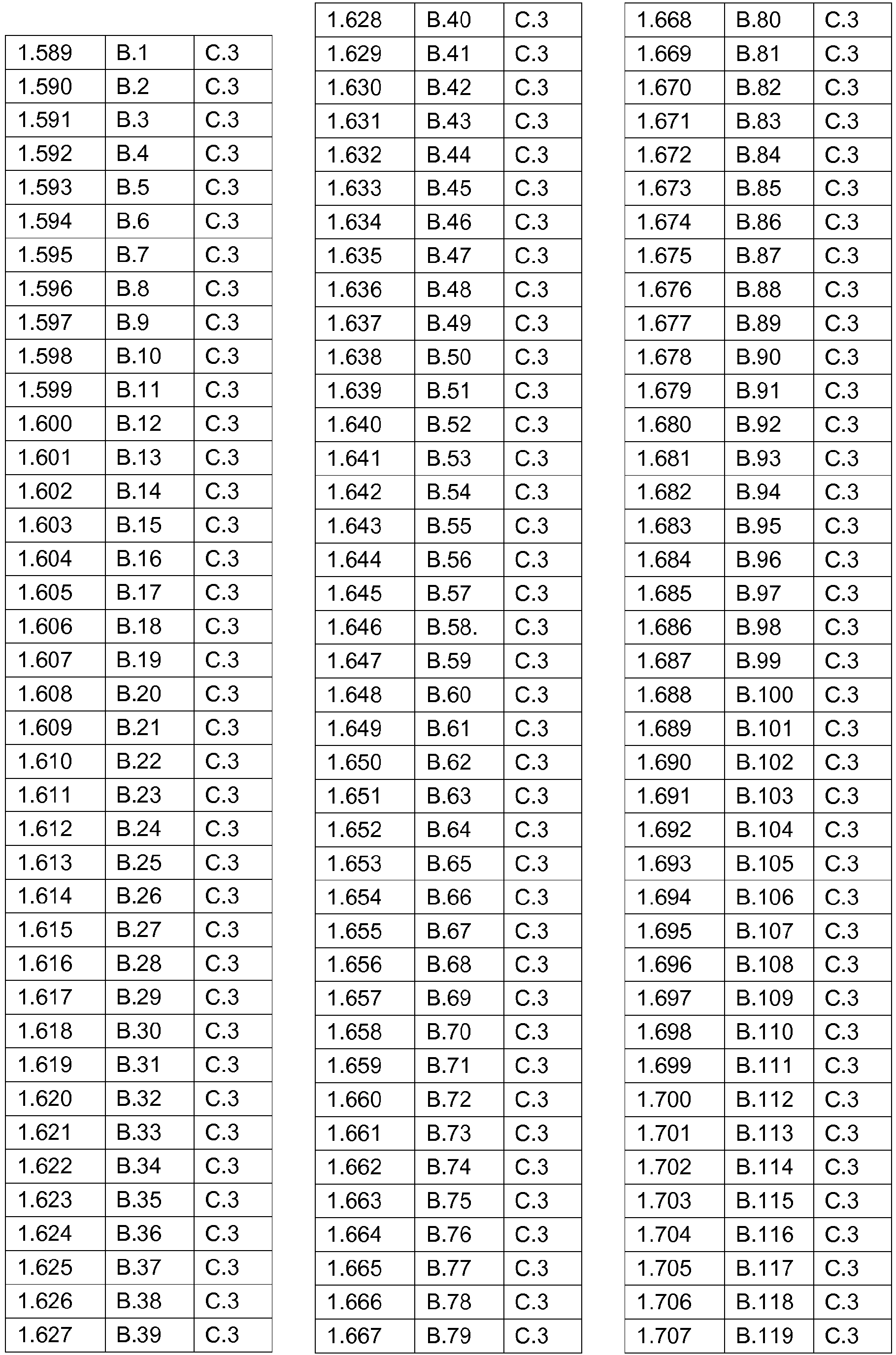

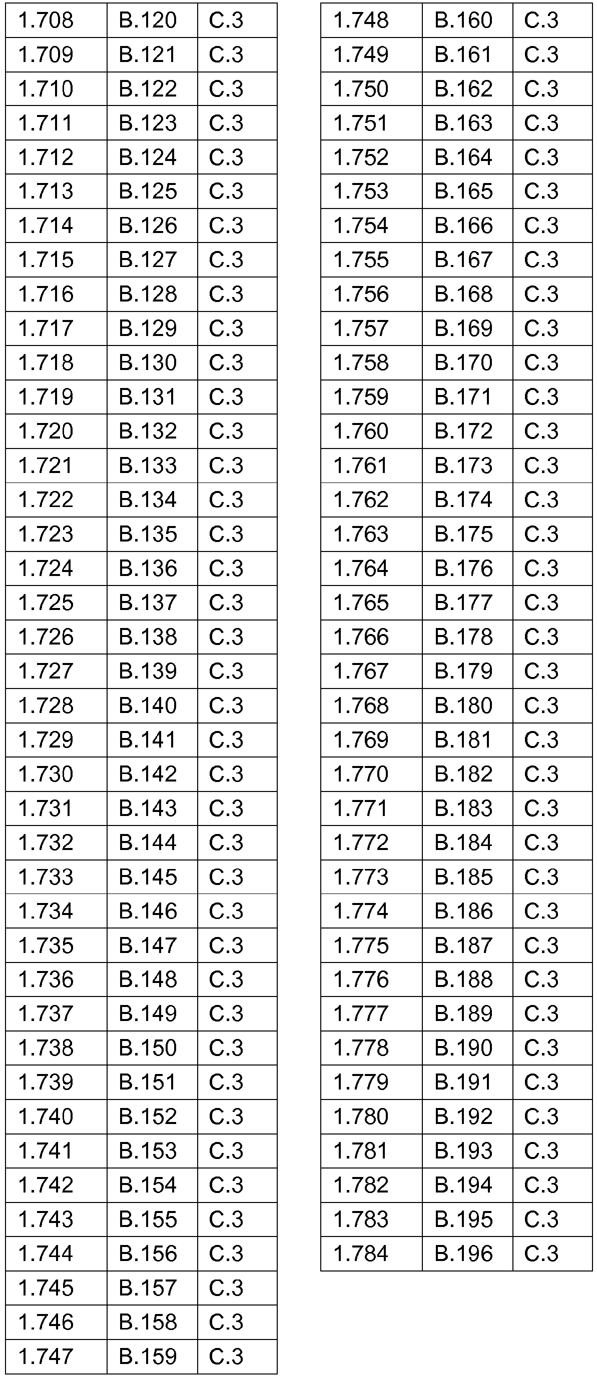

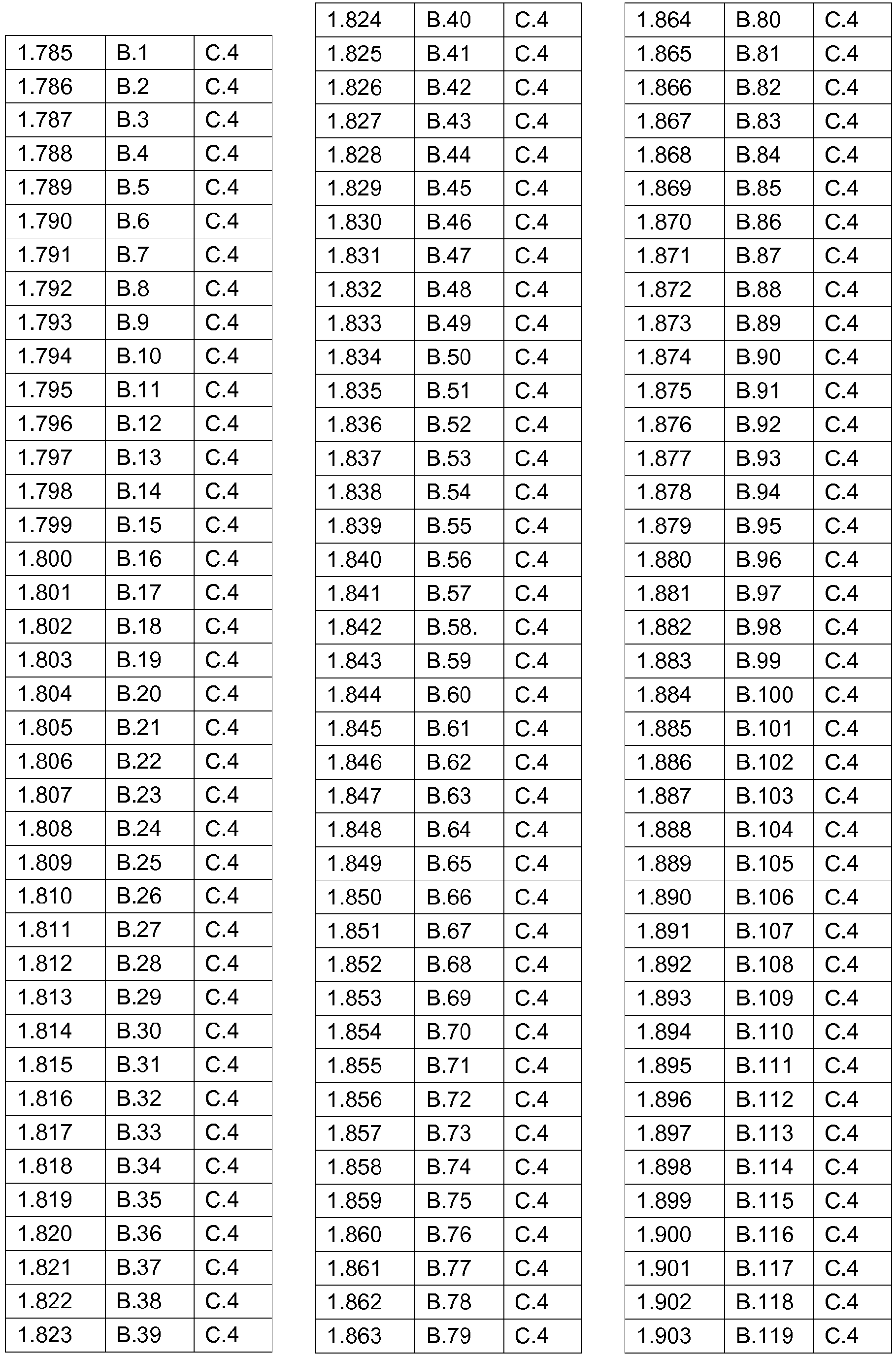

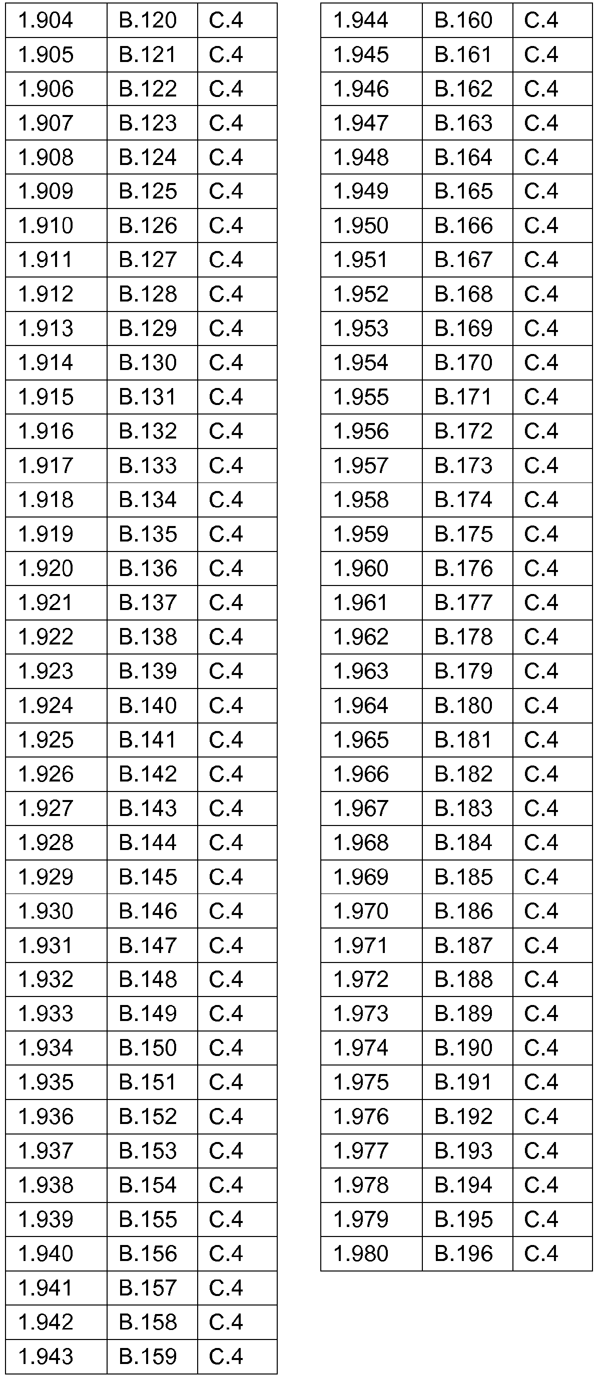

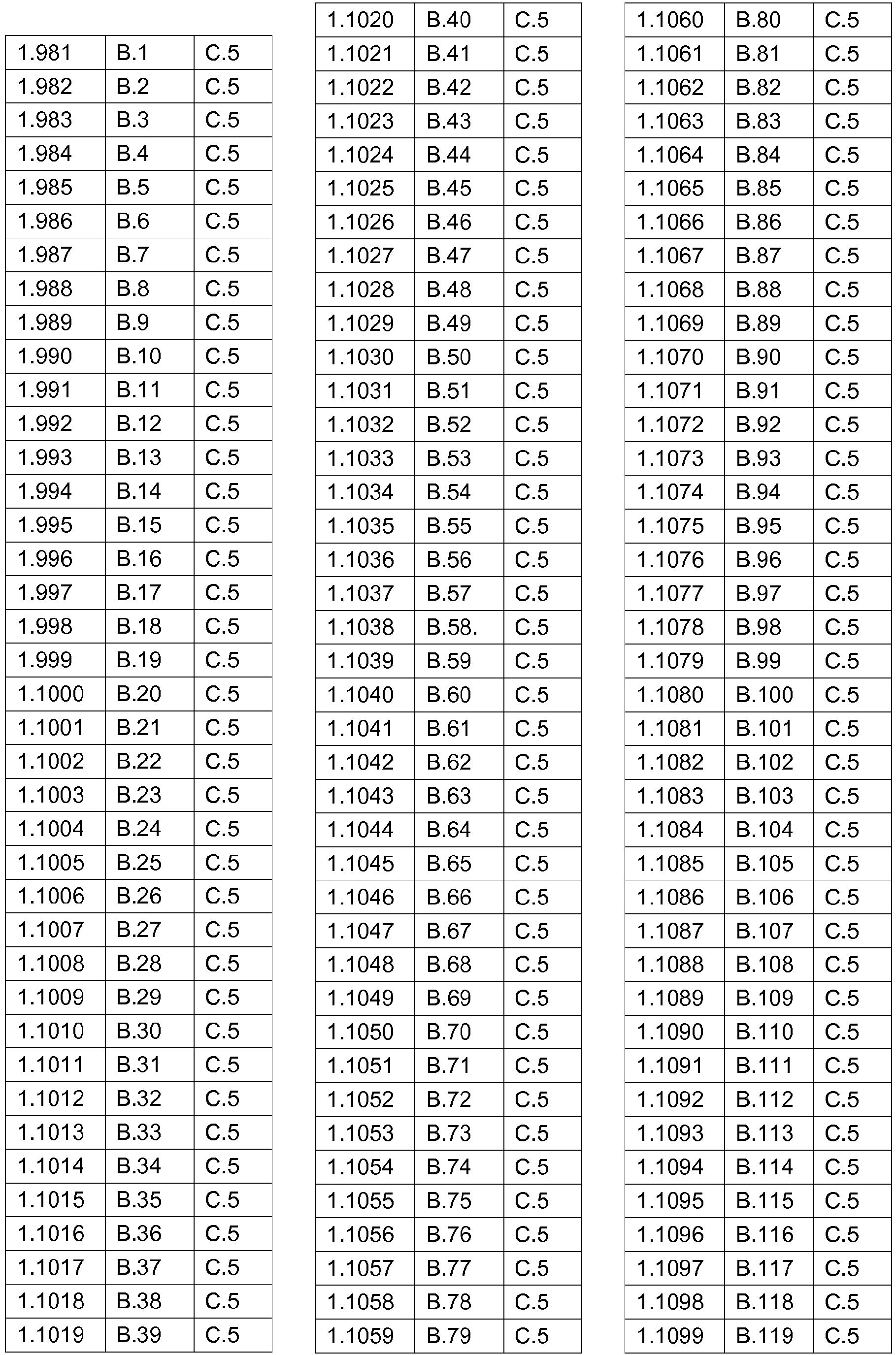

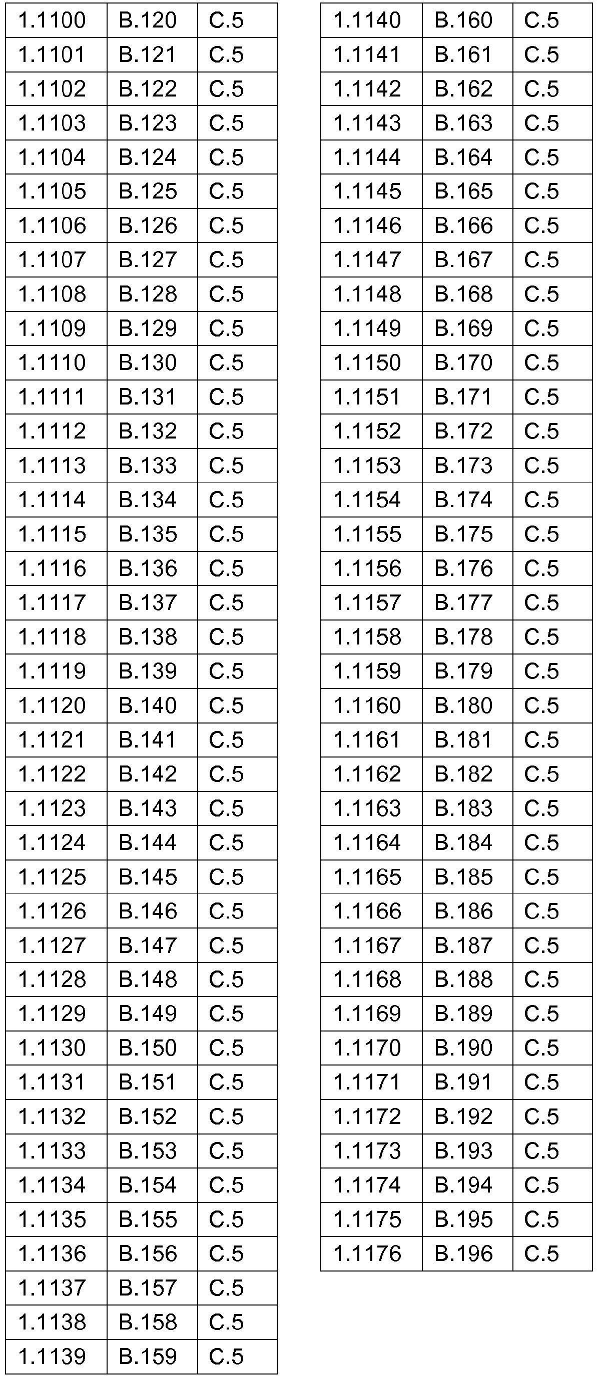

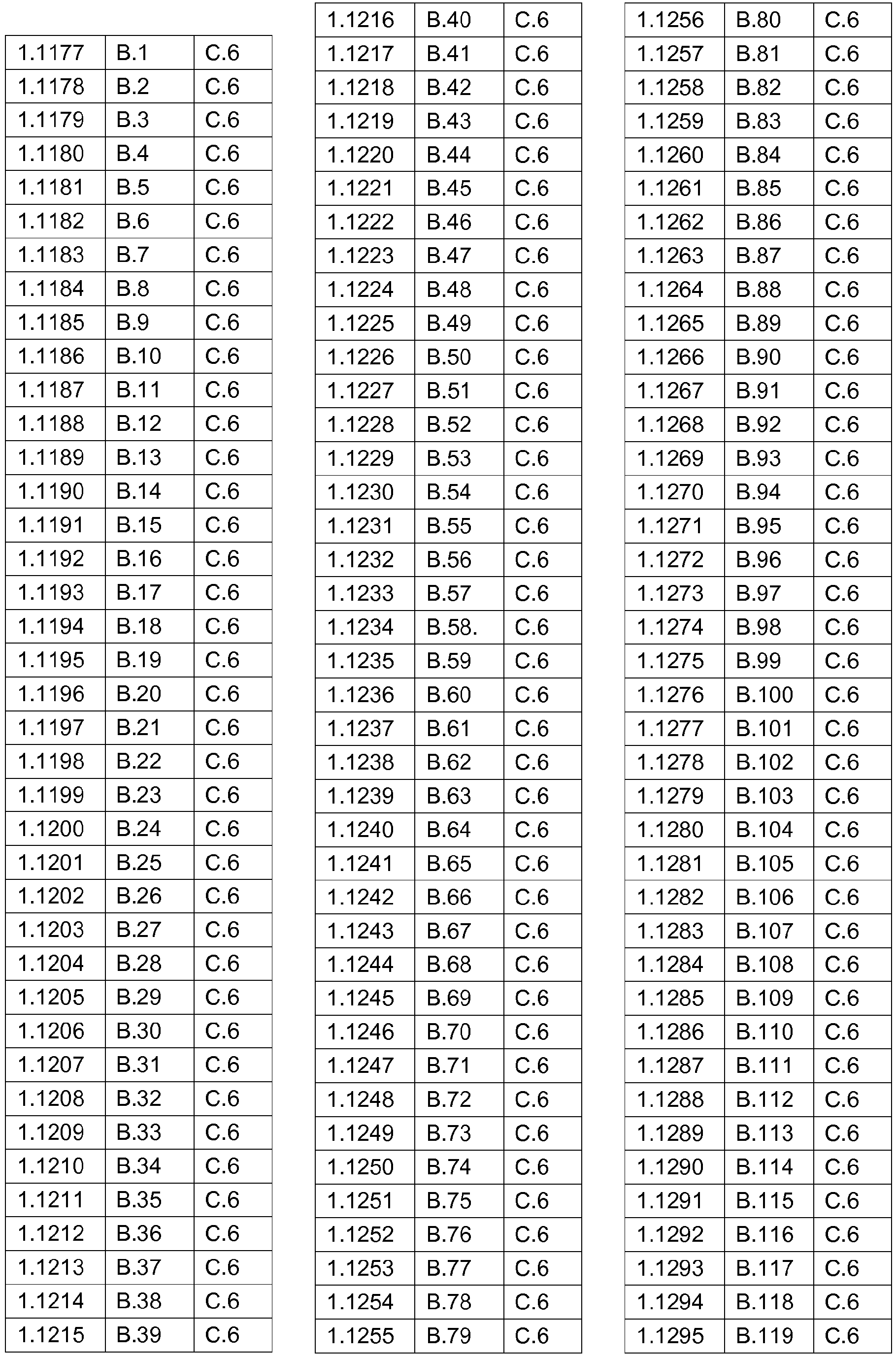

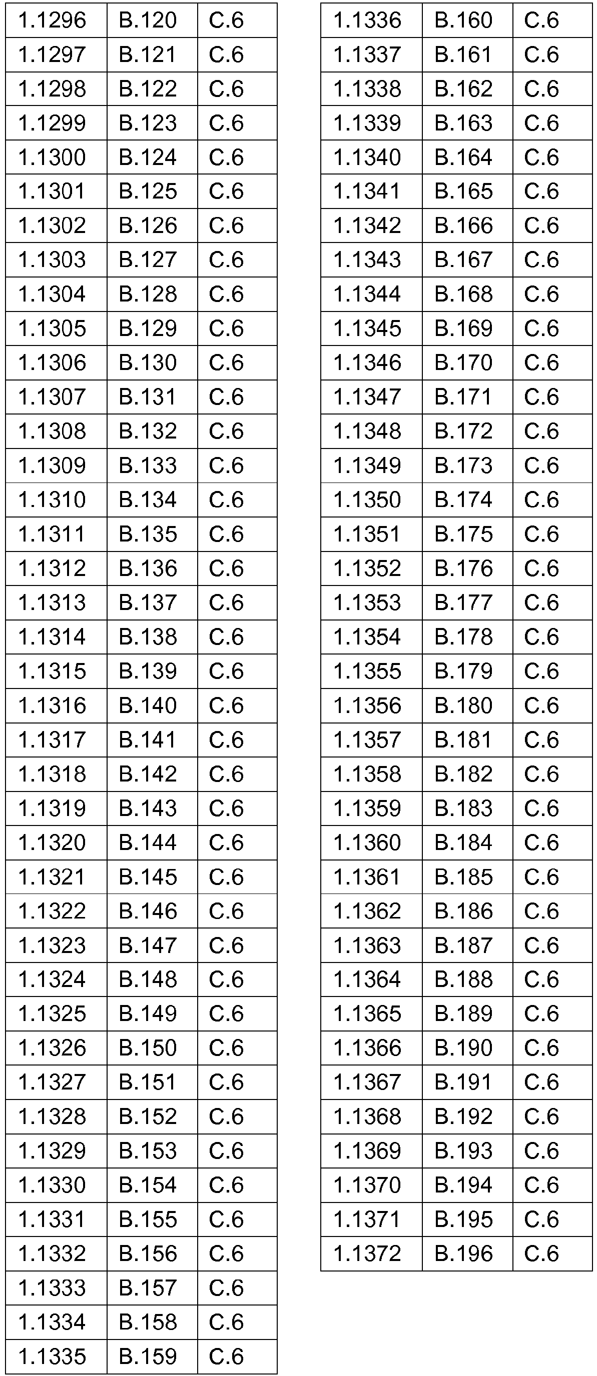

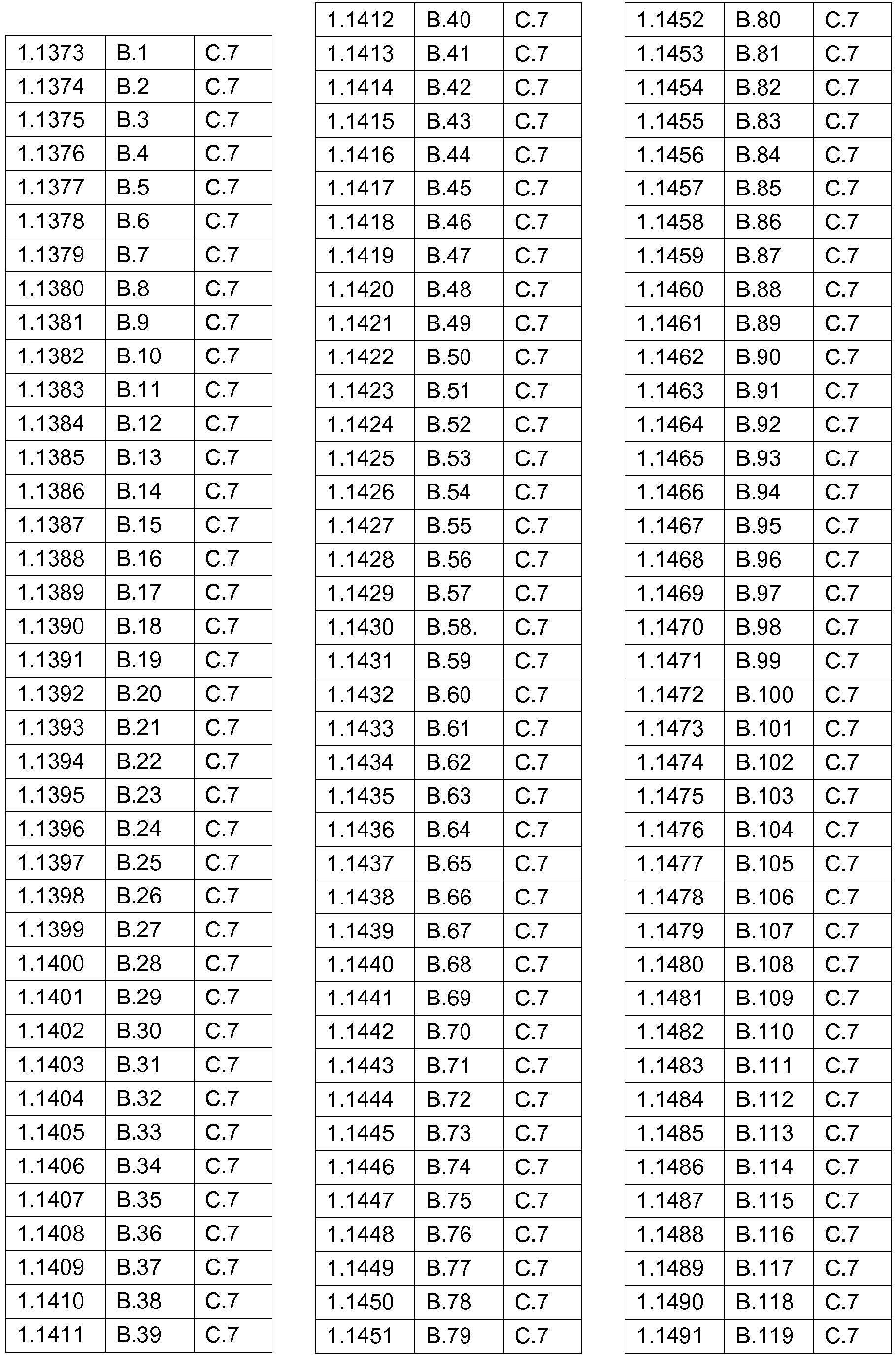

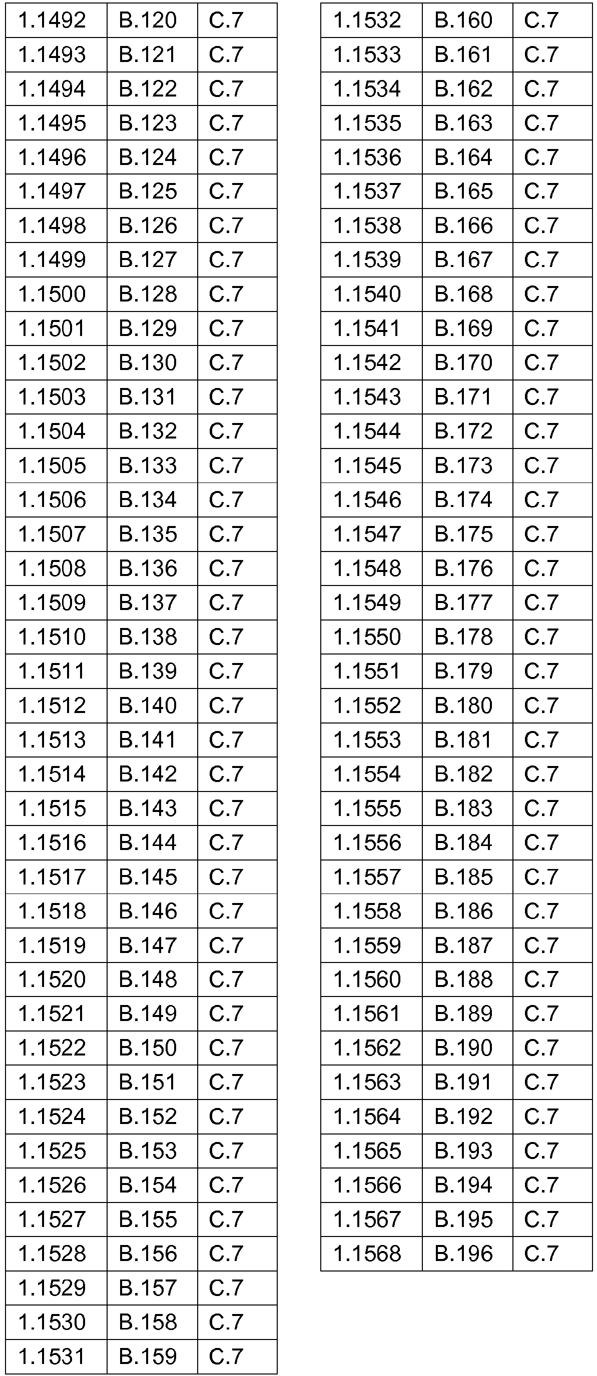

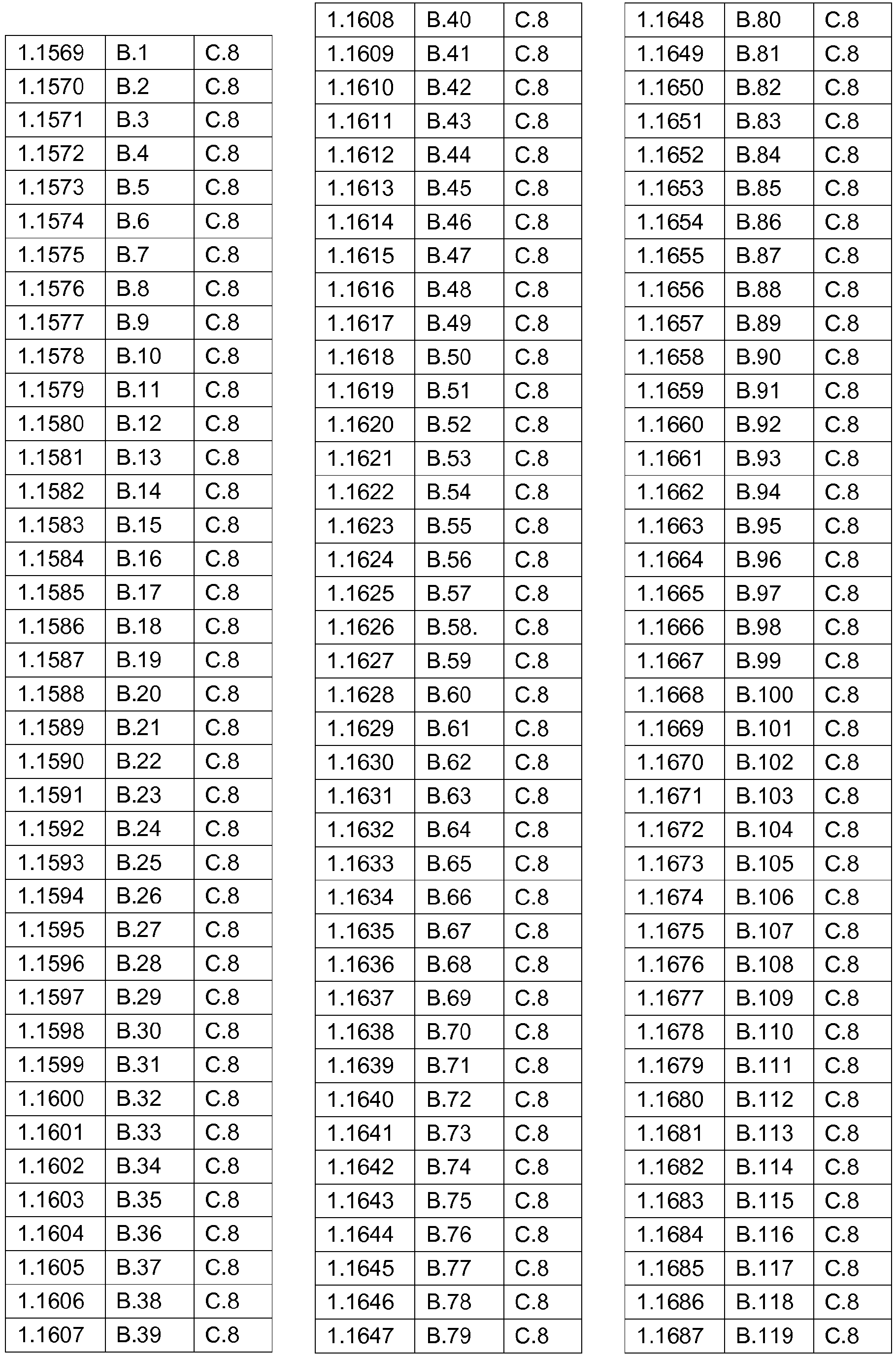

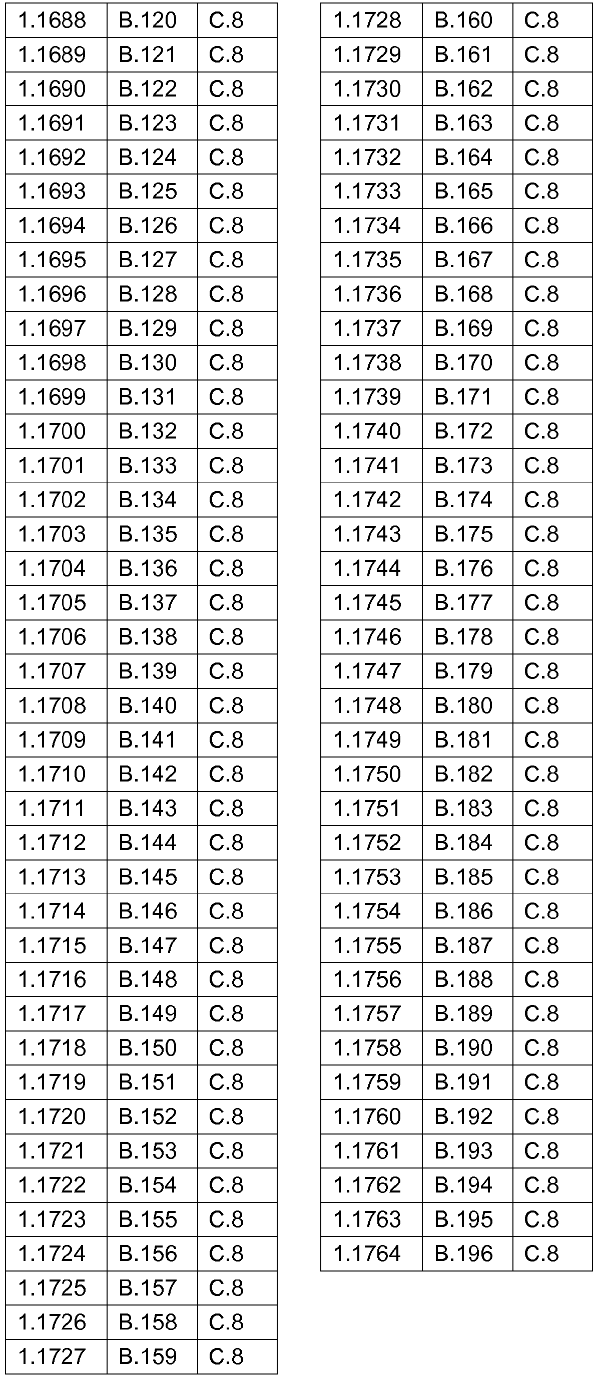

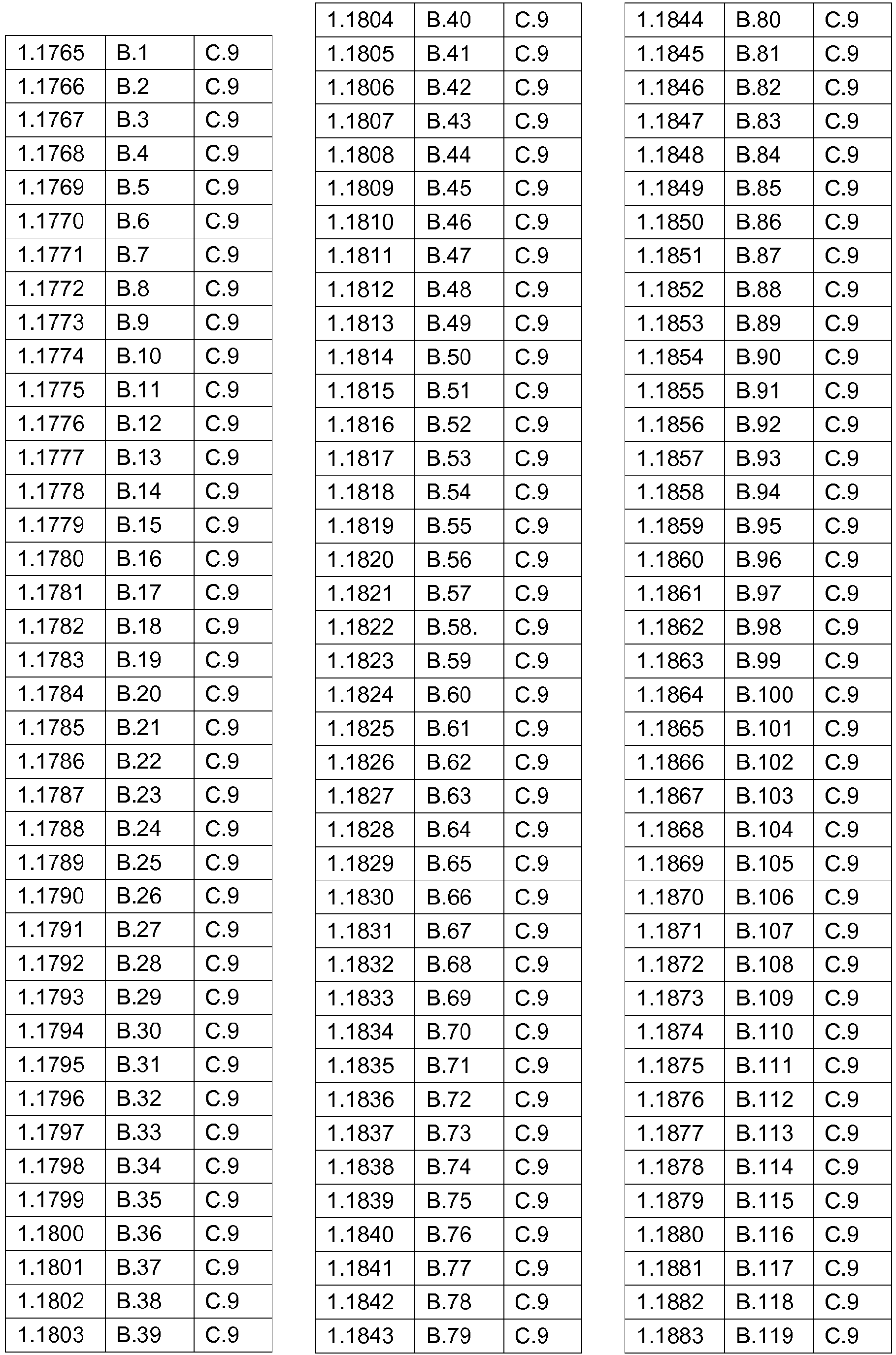

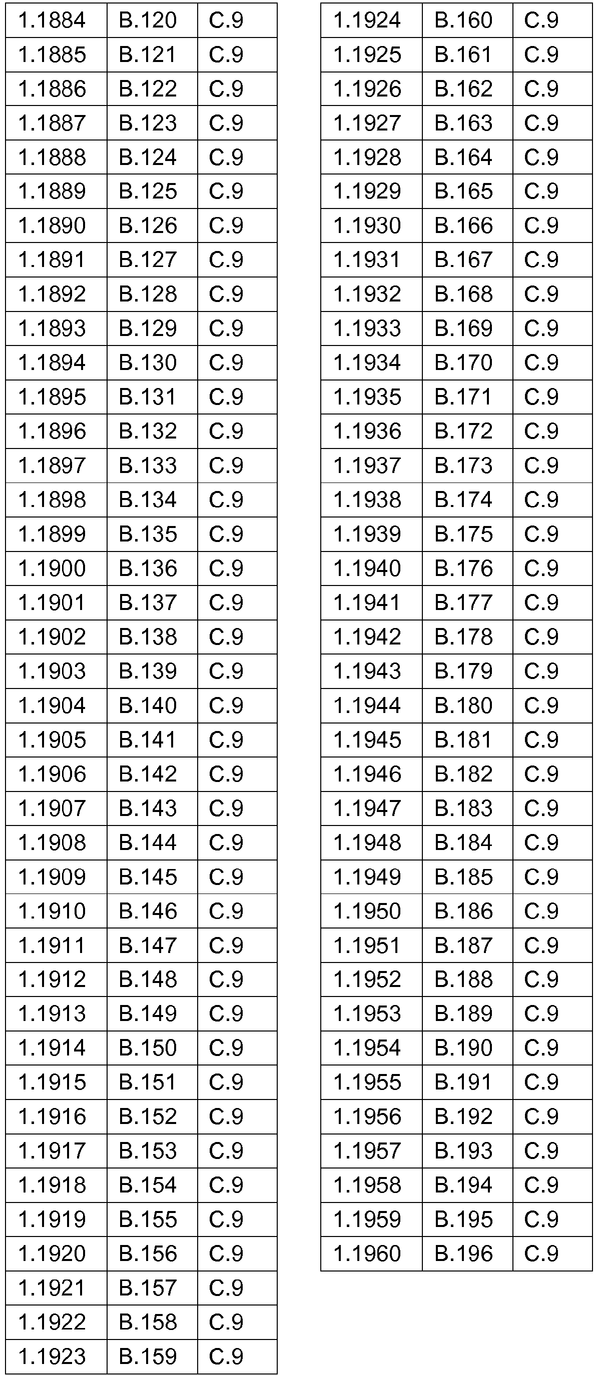

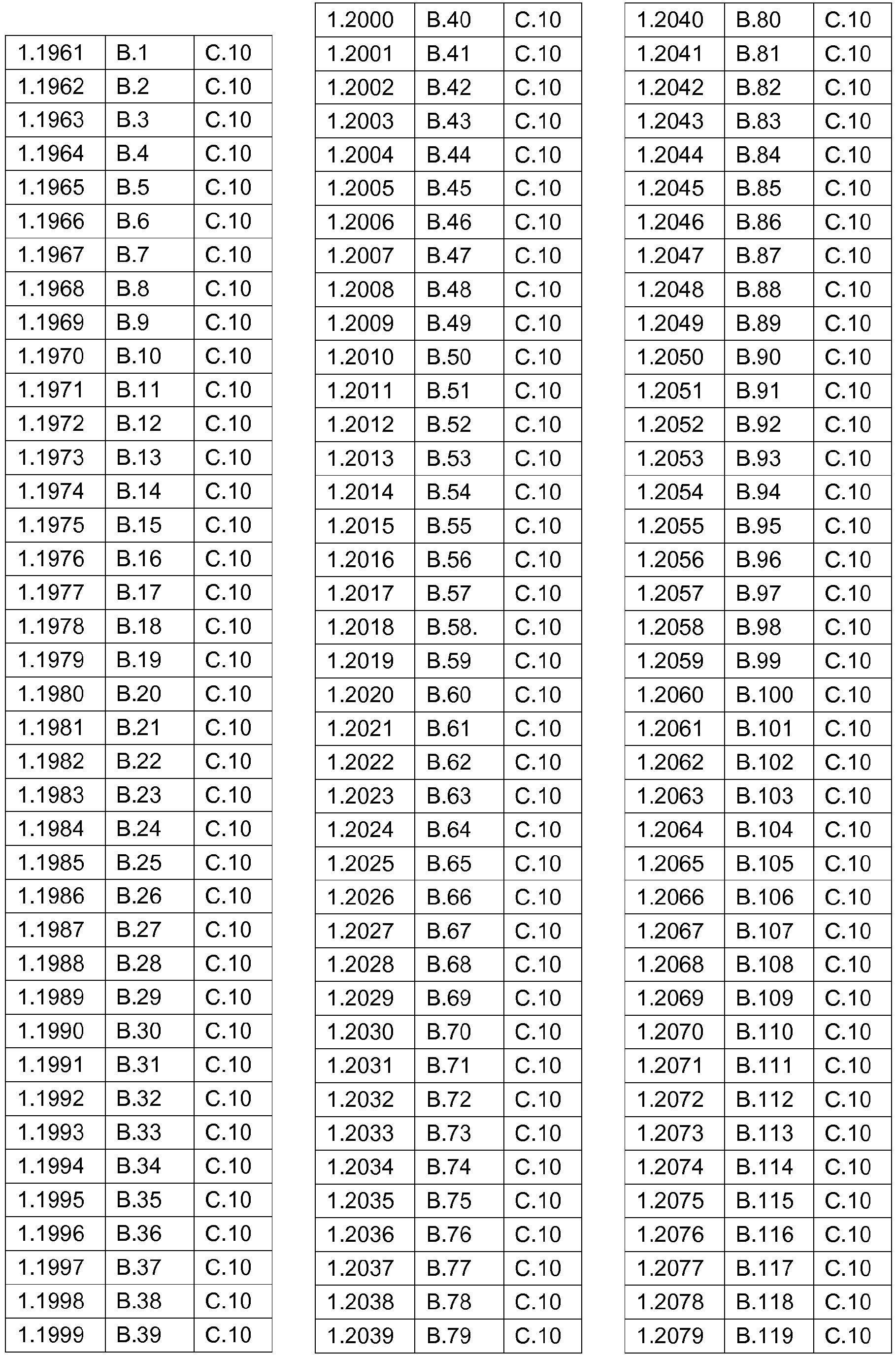

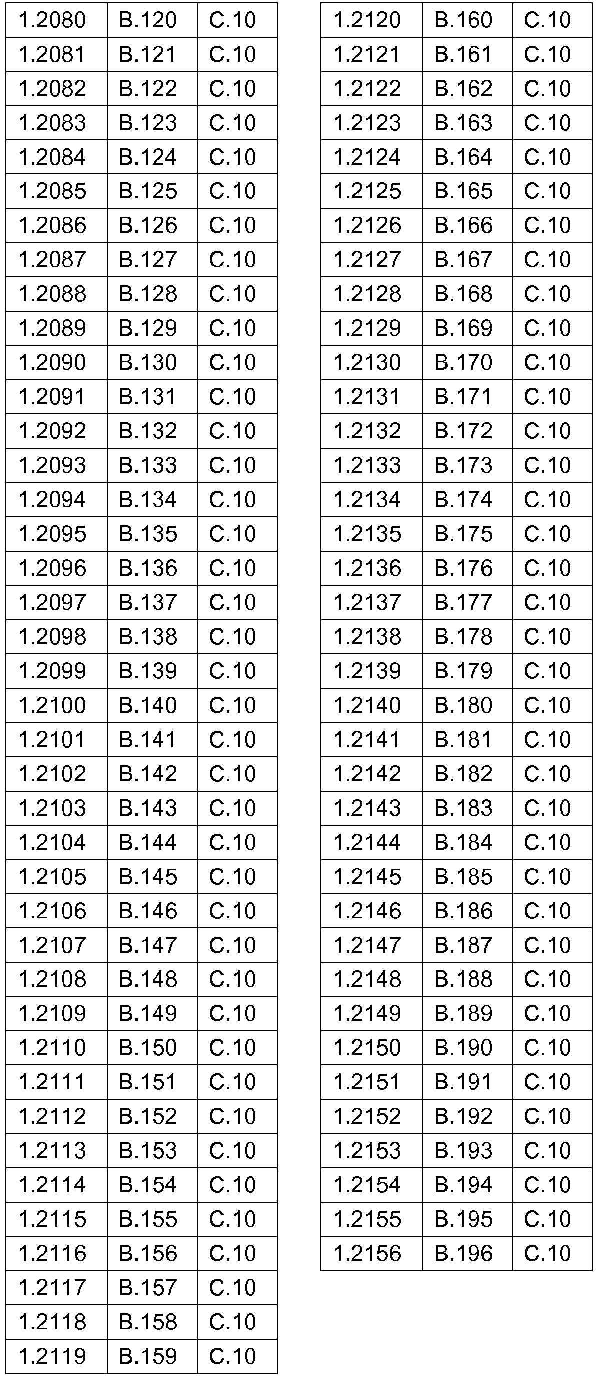

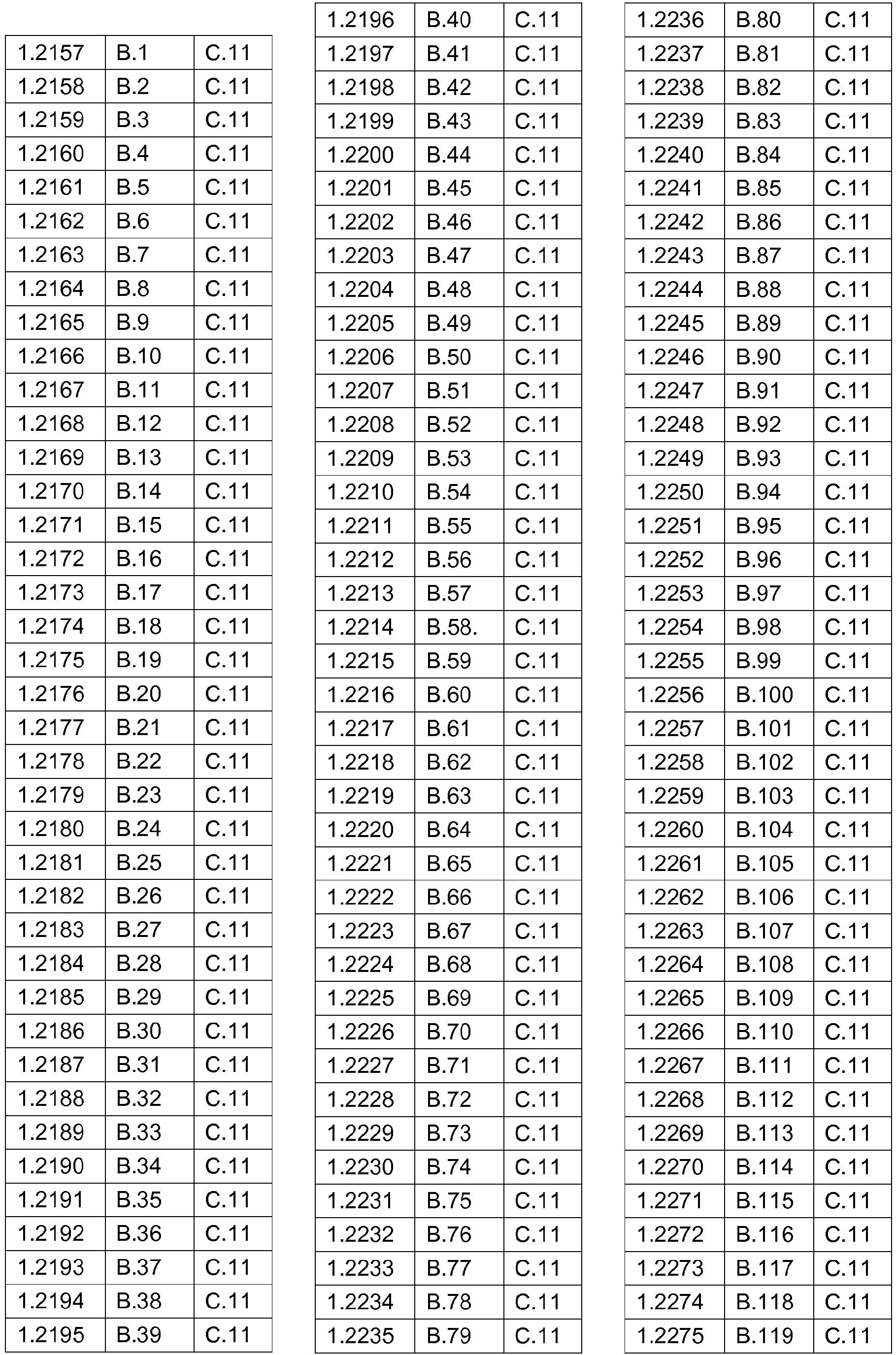

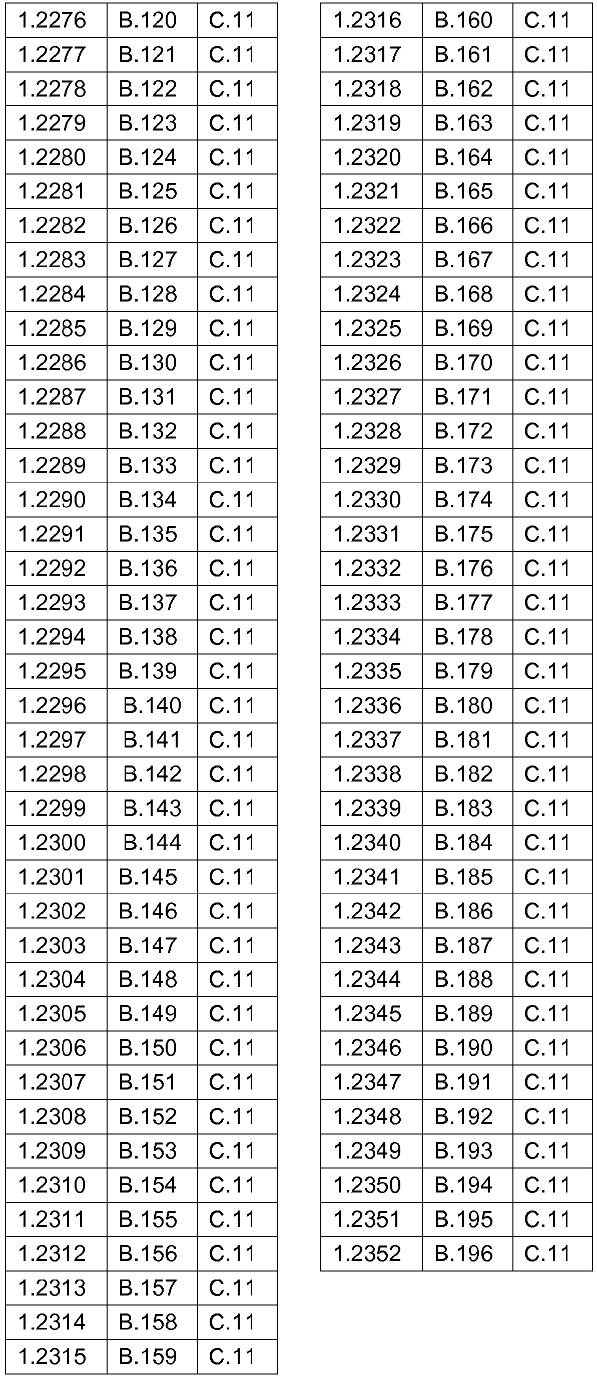

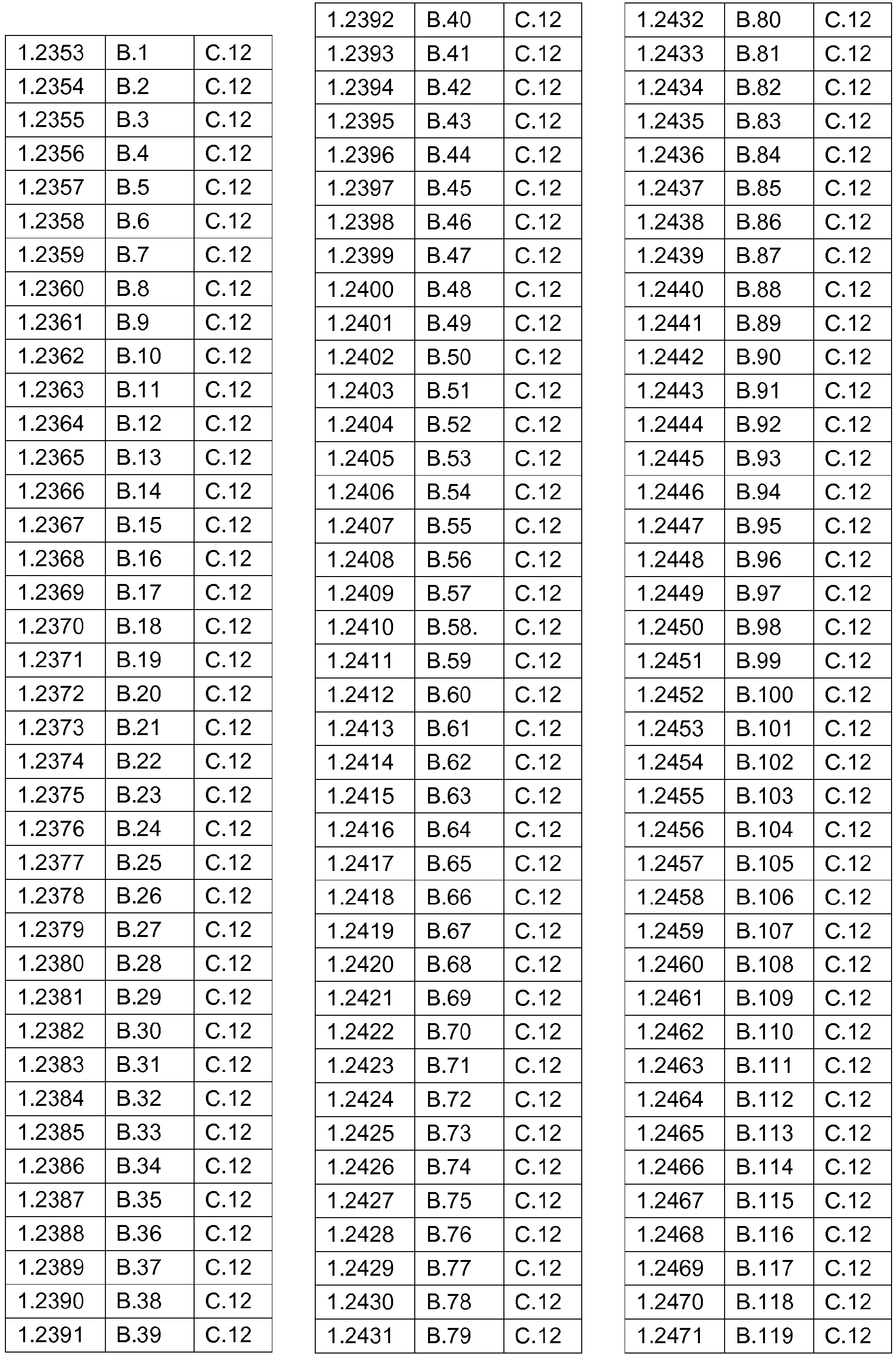

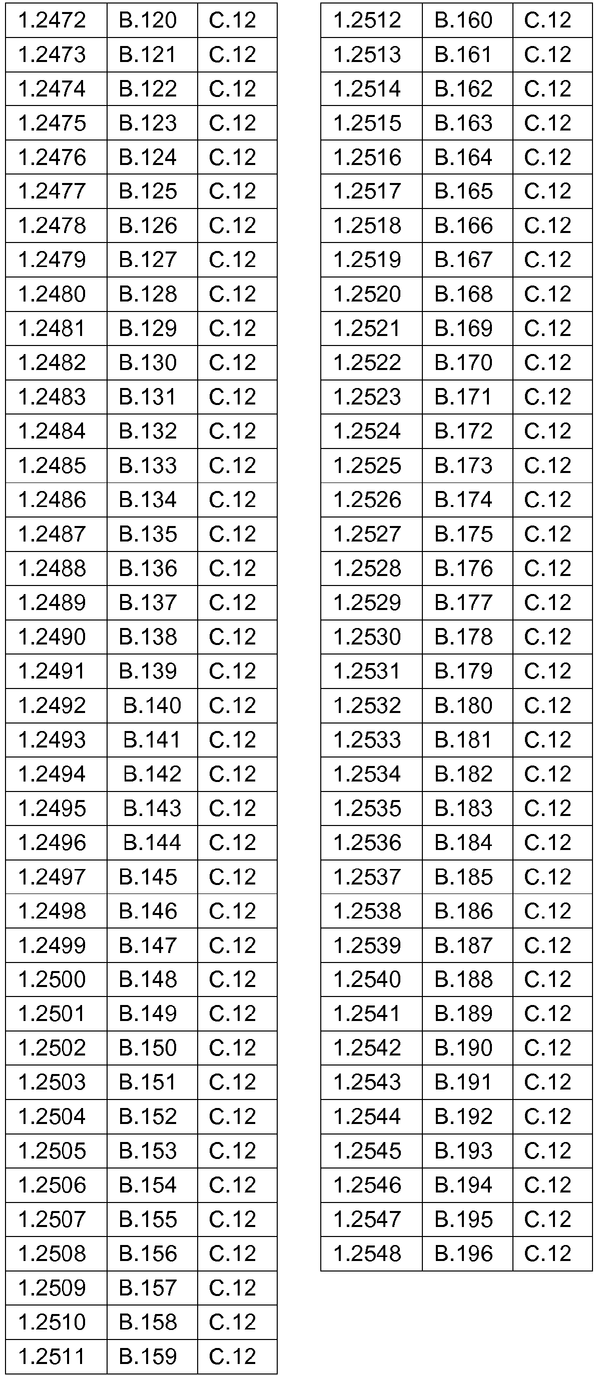

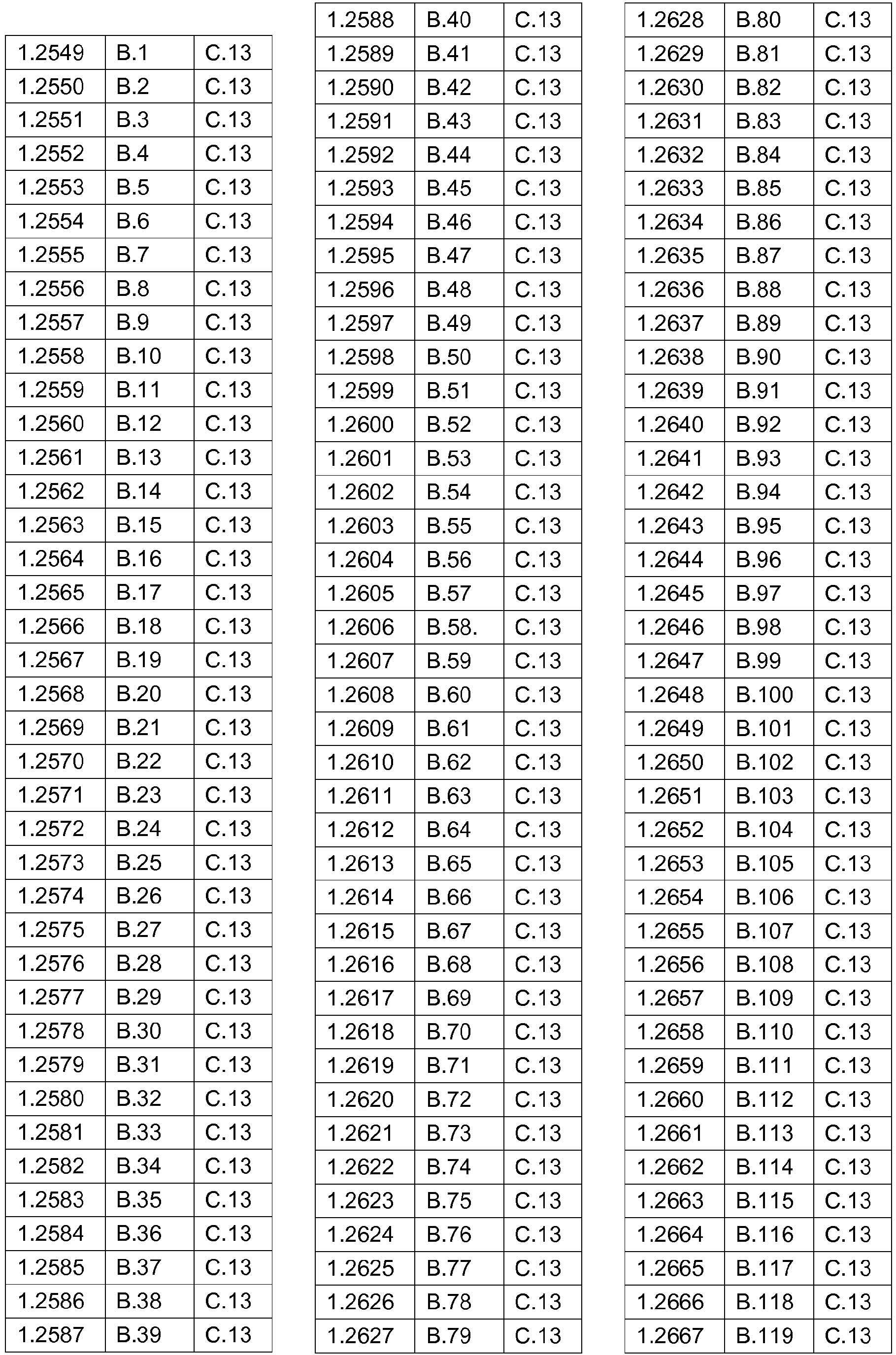

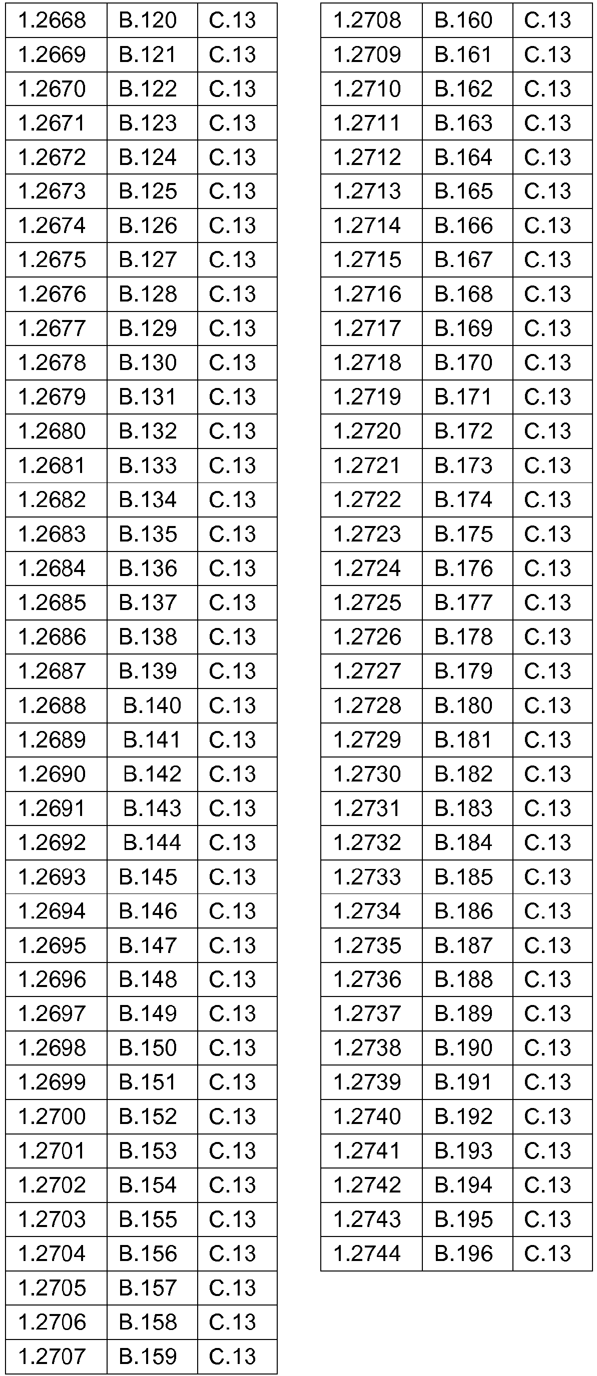

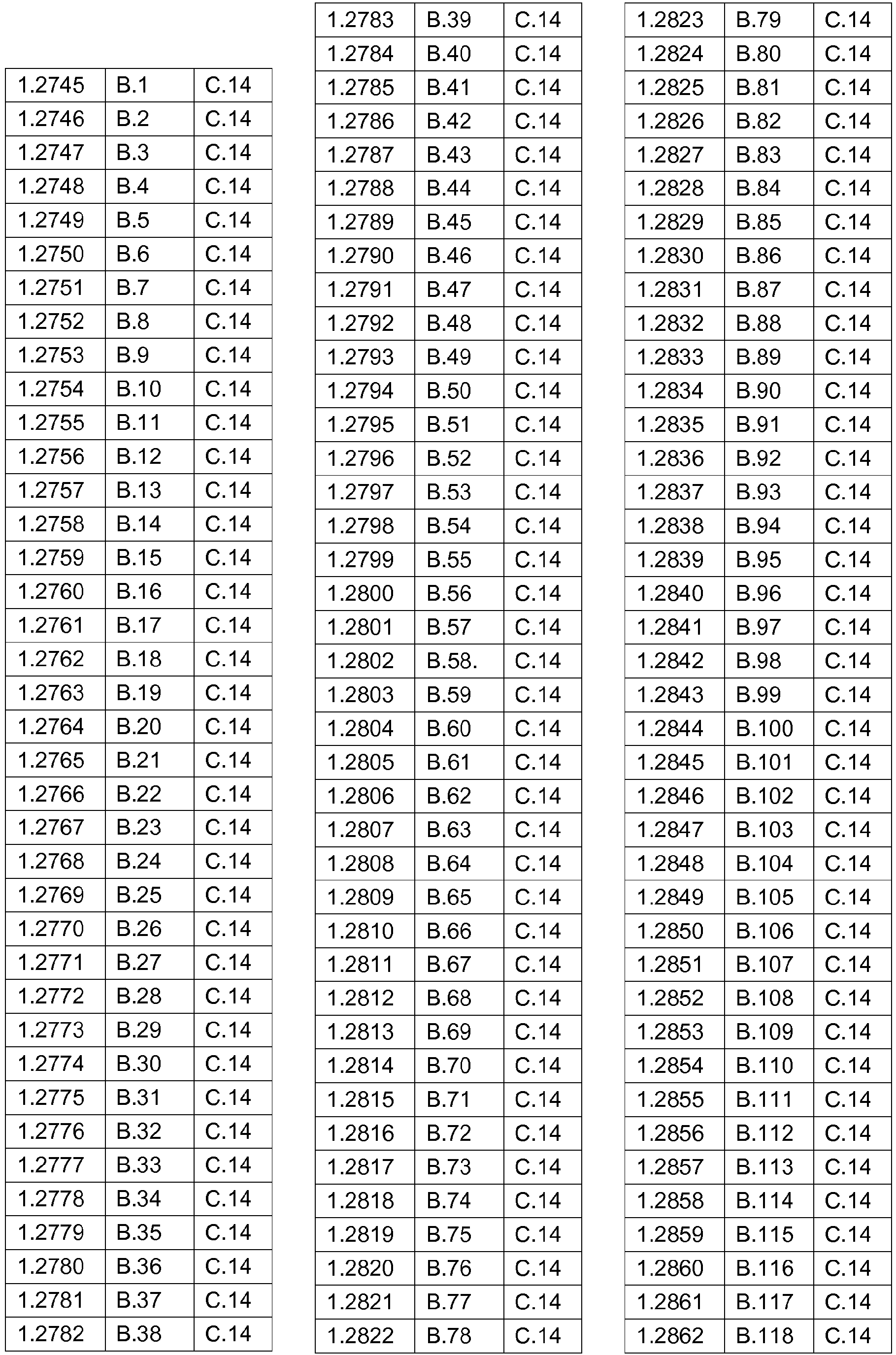

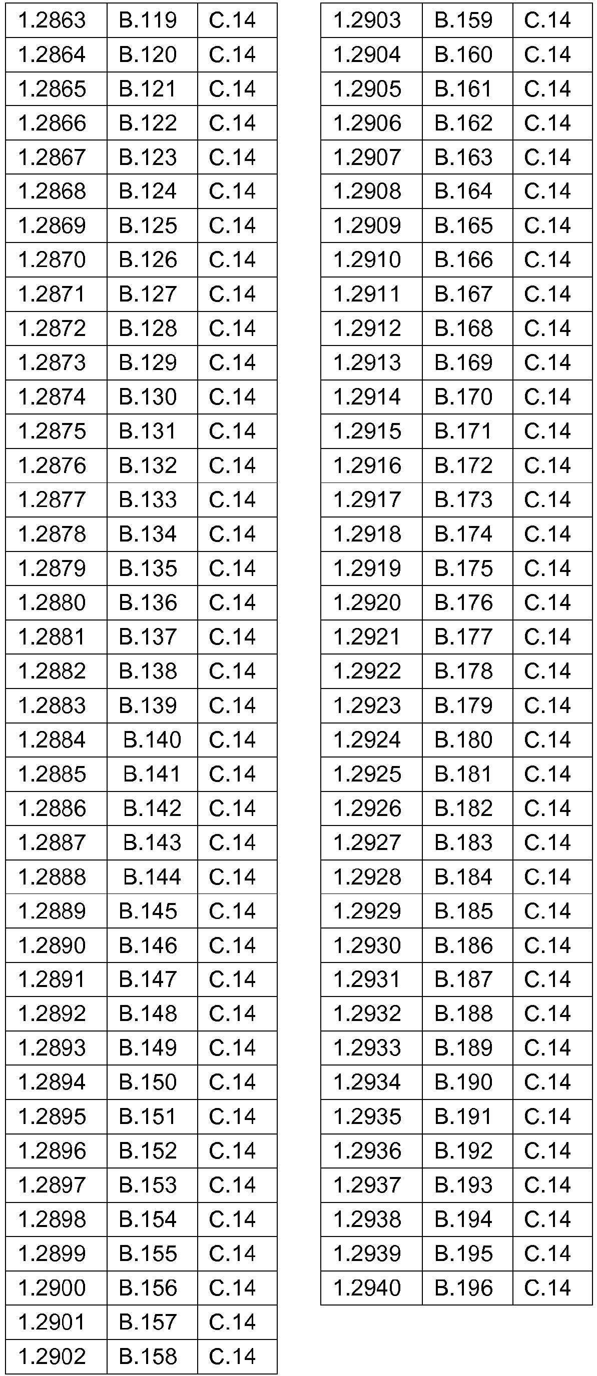

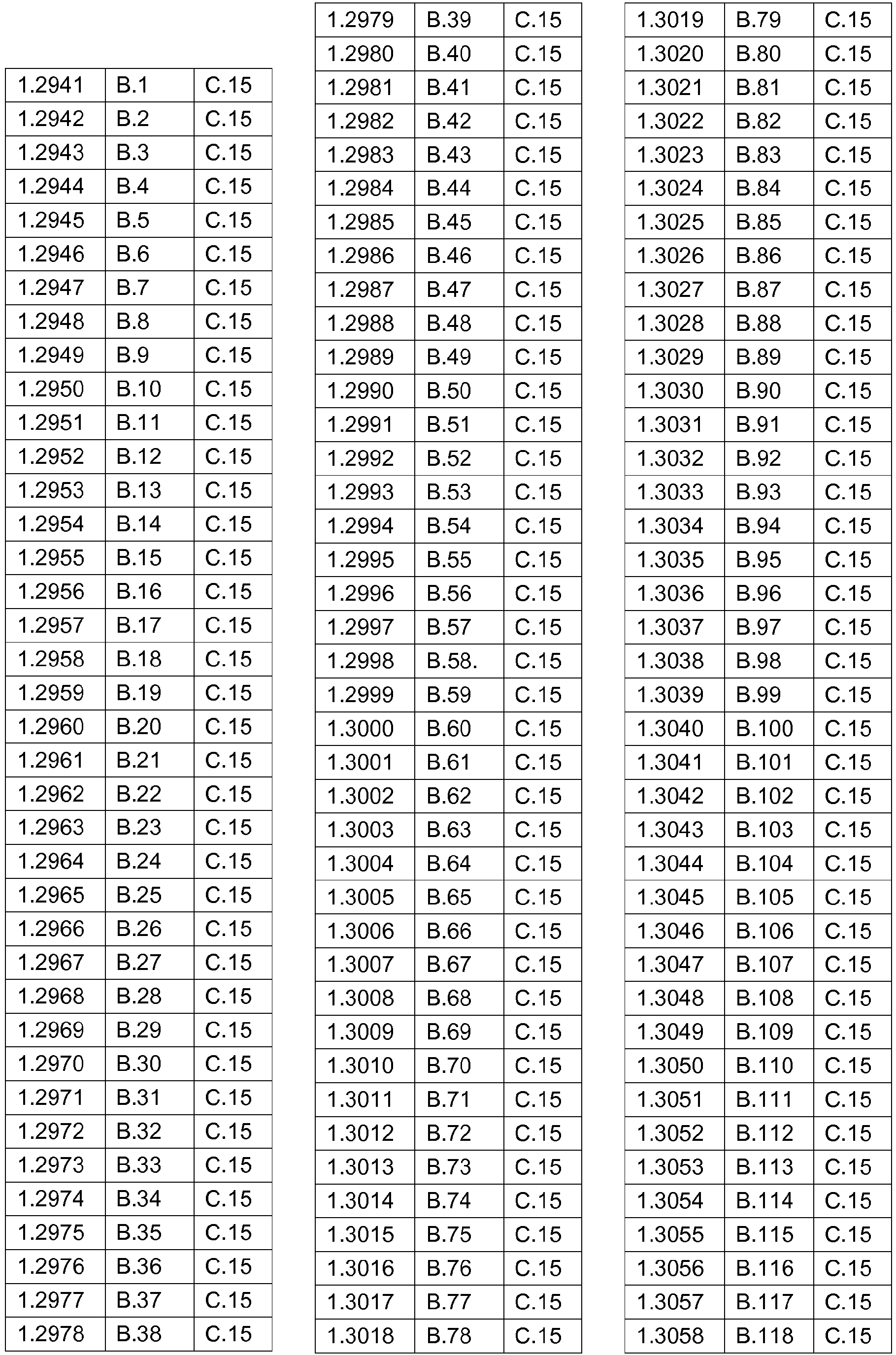

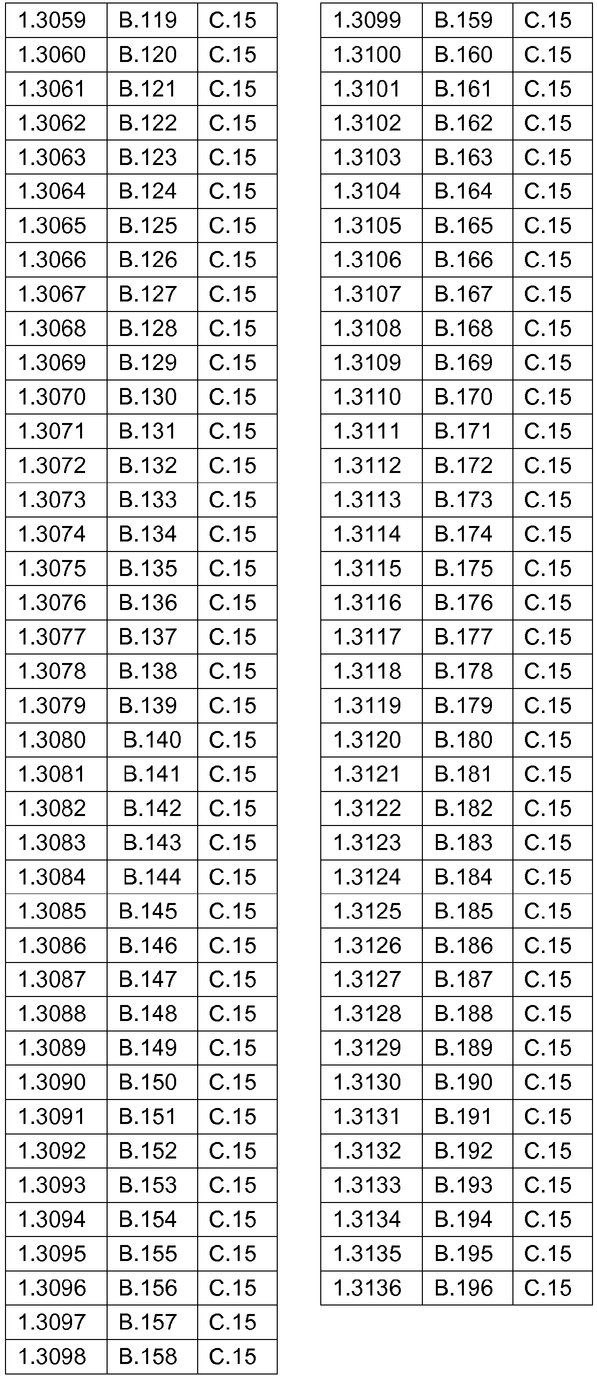

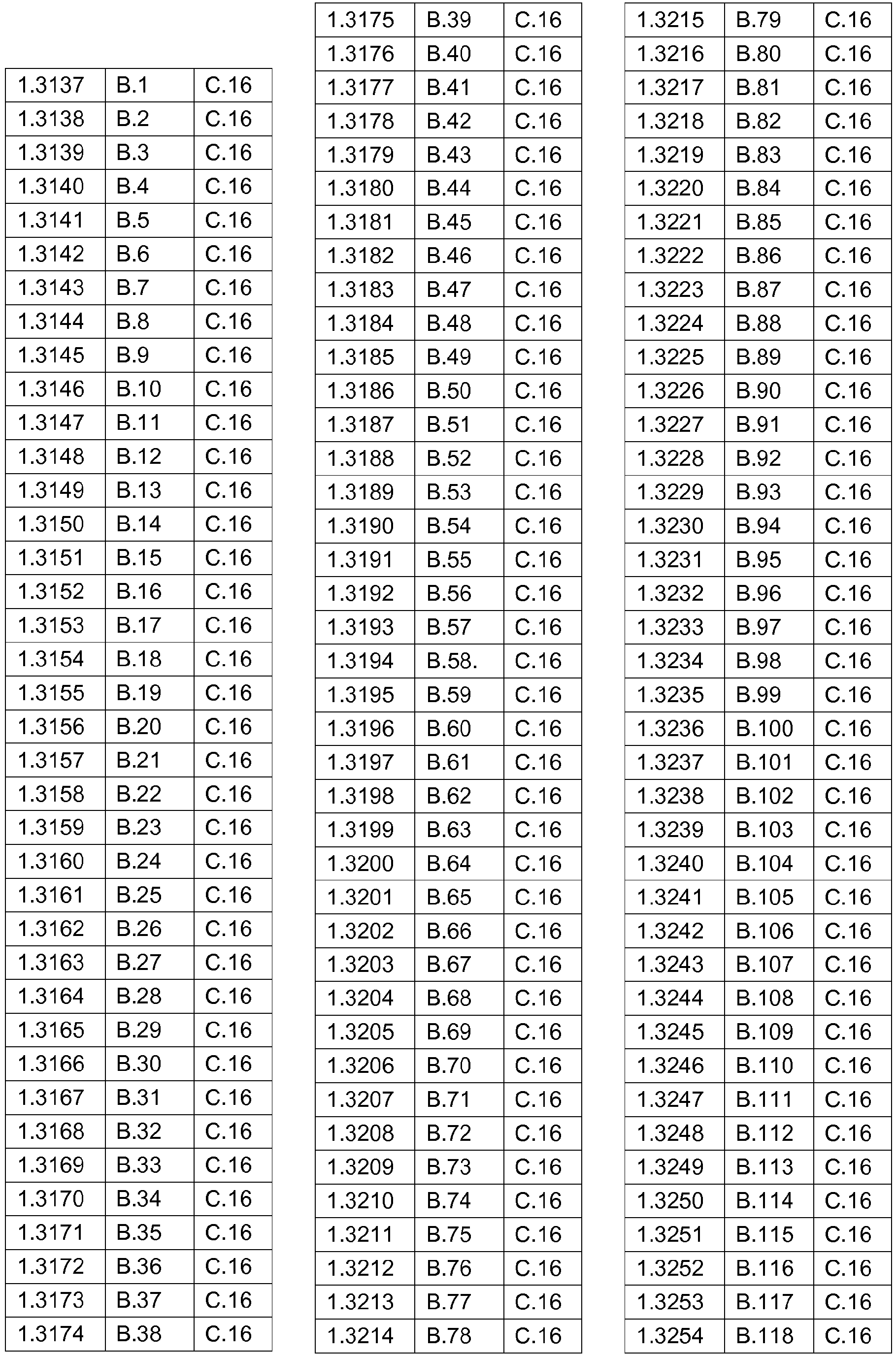

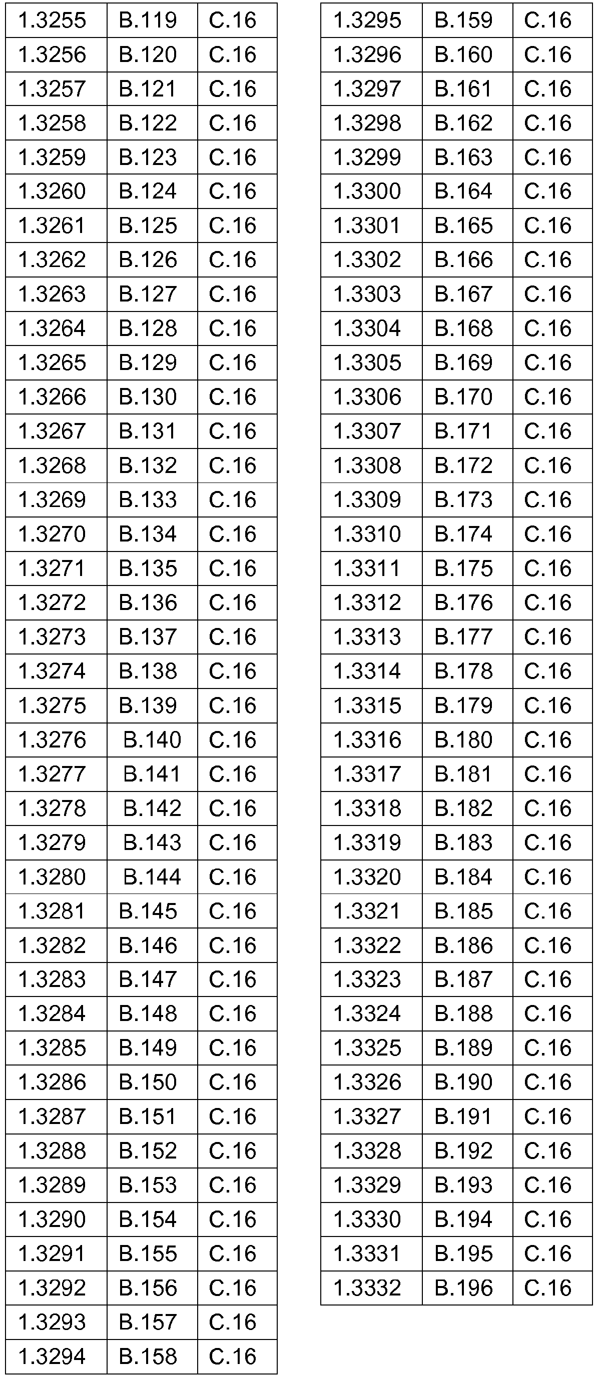

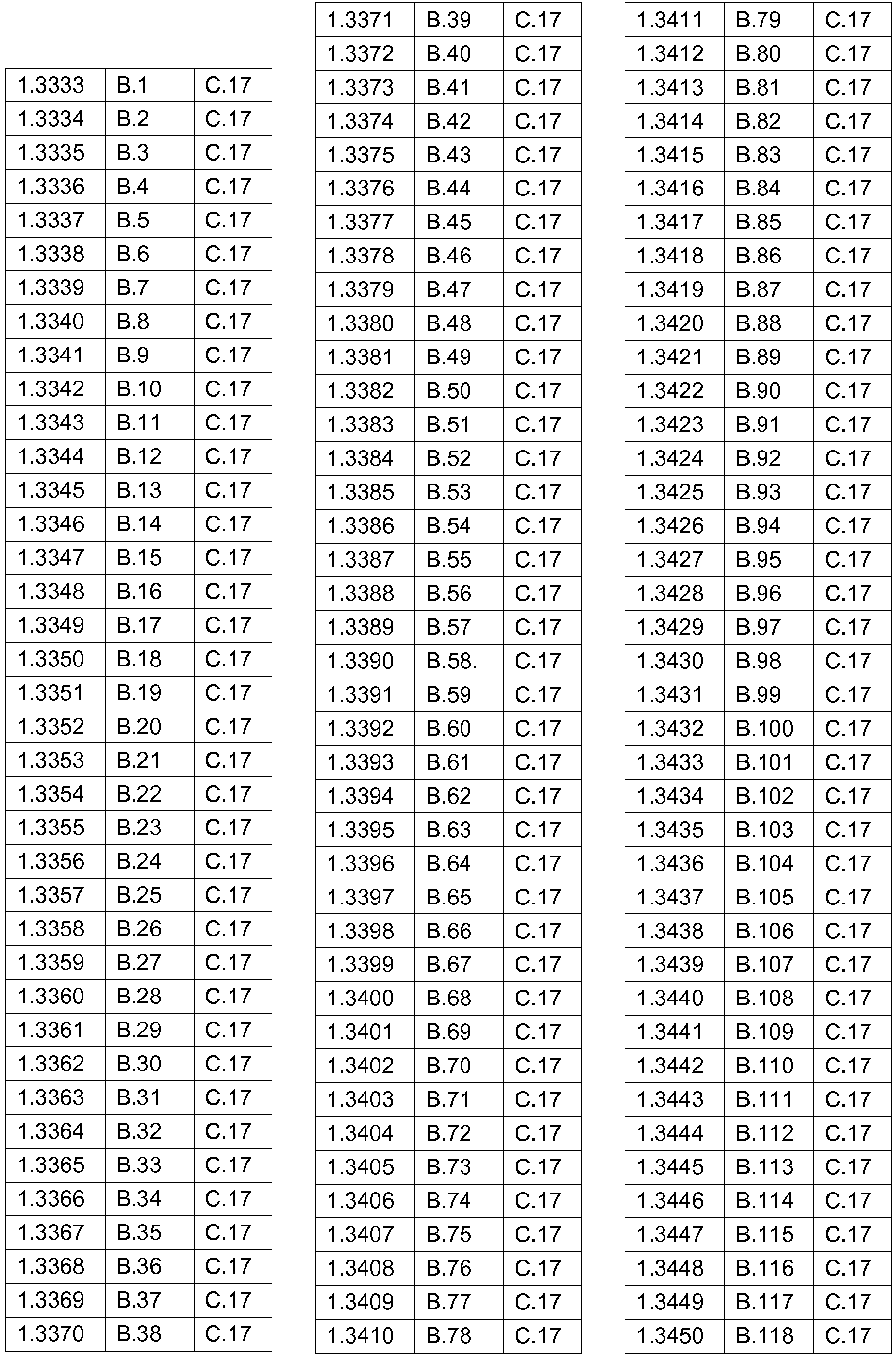

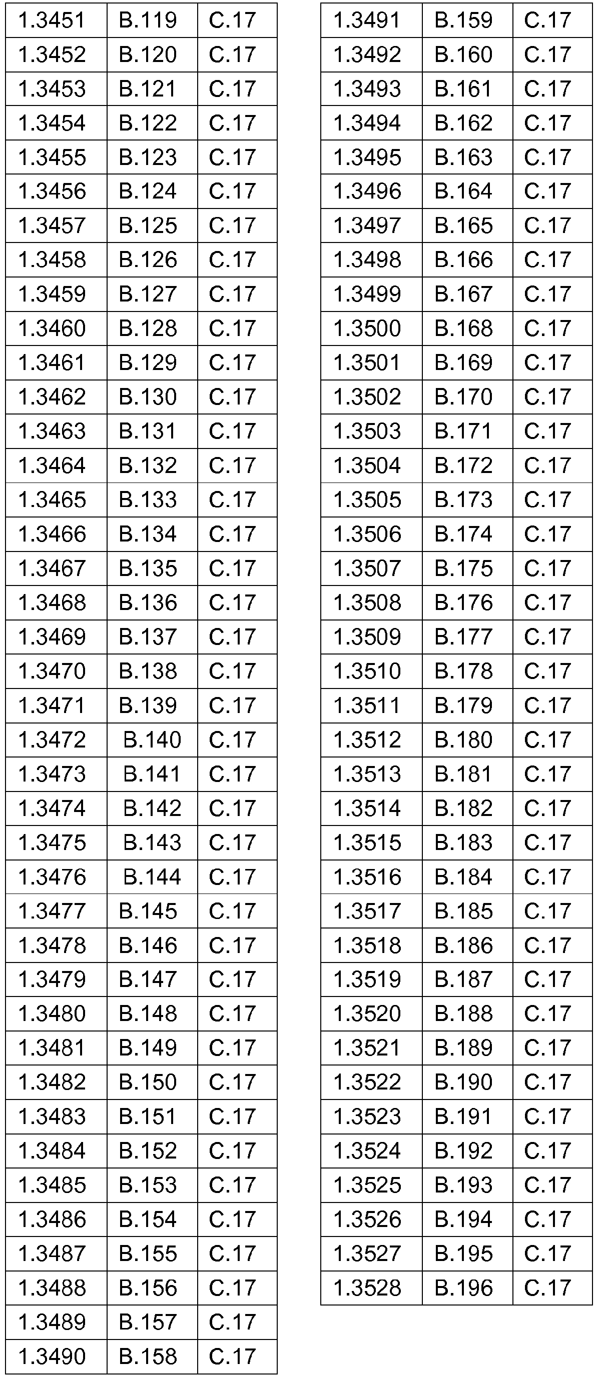

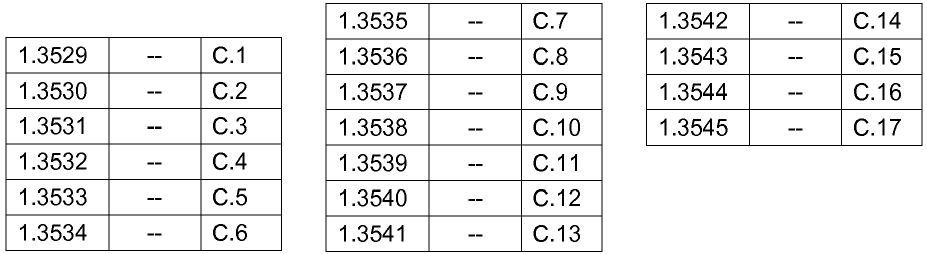

The specific number for each single composition is deductible as follows: Composition 1.200 for example comprises compounds of formula (I) cyhalofop-butyl (B.4) and benoxacor (C.1).

Composition 2.200 for example comprises the compounds of formula (I) (see the definition for compositions 2.1 to 2.3545 below), cyhalofop-butyl (B.4) and benoxacor (C.1).

Composition 7.200 for example comprises compounds of formula (I) imazapyr (B.35), cyhalofop-butyl (B.4) and benoxacor (C.1.

Also especially preferred are compositions 2.1. to 2.3545 which differ from the corresponding compositions 1.1 to 1.3545 only in that they comprise as the active compound A the compounds of formula (Ia).

Also especially preferred are compositions 3.1. to 3.3545 which differ from the corresponding compositions 1.1 to 1.3545 only in that they additionally comprise B.2 clodinafop-propargyl as further herbicide B.

Also especially preferred are compositions 4.1. to 4.3545 which differ from the corresponding compositions 1.1 to 1.3545 only in that they additionally comprise B.8 pinoxaden as further herbicide B.

Also especially preferred are compositions 5.1. to 5.3545 which differ from the corresponding compositions 1.1 to 1.3545 only in that they additionally comprise B.30 imazamox as further herbicide B.

Also especially preferred are compositions 6.1. to 6.3545 which differ from the corresponding compositions 1.1 to 1.3545 only in that they additionally comprise B.32 imazapic as further herbicide B.

Also especially preferred are compositions 7.1. to 7.3545 which differ from the corresponding compositions 1.1 to 1.3545 only in that they additionally comprise B.35 imazapyr as further herbicide B.

Also especially preferred are compositions 8.1. to 8.3545 which differ from the corresponding compositions 1.1 to 1.3545 only in that they additionally comprise B.38 imazaquin as further herbicide B.

Also especially preferred are compositions 9.1. to 9.3545 which differ from the corresponding compositions 1.1 to 1.3545 only in that they additionally comprise B.40 imazethapyr as further herbicide B.

Also especially preferred are compositions 10.1. to 10.3545 which differ from the corresponding compositions 1.1 to 1.3545 only in that they additionally comprise B.51 nicosulfuron as further herbicide B.

Also especially preferred are compositions 11.1. to 11.3545 which differ from the corresponding compositions 1.1 to 1.3545 only in that they additionally comprise B.55 pyribenzoxim as further herbicide B.

Also especially preferred are compositions 12.1. to 12.3545 which differ from the corresponding compositions 1.1 to 1.3545 only in that they additionally comprise B.56 pyriftalid as further herbicide B.

Also especially preferred are compositions 13.1. to 13.3545 which differ from the corresponding compositions 1.1 to 1.3545 only in that they additionally comprise B.64 tritosulfuron as further herbicide B.

Also especially preferred are compositions 14.1. to 14.3545 which differ from the corresponding compositions 1.1 to 1.3545 only in that they additionally comprise B.66 ametryne as further herbicide B.

Also especially preferred are compositions 15.1. to 15.3545 which differ from the corresponding compositions 1.1 to 1.3545 only in that they additionally comprise B.67 atrazine as further herbicide B.

Also especially preferred are compositions 16.1. to 16.3545 which differ from the corresponding compositions 1.1 to 1.3545 only in that they additionally comprise B.68 bentazon as further herbicide B.

Also especially preferred are compositions 17.1. to 17.3545 which differ from the corresponding compositions 1.1 to 1.3545 only in that they additionally comprise B.69 bromoxynil as further herbicide B.

Also especially preferred are compositions 18.1. to 18.3545 which differ from the corresponding compositions 1.1 to 1.3545 only in that they additionally comprise B.73 diuron as further herbicide B.

Also especially preferred are compositions 19.1. to 19.3545 which differ from the corresponding compositions 1.1 to 1.3545 only in that they additionally comprise B.76 isoproturon as further herbicide B.

Also especially preferred are compositions 20.1. to 20.3545 which differ from the corresponding compositions 1.1 to 1.3545 only in that they additionally comprise B.81 simazin as further herbicide B.

Also especially preferred are compositions 21.1. to 21.3545 which differ from the corresponding compositions 1.1 to 1.3545 only in that they additionally comprise B.82 terbuthylazin as further herbicide B.

Also especially preferred are compositions 22.1. to 22.3545 which differ from the corresponding compositions 1.1 to 1.3545 only in that they additionally comprise B.85 acifluorfen as further herbicide B.

Also especially preferred are compositions 23.1. to 23.3545 which differ from the corresponding compositions 1.1 to 1.3545 only in that they additionally comprise B.88 flumioxazin as further herbicide B.

Also especially preferred are compositions 24.1. to 24.3545 which differ from the corresponding compositions 1.1 to 1.3545 only in that they additionally comprise B.89 fomesafen as further herbicide B.

Also especially preferred are compositions 25.1. to 25.3545 which differ from the corresponding compositions 1.1 to 1.3545 only in that they additionally comprise B.94 saflufenacil as further herbicide B.

Also especially preferred are compositions 26.1. to 26.3545 which differ from the corresponding compositions 1.1 to 1.3545 only in that they additionally comprise B.95 sulfentrazone as further herbicide B.

Also especially preferred are compositions 27.1. to 27.3545 which differ from the corresponding compositions 1.1 to 1.3545 only in that they additionally comprise B.98 benzbicyclone as further herbicide B.

Also especially preferred are compositions 28.1. to 28.3545 which differ from the corresponding compositions 1.1 to 1.3545 only in that they additionally comprise B.100 clomazone as further herbicide B.

Also especially preferred are compositions 29.1. to 29.3545 which differ from the corresponding compositions 1.1 to 1.3545 only in that they additionally comprise B.103 isoxaflutole as further herbicide B.

Also especially preferred are compositions 30.1. to 30.3545 which differ from the corresponding compositions 1.1 to 1.3545 only in that they additionally comprise B.103 isoxaflutole and B.67 atrazine as further herbicides B.

Also especially preferred are compositions 31.1. to 31.3545 which differ from the corresponding compositions 1.1 to 1.3545 only in that they additionally comprise B.103 isoxaflutole and B.76 isoproturon as further herbicides B.

Also especially preferred are compositions 32.1. to 32.3545 which differ from the corresponding compositions 1.1 to 1.3545 only in that they additionally comprise B.103 isoxaflutole and B.82 terbutylazin as further herbicides B.

Also especially preferred are compositions 33.1. to 33.3545 which differ from the corresponding compositions 1.1 to 1.3545 only in that they additionally comprise B.104 mesotrione as further herbicide B.

Also especially preferred are compositions 34.1. to 34.3545 which differ from the corresponding compositions 1.1 to 1.3545 only in that they additionally comprise B.104 mesotrione and B.67 atrazine as further herbicides B.

Also especially preferred are compositions 35.1. to 35.3545 which differ from the corresponding compositions 1.1 to 1.3545 only in that they additionally comprise B.104 mesotrione and B.76 isoproturon as further herbicides B.

Also especially preferred are compositions 36.1. to 36.3545 which differ from the corresponding compositions 1.1 to 1.3545 only in that they additionally comprise B.104 mesotrione and B.82 terbutylazin as further herbicides B.

Also especially preferred are compositions 37.1. to 37.3545 which differ from the corresponding compositions 1.1 to 1.3545 only in that they additionally comprise B.106 picolinafen as further herbicide B.

Also especially preferred are compositions 38.1. to 38.3545 which differ from the corresponding compositions 1.1 to 1.3545 only in that they additionally comprise B.107 sulcotrione as further herbicide B.

Also especially preferred are compositions 39.1. to 39.3545 which differ from the corresponding compositions 1.1 to 1.3545 only in that they additionally comprise B. 107 sulcotrione and B.67 atrazine as further herbicides B.

Also especially preferred are compositions 40.1. to 40.3545 which differ from the corresponding compositions 1.1 to 1.3545 only in that they additionally comprise B. 107 sulcotrione and B.76 isoproturon as further herbicides B.

Also especially preferred are compositions 41.1. to 41.3545 which differ from the corresponding compositions 1.1 to 1.3545 only in that they additionally comprise B. 107 sulcotrione and B.82 terbutylazin as further herbicides B.

Also especially preferred are compositions 42.1. to 42.3545 which differ from the corresponding compositions 1.1 to 1.3545 only in that they additionally comprise B.109 tembotrione as further herbicide B.

Also especially preferred are compositions 43.1. to 43.3545 which differ from the corresponding compositions 1.1 to 1.3545 only in that they additionally comprise B.111 topramezone as further herbicide B.

Also especially preferred are compositions 44.1. to 44.3545 which differ from the corresponding compositions 1.1 to 1.3545 only in that they additionally comprise B.111 topramezone and B.67 atrazine as further herbicides B.

Also especially preferred are compositions 45.1. to 45.3545 which differ from the corresponding compositions 1.1 to 1.3545 only in that they additionally comprise B.111 topramezone and B.76 isoproturon as further herbicides B.

Also especially preferred are compositions 46.1. to 46.3545 which differ from the corresponding compositions 1.1 to 1.3545 only in that they additionally comprise B.111 topramezone and B.82 terbutylazin as further herbicides B.

Also especially preferred are compositions 47.1. to 47.3545 which differ from the corresponding compositions 1.1 to 1.3545 only in that they additionally comprise B.116 glyphosate as further herbicide B.

Also especially preferred are compositions 48.1. to 48.3545 which differ from the corresponding compositions 1.1 to 1.3545 only in that they additionally comprise B.116 glyphosate and B.67 atrazine as further herbicides B.

Also especially preferred are compositions 49.1. to 49.3545 which differ from the corresponding compositions 1.1 to 1.3545 only in that they additionally comprise B.116 glyphosate and B.94 saflufenacil as further herbicides B.

Also especially preferred are compositions 50.1. to 50.3545 which differ from the corresponding compositions 1.1 to 1.3545 only in that they additionally comprise B.116 glyphosate and B.103 isoxaflutole as further herbicides B.

Also especially preferred are compositions 51.1. to 51.3545 which differ from the corresponding compositions 1.1 to 1.3545 only in that they additionally comprise B.116 glyphosate and B.128 acetochlor as further herbicides B.

Also especially preferred are compositions 52.1. to 52.3545 which differ from the corresponding compositions 1.1 to 1.3545 only in that they additionally comprise B.116 glyphosate and B.104 mesotrione as further herbicides B.

Also especially preferred are compositions 53.1. to 53.3545 which differ from the corresponding compositions 1.1 to 1.3545 only in that they additionally comprise B.116 glyphosate and B.107 sulcotrione as further herbicides B.

Also especially preferred are compositions 54.1. to 54.3545 which differ from the corresponding compositions 1.1 to 1.3545 only in that they additionally comprise B.116 glyphosate and B.111 topramezone as further herbicides B.

Also especially preferred are compositions 55.1. to 55.3545 which differ from the corresponding compositions 1.1 to 1.3545 only in that they additionally comprise B.122 glufosinate as further herbicide B.

Also especially preferred are compositions 56.1. to 56.3545 which differ from the corresponding compositions 1.1 to 1.3545 only in that they additionally comprise B.126 pendimethalin as further herbicide B.

Also especially preferred are compositions 57.1. to 57.3545 which differ from the corresponding compositions 1.1 to 1.3545 only in that they additionally comprise B.128 acetochlor as further herbicide B.

Also especially preferred are compositions 58.1. to 58.3545 which differ from the corresponding compositions 1.1 to 1.3545 only in that they additionally comprise B.131 dimethenamid-P as further herbicide B.

Also especially preferred are compositions 59.1. to 59.3545 which differ from the corresponding compositions 1.1 to 1.3545 only in that they additionally comprise B.132 fentrazamide as further herbicide B.

Also especially preferred are compositions 60.1. to 60.3545 which differ from the corresponding compositions 1.1 to 1.3545 only in that they additionally comprise B.133 flufenacet as further herbicide B.

Also especially preferred are compositions 61.1. to 61.3545 which differ from the corresponding compositions 1.1 to 1.3545 only in that they additionally comprise B.135 metazachlor as further herbicide B.

Also especially preferred are compositions 62.1. to 62.3545 which differ from the corresponding compositions 1.1 to 1.3545 only in that they additionally comprise B.137 S-metolachlor as further herbicide B.

Also especially preferred are compositions 63.1. to 63.3545 which differ from the corresponding compositions 11.1 to 1.3545 only in that they additionally comprise B.138 pretilachlor as further herbicide B.

Also especially preferred are compositions 64.1. to 64.3545 which differ from the corresponding compositions 1.1 to 1.3545 only in that they additionally comprise B.140 indaziflam as further herbicide B.

Also especially preferred are compositions 65.1. to 65.3545 which differ from the corresponding compositions 1.1 to 1.3545 only in that they additionally comprise B.145 2,4-D as further herbicide B.

Also especially preferred are compositions 66.1. to 66.3545 which differ from the corresponding compositions 1.1 to 1.3545 only in that they additionally comprise B.153 clopyralid as further herbicide B.

Also especially preferred are compositions 67.1. to 67.3545 which differ from the corresponding compositions 1.1 to 1.3545 only in that they additionally comprise B.156 dicamba as further herbicide B.

Also especially preferred are compositions 68.1. to 68.3545 which differ from the corresponding compositions 1.1 to 1.3545 only in that they additionally comprise B.171 MCPA as further herbicide B.

Also especially preferred are compositions 69.1. to 69.3545 which differ from the corresponding compositions 1.1 to 1.3545 only in that they additionally comprise B.174 quinclorac as further herbicide B.

The invention also relates to agrochemical compositions comprising an auxiliary and at least one diaminotriazine compound of formula (I) or a composition according to the invention.

An agrochemical diaminotriazine compound of formula (I) or a composition comprises a pesticidally effective amount of at least one composition according to the invention. The term "effective amount" denotes an amount of the active ingredients, which is sufficient for controlling unwanted plants, especially for controlling unwanted plants in cultivated plants and which does not result in a substantial damage to the treated plants. Such an amount can vary in a broad range and is dependent on various factors, such as the plants to be controlled, the treated cultivated plant or material, the climatic conditions and the specific composition according to the invention used.

The diamonotriazine compounds of formula (I) (compounds A) and optionally B and/or C, their N-oxides, salts or derivatives can be converted into customary types of agrochemical compositions, e. g. solutions, emulsions, suspensions, dusts, powders, pastes, granules, pressings, capsules, and mixtures thereof. Examples for agrochemical composition types are suspensions (e.g. SC, OD, FS), emulsifiable concentrates (e.g. EC), emulsions (e.g. EW, EO, ES, ME), capsules (e.g. CS, ZC), pastes, pastilles, wettable powders or dusts (e.g. WP, SP, WS, DP, DS), pressings (e.g. BR, TB, DT), granules (e.g. WG, SG, GR, FG, GG, MG), insecticidal articles (e.g. LN), as well as gel formulations for the treatment of plant propagation materials such as seeds (e.g. GF). These and further agrochemical compositions types are defined in the "Catalogue of pesticide formulation types and international coding system", Technical Monograph No. 2, 6th Ed. May 2008, CropLife International.

The agrochemical compositions are prepared in a known manner, such as described by Mollet and Grubemann, Formulation technology, Wiley VCH, Weinheim, 2001; or Knowles, New developments in crop protection product formulation, Agrow Reports DS243, T&F Informa, London, 2005.

Suitable auxiliaries are solvents, liquid carriers, solid carriers or fillers, surfactants, dispersants, emulsifiers, wetters, adjuvants, solubilizers, penetration enhancers, protective colloids, adhesion agents, thickeners, humectants, repellents, attractants, feeding stimulants, compatibilizers, bactericides, anti-freezing agents, anti-foaming agents, colorants, tackifiers and binders.

Suitable solvents and liquid carriers are water and organic solvents, such as mineral oil fractions of medium to high boiling point, e.g. kerosene, diesel oil; oils of vegetable or animal origin; aliphatic, cyclic and aromatic hydrocarbons, e. g. toluene, paraffin, tetrahydronaphthalene, alkylated naphthalenes; alcohols, e.g. ethanol, propanol, butanol, benzylalcohol, cyclohexanol; glycols; DMSO; ketones, e.g. cyclohexanone; esters, e.g. lactates, carbonates, fatty acid esters, gamma-butyrolactone; fatty acids; phosphonates; amines; amides, e.g. N-methylpyrrolidone, fatty acid dimethylamides; and mixtures thereof.

Suitable solid carriers or fillers are mineral earths, e.g. silicates, silica gels, talc, kaolins, limestone, lime, chalk, clays, dolomite, diatomaceous earth, bentonite, calcium sulfate, magnesium sulfate, magnesium oxide; polysaccharides, e.g. cellulose, starch; fertilizers, e.g. ammonium sulfate, ammonium phosphate, ammonium nitrate, ureas; products of vegetable origin, e.g. cereal meal, tree bark meal, wood meal, nutshell meal, and mixtures thereof.

Suitable surfactants are surface-active compounds, such as anionic, cationic, nonionic and amphoteric surfactants, block polymers, polyelectrolytes, and mixtures thereof. Such surfactants can be used as emulsifier, dispersant, solubilizer, wetter, penetration enhancer, protective colloid, or adjuvant. Examples of surfactants are listed in McCutcheon's, Vol.1: Emulsifiers & Detergents, McCutcheon's Directories, Glen Rock, USA, 2008 (International Ed. or North American Ed.).

Suitable anionic surfactants are alkali, alkaline earth or ammonium salts of sulfonates, sulfates, phosphates, carboxylates, and mixtures thereof. Examples of sulfonates are alkylarylsulfonates, diphenylsulfonates, alpha-olefin sulfonates, lignine sulfonates, sulfonates of fatty acids and oils, sulfonates of ethoxylated alkylphenols, sulfonates of alkoxylated arylphenols, sulfonates of condensed naphthalenes, sulfonates of dodecyl- and tridecylbenzenes, sulfonates of naphthalenes and alkylnaphthalenes, sulfosuccinates or sulfosuccinamates. Examples of sulfates are sulfates of fatty acids and oils, of ethoxylated alkylphenols, of alcohols, of ethoxylated alcohols, or of fatty acid esters. Examples of phosphates are phosphate esters. Examples of carboxylates are alkyl carboxylates, and carboxylated alcohol or alkylphenol ethoxylates.

Suitable nonionic surfactants are alkoxylates, N-substituted fatty acid amides, amine oxides, esters, sugar-based surfactants, polymeric surfactants, and mixtures thereof. Examples of alkoxylates are compounds such as alcohols, alkylphenols, amines, amides, arylphenols, fatty acids or fatty acid esters which have been alkoxylated with 1 to 50 equivalents. Ethylene oxide and/or propylene oxide may be employed for the alkoxylation, preferably ethylene oxide. Examples of N-substituted fatty acid amides are fatty acid glucamides or fatty acid alkanolamides. Examples of esters are fatty acid esters, glycerol esters or monoglycerides. Examples of sugar-based surfactants are sorbitans, ethoxylated sorbitans, sucrose and glucose esters or alkylpolyglucosides. Examples of polymeric surfactants are home- or copolymers of vinylpyrrolidone, vinylalcohols, or vinylacetate.

Suitable cationic surfactants are quaternary surfactants, for example quaternary ammonium compounds with one or two hydrophobic groups, or salts of long-chain primary amines. Suitable amphoteric surfactants are alkylbetains and imidazolines. Suitable block polymers are block polymers of the A-B or A-B-A type comprising blocks of polyethylene oxide and polypropylene oxide, or of the A-B-C type comprising alkanol, polyethylene oxide and polypropylene oxide. Suitable polyelectrolytes are polyacids or polybases. Examples of polyacids are alkali salts of polyacrylic acid or polyacid comb polymers. Examples of polybases are polyvinylamines or polyethyleneamines.

Suitable adjuvants are compounds, which have a neglectable or even no pesticidal activity themselves, and which improve the biological performance of the compound I on the target. Examples are surfactants, mineral or vegetable oils, and other auxiliaries. Further examples are listed by Knowles, Adjuvants and additives, Agrow Reports DS256, T&F Informa UK, 2006, chapter 5.

Suitable thickeners are polysaccharides (e.g. xanthan gum, carboxymethylcellulose), inorganic clays (organically modified or unmodified), polycarboxylates, and silicates.

Suitable bactericides are bronopol and isothiazolinone derivatives such as alkylisothiazolinones and benzisothiazolinones.

Suitable anti-freezing agents are ethylene glycol, propylene glycol, urea and glycerin.

Suitable anti-foaming agents are silicones, long chain alcohols, and salts of fatty acids.

Suitable colorants (e.g. in red, blue, or green) are pigments of low water solubility and water-soluble dyes. Examples are inorganic colorants (e.g. iron oxide, titan oxide, iron hexacyanoferrate) and organic colorants (e.g. alizarin-, azo- and phthalocyanine colorants).

Suitable tackifiers or binders are polyvinylpyrrolidons, polyvinylacetates, polyvinyl alcohols, polyacrylates, biological or synthetic waxes, and cellulose ethers.

Examples for agrochemical composition types and their preparation are:
i) Water-soluble concentrates (SL, LS)
   10-60 wt% of a compound of formula (I) or compostion according to the invention and 5-15 wt% wetting agent (e.g. alcohol alkoxylates) are dissolved in water and/or in a water-soluble solvent (e.g. alcohols) ad 100 wt%. The active substance dissolves upon dilution with water.
ii) Dispersible concentrates (DC)
   5-25 wt% of a diamonitriazine compound of formula (I) or composition according to the invention and 1-10 wt% dispersant (e. g. polyvinylpyrrolidone) are dissolved in organic solvent (e.g. cyclohexanone) ad 100 wt%. Dilution with water gives a dispersion.
iii) Emulsifiable concentrates (EC)
   15-70 wt% of a diamonitriazine compound of formula (I) or composition according to the invention and 5-10 wt% emulsifiers (e.g. calcium dodecylbenzenesulfonate and castor oil ethoxylate) are dissolved in water-insoluble organic solvent (e.g. aromatic hydrocarbon) ad 100 wt%. Dilution with water gives an emulsion.
iv) Emulsions (EW, EO, ES)
   5-40 wt% of a diamonitriazine compound of formula (I) or composition according to the invention and 1-10 wt% emulsifiers (e.g. calcium dodecylbenzenesulfonate and castor oil ethoxylate) are dissolved in 20-40 wt% water-insoluble organic solvent (e.g. aromatic hydrocarbon). This mixture is introduced into water ad 100 wt% by means of an emulsifying machine and made into a homogeneous emulsion. Dilution with water gives an emulsion.
v) Suspensions (SC, OD, FS)
   In an agitated ball mill, 20-60 wt% of a diamonitriazine compound of formula (I) or composition according to the invention are comminuted with addition of 2-10 wt% dispersants and wetting agents (e.g. sodium lignosulfonate and alcohol ethoxylate), 0,1-2 wt% thickener (e.g. xanthan gum) and water ad 100 wt% to give a fine active substance suspension. Dilution with water gives a stable suspension of the active substance. For FS type composition up to 40 wt% binder (e.g. polyvinylalcohol) is added.
vi) Water-dispersible granules and water-soluble granules (WG, SG)
   50-80 wt% of a diamonitriazine compound of formula (I) or composition according to the invention are ground finely with addition of dispersants and wetting agents (e.g. sodium lignosulfonate and alcohol ethoxylate) ad 100 wt% and prepared as water-dispersible or water-soluble granules by means of technical appliances (e. g. extrusion, spray tower, fluidized bed). Dilution with water gives a stable dispersion or solution of the active substance.
vii) Water-dispersible powders and water-soluble powders (WP, SP, WS)
   50-80 wt% of a compound of formula (I) or composition according to the invention are ground in a rotor-stator mill with addition of 1-5 wt% dispersants (e.g. sodium lignosulfonate), 1-3 wt% wetting agents (e.g. alcohol ethoxylate) and solid carrier (e.g. silica gel) ad 100 wt%. Dilution with water gives a stable dispersion or solution of the active substance.
viii) Gel (GW, GF)
   In an agitated ball mill, 5-25 wt% of a diamonitriazine compound of formula (I) or a composition according to the invention are comminuted with addition of 3-10 wt% dispersants (e.g. sodium lignosulfonate), 1-5 wt% thickener (e.g. carboxymethylcellulose) and water ad 100 wt% to give a fine suspension of the active substance. Dilution with water gives a stable suspension of the active substance.
iv) Microemulsion (ME)
   5-20 wt% of a diamonitriazine compound of formula (I) or a composition according to the invention are added to 5-30 wt% organic solvent blend (e.g. fatty acid dimethylamide and cyclohexanone), 10-25 wt% surfactant blend (e.g. alcohol ethoxylate and arylphenol ethoxylate), and water ad 100 %. This mixture is stirred for 1 h to produce spontaneously a thermodynamically stable microemulsion.
iv) Microcapsules (CS)
   An oil phase comprising 5-50 wt% of a diamonitriazine compound of formula (I) or a composition according to the invention, 0-40 wt% water insoluble organic solvent (e.g. aromatic hydrocarbon), 2-15 wt% acrylic monomers (e.g. methylmethacrylate, methacrylic acid and a di- or triacrylate) are dispersed into an aqueous solution of a protective colloid (e.g. polyvinyl alcohol). Radical polymerization initiated by a radical initiator results in the formation of poly(meth)acrylate microcapsules. Alternatively, an oil phase comprising 5-50 wt% of a diamonitriazine compound of formula (I) according to the invention, 0-40 wt% water insoluble organic solvent (e.g. aromatic hydrocarbon), and an isocyanate monomer (e.g. diphenylmethene-4,4'-diisocyanate) are dispersed into an aqueous solution of a protective colloid (e.g. polyvinyl alcohol). The addition of a polyamine (e.g. hexamethylenediamine) results in the formation of polyurea microcapsules. The monomers amount to 1-10 wt%. The wt% relate to the total CS composition.
ix) Dustable powders (DP, DS)
   1-10 wt% of a diamonitriazine compound of formula (I) or a composition according to the invention are ground finely and mixed intimately with solid carrier (e.g. finely divided kaolin) ad 100 wt%.
x) Granules (GR, FG)
   0.5-30 wt% of a diamonitriazine compound of formula (I) or a composition according to the invention is ground finely and associated with solid carrier (e.g. silicate) ad 100 wt%. Granulation is achieved by extrusion, spray-drying or the fluidized bed.
xi) Ultra-low volume liquids (UL)
   1-50 wt% of a diamonitriazine compound of formula (I) or a composition according to the invention are dissolved in organic solvent (e.g. aromatic hydrocarbon) ad 100 wt%.

The agrochemical compositions types i) to xi) may optionally comprise further auxiliaries, such as 0,1-1 wt% bactericides, 5-15 wt% anti-freezing agents, 0,1-1 wt% anti-foaming agents, and 0,1-1 wt% colorants.

The agrochemical compositions generally comprise between 0.01 and 95%, preferably between 0.1 and 90%, and in particular between 0.5 and 75%, by weight of active substance. The active substances are employed in a purity of from 90% to 100%, preferably from 95% to 100% (according to NMR spectrum).

Solutions for seed treatment (LS), suspoemulsions (SE), flowable concentrates (FS), powders for dry treatment (DS), water-dispersible powders for slurry treatment (WS), water-soluble powders (SS), emulsions (ES), emulsifiable concentrates (EC) and gels (GF) are usually employed for the purposes of treatment of plant propagation materials, particularly seeds. The compositions in question give, after two-to-tenfold dilution, active substance concentrations of from 0.01 to 60% by weight, preferably from 0.1 to 40% by weight, in the ready-to-use preparations. Application can be carried out before or during sowing.

Methods for applying diamonitriazine compounds of formula (I) and compositions thereof, respectively, on to plant propagation material, especially seeds include dressing, coating, pelleting, dusting, soaking and in-furrow application methods of the propagation material. Preferably, compound I or the compositions thereof, respectively, are applied on to the plant propagation material by a method such that germination is not induced, e. g. by seed dressing, pelleting, coating and dusting.

Various types of oils, wetters, adjuvants, fertilizer, or micronutrients, and further pesticides (e.g. herbicides, insecticides, fungicides, growth regulators, safeners) may be added to the active substances or the compositions comprising them as premix or, if appropriate not until immediately prior to use (tank mix). These agents can be admixed with the compositions according to the invention in a weight ratio of 1:100 to 100:1, preferably 1:10 to 10:1.

The user applies the agrochemical composition according to the invention usually from a predosage device, a knapsack sprayer, a spray tank, a spray plane, or an irrigation system. Usually, the agrochemical composition is made up with water, buffer, and/or further auxiliaries to the desired application concentration and the ready-to-use spray liquor or the agrochemical composition according to the invention is thus obtained. Usually, 20 to 2000 liters, preferably 50 to 400 liters, of the ready-to-use spray liquor are applied per hectare of agricultural useful area.

According to one embodiment, either individual components of the agrochemical composition according to the invention or partially premixed components, e. g. agrochemical components comprising compounds of formula (I) and/or active substances from the groups B and/or C may be mixed by the user in a spray tank and further auxiliaries and additives may be added, if appropriate.

In a further embodiment, individual components of the agrochemical composition according to the invention such as parts of a kit or parts of a binary or ternary mixture may be mixed by the user himself in a spray tank and further auxiliaries may be added, if appropriate.

In a further embodiment, either individual components of the agrochemical composition according to the invention or partially premixed components, e. g. components comprising compounds of formula (I) and active substances from the groups B and/or C, can be applied jointly (e.g. after tank mix) or consecutively.

Accordingly, a first embodiment of the invention relates to compositions in the form of a agrochemical composition formulated as a 1-component composition comprising the at least one active compound of formula (I) or the at least one active compound of formula (I) (active compound A) and at least one further active compound selected from the herbicides B and the safeners C and also a solid or liquid carrier and, if appropriate, one or more surfactants.

Accordingly, a second embodiment of the invention relates to compositions in the form of a agrochemical composition formulated as a 2-component composition comprising a first formulation (component) comprising the at least one active compound A, which is a compound of formula (I), a solid or liquid carrier and, if appropriate, one or more surfactants, and a second component comprising at least one further active compound selected from the herbicides B and safeners C, a solid or liquid carrier and, if appropriate, one or more surfactants.

The active compound A, which is a compound of formula (I) and the at least one further active compound B and/or C can be formulated and applied jointly or separately, simultaneously or in succession, before, during or after the emergence of the plants. In case of separate application, the order of the application of the active compounds A, B and/or C is of minor importance. The only thing that is important is that the at least one active compound A and the at least one further active compound B and/or C are present simultaneously at the site of action, i.e. are at the same time in contact with or taken up by the plant to be controlled or to be safened.

The compounds of formula (I) or compositions according to the invention are suitable as herbicides. They are suitable as such or as an appropriately formulated composition (agrochemical composition).

The compounds of formula (I) or compositions according to the invention control vegetation on non-crop areas very efficiently, especially at high rates of application. They act against broad-leafed weeds and grass weeds in crops such as wheat, rice, corn, soybeans and cotton without causing any significant damage to the crop plants. This effect is mainly observed at low rates of application.

The compounds of formula (I) or compositions according to the invention are applied to the plants mainly by spraying the leaves. Here, the application can be carried out using, for example, water as carrier by customary spraying techniques using spray liquor amounts of from about 100 to 1000 l/ha (for example from 300 to 400 l/ha). The herbicidal compositions may also be applied by the low-volume or the ultra-low-volume method, or in the form of microgranules.

Application of the compounds of formula (I) or herbicidal compositions according to the present invention can be done before, during and/or after, preferably during and/or after, the emergence of the undesirable plants.

The compounds of formula (I) or herbicidal compositions according to the present invention can be applied pre- or post-emergence or together with the seed of a crop plant. It is also possible to apply the compounds and compositions by applying seed, pretreated with a composition of the invention, of a crop plant. If the active compounds A and B and, if appropriate C, are less well tolerated by certain crop plants, application techniques may be used in which the herbicidal compositions are sprayed, with the aid of the spraying equipment, in such a way that as far as possible they do not come into contact with the leaves of the sensitive crop plants, while the active compounds reach the leaves of undesirable plants growing underneath, or the bare soil surface (post-directed, lay-by).

In a further embodiment, the composition according to the invention can be applied by treating seed. The treatment of seed comprises essentially all procedures familiar to the person skilled in the art (seed dressing, seed coating, seed dusting, seed soaking, seed film coating, seed multilayer coating, seed encrusting, seed dripping and seed pelleting) based on the compounds of the formula (I) according to the invention or the compositions prepared therefrom. Here, the herbicidal compositions can be applied diluted or undiluted.

The term "seed" comprises seed of all types, such as, for example, corns, seeds, fruits, tubers, seedlings and similar forms. Here, preferably, the term seed describes corns and seeds. The seed used can be seed of the useful plants mentioned above, but also the seed of transgenic plants or plants obtained by customary breeding methods.

Moreover, it may be advantageous to apply the compounds of formula (I) or compositions of the present invention on their own or jointly in combination with other crop protection agents, for example with agents for controlling pests or phytopathogenic fungi or bacteria or with groups of active compounds which regulate growth. Also of interest is the miscibility with mineral salt solutions which are employed for treating nutritional and trace element deficiencies. Non-phytotoxic oils and oil concentrates can also be added.

When employed in plant protection, the amounts of active substances applied, i.e. A (compounds of formula (I)) and B and, if appropriate, C without formulation auxiliaries, are, depending on the kind of effect desired, from 0.001 to 2 kg per ha, preferably from 0.005 to 2 kg per ha, more preferably from 0.05 to 0.9 kg per ha and in particular from 0.1 to 0.75 kg per ha.

In another embodiment of the invention, the application rate of A (compounds of formula (I)) and B and, if appropriate, C, is from 0.001 to 3 kg/ha, preferably from 0.005 to 2.5 kg/ha and in particular from 0.01 to 2 kg/ha of active substance (a.s.).

In another preferred embodiment of the invention, the rates of application of the compounds of formula (I) according to the present invention (total amount of compounds of formula (I)) are from 0.1 g/ha to 3000 g/ha, preferably 10 g/ha to 1000 g/ha, depending on the control target, the season, the target plants and the growth stage.

In another preferred embodiment of the invention, the application rates of the compounds of formula (I) are in the range from 0.1 g/ha to 5000 g/ha and preferably in the range from 1 g/ha to 2500 g/ha or from 5 g/ha to 2000 g/ha.

In another preferred embodiment of the invention, the application rate of the compounds of formula (I) is 0.1 to 1000 g/ha, preferably1 to 750 g/ha, more preferably 5 to 500 g/ha.

The required application rates of herbicidal compounds B are generally in the range of from 0.0005 kg/ha to 2.5 kg/ha and preferably in the range of from 0.005 kg/ha to 2 kg/ha or 0.01 kg/ha to 1.5 kg/h of a.s.

The required application rates of safeners C are generally in the range of from 0.0005 kg/ha to 2.5 kg/ha and preferably in the range of from 0.005 kg/ha to 2 kg/ha or 0.01 kg/ha to 1.5 kg/h of a.s.

In treatment of plant propagation materials such as seeds, e. g. by dusting, coating or drenching seed, amounts of active substance of from 0.1 to 1000 g, preferably from 1 to 1000 g, more preferably from 1 to 100 g and most preferably from 5 to 100 g, per 100 kilogram of plant propagation material (preferably seeds) are generally required.

In another embodiment of the invention, to treat the seed, the amounts of active substances applied, i.e. A and B and, if appropriate, C are generally employed in amounts of from 0.001 to 10 kg per 100 kg of seed.

When used in the protection of materials or stored products, the amount of active substance applied depends on the kind of application area and on the desired effect. Amounts customarily applied in the protection of materials are 0.001 g to 2 kg, preferably 0.005 g to 1 kg, of active substance per cubic meter of treated material.

In the methods of the present invention it is immaterial whether the herbicide compound A of formula (I), and the further herbicide component B and/or the herbicide safener compound C are formulated and applied jointly or separately.
In the case of separate application it is of minor importance, in which order the application takes place. It is only necessary, that the herbicide compound A and the herbicide compound B and/or the herbicide safener compound C are applied in a time frame that allows simultaneous action of the active ingredients on the plants, preferably within a time-frame of at most 14 days, in particular at most 7 days.

Depending on the application method in question, the compositions according to the invention can additionally be employed in a further number of crop plants for eliminating undesirable plants. Examples of suitable crops are the following:
Allium cepa, Ananas comosus, Arachis hypogaea, Asparagus officinalis, Avena sativa, Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus avium, Prunus persica, Pyrus communis, Prunus armeniaca, Prunus cerasus, Prunus dulcis and prunus domestica, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticum aestivum, Triticale, Triticum durum, Vicia faba, Vitis vinifera, Zea mays.

Preferred crops are Arachis hypogaea, Beta vulgaris spec. altissima, Brassica napus var. napus, Brassica oleracea, Citrus limon, Citrus sinensis, Coffea arabica (Coffea canephora, Coffea liberica), Cynodon dactylon, Glycine max, Gossypium hirsutum, (Gossypium arboreum, Gossypium herbaceum, Gossypium vitifolium), Helianthus annuus, Hordeum vulgare, Juglans regia, Lens culinaris, Linum usitatissimum, Lycopersicon lycopersicum, Malus spec., Medicago sativa, Nicotiana tabacum (N.rustica), Olea europaea, Oryza sativa , Phaseolus lunatus, Phaseolus vulgaris, Pistacia vera, Pisum sativum, Prunus dulcis, Saccharum officinarum, Secale cereale, Solanum tuberosum, Sorghum bicolor (s. vulgare), Triticale, Triticum aestivum, Triticum durum, Vicia faba, Vitis vinifera and Zea mays

Especially preferred crops are crops of cereals, corn, soybeans, rice, oilseed rape, cotton, potatoes, peanuts or permanent crops.

The compositions according to the invention can also be used in genetically modified plants. The term "genetically modified plants" is to be understood as plants whose genetic material has been modified by the use of recombinant DNA techniques to include an inserted sequence of DNA that is not native to that plant species' genome or to exhibit a deletion of DNA that was native to that species' genome, wherein the modification(s) cannot readily be obtained by cross breeding, mutagenesis or natural recombination alone. Often, a particular genetically modified plant will be one that has obtained its genetic modification(s) by inheritance through a natural breeding or propagation process from an ancestral plant whose genome was the one directly treated by use of a recombinant DNA technique. Typically, one or more genes have been integrated into the genetic material of a genetically modified plant in order to improve certain properties of the plant. Such genetic modifications also include but are not limited to targeted post-translational modification of protein(s), oligo- or polypeptides. e. g., by inclusion therein of amino acid mutation(s) that permit, decrease, or promote glycosylation or polymer additions such as prenylation, acetylation farnesylation, or PEG moiety attachment.

Plants that have been modified by breeding, mutagenesis or genetic engineering, e.g. have been rendered tolerant to applications of specific classes of herbicides, such as auxinic herbicides such as dicamba or 2,4-D; bleacher herbicides such as 4-hydroxyphenylpyruvate dioxygenase (HPPD) inhibitors or phytoene desaturase (PDS) inhibitors; acetolactate synthase (ALS) inhibitors such as sulfonylureas or imidazolinones; enolpyruvyl shikimate 3-phosphate synthase (EPSP) inhibitors such as glyphosate; glutamine synthetase (GS) inhibitors such as glufosinate; protoporphyrinogen-IX oxidase inhibitors; lipid biosynthesis inhibitors such as acetylCoA carboxylase (ACCase) inhibitors; or oxynil (i. e. bromoxynil or ioxynil) herbicides as a result of conventional methods of breeding or genetic engineering; furthermore, plants have been made resistant to multiple classes of herbicides through multiple genetic modifications, such as resistance to both glyphosate and glufosinate or to both glyphosate and a herbicide from another class such as ALS inhibitors, HPPD inhibitors, auxinic herbicides, or ACCase inhibitors. These herbicide resistance technologies are, for example, described in Pest Management Science 61, 2005, 246; 61, 2005, 258; 61, 2005, 277; 61, 2005, 269; 61, 2005, 286; 64, 2008, 326; 64, 2008, 332; Weed Science 57, 2009, 108; Australian Journal of Agricultural Research 58, 2007, 708; Science 316, 2007, 1185; and references quoted therein. Several cultivated plants have been rendered tolerant to herbicides by mutagenesis and conventional methods of breeding, e. g., Clearfield® summer rape (Canola, BASF SE, Germany) being tolerant to imidazolinones, e. g., imazamox, or ExpressSun® sunflowers (DuPont, USA) being tolerant to sulfonyl ureas, e. g., tribenuron. Genetic engineering methods have been used to render cultivated plants such as soybean, cotton, corn, beets and rape, tolerant to herbicides such as glyphosate, imidazolinones and glufosinate, some of which are under development or commercially available under the brands or trade names RoundupReady® (glyphosate tolerant, Monsanto, USA), Cultivance® (imidazolinone tolerant, BASF SE, Germany) and LibertyLink® (glufosinate tolerant, Bayer CropScience, Germany).

Furthermore, plants are also covered that are by the use of recombinant DNA techniques capable to synthesize one or more insecticidal proteins, especially those known from the bacterial genus Bacillus, particularly from Bacillus thuringiensis, such as delta-endotoxins, e. g., CryIA(b), CryIA(c), CryIF, CryIF(a2), CryIIA(b), CryIIIA, CryIIIB(b1) or Cry9c; vegetative insecticidal proteins (VIP), e. g., VIP1, VIP2, VIP3 or VIP3A; insecticidal proteins of bacteria colonizing nematodes, e. g., Photorhabdus spp. or Xenorhabdus spp.; toxins produced by animals, such as scorpion toxins, arachnid toxins, wasp toxins, or other insect-specific neurotoxins; toxins produced by fungi, such as Streptomycetes toxins, plant lectins, such as pea or barley lectins; agglutinins; proteinase inhibitors, such as trypsin inhibitors, serine protease inhibitors, patatin, cystatin or papain inhibitors; ribosome-inactivating proteins (RIP), such as ricin, maize-RIP, abrin, luffin, saporin or bryodin; steroid metabolism enzymes, such as 3-hydroxysteroid oxidase, ecdysteroid-IDP-glycosyl-transferase, cholesterol oxidases, ecdysone inhibitors or HMG-CoA-reductase; ion channel blockers, such as blockers of sodium or calcium channels; juvenile hormone esterase; diuretic hormone receptors (helicokinin receptors); stilbene synthase, bibenzyl synthase, chitinases or glucanases. In the context of the present invention these insecticidal proteins or toxins are to be understood expressly also as including pre-toxins, hybrid proteins, truncated or otherwise modified proteins. Hybrid proteins are characterized by a new combination of protein domains, (see, e. g., WO 02/015701). Further examples of such toxins or genetically modified plants capable of synthesizing such toxins are disclosed, e. g., in EP-A 374 753, WO 93/007278, WO 95/34656, EP-A 427 529, EP-A 451 878, WO 03/18810 und WO 03/52073. The methods for producing such genetically modified plants are generally known to the person skilled in the art and are described, e. g., in the publications mentioned above. These insecticidal proteins contained in the genetically modified plants impart to the plants producing these proteins tolerance to harmful pests from all taxonomic groups of arthropods, especially to beetles (Coleoptera), two-winged insects (Diptera), and moths (Lepidoptera) and to nematodes (Nematoda). Genetically modified plants capable to synthesize one or more insecticidal proteins are, e. g., described in the publications mentioned above, and some of which are commercially available such as YieldGard® (corn cultivars producing the Cry1Ab toxin), YieldGard® Plus (corn cultivars producing Cry1Ab and Cry3Bb1 toxins), Starlink® (corn cultivars producing the Cry9c toxin), Herculex® RW (corn cultivars producing Cry34Ab1, Cry35Ab1 and the enzyme Phosphinothricin-N-Acetyltransferase [PAT]); NuCOTN® 33B (cotton cultivars producing the Cry1Ac toxin), Bollgard® I (cotton cultivars producing the Cry1Ac toxin), Bollgard® II (cotton cultivars producing Cry1Ac and Cry2Ab2 toxins); VIPCOT® (cotton cultivars producing a VIP-toxin); NewLeaf® (potato cultivars producing the Cry3A toxin); Bt-Xtra®, NatureGard®, KnockOut®, BiteGard®, Protecta®, Bt11 (e. g., Agrisure® CB) and Bt176 from Syngenta Seeds SAS, France, (corn cultivars producing the Cry1Ab toxin and PAT enzyme), MIR604 from Syngenta Seeds SAS, France (corn cultivars producing a modified version of the Cry3A toxin, c.f. WO 03/018810), MON 863 from Monsanto Europe S.A., Belgium (corn cultivars producing the Cry3Bb1 toxin), IPC 531 from Monsanto Europe S.A., Belgium (cotton cultivars producing a modified version of the Cry1Ac toxin) and 1507 from Pioneer Overseas Corporation, Belgium (corn cultivars producing the Cry1 F toxin and PAT enzyme).

Furthermore, plants are also covered that are by the use of recombinant DNA techniques capable to synthesize one or more proteins to increase the resistance or tolerance of those plants to bacterial, viral or fungal pathogens. Examples of such proteins are the so-called "pathogenesis-related proteins" (PR proteins, see, e.g., EP-A 392 225), plant disease resistance genes (e. g., potato cultivars, which express resistance genes acting against Phytophthora infestans derived from the Mexican wild potato, Solanum bulbocastanum) or T4-lysozym (e.g., potato cultivars capable of synthesizing these proteins with increased resistance against bacteria such as Erwinia amylovora). The methods for producing such genetically modified plants are generally known to the person skilled in the art and are described, e.g., in the publications mentioned above.

Furthermore, plants are also covered that are by the use of recombinant DNA techniques capable to synthesize one or more proteins to increase the productivity (e.g., bio-mass production, grain yield, starch content, oil content or protein content), tolerance to drought, salinity or other growth-limiting environmental factors or tolerance to pests and fungal, bacterial or viral pathogens of those plants.

Furthermore, plants are also covered that contain by the use of recombinant DNA techniques a modified amount of ingredients or new ingredients, specifically to improve human or animal nutrition, e. g., oil crops that produce health-promoting long-chain omega-3 fatty acids or unsaturated omega-9 fatty acids (e. g., Nexera® rape, Dow AgroSciences, Canada).

Furthermore, plants are also covered that contain by the use of recombinant DNA techniques a modified amount of ingredients or new ingredients, specifically to improve raw material production, e.g., potatoes that produce increased amounts of amylopectin (e.g. Amflora® potato, BASF SE, Germany).

Furthermore, it has been found that the the compositions according to the invention are also suitable for the defoliation and/or desiccation of plant parts, for which crop plants such as cotton, potato, oilseed rape, sunflower, soybean or field beans, in particular cotton, are suitable. In this regard compositions have been found for the desiccation and/or defoliation of plants, processes for preparing these compositions, and methods for desiccating and/or defoliating plants using the compositions according to the invention.

As desiccants, the compositions according to the invention are suitable in particular for desiccating the above-ground parts of crop plants such as potato, oilseed rape, sunflower and soybean, but also cereals. This makes possible the fully mechanical harvesting of these important crop plants.

Also of economic interest is the facilitation of harvesting, which is made possible by concentrating within a certain period of time the dehiscence, or reduction of adhesion to the tree, in citrus fruit, olives and other species and varieties of pomaceous fruit, stone fruit and nuts. The same mechanism, i.e. the promotion of the development of abscission tissue between fruit part or leaf part and shoot part of the plants is also essential for the controlled defoliation of useful plants, in particular cotton.

Moreover, a shortening of the time interval in which the individual cotton plants mature leads to an increased fiber quality after harvesting.

Another aspect of the invention is an agrochemical composition comprising a herbicidal active amount of at least one compound of formula (I) as defined above and at least one inert liquid and/or solid carrier and, if appropriate, at least one surface-active substances.

A further aspect of the invention is a method of controlling undesired vegetation, which comprises allowing a herbicidally active amount of at least one compound of formula (I) as as defined above to act on plants, their environment or on seed.

A further aspect of the invention in is the use of a compound of formula (I) as defined aboven as a herbicide or for the desiccation/defoliation of plants.

The preparation of the diaminotriazine compounds of formula (I) is illustrated by examples. The following abbreviations are used herein:
- CH: Cyclohexane
- EtOAc: Ethyl acetate
- eq: mol equivalent
- HPLC: High performance liquid chromatography
- MeOH: Methanol
- MS: Mass spectrometry
- THF: tetrahydrofurane
The term room temperature indicates ambient temperature which is 22 to 23 °C. The products shown below were characterized by the mass ([m/z]) or retention time (RT; [min.]) determined by HPLC-MS spectrometry.
HPLC-MS = high performance liquid chromatography-coupled mass spectrometry; HPLC column:
RP-18 column (Chromolith Speed ROD from Merck KgaA, Germany), 50*4.6 mm; mobile phase: acetonitrile + 0.1 % trifluoroacetic acid (TFA)/water + 0.1 % TFA using a gradient from 5:95 to 100:0 over 5 minutes at 40°C, flow rate 1.8 ml/min.
   MS: quadrupole electrospray ionization, 80 V (positive mode).

### A Preparation examples

### Example 1: 6-pyrrolidin-1-yl-N4-(2,3,5,6-tetrafluoro-4-pyridyl)-1,3,5-triazine-2,4-diamine

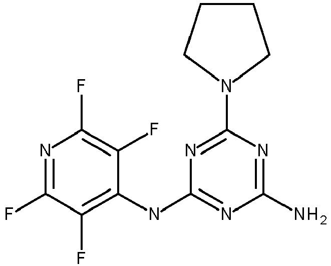

### 1.1 4-chloro-6-pyrrolidin-1-yl-1,3,5-triazin-2-amine

To a solution of cyanuric chloride (5 g, 27 mmol, 2 eq) dissolved in dry THF (100 mL) cooled to 0°C diisopropyethylamine (2.6 mL, 15 mmol, 1.1 eq) was added and the mixture was cooled to -10°C. After stirring for 15 min, a solution of pyrrolidine (1.13 mL, 14 mmol, 1 eq.) in dry THF (20 mL) was slowly added at this temperature over a period of 1 hour. After stirring for another one hour, a 25% w/w aqueous solution of ammonia (8.5 mL, 4 eq.) was added and the mixture was allowed to warm to room temperature and stirred for 16 h. Then, the mixture was washed with water (100 mL) and extracted with EtOAc (2 x 50 mL). The combined organic extracts were washed with brine (100 mL), dried (Na₂SO₄) and rotary evaporated. Chromatography (CH₂Cl₂:MeOH, 100:0 to 98:2 to 95:5) gave the desired chloro-triazine (2.7 g, 13.6 mmol, 97%) as a white solid.
LC/MS RT: 0.761, LC/MS (m/z): 200.1

### 1.2 6-pyrrolidin-1-yl-N4-(2,3,5,6-tetrafluoro-4-pyridyl)-1,3,5-triazine-2,4-diamine

A mixture of the 4-chloro-6-pyrrolidin-1-yl-1,3,5-triazin-2-amine (400 mg, 2 mmol, 1 eq), 4-Amino-2,3,5,6-tetrafluoropyridine (366 mg, 2.2 mmol, 1.1 eq), potassium tert-butoxide (674 mg, 5.7 mmol, 3 eq) and [1,1'-Bis(di-cyclohexylphosphino)-ferrocene] dichloropalladium(II) (163 mg, 0.2 mmol, 0.1 eq) in dioxane (15 mL) was heated to 100 °C for 16 h. Then, the mixture was cooled to room temperature and water (30 mL) was added and the mixture was extracted with EtOAc (3 x 30 mL). The combined organic extracts were washed with brine (100 mL), dried (Na₂SO₄) and solvent was removed at reduced pressure by a rotary evaporator. Chromatography (CH: EtOAC, 100:0 gradient to 50:50 v/v) yielded the desired product (45 mgs, 0.14 mmol, 7%) as a beige solid.
LC/MS RT: 0.847, LC/MS (m/z): 330.5

### B Use examples

The herbicidal activity of the azines of formula (I) was demonstrated by the following greenhouse experiments:

The culture containers used were plastic flowerpots containing loamy sand with approximately 3.0% of humus as the substrate. The seeds of the test plants were sown separately for each species.
For the pre-emergence treatment, the active ingredients, which had been suspended or emulsified in water, were applied directly after sowing by means of finely distributing nozzles. The containers were irrigated gently to promote germination and growth and subsequently covered with transparent plastic hoods until the plants had rooted. This cover caused uniform germination of the test plants, unless this had been impaired by the active ingredients.
For the post-emergence treatment, the test plants were first grown to a height of 3 to 15 cm, depending on the plant habit, and only then treated with the active ingredients which had been suspended or emulsified in water. For this purpose, the test plants were either sown directly and grown in the same containers, or they were first grown separately as seedlings and transplanted into the test containers a few days prior to treatment.
Depending on the species, the plants were kept at 10 - 25°C or 20 - 35°C, respectively.
The test period extended over 2 to 4 weeks. During this time, the plants were tended, and their response to the individual treatments was evaluated.

Evaluation was carried out using a scale from 0 to 100. 100 means no emergence of the plants, or complete destruction of at least the aerial moieties, and 0 means no damage, or normal course of growth. A moderate herbicidal activity is given at values of at least 60, a good herbicidal activity is given at values of at least 70, and a very good herbicidal activity is given at values of at least 85.

The plants used in the greenhouse experiments were of the following species:

| Bayer code | Scientific name |
|---|---|
| ABUTH | Abutilon theophrasti |
| ALOMY | Alopecurus myosuroides |
| AMARE | Amaranthus retroflexus |
| APESV | Apera spica-venti |
| ECHCG | Echinocloa crus-galli |
| POLCO | Polygonum convolvulus |
| SETFA | Setaria faberi |
| SETVI | Setaria viridis |
| STEME | Stellaria media |
| VIOAR | Viola arvensis |

The compound of Example 1 applied by pre-emergence method at an application rate of 0.5 kg/ha, showed very good herbicidal activity against APESV, AMARE and ALOMY.

## Claims

1. A diaminotriazine compound of formula (I) 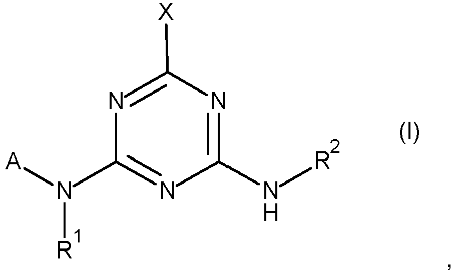 wherein
A is 5- or 6-membered monocyclic heteroaryl, which comprises 1, 2 or 3 heteroatoms as ring members, which are selected from O, S and N, where heteroaryl is substituted by 1, 2, 3 or 4 substituents R^{A}, which are identical or different and which are selected from the group consisting of halogen, OH, CN, amino, NO₂, C₁-C₆-alkyl, C₂-C₆-alkenyl, C₂-C₆-alkynyl, C₁-C₆-alkoxy, C₂-C₆-alkenyloxy, C₂-C₆-alkynyloxy, (C₁-C₆-alkoxy)-C₁-C₆-alkyl, (C₁-C₆-alkoxy)-C₁-C₆-alkoxy, (C₁-C₆-alkoxy)-C₂-C₆-alkenyl, (C₁-C₆-alkoxy)-C₂-C₆-alkynyl, C₁-C₆-alkylthio, (C₁-C₆-alkyl)sulfinyl, (C₁-C₆-alkyl)sulfonyl, (C₁-C₆-alkyl)amino, di(C₁-C₆-alkyl)amino, (C₁-C₆-alkyl)-carbonyl, (C₁-C₆-alkoxy)-carbonyl, (C₁-C₆-alkyl)-carbonyloxy, C₃-C₆-cycloalkyl, C₃-C₆-cycloalkoxy, (C₃-C₆-cycloalkyl)-C₁-C₄-alkyl, (C₃-C₆-cycloalkyl)-C₁-C₄-alkoxy, where the aliphatic and cycloaliphatic parts of the 22 aforementioned radicals are unsubstituted, partly or completely halogenated and where the cycloaliphatic parts of the last 4 mentioned radicals may carry 1, 2, 3, 4, 5 or 6 methyl groups;
R¹ is selected from the group consisting of H, OH, S(O)₂NH₂, CN, C₁-C₆-alkyl, C₂-C₆-alkenyl, C₂-C₆-alkynyl, (C₃-C₆-cycloalkyl)-C₁-C₄-alkyl, C₁-C₆-alkoxy, (C₁-C₆-alkoxy)-C₁-C₆-alkyl, (C₁-C₆-alkyl)-carbonyl, (C₁-C₆-alkoxy)carbonyl, (C₁-C₆-alkyl)sulfonyl, (C₁-C₆-alkylamino)carbonyl, di(C₁-C₆-alkyl)aminocarbonyl, (C₁-C₆-alkylamino)sulfonyl, di(C₁-C₆-alkyl)aminosulfonyl and (C₁-C₆-alkoxy)sulfonyl, where the aliphatic and cycloaliphatic parts of the 14 aforementioned radicals are unsubstituted, partly or completely halogenated, phenyl, phenyl-C₁-C₆-alkyl, phenylsulfonyl, phenylaminosulfonyl, phenylcarbonyl and phenoxycarbonyl,
wherein phenyl in the last 6 mentioned radicals are unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy;
R² is selected from the group consisting of H, OH, S(O)₂NH₂, CN, C₁-C₆-alkyl, C₂-C₆-alkenyl, C₂-C₆-alkynyl, (C₃-C₆-cycloalkyl)-C₁-C₄-alkyl, C₁-C₆-alkoxy, (C₁-C₆-alkoxy)-C₁-C₆-alkyl, (C₁-C₆-alkyl)carbonyl, (C₁-C₆-alkoxy)carbonyl, (C₁-C₆-alkyl)sulfonyl, (C₁-C₆-alkylamino)carbonyl, di(C₁-C₆-alkyl)amino-carbonyl, (C₁-C₆-alkylamino)sulfonyl, di(C₁-C₆-alkyl)aminosulfonyl and (C₁-C₆-alkoxy)sulfonyl, where the aliphatic and cycloaliphatic parts of the 14 aforementioned radicals are unsubstituted, partly or completely halogenated,
phenyl, phenylsulfonyl, phenylaminosulfonyl, phenyl-C₁-C₆ alkyl, phenoxy, phenylcarbonyl and phenoxycarbonyl,
wherein phenyl in the last 6 mentioned radicals is unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy;
X is NR^{3a}R^{3b}, OR^{3c} or S(O)ₖR^{3d},
wherein R^{3a}, R^{3b}, R^{3c} or R^{3d} are independently of one another are selected from the group consisting of H, CN, S(O)₂NH₂, C₁-C₆-alkyl, C₂-C₆-alkenyl, C₂-C₆-alkynyl, C₃-C₆-cycloalkyl, (C₃-C₆-cycloalkyl)-C₁-C₆-alkyl, (C₁-C₆-alkoxy)-C₁-C₆-alkyl, (C₁-C₆-alkyl)-carbonyl, (C₁-C₆-alkoxy)carbonyl, (C₁-C₆-alkyl)sulfonyl, (C₁-C₆-alkylamino)carbonyl, di(C₁-C₆-alkyl)aminocarbonyl, (C₁-C₆-alkylamino)sulfonyl, di(C₁-C₆-alkyl)aminosulfonyl and (C₁-C₆-alkoxy)sulfonyl, where the aliphatic and cycloaliphatic parts of the 14 aforementioned radicals are unsubstituted, partly or completely halogenated,
phenyl, phenylsulfonyl, phenyl-C₁-C₆ alkyl, phenylaminosulfonyl, phenylcarbonyl and phenoxycarbonyl, wherein phenyl in the last 6 mentioned radicals is unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy, or R^{3a}, R^{3b} together with the nitrogen atom, to which they are bound, form an N-bound, mono - or bicyclic heterocyclic radical, which may have1, 2, 3 or 4 further heteroatoms which are selected from N, O and S, which is unsubstituted or substituted by one or more identical or different substituents selected from the group consisting of halogen, CN, NO₂, C₁-C₆-alkyl, C₁-C₆-haloalkyl, C₁-C₆-alkoxy and C₁-C₆-haloalkoxy,
one of R^{3a}, R^{3b} may also be OH, C₁-C₆-alkoxy, C₃-C₆-cycloalkoxy, (C₃-C₆-cycloalkyl)-C₁-C₄-alkoxy C₂-C₆-alkenyloxy, C₂-C₆-alkynyloxy, (C₁-C₆-alkoxy)-C₁-C₆-alkoxy, where the aliphatic and cycloaliphatic parts of the 7 aforementioned radicals are unsubstituted, partly or completely halogenated,
or phenoxy, which is unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy; k is 0, 1 or 2,
including their agriculturally acceptable salts.

2. The compound of claim 1, wherein A is a 6-membered heteroaryl having 1 to 3 nitrogen atoms as ring members, where heteroaryl is substituted by 1, 2, 3 or 4 identical or different radicals R^{A}.

3. The compound of claim 2, wherein A is pyridyl, in particular 4-pyridyl or 2-pyridyl, which is substituted by 1, 2, 3 or 4 identical or different radicals R^{A}.

4. The compound of any of the preceding claims, wherein R^{A} is selected from the group consisting of halogen, CN, C₁-C₆-alkyl, C₁-C₆-alkoxy, C₁-C₆-haloalkyl, C₃-C₆-cycloalkyl, C₃-C₆-cycloalkoxy, (C₃-C₆-cycloalkyl)methoxy, C₂-C₆-alkynyl, C₂-C₆-alkenyl, C₂-C₆-alkynyloxy, C₂-C₆-alkenyloxy and C₁-C₆-haloalkoxy.

5. The compound of any of the preceding claims, wherein R^{A} is halogen, C₁-C₄-haloalkyl, C₁-C₄-alkoxy, C₁-C₄-haloalkoxy and CN.

6. The compound of claim 5, wherein A is 2,3,5,6-tetraflouro-4-pyridyl or 4-chloro-3,5,6-trifluoro-2-pyridyl.

7. The compound of any of the preceding claims, wherein R¹ is selected from the group consisting of H, CN, C₁-C₆-alkyl, (C₁-C₆-alkoxy)-C₁-C₆-alkyl, (C₁-C₆-alkyl)carbonyl, (C₁-C₆-alkyl)sulfonyl, C₁-C₆-alkoxy, (C₁-C₆-alkoxy)carbonyl, (C₁-C₆-alkylamino)carbonyl, di(C₁-C₆-alkyl)aminocarbonyl, (C₁-C₆-alkylamino)sulfonyl, di(C₁-C₆-alkyl)aminosulfonyl, where the aliphatic parts of the 10 aforementioned radicals are unsubstituted, partly or completely halogenated,
phenyl, phenylcarbonyl and phenyl-C₁-C₆ alkyl,
wherein phenyl in the last 3 mentioned radical is unsubstituted or substituted by 1, 2, 3, 4, or 5 identical or different substituents selected from the group consisting of halogen, CN, NO₂, C₁-C₆-alkyl, C₁-C₆-haloalkyl, C₁-C₆-alkoxy and C₁-C₆-haloalkoxy.

8. The compound of any of the preceding claims, wherein R² is selected from the group consisting of H, CN, C₁-C₆-alkyl, (C₁-C₆-alkoxy)-C₁-C₆-alkyl, (C₁-C₆-alkyl)carbonyl, (C₁-C₆-alkyl)sulfonyl, (C₁-C₆-alkoxy)carbonyl, (C₁-C₆-alkylamino)carbonyl, di(C₁-C₆-alkyl)aminocarbonyl, (C₁-C₆-alkylamino)sulfonyl, di(C₁-C₆-alkyl)aminosulfonyl, where the aliphatic parts of the 9 aforementioned radicals are unsubstituted, partly or completely halogenated,
phenyl, phenylcarbonyl and C₁-C₆ alkylphenyl,
wherein phenyl in the last 3 mentioned radical is unsubstituted or substituted by 1, 2, 3, 4, or 5 identical or different substituents selected from the group consisting of halogen, CN, NO₂, C₁-C₆-alkyl, C₁-C₆-haloalkyl, C₁-C₆-alkoxy and C₁-C₆-haloalkoxy.

9. The compound of any of the preceding claims, wherein R¹ is selected from the group consisting of H, CN, C₁-C₆-alkyl, C₁-C₆-haloalkyl, (C₁-C₆-alkoxy)-C₁-C₆-alkyl, C₁-C₆-alkoxy, (C₁-C₆-alkyl)carbonyl, (C₁-C₆-haloalkyl)carbonyl, (C₁-C₆-alkyl)sulfonyl or (C₁-C₆-haloalkyl)sulfonyl.

10. The compound of any of the preceding claims, wherein R² is selected from the group consisting of H, CN, C₁-C₄-alkyl, (C₁-C₄-alkoxy)-C₁-C₄-alkyl, (C₁-C₄-alkyl)carbonyl or (C₁-C₄-alkyl)sulfonyl.

11. The compound of any of the preceding claims, wherein R¹ is H.

12. The compound of any of the preceding claims, wherein R² is H.

13. The compound of any of the preceding claims, wherein
X is OR^{3c}, wherein
R^{3c} is selected from the group consisting of H, C₁-C₆-alkyl, C₃-C₆-cycloalkyl, (C₁-C₆-alkoxy)-C₁-C₆-alkyl, (C₁-C₆-alkyl)-carbonyl, (C₁-C₆-alkoxy)carbonyl, (C₁-C₆-alkyl)sulfonyl, where the aliphatic parts of the 6 aforementioned radicals are unsubstituted, partly or completely halogenated, phenyl, phenylsulfonyl or phenyl-C₁-C₆ alkyl,
wherein phenyl in the last 3 mentioned radicals is unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy.

14. The compound of any of claims 1 to 12, wherein
X is S(O)ₖR^{3d}, wherein
R^{3d} is selected from the group consisting of H, CN, C₁-C₆-alkyl, C₃-C₆-cycloalkyl, C₃-C₆-cyxloalkyl-C₁-C₆-alkyl, (C₁-C₆-alkoxy)-C₁-C₆-alkyl, (C₁-C₆-alkyl)-carbonyl, (C₁-C₆-alkoxy)carbonyl, (C₁-C₆-alkyl)sulfonyl, where the aliphatic parts of the 7 aforementioned radicals are unsubstituted, partly or completely halogenated,
phenyl, phenylsulfonyl or phenyl-C₁-C₆ alkyl,
wherein phenyl in the last 3 mentioned radicals is unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy, and
k is 0, 1 or 2.

15. The compound of any of claims 1 to 12, wherein
X is NR^{3a}R^{3b}, wherein
R^{3a} R^{3b} are independently of one another H, CN, S(O)₂NH₂, C₁-C₆-alkyl, C₂-C₆-alkenyl, C₂-C₆-alkynyl, C₃-C₆-cycloalkyl, C₃-C₆-cycloalkyl-C₁-C₆-alkyl, (C₁-C₆-alkoxy)-C₁-C₆-alkyl, (C₁-C₆-alkyl)-carbonyl, (C₁-C₆-alkoxy)carbonyl, (C₁-C₆-alkyl)sulfonyl, C₁-C₆-alkylamino)carbonyl, di(C₁-C₆-alkyl)aminocarbonyl, (C₁-C₆-alkylamino)sulfonyl, di(C₁-C₆-alkyl)aminosulfonyl and (C₁-C₆-alkoxy)sulfonylwhere the aliphatic parts of the 15 aforementioned radicals are unsubstituted, partly or completely halogenated, phenyl, phenylsulfonyl or phenyl-C₁-C₆ alkyl,
wherein phenyl in the last 3 mentioned radicals is unsubstituted or substituted by 1, 2, 3, 4 or 5 identical or different substituents selected from the group consisting of halogen, CN, NO₂, C₁-C₆-alkyl, C₁-C₆-haloalkyl, C₁-C₆-alkoxy and C₁-C₆-haloalkoxy,
one of R^{a}, R^{b} may also be OH, C₁-C₆-alkoxy, C₃-C₆-cycloalkoxy, (C₁-C₆-alkoxy)-C₁-C₆-alkoxy, where the aliphatic and cycloaliphatic parts of the 3 aforementioned radicals are unsubstituted, partly or completely halogenated,
or
R^{3a}, R^{3b} together with the nitrogen atom, to which they are bound, form an N-bound saturated or unsaturated mono - or bicyclic heterocyclic radical, which may have 1, 2, 3 or 4 further heteroatoms which are selected from N,
O and S, which is substituted or unsubstituted by one or more identical or different substituents selected from the group consisting of halogen, CN, NO₂, C₁-C₆-alkyl, C₁-C₆-haloalkyl, C₁-C₆-alkoxy, (C₁-C₆-alkoxy)-C₁-C₆-alkyl and C₁-C₆-haloalkoxy.

16. The compound of claim 15, wherein
X is NR^{3a}R^{3b}, wherein
R^{3a}R^{3b} together with the nitrogen atom, to which they are bound, form an N-bound saturated or unsaturated mono - or bicyclic heterocyclic radical, which is selected from the group consisting of 1-aziridinyl, 1-azetidinyl, 1-piperidinyl, 1-pyrrolidinyl, azepan-1-yl, azocan-1-yl, morpholin-4-yl, isoxazolidine-2-yl, oxazolidine-3-yl, piperazine-1-yl, octahydroisoindol-2-yl, octahydroindol-1-yl, octahydro-2H-quinolin-1-yl, azabicyclo[2.2.1]heptan-3-yl and azabicyclo[2.2.1]heptan-7-yl, where the aforementioned radicals are unsubstituted or substituted by one or more identical or different substituents selected from the group consisting of halogen, CN, NO₂, C₁-C₂-alkyl, C₁-C₂-haloalkyl, (C₁-C₂-alkoxy)-C₁-C₂-alkyl, C₁-C₂-alkoxy and C₁-C₂-haloalkoxy.

17. An agrochemical composition comprising a herbicidal active amount of at least one compound as claimed in any of claims 1 to 16 and at least one inert liquid and/or solid carrier and, if appropriate, at least one surface-active substances.

18. A method of controlling undesired vegetation, which comprises allowing a herbicidally active amount of at least one compound as claimed in any of claims 1 16 to act on plants, their environment or on seed.

19. The use of a compound as claimed in any of claims 1 to 16 as a herbicide or for the desiccation/defoliation of plants.

## Patentansprüche

1. Diaminotriazinverbindung der Formel (I) 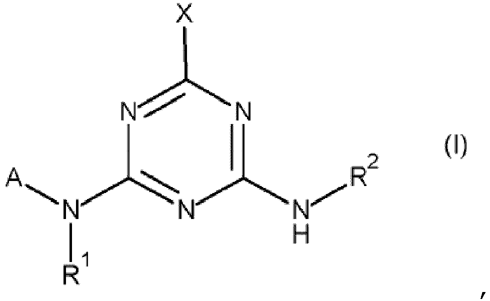 mit den folgenden Bedeutungen:
A bedeutet 5- oder 6-gliedriges monocyclisches Heteroaryl das 1, 2 oder 3 Heteroatome als Ringglieder umfasst, die aus 0, S und N ausgewählt sind, wobei Heteroaryl durch 1, 2, 3 oder 4 Substituenten R^{A} substituiert ist, die gleich oder verschieden sind und die aus der Gruppe bestehend aus Halogen, OH, CN, Amino, NO₂, C₁-C₆-Alkyl, C₂-C₆-Alkenyl, C₂-C₆-Alkinyl, C₁-C₆-Alkoxy, C₂-C₆-Alkenyloxy, C₂-C₆-Alkinyloxy, (C₁-C₆-Alkoxy)-C₁-C₆-alkyl, (C₁-C₆-Alkoxy)-C₁-C₆-alkoxy, (C₁-C₆-Alkoxy)-C₂-C₆-alkenyl, (C₁-C₆-Alkoxy)-C₂-C₆-alkinyl), C₁-C₆-Alkylthio, (C₁-C₆-Alkyl)sulfinyl), (C₁-C₆-Alkyl)sulfonyl, (C₁-C₆-Alkyl) amino, Di-(C₁-C₆-alkyl)amino, (C₁-C₆-Alkyl) carbonyl, (C₁-C₆-Alkoxy)carbonyl, (C₁-C₆-Alkyl)carbonyloxy, C₃-C₆-Cycloalkyl, C₃-C₆-Cycloalkoxy, (C₃-C₆-Cycloalkyl) -C₁-C₄-alkyl, (C₃-C₆-Cycloalkyl)-C₁-C4-alkoxy ausgewählt sind, wobei die aliphatischen und cycloaliphatischen Teile der 22 oben genannten Reste unsubstituiert oder teilweise oder vollständig halogeniert sind, und wobei die cycloaliphatischen Teile der letzten 4 genannten Reste 1, 2, 3, 4, 5 oder 6 Methylgruppen tragen können;
R¹ ist ausgewählt aus der Gruppe bestehend aus H, OH, S(O)₂NH₂, CN, C₁-C₆-Alkyl, C₂-C₆-Alkenyl, C₂-C₆-Alkinyl, (C₃-C₆-Cycloalkyl) -C₁-C₄-alkyl, C₁-C₆-Alkoxy, (C₁-C₆-Alkoxy)-C₁-C₆-alkyl, (C₁-C₆-Alkyl) carbonyl, (C₁-C₆-Alkoxy)carbonyl, (C₁-C₆-Alkyl)sulfonyl, (C₁-C₆-Alkylamino)carbonyl, Di-(C₁-C₆-alkyl)aminocarbonyl, (C₁-C₆-Alkylamino)sulfonyl, Di-(C₁-C₆-alkyl)aminosulfonyl und (C₁-C₆-Alkoxy)sulfonyl, wobei die aliphatischen und cycloaliphatischen Teile der 14 oben genannten Reste unsubstituiert oder teilweise oder vollständig halogeniert sind, Phenyl, Phenyl-C₁-C₆-alkyl, Phenylsulfonyl, Phenylaminosulfonyl, Phenylcarbonyl und Phenoxycarbonyl,
wobei Phenyl in den letzten 6 genannten Resten unsubstituiert oder durch 1, 2, 3, 4 oder 5 gleiche oder verschiedene Substituenten, ausgewählt aus der Gruppe bestehend aus Halogen, CN, NO₂, C₁-C₆-Alkyl, C₁-C₆-Halogenalkyl, C₁-C₆-Alkoxy und C₁-C₆-Halogenalkoxy, substituiert sind;
R² ist ausgewählt aus der Gruppe bestehend aus H, OH, S(O)₂NH₂, CN, C₁-C₆-Alkyl, C₂-C₆-Alkenyl, C₂-C₆-Alkinyl, (C₃-C₆-Cycloalkyl)-C₁-C₄-alkyl, C₁-C₆-Alkoxy, (C₁-C₆-Alkoxy)-C₁-C₆-alkyl, (C₁-C₆-Alkyl) carbonyl, (C₁-C₆-Alkoxy)carbonyl, (C₁-C₆-Alkyl)sulfonyl, (C₁-C₆-Alkylamino)carbonyl, Di-(C₁-C₆-alkyl)aminocarbonyl, (C₁-C₆-Alkylamino)sulfonyl, Di-(C₁-C₆-alkyl)aminosulfonyl und (C₁-C₆-Alkoxy)sulfonyl, wobei die aliphatischen und cycloaliphatischen Teile der 14 oben genannten Reste unsubstituiert oder teilweise oder vollständig halogeniert sind,
Phenyl, Phenylsulfonyl, Phenylaminosulfonyl, Phenyl-C₁-C₆-alkyl, Phenoxy, Phenylcarbonyl und Phenoxycarbonyl,
wobei Phenyl in den letzten 6 genannten Resten unsubstituiert oder durch 1, 2, 3, 4 oder 5 gleiche oder verschiedene Substituenten, ausgewählt aus der Gruppe bestehend aus Halogen, CN, NO₂, C₁-C₆-Alkyl, C₁-C₆-Halogenalkyl, C₁-C₆-Alkoxy und C₁-C₆-Halogenalkoxy, substituiert ist;
X bedeutet NR^{3a}R^{3b}, OR^{3c} oder S(O)ₖR^{3d},
wobei R^{3a}, R^{3b}, R^{3c} oder R^{3d} unabhängig voneinander aus der Gruppe bestehend aus H, CN, S(O)₂NH₂, C₁-C₆-Alkyl, C₂-C₆-Alkenyl, C₂-C₆-Alkinyl, C₃-C₆-Cycloalkyl, (C₃-C₆-Cycloalkyl)-C₁-C₆-alkyl, (C₁-C₆-Alkoxy)-C₁-C₆-alkyl, (C₁-C₆-Alkyl) carbonyl, (C₁-C₆-Alkoxy)carbonyl, (C₁-C₆-Alkyl)sulfonyl, (C₁-C₆-Alkylamino)carbonyl, Di-(C₁-C₆-alkyl)aminocarbonyl, (C₁-C₆-Alkylamino)sulfonyl, Di-(C₁-C₆-alkyl)aminosulfonyl und (C₁-C₆-Alkoxy)sulfonyl ausgewählt sind, wobei die aliphatischen und cycloaliphatischen Teile der 14 oben genannten Reste unsubstituiert oder teilweise oder vollständig halogeniert sind,
Phenyl, Phenylsulfonyl, Phenyl-C₁-C₆-alkyl, Phenylaminosulfonyl, Phenylcarbonyl und Phenoxycarbonyl, wobei Phenyl in den letzten 6 genannten Resten unsubstituiert oder durch 1, 2, 3, 4 oder 5 gleiche oder verschiedene Substituenten, ausgewählt aus der Gruppe bestehend aus Halogen, CN, NO₂, C₁-C₆-Alkyl, C₁-C₆-Halogenalkyl, C₁-C₆-Alkoxy und C₁-C₆-Halogenalkoxy, substituiert ist, oder
R^{3a}, R^{3b} gemeinsam mit dem Stickstoffatom, an das sie gebunden sind, einen N-gebundenen mono- oder bicyclischen heterocyclischen Rest bilden, der 1, 2, 3 oder 4 weitere Heteroatome, die aus N, 0 und S ausgewählt sind, aufweisen kann und der unsubstituiert oder durch einen oder mehrere gleiche oder verschiedene Substituenten, ausgewählt aus der Gruppe bestehend aus Halogen, CN, NO₂, C₁-C₆-Alkyl, C₁-C₆-Halogenalkyl, C₁-C₆-Alkoxy und C₁-C₆-Halogenalkoxy, substituiert ist,
eines von R^{3a}, R^{3b} kann auch OH, C₁-C₆-Alkoxy, C₃-C₆-Cycloalkoxy, (C₃-C₆-Cycloalkyl) -C₁-C₄-alkoxy, C₂-C₆-Alkenyloxy, C₂-C₆-Alkinyloxy, (C₁-C₆-Alkoxy)-C₁-C₆-alkoxy bedeuten, wobei die aliphatischen und cycloaliphatischen Teile der 7 oben genannten Reste unsubstituiert oder teilweise oder vollständig halogeniert sind, oder Phenoxy, das unsubstituiert oder durch 1, 2, 3, 4 oder 5 gleiche oder verschiedene Substituenten, ausgewählt aus der Gruppe bestehend aus Halogen, CN, NO₂, C₁-C₆-Alkyl, C₁-C₆-Halogenalkyl, C₁-C₆-Alkoxy und C₁-C₆-Halogenalkoxy, substituiert ist;
k 0, 1 oder 2 bedeutet,
einschließlich ihrer landwirtschaftlich annehmbaren Salze.

2. Verbindung nach Anspruch 1, wobei A ein 6-gliedriges Heteroaryl mit 1 bis 3 Stickstoffatomen als Ringglieder bedeutet, wobei Heteroaryl durch 1, 2, 3 oder 4 gleiche oder verschiedene Reste R^{A} substituiert ist.

3. Verbindung nach Anspruch 2, wobei A Pyridyl, insbesondere 4-Pyridyl oder 2-Pyridyl, bedeutet, das durch 1, 2, 3 oder 4 gleiche oder verschiedene Reste R^{A} substituiert ist.

4. Verbindung nach einem der vorhergehenden Ansprüche, wobei R^{A} aus der Gruppe bestehend aus Halogen, CN, C₁-C₆-Alkyl, C₁-C₆-Alkoxy, C₁-C₆-Halogenalkyl, C₃-C₆-Cycloalkyl, C₃-C₆-Cycloalkoxy, (C₃-C₆-Cycloalkyl)methoxy, C₂-C₆-Alkinyl, C₂-C₆-Alkenyl, C₂-C₆-Alkinyloxy, C₂-C₆-Alkenyloxy und C₁-C₆-Halogenalkoxy ausgewählt ist.

5. Verbindung nach einem der vorhergehenden Ansprüche, wobei R^{A} Halogen, C₁-C₄-Halogenalkyl, C₁-C₄-Alkoxy, C₁-C₄-Halogenalkoxy und CN bedeutet.

6. Verbindung nach Anspruch 5, wobei A 2,3,5,6-Tetrafluor-4-pyridyl oder 4-Chlor-3,5,6-trifluor-2-pyridyl bedeutet.

7. Verbindung nach einem der vorhergehenden Ansprüche, wobei R¹ aus der Gruppe bestehend aus H, CN, C₁-C₆-Alkyl, (C₁-C₆-Alkoxy)-C₁-C₆-alkyl, (C₁-C₆-Alkyl) carbonyl, (C₁-C₆-Alkyl)sulfonyl, C₁-C₆-Alkoxy, (C₁-C₆-Alkoxy)carbonyl, (C₁-C₆-Alkylamino)carbonyl, Di- (C₁-C₆-alkyl) aminocarbonyl, (C₁-C₆-Alkylamino)sulfonyl, Di-(C₁-C₆-alkyl)aminosulfonyl, wobei die aliphatischen Teile der 10 oben genannten Reste unsubstituiert oder teilweise oder vollständig halogeniert sind,
Phenyl, Phenylcarbonyl und Phenyl-C₁-C₆-alkyl, wobei Phenyl in den letzten 3 genannten Resten unsubstituiert oder durch 1, 2, 3, 4 oder 5 gleiche oder verschiedene Substituenten, ausgewählt aus der Gruppe bestehend aus Halogen, CN, NO₂, C₁-C₆-Alkyl, C₁-C₆-Halogenalkyl, C₁-C₆-Alkoxy und C₁-C₆-Halogenalkoxy, substituiert ist, ausgewählt ist.

8. Verbindung nach einem der vorhergehenden Ansprüche, wobei R² aus der Gruppe bestehend aus H, CN, C₁-C₆-Alkyl, (C₁-C₆-Alkoxy)-C₁-C₆-alkyl, (C₁-C₆-Alkyl) carbonyl, (C₁-C₆-Alkyl)sulfonyl, (C₁-C₆-Alkoxy)carbonyl, (C₁-C₆-Alkylamino)carbonyl, Di-(C₁-C₆-alkyl)aminocarbonyl, (C₁-C₆-Alkylamino)sulfonyl, Di-(C₁-C₆-alkyl)aminosulfonyl, wobei die aliphatischen Teile der 9 oben genannten Reste unsubstituiert oder teilweise oder vollständig halogeniert sind,
Phenyl, Phenylcarbonyl und C₁-C₆-alkylphenyl,
wobei Phenyl in den letzten 3 genannten Resten unsubstituiert oder durch 1, 2, 3, 4 oder 5 gleiche oder verschiedene Substituenten, ausgewählt aus der Gruppe bestehend aus Halogen, CN, NO₂, C₁-C₆-Alkyl, C₁-C₆-Halogenalkyl, C₁-C₆-Alkoxy und C₁-C₆-Halogenalkoxy, substituiert ist, ausgewählt ist.

9. Verbindung nach einem der vorhergehenden Ansprüche, wobei R¹ aus der Gruppe bestehend aus H, CN, C₁-C₆-Alkyl, C₁-C₆-Halogenalkyl, (C₁-C₆-Alkoxy)-C₁-C₆-alkyl, C₁-C₆-Alkoxy, (C₁-C₆-Alkyl) carbonyl, (C₁-C₆-Halogenalkyl)carbonyl, (C₁-C₆-Alkyl)sulfonyl oder (C₁-C₆-Halogenalkyl)sulfonyl ausgewählt ist.

10. Verbindung nach einem der vorhergehenden Ansprüche, wobei R² aus der Gruppe bestehend aus H, CN, C₁-C₄-Alkyl, (C₁-C₄-Alkoxy)-C₁-C₄-alkyl, (C₁-C₄-Alkyl)carbonyl oder (C₁-C₄-Alkyl)sulfonyl ausgewählt ist.

11. Verbindung nach einem der vorhergehenden Ansprüche, wobei R¹ H bedeutet.

12. Verbindung nach einem der vorhergehenden Ansprüche, wobei R² H bedeutet.

13. Verbindung nach einem der vorhergehenden Ansprüche, wobei
X OR^{3c} bedeutet, wobei
R^{3c} aus der Gruppe bestehend aus H, C₁-C₆-Alkyl, C₃-C₆-Cycloalkyl, (C₁-C₆-Alkoxy)-C₁-C₆-alkyl, (C₁-C₆-Alkyl) carbonyl, (C₁-C₆-Alkoxy)carbonyl, (C₁-C₆-Alkyl)sulfonyl, wobei die aliphatischen Teile der 6 oben genannten Reste unsubstituiert oder teilweise oder vollständig halogeniert sind, Phenyl, Phenylsulfonyl oder Phenyl-C₁-C₆-alkyl, wobei Phenyl in den letzten 3 genannten Resten unsubstituiert oder durch 1, 2, 3, 4 oder 5 gleiche oder verschiedene Substituenten, ausgewählt aus der Gruppe bestehend aus Halogen, CN NO₂, C₁-C₆-Alkyl, C₁-C₆-Halogenalkyl C₁-C₆-Alkoxy und C₁-C₆-Halogenalkoxy, substituiert ist, ausgewählt ist.

14. Verbindung nach einem der Ansprüche 1 bis 12, wobei
X S(O)ₖR^{3d} bedeutet, wobei
R^{3d} aus der Gruppe bestehend aus H, CN, C₁-C₆-Alkyl, C₃-C₆-Cycloalkyl, C₃-C₆-Cycloalkyl-C₁-C₆-alkyl, (C₁-C₆-Alkoxy)-C₁-C₆-alkyl, (C₁-C₆-Alkyl) carbonyl, (C₁-C₆-Alkoxy)carbonyl, (C₁-C₆-Alkyl)sulfonyl, wobei die aliphatischen Teile der 7 oben genannten Reste unsubstituiert oder teilweise oder vollständig halogeniert sind, Phenyl, Phenylsulfonyl oder Phenyl-C₁-C₆-alkyl,
wobei Phenyl in den letzten 3 genannten Resten unsubstituiert oder durch 1, 2, 3, 4 oder 5 gleiche oder verschiedene Substituenten, ausgewählt aus der Gruppe bestehend aus Halogen, CN, NO₂, C₁-C₆-Alkyl, C₁-C₆-Halogenalkyl, C₁-C₆-Alkoxy und C₁-C₆-Halogenalkoxy, substituiert ist, ausgewählt ist, und
k 0, 1 oder 2 bedeutet.

15. Verbindung nach einem der Ansprüche 1 bis 12, wobei
X NR^{3a}R^{3b} bedeutet, wobei
R^{3a}R^{3b} unabhängig voneinander H, CN, S(O)₂NH₂, C₁-C₆-Alkyl, C₂-C₆-Alkenyl, C₂-C₆-Alkinyl, C₃-C₆-Cycloalkyl, C₃-C₆-Cycloalkyl-C₁-C₆-alkyl, (C₁-C₆-Alkoxy) -C₁-C₆-alkyl, (C₁-C₆-Alkyl) carbonyl, (C₁-C₆-Alkoxy)carbonyl, (C₁-C₆-Alkyl)sulfonyl, C₁-C₆-Alkylamino)carbonyl, Di-(C₁-C₆-alkyl)aminocarbonyl, (C₁-C₆-Alkylamino)sulfonyl, Di-(C₁-C₆-alkyl)aminosulfonyl und (C₁-C₆-alkoxy)sulfonyl, wobei die aliphatischen Teile der 15 oben genannten Reste unsubstituiert oder teilweise oder vollständig halogeniert sind, Phenyl, Phenylsulfonyl oder Phenyl-C₁-C₆-alkyl, wobei Phenyl in den letzten 3 genannten Resten unsubstituiert oder durch 1, 2, 3, 4 oder 5 gleiche oder verschiedene Substituenten, ausgewählt aus der Gruppe bestehend aus Halogen, CN, NO₂, C₁-C₆-Alkyl, C₁-C₆-Halogenalkyl, C₁-C₆-Alkoxy und C₁-C₆-Halogenalkoxy, substituiert ist,
wobei eines von R^{a}, R^{b} auch OH, C₁-C₆-Alkoxy, C₃-C₆-Cycloalkoxy, (C₁-C₆-Alkoxy)-C₁-C₆-alkoxy, sein kann, wobei die aliphatischen und cycloaliphatischen Teile der 3 oben genannten Reste unsubstituiert oder teilweise oder vollständig halogeniert sind, bedeuten,
oder
R^{3a}, R^{3b} gemeinsam mit dem Stickstoffatom, an das sie gebunden sind, einen N-gebundenen gesättigten oder ungesättigten mono- oder bicyclischen heterocyclischen Rest, der 1, 2, 3 oder 4 weitere Heteroatome, die aus N, 0 und S ausgewählt sind, aufweisen kann und der unsubstituiert oder durch einen oder mehrere gleiche oder verschiedene Substituenten, ausgewählt aus der Gruppe bestehend aus Halogen, CN, NO₂, C₁-C₆-Alkyl, C₁-C₆-Halogenalkyl, C₁-C₆-Alkoxy, (C₁-C₆-Alkoxy)-C₁-C₆-alkyl und C₁-C₆-Halogenalkoxy substituiert sein kann, bilden.

16. Verbindung nach Anspruch 15, wobei
X NR^{3a}R^{3b} bedeutet, wobei
R^{3a}R^{3b} gemeinsam mit dem Stickstoffatom, an das sie gebunden sind, einen N-gebundenen gesättigten oder ungesättigten mono- oder bicyclischen heterocyclischen Rest bilden, der aus der Gruppe bestehend aus 1-Aziridinyl, 1-Azetidinyl, 1-Piperidinyl, 1-Pyrrolidinyl, Azepan-1-yl, Azocan-1-yl, Morpholin-4-yl, Isoxazolidin-2-yl, Oxazolidin-3-yl, Piperazin-1-yl, Octahydroisoindol-2-yl, Octahydroindol-1-yl, Octahydro-2H-chinolin-1-yl, Azabicyclo[2.2.1]heptan-3-yl und Azabicyclo[2.2.1]heptan-7-yl, ausgewählt ist, wobei die oben genannten Reste unsubstituiert oder durch einen oder mehrere gleiche oder verschiedene Substituenten, ausgewählt aus der Gruppe bestehend aus Halogen, CN, NO₂, C₁-C₂-Alkyl, C₁-C₂-Halogenalkyl, (C₁-C₂-Alkoxy) -C₁-C₂-alkyl, C₁-C₂-Alkoxy und C₁-C₂-Halogenalkoxy substituiert sind.

17. Agrarchemische Zusammensetzung umfassend eine herbizid wirksame Menge an mindestens einer Verbindung nach einem der Ansprüche 1 bis 16 und mindestens einen inerten flüssigen und/oder festen Träger sowie gegebenenfalls mindestens eine grenzflächenaktive Substanz.

18. Verfahren zum Bekämpfen von unerwünschtem Pflanzenwuchs, bei dem man eine herbizid wirksame Menge an mindestens einer Verbindung nach einem der Ansprüche 1 bis 16 auf Pflanzen, ihre Umwelt oder auf Saatgut einwirken lässt.

19. Verwendung einer Verbindung nach einem der Ansprüche 1 bis 16 als Herbizid oder für die Austrocknung/Entlaubung von Pflanzen.

## Revendications

1. Composé de diaminotriazine de formule (I) 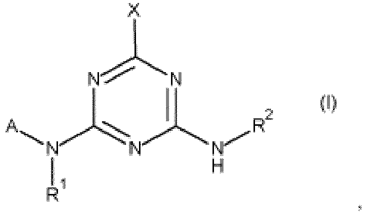 dans lequel
A est un hétéroaryle monocyclique à 5 ou 6 chaînons, qui comprend 1, 2 ou 3 hétéroatomes en tant que chaînons du cycle, qui sont choisis parmi 0, S et N, où l'hétéroaryle est substitué par 1, 2, 3 ou 4 substituants R^{A}, qui sont identiques ou différents et qui sont choisis dans le groupe constitué d'un halogène, de OH, CN, un groupe amine, NO₂, un alkyle en C₁-C₆, un alcényle en C₂-C₆, un alcynyle en C₂-C₆, un alcoxy en C₁-C₆, un alcényloxy en C₂-C₆, un alcynyloxy en C₂-C₆, un (alcoxy en C₁-C₆)-alkyle en C₁-C₆, un (alcoxy en C₁-C₆)-alcoxy en C₁-C₆, un (alcoxy en C₁-C₆)-alcényle en C₂-C₆, un (alcoxy en C₁-C₆)-alcynyle en C₂-C₆, un alkylthio en C₁-C₆, un (alkyle en C₁-C₆)sulfinyle, un (alkyle en C₁-C₆)sulfonyle, une (alkyle en C₁-C₆)amine, une di(alkyle en C₁-C₆)amine, un (alkyle en C₁-C₆)-carbonyle, un (alcoxy en C₁-C₆)-carbonyle, un (alkyle en C₁-C₆) -carbonyloxy, un cycloalkyle en C₃-C₆, un cycloalcoxy en C₃-C₆, un (cycloalkyle en C₃-C₆)-alkyle en C₁-C₄, un (cycloalkyle en C₃-C₆)-alcoxy en C₁-C₄, où les parties aliphatiques et cycloaliphatiques des 22 radicaux susmentionnés sont non substituées, partiellement ou complètement halogénées et où les parties cycloaliphatiques des 4 derniers radicaux mentionnés peuvent porter 1, 2, 3, 4, 5 ou 6 groupes méthyle ;
R¹ est choisi dans le groupe constitué de H, OH, S(O)₂NH₂, CN, un alkyle en C₁-C₆, un alcényle en C₂-C₆, un alcynyle en C₂-C₆, un (cycloalkyle en C₃-C₆)-alkyle en C₁-C₄, un alcoxy en C₁-C₆, un (alcoxy en C₁-C₆)-alkyle en C₁-C₆, un (alkyle en C₁-C₆)-carbonyle, un (alcoxy en C₁-C₆)carbonyle, un (alkyle en C₁-C₆)sulfonyle, un (alkylamine en C₁-C₆)carbonyle, un di(alkyle en C₁-C₆)aminocarbonyle, un (alkylamine en C₁-C₆)sulfonyle, un di(alkyle en C₁-C₆) aminosulfonyle et un (alcoxy en C₁-C₆)sulfonyle, où les parties aliphatiques et cycloaliphatiques des 14 radicaux susmentionnés sont non substituées, partiellement ou complètement halogénées, du phényle, du phényl-alkyle en C₁-C₆, du phénylsulfonyle, du phénylaminosulfonyle, du phénylcarbonyle et du phénoxycarbonyle, où le phényle dans les 6 derniers radicaux mentionnés est non substitué ou substitué par 1, 2, 3, 4 ou 5 substituants identiques ou différents choisis dans le groupe constitué d'un halogène, de CN, NO₂, un alkyle en C₁-C₆, un haloalkyle en C₁-C₆, un alcoxy en C₁-C₆ et un haloalcoxy en C₁-C₆ ;
R² est choisi dans le groupe constitué de H, OH, S(O)₂NH₂, CN, un alkyle en C₁-C₆, un alcényle en C₂-C₆, un alcynyle en C₂-C₆, un (cycloalkyle en C₃-C₆)-alkyle en C₁-C₄, un alcoxy en C₁-C₆, un (alcoxy en C₁-C₆)-alkyle en C₁-C₆, un (alkyle en C₁-C₆)carbonyle, un (alcoxy en C₁-C₆)carbonyle, un (alkyle en C₁-C₆)sulfonyle, un (alkylamine en C₁-C₆)carbonyle, un di (alkyle en C₁-C₆)aminocarbonyle, un (alkylamine en C₁-C₆)sulfonyle, un di(alkyle en C₁-C₆)aminosulfonyle et un (alcoxy en C₁-C₆)sulfonyle, où les parties aliphatiques et cycloaliphatiques des 14 radicaux susmentionnés sont non substituées, partiellement ou complètement halogénées, du phényle, du phénylsulfonyle, du phénylaminosulfonyle, du phényl-alkyle en C₁-C₆, du phénoxy, du phénylcarbonyle et du phénoxycarbonyle,
où le phényle dans les 6 derniers radicaux mentionnés est non substitué ou substitué par 1, 2, 3, 4 ou 5 substituants identiques ou différents choisis dans le groupe constitué d'un halogène, de CN, NO₂, un alkyle en C₁-C₆, un haloalkyle en C₁-C₆, un alcoxy en C₁-C₆ et un haloalcoxy en C₁-C₆ ;
X représente NR^{3a}R^{3b}, OR^{3c} ou S(O)ₖR^{3d},
dans lequel R^{3a}, R^{3b}, R^{3c} ou R^{3d} sont choisis, indépendamment l'un de l'autre, dans le groupe constitué de H, CN, S(O)₂NH₂, un alkyle en C₁-C₆, un alcényle en C₂-C₆, un alcynyle en C₂-C₆, un cycloalkyle en C₃-C₆, un (cycloalkyle en C₃-C₆)-alkyle en C₁-C₆, un (alcoxy en C₁-C₆)-alkyle en C₁-C₆, un (alkyle en C₁-C₆)-carbonyle, un (alcoxy en C₁-C₆)carbonyle, un (alkyle en C₁-C₆)sulfonyle, un (alkylamine en C₁-C₆)carbonyle, un di (alkyle en C₁-C₆) aminocarbonyle, un (alkylamine en C₁-C₆)sulfonyle, un di (alkyle en C₁-C₆)aminosulfonyle et un (alcoxy en C₁-C₆)sulfonyle, où les parties aliphatiques et cycloaliphatiques des 14 radicaux susmentionnés sont non substituées, partiellement ou complètement halogénées, du phényle, du phénylsulfonyle, du phényl-alkyle en C₁-C₆, du phénylaminosulfonyle, du phénylcarbonyle et du phénoxycarbonyle, dans lequel le phényle dans les 6 derniers radicaux mentionnés est non substitué ou substitué par 1, 2, 3, 4 ou 5 substituants identiques ou différents choisis dans le groupe constitué d'un halogène, de CN, NO₂, un alkyle en C₁-C₆, un haloalkyle en C₁-C₆, un alcoxy en C₁-C₆ et un haloalcoxy en C₁-C₆, ou R^{3a} et R^{3b} forment, conjointement avec l'atome d'azote auquel ils sont liés, un radical hétérocyclique, mono- ou bicyclique lié à N, qui peut comporter 1, 2, 3 ou 4 autres hétéroatomes qui sont choisis parmi N, O et S, qui est non substitué ou substitué par un ou plusieurs substituants identiques ou différents choisis dans le groupe constitué d'un halogène, de CN, NO₂, un alkyle en C₁-C₆, un haloalkyle en C₁-C₆, un alcoxy en C₁-C₆ et un haloalcoxy en C₁-C₆ ; l'un des R^{3a} et R^{3b} peut également représenter OH, un alcoxy en C₁-C₆, un cycloalcoxy en C₃-C₆, un (cycloalkyle en C₃-C₆)-alcoxy en C₁-C₄, un alcényloxy en C₂-C₆, un alcynyloxy en C₂-C₆, un (alcoxy en C₁-C₆)-alcoxy en C₁-C₆, où les parties aliphatiques et cycloaliphatiques des 7 radicaux susmentionnés sont non substituées, partiellement ou complètement halogénées, ou du phénoxy, qui est non substitué ou substitué par 1, 2, 3, 4 ou 5 substituants identiques ou différents choisis dans le groupe constitué d'un halogène, de CN, NO₂, un alkyle en C₁-C₆, un haloalkyle en C₁-C₆, un alcoxy en C₁-C₆ et un haloalcoxy en C₁-C₆ ;
k est égal à 0, 1 ou 2,
y compris leurs sels de qualité agricole.

2. Composé selon la revendication 1, dans lequel A est un hétéroaryle à 6 chaînons comportant de 1 à 3 atomes d'azote en tant que chaînons du cycle, où l'hétéroaryle est substitué par 1, 2, 3 ou 4 radicaux R^{A} identiques ou différents.

3. Composé selon la revendication 2, dans lequel A est un pyridyle, en particulier le 4-pyridyle ou le 2-pyridyle, qui est substitué par 1, 2, 3 ou 4 radicaux R^{A} identiques ou différents.

4. Composé selon l'une quelconque des revendications précédentes, dans lequel R^{A} est choisi dans le groupe constitué d'un halogène, de CN, un alkyle en C₁-C₆, un alcoxy en C₁-C₆, un haloalkyle en C₁-C₆, un cycloalkyle en C₃-C₆, un cycloalcoxy en C₃-C₆, un (cycloalkyle en C₃-C₆)méthoxy, un alcynyle en C₂-C₆, un alcényle en C₂-C₆, un alcynyloxy en C₂-C₆, un alcényloxy en C₂-C₆ et un haloalcoxy en C₁-C₆.

5. Composé selon l'une quelconque des revendications précédentes, dans lequel R^{A} est un halogène, un haloalkyle en C₁-C₆, un alcoxy en C₁-C₄, un haloalcoxy en C₁-C₄ et CN.

6. Composé selon la revendication 5, dans lequel A est le 2,3,5,6-tétrafluoro-4-pyridyle ou le 4-chloro-3,5,6-trifluoro-2-pyridyle.

7. Composé selon l'une quelconque des revendications précédentes, dans lequel R¹ est choisi dans le groupe constitué de H, CN, un alkyle en C₁-C₆, un (alcoxy en C₁-C₆)-alkyle en C₁-C₆, un (alkyle en C₁-C₆) carbonyle, un (alkyle en C₁-C₆)sulfonyle, un alcoxy en C₁-C₆, un (alcoxy en C₁-C₆)carbonyle, un (alkylamine en C₁-C₆)carbonyle, un di(alkyle en C₁-C₆) aminocarbonyle, un (alkylamine en C₁-C₆)sulfonyle, un di(alkyle en C₁-C₆)aminosulfonyle, où les parties aliphatiques des 10 radicaux susmentionnés sont non substituées, partiellement ou complètement halogénées,
du phényle, du phénylcarbonyle et du phényl-alkyle en C₁-C₆,
dans lequel le phényle dans les 3 derniers radicaux mentionnés est non substitué ou substitué par 1, 2, 3, 4 ou 5 substituants identiques ou différents choisis dans le groupe constitué d'un halogène, de CN, NO₂, un alkyle en C₁-C₆, un haloalkyle en C₁-C₆, un alcoxy en C₁-C₆ et un haloalcoxy en C₁-C₆.

8. Composé selon l'une quelconque des revendications précédentes, dans lequel R² est choisi dans le groupe constitué de H, CN, un alkyle en C₁-C₆, un (alcoxy en C₁-C₆)-alkyle en C₁-C₆, un (alkyle en C₁-C₆) carbonyle, un (alkyle en C₁-C₆)sulfonyle, un (alcoxy en C₁-C₆) carbonyle, un (alkylamine en C₁-C₆) carbonyle, un di(alkyle en C₁-C₆)aminocarbonyle, un (alkylamine en C₁-C₆)sulfonyle et un di (alkyle en C₁-C₆)aminosulfonyle, où les parties aliphatiques des 9 radicaux susmentionnés sont non substituées, partiellement ou complètement halogénées,
du phényle, du phénylcarbonyle et de l'alkylphényle en C₁-C₆,
dans lequel le phényle dans les 3 derniers radicaux mentionnés est non substitué ou substitué par 1, 2, 3, 4 ou 5 substituants identiques ou différents choisis dans le groupe constitué d'un halogène, de CN, NO₂, un alkyle en C₁-C₆, un haloalkyle en C₁-C₆, un alcoxy en C₁-C₆ et un haloalcoxy en C₁-C₆.

9. Composé selon l'une quelconque des revendications précédentes, dans lequel R¹ est choisi dans le groupe constitué de H, CN, un alkyle en C₁-C₄, un haloalkyle en C₁-C₆, un (alcoxy en C₁-C₆)-alkyle en C₁-C₆, un alcoxy en C₁-C₆, un (alkyle en C₁-C₆)carbonyle, un (haloalkyle en C₁-C₆)carbonyle, un (alkyle en C₁-C₆)sulfonyle ou un (haloalkyle en C₁-C₆)sulfonyle.

10. Composé selon l'une quelconque des revendications précédentes, dans lequel R² est choisi dans le groupe constitué de H, CN, un alkyle en C₁-C₄, un (alcoxy en C₁-C₄)-alkyle en C₁-C₄, un (alkyle en C₁-C₄)carbonyle ou un (alkyle en C₁-C₄)sulfonyle.

11. Composé selon l'une quelconque des revendications précédentes, dans lequel R¹ est H.

12. Composé selon l'une quelconque des revendications précédentes, dans lequel R² est H.

13. Composé selon l'une quelconque des revendications précédentes, dans lequel
X est OR^{3c}, dans lequel
R^{3c} est choisi dans le groupe constitué de H, un alkyle en C₁-C₆, un cycloalkyle en C₃-C₆, un (alcoxy en C₁-C₆)-alkyle en C₁-C₆, un (alkyle en C₁-C₆)-carbonyle, un (alcoxy en C₁-C₆) carbonyle, un (alkyle en C₁-C₆)sulfonyle, où les parties aliphatiques des 6 radicaux susmentionnés sont non substituées, partiellement ou complètement halogénées,
du phényle, du phénylsulfonyle ou du phényl-alkyle en C₁-C₆,
dans lequel le phényle dans les 3 derniers radicaux mentionnés est non substitué ou substitué par 1, 2, 3, 4 ou 5 substituants identiques ou différents choisis dans le groupe constitué d'un halogène, de CN, NO₂, un alkyle en C₁-C₆, un haloalkyle en C₁-C₆, un alcoxy en C₁-C₆ et un haloalcoxy en C₁-C₆.

14. Composé selon l'une quelconque des revendications 1 à 12, dans lequel
X est S(O)ₖR^{3d}, dans lequel
R^{3d} est choisi dans le groupe constitué de H, CN, un alkyle en C₁-C₆, un cycloalkyle en C₃-C₆, un cycloalkyle en C₃-C₆-alkyle en C₁-C₆, un (alcoxy en C₁-C₆)-alkyle en C₁-C₆, un (alkyle en C₁-C₆) carbonyle, un (alcoxy en C₁-C₆) carbonyle, un (alkyle en C₁-C₆)sulfonyle, où les parties aliphatiques des 7 radicaux susmentionnés sont non substituées, partiellement ou complètement halogénées,
du phényle, du phénylsulfonyle ou du phényl-alkyle en C₁-C₆,
dans lequel le phényle dans les 3 derniers radicaux mentionnés est non substitué ou substitué par 1, 2, 3, 4 ou 5 substituants identiques ou différents choisis dans le groupe constitué d'un halogène, de CN, NO₂, un alkyle en C₁-C₆, un haloalkyle en C₁-C₆, un alcoxy en C₁-C₆ et un haloalcoxy en C₁-C₆, et
k est égal à 0, 1 ou 2.

15. Composé selon l'une quelconque des revendications 1 à 12, dans lequel
X est NR^{3a}R^{3b}, dans lequel
R^{3a} et R^{3b} représentent, indépendamment l'un de l'autre, H, CN, S(O)₂NH₂, un alkyle en C₁-C₆, un alcényle en C₂-C₆, un alcynyle en C₂-C₆, un cycloalkyle en C₃-C₆, un cycloalkyle en C₃-C₆-alkyle en C₁-C₆, un (alcoxy en C₁-C₆)-alkyle en C₁-C₆, un (alkyle en C₁-C₆)-carbonyle, un (alcoxy en C₁-C₆)carbonyle, un (alkyle en C₁-C₆) sulfonyle, un (alkylamine en C₁-C₆) carbonyle, un di (alkyle en C₁-C₆)-aminocarbonyle, un (alkylamine en C₁-C₆)sulfonyle, un di (alkyle en C₁-C₆) aminosulfonyle et un (alcoxy en C₁-C₆)sulfonyle où les parties aliphatiques des 15 radicaux susmentionnés sont non substituées, partiellement ou complètement halogénées,
du phényle, du phénylsulfonyle ou du phényl-alkyle en C₁-C₆,
dans lequel le phényle dans les 3 derniers radicaux mentionnés est non substitué ou substitué par 1, 2, 3, 4 ou 5 substituants identiques ou différents choisis dans le groupe constitué d'un halogène, de CN, NO₂, un alkyle en C₁-C₆, un haloalkyle en C₁-C₆, un alcoxy en C₁-C₆ et un haloalcoxy en C₁-C₆,
l'un parmi R^{a} et R^{b} peut aussi être OH, un alcoxy en C₁-C₆, un cycloalcoxy en C₃-C₆, un (alcoxy en C₁-C₆)-alcoxy en C₁-C₆, où les parties aliphatiques et cycloaliphatiques des 3 radicaux susmentionnés sont non substituées, partiellement ou complètement halogénées,
ou
R^{3a} et R^{3b}, conjointement avec l'atome d'azote auquel ils sont liés, forment un radical hétérocylique mono- ou bicyclique saturé ou insaturé lié à N, qui peut comporter 1, 2, 3 ou 4 autres hétéroatomes qui sont choisis parmi N, 0 et S, qui est substitué ou non substitué par un ou plusieurs substituants identiques ou différents choisis dans le groupe constitué d'un halogène, de CN, NO₂, un alkyle en C₁-C₆, un haloalkyle en C₁-C₆, un alcoxy en C₁-C₆, un (alcoxy en C₁-C₆)-alkyle en C₁-C₆ et un haloalcoxy en C₁-C₆.

16. Composé selon la revendication 15, dans lequel
X représente NR^{3a}R^{3b}, dans lequel
R^{3a} et R^{3b}, conjointement avec l'atome d'azote auquel ils sont liés, forment un radical hétérocyclique mono- ou bicyclique saturé ou insaturé lié à N, qui est choisi dans le groupe constitué du 1-aziridinyle, du 1-azétidinyle, du 1-pipéridinyle, du 1-pyrrolidinyle, de l'azépan-1-yle, de l'azocan-1-yle, du morpholin-4-yle, de l'isoxazolidine-2-yle, de l'oxazolidine-3-yle, de la pipérazine-1-yle, de l'octahydroisoindol-2-yle, de l'octahydroindol-1-yle, de l'octahydro-2H-quinolin-1-yle, de l'azabicyclo[2.2.1]heptan-3-yle et de l'azabicyclo[2.2.1]heptan-7-yle, où les radicaux susmentionnés sont non substitués ou substitués par un ou plusieurs substituants identiques ou différents choisis dans le groupe constitué d'un halogène, de CN, NO₂, un alkyle en C₁-C₂, un haloalkyle en C₁-C₂, un (alcoxy en C₁-C₂)-alkyle en C₁-C₂, un alcoxy en C₁-C₂ et un haloalcoxy en C₁-C₂.

17. Composition agrochimique comprenant une quantité herbicide active d'au moins un composé selon l'une quelconque des revendications 1 à 16 et au moins un support liquide et/ou solide inerte et, si nécessaire, au moins une substance tensioactive.

18. Procédé de lutte contre la végétation indésirable, qui implique de laisser agir une quantité herbicide active d'au moins un composé selon l'une quelconque des revendications 1 à 16 sur des plantes, leur environnement ou sur des graines.

19. Utilisation d'un composé selon l'une quelconque des revendications 1 à 16 en tant qu'herbicide ou à des fins de desséchement/défoliation de plantes.
